# FASCICULE DE BREVET EUROPEEN

(11) **EP 1 379 514 B1**
(45) Date de publication et mention de la délivrance du brevet: **15.07.2009**
(21) Numéro de dépôt: 02761921.2
(22) Date de dépôt: 09.04.2002
(51) Int. Cl.: C07D 277/28, A61K 31/33, A61P 43/00, C07D 277/34, C07D 417/04, C07D 417/06, C07D 233/50, C07D 401/06, C07D 417/14, C07D 233/54

(54) **DERIVES D'HETEROCYCLES A 5 CHAINONS, LEUR PREPARATION ET LEUR APPLICATION COMME MEDICAMENTS**
FÜNFGLIEDERIGE HETERORINGDERATIVE UND DEREN VERWENDUNG ALS MAO INHIBITOREN UND/ODER ALS INHIBITOREN DER LIPIDPEROXIDIERUNG
5-MEMBERED HETEROCYCLES, PREPARATION AND APPLICATION THEREOF AS MEDICAMENTS

(30) Priorité: 10.04.2001 FR 0104943; 14.02.2002 FR 0201811
(43) Date de publication de la demande: 14.01.2004
(73) Titulaire: IPSEN PHARMA, 92100 Boulogne-Billancourt (FR)
(72) Inventeur: HARNETT, Jeremiah, F-91190 Gif-sur-Yvette (FR); BIGG, Dennis, F-91190 Gif-sur-Yvette (FR); LIBERATORE, Anne-Marie, F-78610 Auffargis (FR); ROLLAND, Alain, F-91120 Palaiseau (FR)
(74) Mandataire: Bourgouin, André
(86) Numéro de dépôt international: PCT/FR2002/001218
(87) Numéro de publication internationale: WO 2002/083656

(56) Documents cités:
- WO-A-01/26656
- WO-A-97/13768
- WO-A-98/58934
- US-A- 5 326 770

## Description

La présente invention concerne certains composés de formule générale **(I)** décrite ci-après, lesdits composés en tant que médicaments ainsi que leur utilisation pour préparer un médicament destiné à inhiber les monoamine oxydases (MAO) et/ou peroxydation lipidique et/ou à agir en tant que modulateurs des canaux sodiques.

Les composés évoqués ci-dessus présentent souvent 2 ou 3 des activités citées ci-dessus, lesquelles leur confèrent des propriétés pharmacologiques avantageuses.

En effet, compte tenu du rôle potentiel des MAO et des ROS (« *reactive oxygen species* » ou espèces réactives de l'oxygène, à l'origine de la peroxydation lipidique) en physiopathologie, les nouveaux dérivés décrits répondant à la formule générale (I) peuvent produire des effets bénéfiques ou favorables dans le traitement de pathologies où ces enzymes et/ou ces espèces radicalaires sont impliquées. Notamment :
- les troubles du système nerveux central ou périphérique comme par exemple les maladies neurologiques où l'on peut notamment citer la maladie de Parkinson, les traumatismes cérébraux ou de la moelle épinière, l'infarctus cérébral, l'hémorragie sub arachnoïde, l'épilepsie, le vieillissement, les démences séniles, la maladie d'Alzheimer, la chorée de Huntington, la sclérose latérale amyotrophique, les neuropathies périphériques, la douleur ;
- la schizophrénie, les dépressions, les psychoses ;
- les troubles de la mémoire et de l'humeur ;
- les pathologies comme par exemple la migraine ;
- les troubles du comportement, la boulimie et l'anorexie ;
- les maladies auto-immunes et virales comme par exemple le lupus, le sida, les infections parasitaires et virales, le diabète et ses complications, la sclérose en plaques.
- l'addiction aux substances toxiques ;
- les pathologies inflammatoires et prolifératives ;
- et plus généralement toutes les pathologies caractérisées par une production excessive des ROS et/ou une participation des MAO.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des MAO (Goodman & Gilman's : The pharmacological basis of therapeutics , 9th ed., 1995, 431-519).

L'intérêt d'une combinaison des activités inhibitrice de MAO et inhibitrice de la peroxydation lipidique est par exemple bien illustré dans la maladie de Parkinson. Cette pathologie est caractérisée par une perte des neurones dopaminergiques de la voie nigrostriatal dont la cause serait en partie liée à un stress oxydatif dû aux ROS. De la dopamine exogène à partir de L Dopa est utilisé en thérapeutique afin de maintenir des taux suffisants de dopamine. Les inhibiteurs de MAO sont aussi utilisés avec la L Dopa pour éviter sa dégradation métabolique mais n'agissent pas sur les ROS. Des composés agissant à la fois sur les MAO et les ROS auront donc un avantage certain.

Par ailleurs, le caractère de modulateur des canaux sodiques est très utile pour des indications thérapeutiques comme :
- le traitement ou la prévention de la douleur, et notamment :
   ❖ des douleurs post-opératoires,
   ❖ de la migraine,
   ❖ des douleurs neuropathiques telles que la névralgie du trijumeau, la douleur post-herpétique, les neuropathies diabétiques, les névralgies glosso-pharyngiennes, les radiculopathies et neuropathies secondaires à des infiltrations métastatiques, l'adiposis dolorosa et les douleurs liées aux brûlures,
   ❖ des douleurs centrales consécutives aux accidents cérébraux vasculaires, lésions thalamiques et sclérose en plaques, et
   ❖ des douleurs chroniques inflammatoires ou liées à un cancer ;
- le traitement de l'épilepsie ;
- le traitement de troubles liés à la neurodégénération, et en particulier :
   ❖ des accidents cérébraux vasculaires,
   ❖ du traumatisme cérébral, et
   ❖ de maladies neurodégénératives telles que la maladie d'Alzheimer, la maladie de Parkinson et la sclérose latérale amyotrophique ;
- le traitement des troubles bipolaires et du syndrome du colon irritable.

Les avantages concrets de la présence dans un composé d'au moins une de ces activités ressortent donc clairement de ce qui précède.

La demande de brevet européen EP 432 740 décrit des dérivés d'hydroxyphénylthiazoles, lesquels peuvent être utilisés dans le traitement des maladies inflammatoires, en particulier les maladies rhumatismales. Ces dérivés d'hydroxyphénylthiazoles montrent des propriétés de piégeurs de radicaux libres et d'inhibiteurs du métabolisme de l'acide arachidonique (ils inhibent la lipoxygénase et la cyclooxygénase).

D'autres dérivés d'hydroxyphénylthiazoles ou d'hydroxyphényloxazoles sont décrits dans la demande de brevet PCT WO 99/09829. Ceux-ci possèdent des propriétés analgésiques.

Un certain nombre de dérivés d'imidazoles de structures proches ou identiques à celles de composés répondant à la formule générale **(I)** selon l'invention ont par ailleurs été décrits par la demanderesse dans la demande de brevet PCT WO 99/64401 comme agonistes ou antagonistes de la somatostatine. Lesdits dérivés d'imidazoles possèdent cependant des propriétés thérapeutiques dans des domaines différents de ceux indiqués ci-dessus (la suppression de l'hormone de croissance et le traitement de l'acromégalie, le traitement de la resténose, l'inhibition de la sécrétion d'acide gastrique et la prévention des saignements gastro-intestinaux notamment).

Les documents WO 97/13768 et US 5 326 770 décrivent également des composés ayant une activité au niveau des recepteurs MAO.

Par ailleurs, les composés de formule générale **(A1)** 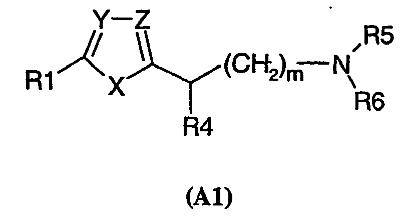 dans laquelle
R1 représente l'un des radicaux aryle, hétéroaryle, aralkyle ou cycloalkyle optionnellement substitués par un à trois substituants choisis indépendamment parmi un atome halogène, le radical CF₃, CN, OH, alkyle ou alkoxy, SO₂R9 avec R9 représentant NH₂ ou NHCH₃ ;
X représente NR2, R2 représentant H ou alkyle ;
Y représente N ou CR3 ;
Z représente CR3 ou N ;
à la condition toutefois que Y et Z ne sont pas tous deux CR3 ou N en même temps ;
R3 représente H, alkyle, halogène, hydroxyalkyle ou phényle éventuellement substitué par 1 à 3 subtituants choisis parmi H, CF₃, CN, SO₂NH₂, OH, alkyle ou alkoxy ;
m représente 0, 1 ou 2 ;
R4 représente H ou alkyle ;
lorsque Z représente CR3, alors R3 et R4 peuvent aussi représenter ensemble -(CH₂)ₙ₁- avec n1 entier de 2 à 4 ou R2 et R4 peuvent aussi représenter ensemble -(CH₂)ₙ₂- avec n2 entier de 2 à 4 ;
R5 et R6 représentent indépendamment H, alkyle, alkoxy, aryle ou aralkyle ;
NR5R6 pouvant aussi représenter ensemble (notamment) :
- le radical 2-(1,2,3,4-tétrahydroquinolyl) éventuellement substitué,
- un radical 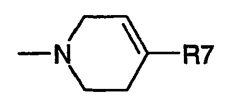 dans lequel R7 représente l'un des radicaux phényle, benzyle ou phénéthyle dans lequels le cycle phényle peut être substitué ;
- un radical 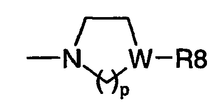
dans lequel p est un entier de 1 à 3,
W est N et R8 représente H, CF₃, l'un des radicaux phényle, pyridyle ou pyrimidinyle éventuellement substitués de 1 à 2 fois par des radicaux choisis parmi halogène, OH, alkyle ou alkoxy, ou
W est CH et R8 représente phényle éventuellement substitué ou aralkyle éventuellement substitué sur le groupe aryle ;
ont été décrits dans la demande de brevet PCT WO 96/16040 en tant qu'agonistes partiels ou antagonistes des sous-récepteurs de la dopamine du cerveau ou en tant que formes prodrogues de tels agonistes partiels ou antagonistes. Ces composés présenteraient de ce fait des propriétés intéressantes dans le diagnostic et le traitement de désordres affectifs tels que la schizophrénie et la dépression ainsi que certains désordres du mouvement tels que la maladie de Parkinson.

Il a également été décrit dans la demande de brevet PCT WO 98/27108 que certains amides de formule générale **(A2)** 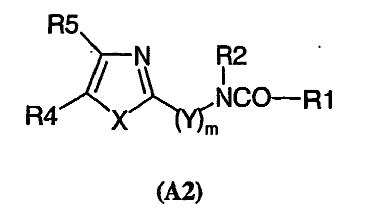 dans laquelle :
R1 représente notamment un radical alkyle, phényle éventuellement substitué ou aryle hétérocyclique éventuellement substitué ;
R2 représente H ou phénylalkyle ;
R4 représente H, quinolyle, 3-4-méthylènedioxyphényle ou l'un des radicaux phényle ou pyridyle éventuellement substitués, par un ou des radicaux choisis notamment parmi alkyle, alkoxy, alkylthio, hydroxy éventuellement protégé, amino, alkylamino, dialkylamino ;
R5 représente H ou un radical imidazolyle, phényle, nitrophényle, phénylalkyle, ou encore un radical -CO-N(R7)(R8), dans lequel R7 et R8 représentent indépendamment H, phényle, phénylalkyle, alkyle ou alkoxy ;
ou R4 et R5 en combinaison forment un groupe de formule -CH=CH-CH=CH- ;
Y est un radical phénylène substitué par un radical phényle, phénoxy ou phénylalkoxy, ou un groupe de formule -CH(R3)-, dans laquelle R3 représente H ou un radical de formule -(CH₂)ₙ-R6, dans laquelle R6 représente un radical hydroxy éventuellement protégé, acyle, carboxy, acylamino, alkoxy, phénylalkoxy, alkylthio, phényle éventuellement substitué, pyridyle éventuellement substitué, pyrazinyle, pyrimidinyle, furyle, imidazolyle, naphtyle, N-alkylindolyle ou 3,4-méthylènedioxyphényle et n est un entier de 0 à 3 ;
R2 et R3 pris ensemble avec les atomes de carbone qui les portent pouvant former un groupe phényle ;
X représente S ou NR9 ;
R9 représentant H, un radical alkyle ou cycloalkyle, ou encore un radical benzyle éventuellement substitué une fois sur sa partie phényle par H, alkyle ou alkoxy ; sont des inhibiteurs des NO synthases et peuvent être utilisés pour traiter des maladies qui comprennent notamment l'ischémie cardiovasculaire ou cérébrale, l'hémorragie cérébrale, les troubles du système nerveux central, la maladie d'Alzheimer, la sclérose en plaques, le diabète, l'hépatite, la migraine, l'arthrite rhumatoïde et l'ostéoporose.

Dans un domaine différent, la demanderesse a elle-même précédemment décrit dans la demande de brevet PCT WO 98/58934 des dérivés d'amidines ayant la faculté d'inhiber les NO Synthases et/ou la peroxydation lipidique.

La demanderesse a plus récemment décrit dans la demande de brevet PCT/FR00/02805 que certains intermédiaires des premières étapes de synthèse des amidines décrites dans la demande de brevet PCT WO 98/58934, et plus généralement certains dérivés d'hétérocycles à cinq chaînons, à savoir les produits de formule générale (I) définie ci-après, possèdent au moins l'une des trois propriétés choisies parmi les propriétés suivants (et souvent même deux de ces trois propriétés voire parfois les trois à la fois) :
- des propriétés d'inhibition des MAO ;
- des propriétés d'inhibition de la peroxydation lipidique ; et
- des propriétés de modulation des canaux sodiques.

Ces propriétés avantageuses offrent l'intérêt d'ouvrir à de tels composés de nombreuses applications, en particulier dans le traitement des maladies neurodégénératives, et notamment celles indiquées précédemment, de la douleur ou de l'épilepsie.

Selon la demande de brevet PCT WO 01/26656, les composés répondant à la formule générale **(I)** 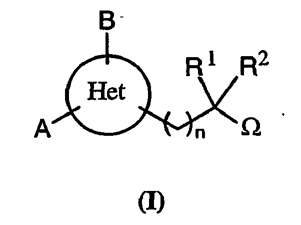 sous forme racémique, d'énantiomère ou toute combinaison de ces formes, dans laquelle Het est un hétérocycle à 5 chaînons comportant 2 hétéroatomes et tel que la formule générale **(I)** corresponde exclusivement à l'une des sous-formules suivantes : 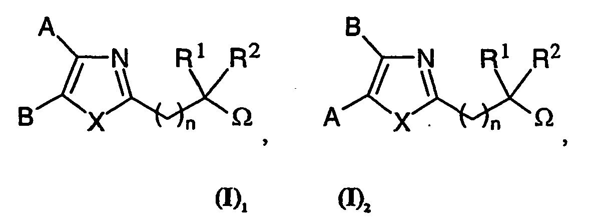 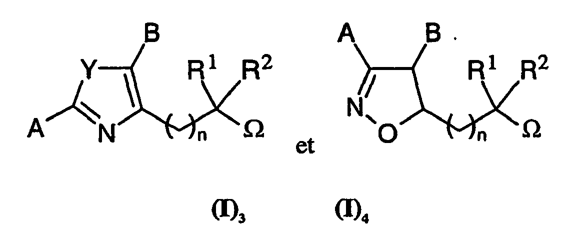 dan lesquelles
A représente
soit un radical 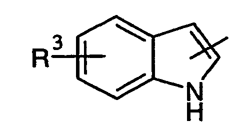 dans lequel R³ représente un atome d'hydrogène, le groupe OH ou un radical alkoxy ou alkyle,
soit un radical 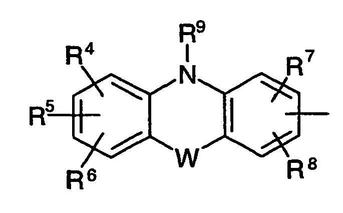 dans lequel R⁴, R⁵, R⁶, R⁷ et R⁸ représentent, indépendamment, un atome d'hydrogène, un halogène, le groupe OH ou un radical alkyle, alkoxy, cyano, nitro ou NR¹⁰R¹¹, R¹⁰ et R¹¹ représentant, indépendamment, un atome d'hydrogène, un radical alkyle ou un groupe -COR¹², ou bien R¹⁰ et R¹¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R¹² représentant un atome d'hydrogène ou un radical alkyle, alkoxy ou NR¹³R¹⁴,
R¹³ et R¹⁴ représentant, indépendamment, un atome d'hydrogène ou un radical alkyle, ou bien R¹³ et R¹⁴ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R⁹ représente un atome d'hydrogène, un radical alkyle ou un groupe -COR¹⁵,
R¹⁵ représentant un atome d'hydrogène ou un radical alkyle, alkoxy ou NR¹⁶R¹⁷, R¹⁶ et R¹⁷ représentant, indépendamment, un atome d'hydrogène ou un radical alkyle, ou bien R¹⁶ et R¹⁷ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
et W n'existe pas, ou représente une liaison, ou -O-, -S- ou -NR¹⁸-, dans lequel R¹⁸ représente un atome d'hydrogène ou un radical alkyle ;
soit un radical 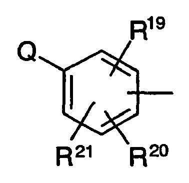 dans lequel Q représente H, -OR²², -SR²², -NR²³R²⁴, un radical phényle éventuellement substitué par un ou des substituants choisis indépendamment parmi un atome halogène, un radical OH, cyano, nitro, alkyle, alkoxy ou -NR¹⁰R¹¹ et un groupe de deux substituants représentant ensemble un radical méthylène dioxy ou éthylènedioxy, ou encore Q représente un radical -COPh, -SO₂Ph ou -CH₂Ph, ledit radical -COPh, -SO₂Ph ou -CH₂Ph étant éventuellement substitué sur sa partie aromatique par un ou des substituants choisis indépendamment parmi un radical alkyle ou alkoxy et un atome halogène,
R¹⁰ et R¹¹ représentant, indépendamment, un atome d'hydrogène, un radical alkyle ou un groupe -COR¹², ou bien R¹⁰ et R¹¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R¹² représentant un atome d'hydrogène, un radical alkyle ou alkoxy ou NR¹³R¹⁴,
R¹³ et R¹⁴ représentant, indépendamment, un atome d'hydrogène ou un radical alkyle, ou bien R¹³ et R¹⁴ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycles pouvant être par exemple azétidine, pyrrolidine, pipéridine, pipérazine, morpholine ou thiomorpholine,
R²² représentant un atome d'hydrogène, un radical alkyle ou un radical aryle éventuellement substitué par un ou plusieurs substituants choisis parmi les radicaux alkyle, OH, halogène, nitro et alkoxy,
R²³ et R²⁴ représentant, indépendamment, un atome d'hydrogène, un radical alkyle ou un radical -CO-R²⁵,
R²⁵ représentant un radical alkyle,
et R¹⁹, R²⁰ et R²¹ représentent, indépendamment, un hydrogène, un halogène, le groupe OH ou SR²⁶, ou un radical alkyle, cycloalkyle, alkényle, alkoxy, cyano, nitro, -S₂ONHR⁴⁹, -CONHR²⁵, -S(O)_{q}R⁵⁶, -NH(CO)R⁵⁷, -CF₃, -OCF₃ ou NR²⁷R²⁸,
R²⁶ représentant un atome d'hydrogène ou un radical alkyle,
R²⁷ et R²⁸ représentant, indépendamment, un atome d'hydrogène, un radical alkyle ou un groupe -COR²⁹, ou bien R²⁷ et R²⁸ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R⁴⁹ et R⁵⁵ représentant, indépendamment à chaque fois qu'ils interviennent, un atome d'hydrogène ou un radical alkyle ou alkylcarbonyle,
q représentant un entier de 0 à 2,
R⁵⁶ et R⁵⁷ représentant, indépendamment à chaque fois qu'ils interviennent, un atome d'hydrogène ou un radical alkyle ou alkoxy,
R²⁹ représentant un atome d'hydrogène, un radical alkyle, alkoxy ou -NR³⁰R³¹,
R³⁰ et R³¹ représentant, indépendamment, un atome d'hydrogène ou un radical alkyle, ou bien R³⁰ et R³¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
soit un radical 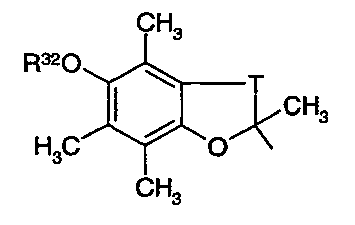 dans lequel R³² représente un atome d'hydrogène ou un radical alkyle,
et T représente un radical -(CH₂)ₘ- avec m = 1 ou 2,
soit enfin un radical 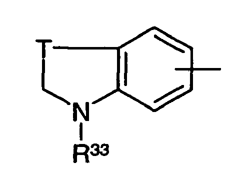 dans lequel R³³ représente un atome d'hydrogène ou un radical alkyle, -Σ-NR³⁴R³⁵ ou -Σ-CHR³⁶R³⁷,
E représentant un radical alkylène linéaire ou ramifié comptant de 1 à 6 atomes de carbone,
R³⁴ et R³⁵ représentant, indépendamment, un atome d'hydrogène ou un radical alkyle,
R³⁶ et R³⁷ représentant, indépendamment, un atome d'hydrogène ou un radical aryle carbocyclique ou hétérocyclique éventuellement substitué par un ou plusieurs substituants choisis parmi les radicaux alkyle, OH, halogène, nitro, alkoxy ou NR¹⁰R¹¹,
R¹⁰ et R¹¹ représentant, indépendamment, un atome d'hydrogène, un radical alkyle ou un groupe -COR¹², ou bien R¹⁰ et R¹¹ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
R¹² représentant un atome d'hydrogène ou un radical alkyle, alkoxy ou NR¹³R¹⁴,
R¹³ et R¹⁴ représentant, indépendamment, un atome d'hydrogène ou un radical alkyle, ou bien R¹³ et R¹⁴ formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
et T représente un radical -(CH₂)ₘ- avec m = 1 ou 2,
ou encore A représente un radical alkyle, cycloalkyle ou cycloalkylalkyle ;
X représente S ou NR³⁸,
R³⁸ représentant un atome d'hydrogène ou un radical alkyle, cyanoalkyle, aralkyle, alkylcarbonyle ou aralkylcarbonyle,
Y représente O ou S ;
R¹ représente un atome d'hydrogène, un radical alkyle, aminoalkyle, alkoxyalkyle, cycloalkyle, cycloalkylalkyle, trifluorométhylalkyle, alkényle, allènyle, allènylalkyle, alkynyle, cyanoalkyle, -(CH₂)_{g}-Z¹R³⁹, -(CH₂)_{g}-COR⁴⁰, -(CH₂)_{g}-NHCOR⁷⁰, aryle, aralkyle, arylcarbonyle, hétéroarylalkyle ou aralkylcarbonyle, le groupement aryle des radicaux aryle, aralkyle, arylcarbonyle, hétéroarylalkyle ou aralkylcarbonyle étant lui-même éventuellement substitué par un ou plusieurs substituants choisis parmi le groupe constitué des radicaux alkyle, halogène, alkoxy, nitro, cyano, cyanoalkyle, amino, alkylamino, dialkylamino, -(CH₂)ₖ-Z²R³⁹ ou -(CH₂)ₖ-COR⁴⁰,
Z¹ et Z² représentant une liaison, -O-, -NR⁴¹- ou -S-,
R³⁹ et R⁴¹ représentant, indépendamment à chaque fois qu'ils interviennent, un atome d'hydrogène ou un radical alkyle, alkényle, alkynyle ou cyanoalkyle,
R⁴⁰ représentant, indépendamment à chaque fois qu'il intervient, un atome d'hydrogène ou un radical alkyle, allènyle, allènylalkyle, alkényle, alkynyle, cyanoalkyle, alkoxy ou NR⁴²R⁴³,
R⁴² et R⁴³ représentant, indépendamment, indépendamment à chaque fois qu'ils interviennent, un atome d'hydrogène ou un radical alkyle, allènyle, allènylalkyle, alkényle, alkynyle ou cyanoalkyle,
et R² représente un atome d'hydrogène, un radical alkyle, aminoalkyle, alkoxyalkyle, cycloalkyle, cycloalkylalkyle, trifluorométhylalkyle ou -(CH₂)_{g}-NHCOR⁷¹, ou encore l'un des radicaux aralkyle ou hétéroarylalkyle éventuellement substitués sur le groupe aryle ou hétéroaryle par un ou des groupes choisis indépendamment parmi le groupe composé d'un atome halogène et d'un radical alkyle, alkoxy, hydroxy, cyano, nitro, amino, alkylamino ou dialkylamino,
R⁷⁰ et R⁷¹ représentant indépendamment un radical alkyle ou alkoxy ;
ou bien R¹ et R², pris ensemble avec l'atome de carbone qui les porte, forment un carbocycle de 3 à 7 chaînons ;
B représente un atome d'hydrogène, un radical alkyle, un radical -(CH₂)_{g}-Z³R⁴⁴ ou un radical aryle carbocyclique éventuellement substitué de 1 à 3 fois par des radicaux choisis parmi le groupe composé d'un atome halogène, d'un radical alkyle ou alkoxy linéaire ou ramifié comptant de 1 à 6 atomes de carbone, d'un radical hydroxy, cyano ou nitro, d'un radical amino, alkylamino ou dialkylamino et d'un radical aryle carbocyclique,
Z³ représentant une liaison, -O-, -NR⁴⁵- ou -S-,
R⁴⁴ et R⁴⁵ représentant, indépendamment, un atome d'hydrogène ou un radical alkyle, alkényle, alkynyle, allènyle, allènylalkyle ou cyanoalkyle ;
Ω représente l'un des radicaux NR⁴⁶R⁴⁷ ou OR⁴⁸, dans lesquels :
R⁴⁶ et R⁴⁷ représentent, indépendamment, un atome d'hydrogène ou un radical alkyle, cycloalkyle, cycloalkylalkyle, alkényle, alkynyle, allènyle, allènylalkyle, cyanoalkyle, -(CH₂)_{g}-Z⁴R⁵⁰, -(CH₂)ₖ-COR⁵¹, -(CH₂)ₖ-COOR⁵¹, -(CH₂)ₖ-CONHR⁵¹ ou -SO₂R⁵¹, ou encore un radical choisi parmi les radicaux aryle, aralkyle, aryloxyalkyle, arylcarbonyle, arylimino, aralkylcarbonyle, hétéroaryle et en particulier pyridinyle, pyridinylalkyle ou pyridinylcarbonyle, le groupement aryle ou hétéroaryle desdits radicaux aryle, aralkyle, aryloxyalkyle, arylcarbonyle, arylimino, aralkylcarbonyle, hétéroaryle, pyridinylalkyle ou pyridinylcarbonyle étant éventuellement substitué par un ou des substituants choisis indépendamment parmi halogène, alkyle, alkoxy, hydroxy, nitro, cyano, cyanoalkyle, amino, alkylamino, dialkylamino, -(CH₂)ₖ-Z⁵R⁵⁰, -(CH₂)ₖ-COR⁵¹ et -(CH₂)ₖ-COOR⁵¹, Z⁴ et Z⁵ représentant une liaison, -O-, -NR⁵²- ou -S-,
ou R⁴⁶ et R⁴⁷ pris ensemble forment avec l'atome d'azote un hétérocycle non aromatique de 4 à 8 chaînons, les éléments de la chaîne étant choisis parmi un groupe composé de -CH(R⁵³)-, -NR⁵⁴-, -O-, -S- et -CO-, ledit hétérocycle pouvant être par exemple une azétidine, une pipérazine, une homopipérazine, une 3,5-dioxopipérazine, une pipéridine, une pyrrolidine, une morpholine ou une thiomorpholine,
R⁵⁰ et R⁵², représentant, indépendamment à chaque fois qu'ils interviennent, un atome d'hydrogène ou un radical alkyle, alkényle, alkynyle, allènyle, allènylalkyle ou cyanoalkyle,
R⁵¹ représentant, indépendamment à chaque fois qu'il intervient, un atome d'hydrogène, l'un des radicaux cycloalkyle ou cycloalkylalkyle dans lesquels le radical cycloalkyle compte de 3 à 7 atomes de carbone, un radical alkyle linéaire ou ramifié comptant de 1 à 8 atomes de carbone, un radical alkényle, alkynyle, allènyle, allènylalkyle, cyanoalkyle, alkoxyalkyle ou NR⁵⁸R⁵⁹, ou encore un radical aryle ou aralkyle, ledit radical aryle ou aralkyle pouvant être substitué par un ou des substituants choisis indépendamment parmi un atome halogène et un radical alkyle ou alkoxy,
R⁵⁸ et R⁵⁹ représentant, indépendamment, un atome d'hydrogène ou un radical alkyle, alkényle, alkynyle, allènyle, allènylalkyle ou cyanoalkyle,
R⁵³ et R⁵⁴ représentant, indépendamment, un atome d'hydrogène ou un radical -(CH₂)ₖ-Z⁷R⁶⁰ ou -(CH₂)ₖ-COR⁶¹,
Z⁷ représentant une liaison, -O-, -NR⁶²- ou -S-,
R⁶⁰ et R⁶² représentant, indépendamment, un atome d'hydrogène ou un radical alkyle, alkényle, allènyle, allènylalkyle, alkynyle, cyanoalkyle, aryle, aralkyle, arylcarbonyle, aralkylcarbonyle, pyridinyle, pyridinylalkyle ou pyridinylcarbonyle, le groupement aryle ou pyridinyle des radicaux aryle, aralkyle, arylcarbonyle, aralkylcarbonyle, pyridinyle, pyridinylalkyle ou pyridinylcarbonyle étant éventuellement substitué par un ou plusieurs substituants choisis parmi le groupe constitué des radicaux alkyle, halogène, nitro, alkoxy, cyano, cyanoalkyle, -(CH₂)ₖ-Z⁸R⁶³ et -(CH₂)ₖ-COR⁶⁴,
R⁶¹ représentant un atome d'hydrogène, un radical alkyle, allènyle, allènylalkyle, alkényle, alkynyle, cyanoalkyle, alkoxy ou NR⁶⁵R⁶⁶,
R⁶⁵ et R⁶⁶ représentant, indépendamment, un atome d'hydrogène ou un radical alkyle, allènyle, allènylalkyle, alkényle, alkynyle ou cyanoalkyle,
Z⁸ représentant une liaison, -O-, -NR⁶⁷- ou -S-,
R⁶³ et R⁶⁷ représentant, indépendamment, un atome d'hydrogène, un radical alkyle, allènyle, allènylalkyle, alkényle, alkynyle ou cyanoalkyle,
R⁶⁴ représentant un atome d'hydrogène, un radical alkyle, allènylalkyle, alkényle, alkényle, alkynyle, cyanoalkyle, alkoxy ou NR⁶⁸R⁶⁹,
R⁶⁸ et R⁶⁹ représentant, indépendamment, un atome d'hydrogène ou un radical alkyle, allènyle, allènylalkyle, alkényle, alkynyle ou cyanoalkyle,
et R⁴⁸ représente un atome d'hydrogène ou un radical alkyle, alkynyle ou cyanoalkyle ;
g et p, chaque fois qu'ils interviennent, étant indépendamment des entiers de 1 à 6, et k et n, chaque fois qu'ils interviennent, étant indépendamment des entiers de 0 à 6 ;
étant entendu que lorsque Het est tel que le composé de formule générale (I) réponde à la sous-formule générale **(I)₄,** alors :
A représente le radical 4-hydroxy-2,3-di-tertiobutyl-phényle ;
B, R¹ et R² représentent tous H ; et enfin
Ω représente OH ;
ou des sels pharmaceutiquement acceptables de composés de formule générale **(I)** ; peuvent être utilisés pour préparer un médicament destiné à avoir au moins l'une des trois activités suivantes :
- inhiber les monoamine oxydases, en particulier la monoamine oxydase B,
- inhiber la peroxydation lipidique,
- avoir une activité modulatrice vis-à-vis des canaux sodiques.

Ceci permet aux composés de formule générale **(I)** d'être utiles dans le traitement des maladies citées précédemment comme étant reliées aux MAO, à la peroxydation lipidique et aux canaux sodiques.

Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par allènyle, on entend le radical -CH=C=CH₂. Par aryle carbocyclique ou hétérocyclique, on entend un système carbocyclique (en particulier, le radical phényle qui peut être noté de façon abrégée Ph) ou hétérocyclique comprenant au moins un cycle aromatique, un système étant dit hétérocyclique lorsque l'un au moins des cycles qui le composent comporte un hétéroatome (O, N ou S). Par hétérocycle, on entend un système mono- ou polycyclique ledit système comprenant au moins un hétéroatome choisi parmi O, N et S et étant saturé, partiellement ou totalement insaturé ou aromatique. Par hétéroaryle, on entend un hétérocycle tel que défini précédemment dans lequel l'un au moins des cycles qui le composent est aromatique. Par haloalkyle, on entend un radical alkyle dont au moins l'un des atomes d'hydrogène (et éventuellement tous) est remplacé par un atome halogène.

De plus, lorsqu'il n'est pas donné plus de précision, on entend par un radical éventuellement substitué un radical comportant un ou des substituants choisis indépendamment parmi le groupe composé d'un atome halogène et des radicaux alkyle et alkoxy.

Par radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle, on entend respectivement les radicaux alkylthio, alkoxy, haloalkyle, alkoxyalkyle, trifluorométhylalkyle, cycloalkylalkyle, haloalkoxy, aminoalkyle, alkényle, alkynyle, allènylalkyle, cyanoalkyle et aralkyle dont le radical alkyle (les radicaux alkyle) a (ont) la (les) signification(s) indiquée(s) précédemment.

Par hétérocycle, on entend notamment les radicaux thiophène, pipéridine, pipérazine, quinoline, indoline et indole. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

La présente invention concerne une sélection de composés de formule générale **(I)** représentée ci-dessus, à savoir les composés suivants :
- 2,6-di*tert*-butyl-4-{2-[2-(méthylamino)éthyl]-1,3-thiazol-4-yl}phénol (ci-après composé 1) ;
- 2,6-di*tert*-butyl-4-[4-(hydroxyméthyl)-1,3-oxazol-2-yl]phénol (ci-après composé 2) ;
- 2,6-di*tert*-butyl-4-{2-[1-(méthylamino)éthyl]-1,3-thiazol-4-yl}phénol (ci-après composé 3) ;
- 2,6-di*tert*-butyl-4-[2-(méthoxyméthyl)-1,3-thiazol-4-yl]phénol (ci-après composé 4) ;
- 2,6-di*tert*-butyl-4-{4-[(méthylamino)méthyl]-1,3-oxazol-2-yl}phénol (ci-après composé 5) ;
- N-{[4-(3,5-di*tert*-butyl-4-hydroxyphényl)-1,3-thiazol-2-yl]méthyl}acétamide (ci-après composé 6) ;
- [4-(3,5-di*tert*-butyl-4-hydroxyphényl)-1,3-thiazol-2-yl]méthylcarbamate d'éthyle (ci-après composé 7) ;
- 2,6-di*tert*-butyl-4-[2-(morpholin-4-ylméthyl)-1,3-thiazol-4-yl]phénol (ci-après composé 8) ;
- 2,6-di*tert*-butyl-4-[2-(thiomorpholin-4-ylméthyl)-1,3-thiazol-4-yl]phénol (ci-après composé 9) ;
- 4-[2-(anilinométhyl)-1,3-thiazol-4-yl]-2,6-di*tert*-butylphénol (ci-après composé 10) ;
- 2,6-di*tert*-butyl-4-(2-{[[2-(diméthylamino)éthyl](méthyl)amino]méthyl}-1,3-thiazol-4-yl)phénol (ci-après composé 11) ;
- 2,6-di*tert*-butyl-4-{5-méthyl-2-[(méthylamino)méthyl]-1,3-thiazol-4-yl}phénol (ci-après composé 12) ;
- 1-[4-(10*H*-phénothiazin-2-yl)-1,3-thiazol-2-yl]méthanamine (ci-après composé 13) ;
- *N*-{[4-(3,5-di*tert*-butyl-4-hydroxyphényl)-1,3-thiazol-2-yl]méthyl}-*N*-méthylacétamide (ci-après composé 14) ;
- 1-[4-(3,5-di*tert*-butyl-4-méthoxyphényl)-1,3-thiaol-2-yl]-N-méthylméthanamine (ci-après composé 15) ;
- 2,6-di*tert*-butyl-4-{2-[(éthylamino)méthyl]-1,3-thiazol-4-yl}phénol (ci-après composé 16) ;
- 2,6-di*tert*-butyl-4-{2-[(4-phénylpipérazin-1-yl)méthyl]-1,3-thiazol-4-yl}phénol (ci-après composé 17) ;
- 2,6-di*tert*-butyl-4-{2-[(4-méthyl-1,4-diazépan-1-yl)méthyl]-1,3-thiazol-4-yl}phénol (ci-après composé 18) ;
- N-{1-[4-(4-anilinophényl)-1,3-thiazol-2-yl]éthyl}-N-méthylamine (ci-après composé 19) ;
- 2,6-di*tert*-butyl-4-{2-[(isopropylamino)méthyl]-1,3-thiazol-4-yl}phénol (ci-après composé 20) ;
- 2,6-di*tert*-butyl-4-{2-[(cyclohexylamino)méthyl]-1,3-thiazol-4-yl}phénol (ci-après composé 21) ;
- 2,6-di*tert*-butyl-4-{2-[(4-isopropylpipérazin-1-yl)méthyl]-1,3-thiazol-4-yl}phénol (ci-après composé 22) ;
- *N*-méthyl-1-[4-(10*H*-phénothiazin-2-yl)-1,3-thiazol-2-yl]éthanamine (ci-après composé 23) ;
- 2,6-di*tert*-butyl-4-{2-[(4-éthylpipérazin-1-yl)méthyl]-1,3-thiazol-4-yl}phénol (ci-après composé 24) ;
- *N*-{[4-(4-anilinophényl)-1,3-thiazol-2-yl]méthyl}-*N*-éthylamine (ci-après composé 25) ;
- *N*-{-[4-(10*H*-phénothiazin-2-yl)-1,3-thiazol-2-yl]méthyl}éthanamine (ci-après composé 26) ;
- 2,6-di*tert*-butyl-4-(2-{[4-(diméthylamino)pipéridin-1-yl]méthyl}-1,3-thiazol-4-yl)phénol (ci-après composé 27) ;
- 1-{[4-(3,5-di*tert*-butyl-4-hydroxyphényl)-1,3-thiazol-2-yl]méthyl}pipéridin-4-ol (ci-après composé 28) ;
- 2-[4-(1,1'-biphényl-4-yl)-1*H*-imidazol-2-yl]éthylcarbamate de 4-méthylpentyle (ci-après composé 29) ;
- 2-[4-(4-pyrrolidin-1-ylphényl)-1*H*-imidazol-2-yl]éthylcarbamate de 3,3-diméthylbutyle (ci-après composé 30) ;
- 2-[4-(1,1'-biphényl-4-yl)-1*H*-imidazol-2-yl]éthylcarbamate d'isopentyle (ci-après composé 31) ;
- 2-[4-(4'-bromo-1,1'-biphényl-4-yl)-1*H*-imidazol-2-yl]éthylcarbamate d'hexyle (ci-après composé 32) ;
- 2-[4-(4-*tert*-butylphényl)-1*H*-imidazol-2-yl]éthylcarbamate de benzyle (ci-après composé 33) ;
- 2-[4-(1,1'-biphényl-4-yl)-1*H*-imidazol-2-yl]éthylcarbamate de 3,3-diméthylbutyle (ci-après composé 34) ;
- 2-[4-(4-pyrrolidin-1-ylphényl)-1*H*-imidazol-2-yl]éthylcarbamate d'hexyle (ci-après composé 35) ;
- 2-[4-(3,5-di*tert*-butyl-4-hydroxyphényl)-1*H*-imidazol-2-yl]éthylcarbamate d'hexyle (ci-après composé 37) ;
- 2-[4-(3,5-di*tert*-butyl-4-hydroxyphényl)-1*H*-imidazol-2-yl]éthylcarbamate de 3,3-diméthylbutyle (ci-après composé 38) ;
- 2-[4-(4-méthoxyphényl)-1*H*-imidazol-2-yl]éthylcarbamate de 3,3-diméthylbutyle (ci-après composé 39) ;
- 2-[4-(3,5-di*tert*-butyl-4-hydroxyphényl)-1*H*-imidazol-2-yl]éthylcarbamate de benzyle (ci-après composé 40) ;
- 2-[4-(4-pyrrolidin-1-ylphényl)-1*H*-imidazol-2-yl]éthylcarbamate de benzyle (ci-après composé 41) ;
- 2-[4-(1,1'-biphényl-4-yl)-1*H*-imidazol-2-yl]éthylcarbamate de 2-phényléthyle (ci-après composé 42) ;
- 2-[4-(4'-fluoro-1,1'-biphényl-4-yl)-1*H*-imidazol-2-yl]éthylcarbamate de butyle (ci-après composé 43) ;
- 2-[4-(1,1'-biphényl-4-yl)-5-méthyl-1*H*-imidazol-2-yl]éthylcarbamate de butyle (ci-après composé 44) ;
- 2-[4-(4'-méthyl-1,1'-biphényl-4-yl)-1*H*-imidazol-2-yl]éthylcarbamate de butyle (ci-après composé 45) ;
- 2-[4-(4'-chloro-1,1'-biphényl-4-yl)-1*H*-imidazol-2-yl]éthylcarbamate de butyle (ci-après composé 46) ;
- 2-[4-(2'-fluoro-1,1'-biphényl-4-yl)-1*H*-imidazol-2-yl]éthylcarbamate de butyle (ci-après composé 47) ;
- 2-[4-(2',4'-difluoro-1,1'-biphényl-4-yl)-1*H*-imidazol-2-yl]éthylcarbamate de butyle (ci-après composé 49) ;
- 2,6-di-*tert*-butyl-4-{2-[(propylamino)méthyl]-1,3-thiazol-4-yl}phénol (ci-après composé 50) ;
- *N*-{[4-(10*H*-phénothiazin-2-yl)-1,3-thiazol-2-yl]méthyl}-*N*-propylamine (ci-après composé 51) ;
- *N*-{[4-(10*H*-phénothiazin-2-yl)-1,3-thiazol-2-yl]méthyllbutan-1-amine (ci-après composé 52) ;
- *N*-{[4-(10*H*-phénothiazin-2-yl)-1,3-thiazol-2-yl]méthyl}pentan-1-amine (ci-après composé 53) ;
- 1-{[4-(3,5-di-*tert*-butyl-4-hydroxyphényl)-1,3-thiazol-2-yl]méthyl}pipéridin-3-ol (ci-après composé 54) ;
- 1-{[4-(3,5-di-*tert*-butyl-4-hydroxyphényl)-1,3-thiazol-2-yl]méthyl}pyrrolidin-3-ol (ci-après composé 55) ;
- [4-(10*H*-phénothiazin-2-yl)-1,3-thiazol-2-yl]méthanol (ci-après composé 56) ;
- *N,N*-diméthyl-*N*-{[4-(10*H*-phénothiazin-2-yl)-1,3-thiazol-2-yl]méthyl}amine (ci-après composé 57) ;
- 2-{2-[(4-méthylpipérazin-1-yl)méthyl]-1,3-thiazol-4-yl}-10*H*-phénothiazine (ci-après composé 58) ;
- 2-[2-(pipéridin-1-ylméthyl)-1,3-thiazol-4-yl]-10*H*-phénothiazine (ci-après composé 59) ;
- 2-[2-(pipérazin-1-ylméthyl)-1,3-thiazol-4-yl]-10*H*-phénothinine (ci-après composé 60) ;
- 1-{[4-(3,5-*tert*-butyl4-hydroxyphényl)-1,3-thiazol-2-yl]méthyl}azétidin-3-ol (ci-après composé 61) ;
- 2-[2-(morpholin-4-ylméthyl)-1,3-thiazol-4-yl]-10*H*-phénothiazine (ci-après composé 62) ;
- 2-[2-(thiomorpholin-4-ylméthyl)-1,3-thiazol-4-yl]-10*H*-phénothinine (ci-après composé 63) ;
- 2-{2-[(4-méthyl-1,4-diazépan-1-yl)méthyl]-1,3-thiazol-4-yl}-10*H*-phénothiazine (ci-après composé 64) ;
- (3*R*)-1-{[4-(3,5-di-tert-butyl-4-hydroxyphényl)-1,3-thiazol-2-yl]méthyl}pyrrolidin-3-ol (ci-après composé 65) ;
- (3*S*)-1-{[4-(3,5-di-tert-butyl-4-hydroxyphényl)-1,3-thiazol-2-yl]méthyl}pyrrolidin-3-ol (ci-après composé 66) ;
- 2,6-di-*tert*-butyl-4-[2-(pyrrolidin-1-ylméthyl)-1,3-thiazol-4-yl]phénol (ci-après composé 67) ;
- 2,6-di-tert-butyl-4-{2-[(butylamino)méthyl]-1,3-thiazol-4-yl}phénol (ci-après composé 68) ;
- 2-{2-[(4-éthylpipérazin-1-yl)méthyl]-1,3-thiazol-4-yl}-10*H*-phénothinine (ci-après composé-69) ;
- *N*-méthyl-*N*-{[4-(10*H*-phénothiazin-2-yl)-1*H*-imidazol-2-yl]méthyl}amine (ci-après composé 70) ;
- [4-(10*H*-phénothiazin-2-yl)-1,3-thiazol-2-yl]méthylcarbamate de méthyle (ci-après composé 71) ;
- [4-(10*H*-phénothiazin-2-yl)-1,3-thiazol-2-yl]méthylcarbamate de butyle (ci-après composé 72) ;
- *N*-néopentyl-*N*-{[4-(10*H*-phénothiazin-2-yl)-1,3-thiazol-2-yl]méthyl}amine (ci-après composé 73) ;
- 1-{[4-(10*H*-phénothiazin-2-yl)-1,3-thiazol-2-yl]méthyl}pipéridin-4-ol (ci-après composé 74) ;
- *N*-{[4-(10*H*-phénothiazin-2-yl)-1,3-thiazol-2-yl]méthyl}acétamide (ci-après composé 75) ;
- *N*-{[4-(10*H*-phénothiazin-2-yl)-1,3-thiazol-2-yl]méthyl}butanamide (ci-après composé 76) ;
- 2,6-di-*tert*-butyl-4-{2-[(4-propylpipérazin-1-yl)méthyl]-1,3-thiazol-4-yl}phénol (ci-après composé 77) ;
- 2,6-di-*tert*-butyl-4-{2-[2-méthyl-1-(méthylamino)propyl]-1,3-thiazol-4-yl}phénol (ci-après composé 78) ;
- *N*,2-diméthyl-1-[4-(10*H*-phénothiazin-2-yl)-1,3-thiazol-2-yl]propan-1-amine (ci-après composé 79) ;
- *N*-{[4-(10*H*-phénothiazin-2-yl)-1,3-thiazol-2-yl]méthyl}hexanamide (ci-après composé 80) ;
- (3*R*)-1-{[4-(10*H*-phénothiazin-2-yl)-1,3-thiaol-2-yl]méthyl}pyrrolidin-3-ol (ci-après composé 81) ;
- (3*S*)-1-{[4-(10*H*-phénothiazin-2-yl)-1,3-thiaol-2-yl]méthyl}pyrrolidin-3-ol (ci-après composé 82) ;
- 1-{[4-(10*H*-phénothiazin-2-yl)-1,3-thiazol-2-yl]méthyl}azétidin-3-ol (ci-après composé 83) ;
- 2-{2-[(4-propylpipérain-1-yl)méthyl]-1,3-thiazol-4-yl}-10*H*-phénothiazine (ci-après composé 84) ;
- 2-{2-[(4-acétylpipérain-1-yl)méthyl]-1,3-thiazol-4-yl}-10*H*-phénothiazine (ci-après composé 85) ;
- 2-{2-[(4-butylpipérazin-1-yl)méthyl]-1,3-thiazol-4-yl}-10*H*-phénothiazine (ci-après composé 86) ;
- 4-{[4-(10*H*-phénothiazin-2-yl)-1,3-thiazol-2-yl]méthyl}pipérazine-1-carboxylate de méthyle (ci-après composé 87) ;
- 4-[2-(aminométhyl)-1H-imidazol-4-yl]-2,6-di-*tert*-butylphénol (ci-après composé 88) ;
- 4-{2-[(benzylamino)méthyl]-1,3-thiazol-4-yl}-2,6-di-*tert*-butylphénol (ci-après composé 89) ;
- 4-{2-[(4-acétylpipérazin-1-yl)méthyl]-1,3-thiazol-4-yl}-2,6-di-*tert*-butylphénol (ci-après composé 90) ;
- *N*-méthyl-N-{[4-(10*H*-phénoxazin-2-yl)-1,3-thiazol-2-yl]méthyl}amine (ci-après composé 91) ;
- 4-[2-(azétidin-1-ylméthyl)-1,3-thiazol-4-yl]-2,6-di-*tert*-butylphénol (ci-après composé 92) ;
- 2,6-di-*tert*-butyl-4-{2-[(4-butylpipérazin-1-yl)méthyl]-1,3-thiazol-4-yl}phénol (ci-après composé 93) ;
- 2-[4-(3'-chloro-1,1'-biphényl-4-yl)-1*H*-imidazol-2-yl]éthylcarbamate de butyle (ci-après composé 94) ;
- 2-[4-(3'-fluoro-1,1'-biphényl-4-yl)-1*H*-imidazol-2-yl]éthylcarbamate de butyle (ci-après composé 95) ;
- 2-[4-(4-isobutylphényl)-1*H*-imidazol-2-yl]éthylcarbamate de butyle (ci-après composé 96) ;
- 2-[4-(4-isobutylphényl)-1*H*-imidazol-2-yl]éthylcarbamate de benzyle (ci-après composé 97) ;
- 2-[4-(3'-chloro-4'-fluoro-1,1'-biphényl-4-yl)-1*H*-imidazol-2-yl]éthylcarbamate de butyle (ci-après composé 98) ;
- 2-[4-(3',4'-dichloro-1,1'-biphényl-4-yl)-1*H*-imidazol-2-yl]éthylcarbamate de butyle (ci-après composé 99) ;
- 2-[4-(4-propylphényl)-1*H*-imidazol-2-yl]éthylcarbamate de butyle (ci-après composé 100) ;
- 2-[4-(4-éthylphényl)-1*H*-imidazol-2-yl]éthylcarbamate de butyle (ci-après composé 101) ;
- 2-[4-(4'-cyano-1,1'-biphényl-4-yl)-1*H*-imidazol-2-yl]éthylcarbamate de butyle (ci-après composé 102) ;
- 2-[4-(1,1'-biphényl-4-yl)-5-éthyl-1*H*-imidazol-2-yl]éthylcarbamate de butyle (ci-après composé 104) ;
- 2-[4-(2'-chloro-1,1'-biphényl-4-yl)-1*H*-imidazol-2-yl]éthylcarbamate de butyle (ci-après composé 105) ;
- 2-[4-(2',3'-difluoro-1,1'-biphényl-4-yl)-1*H*-imidazol-2-yl]éthylcarbamate de butyle (ci-après composé 106) ;
- 2-[4-(2'-bromo-1,1'-biphényl-4-yl)-1*H*-imidazol-2-yl]éthylcarbamate de butyle (ci-après composé 107) ;
- 2-[4-(3',5'-difluoro-1,1'-biphényl-4-yl)-1*H*-imidazol-2-yl]éthylcarbamate de butyle (ci-après composé 108) ;
- 2-[4-(2'-méthoxy-1,1'-biphényl-4-yl)-1*H*-imidazol-2-yl]éthylcarbamate de butyle (ci-après composé 109) ;
- 2-[4-(3'-nitro-1,1'-biphényl--4-yl)-1*H*-imidazol-2-yl]éthylcarbamate de butyle (ci-après composé 110) ;
- 2-[4-(2',5'-difluoro-1,1'-biphényl-4-yl)-1*H*-imidazol-2-yl]éthylcarbamate de butyle (ci-après composé 111) ;
- 2-[4-(3'-méthoxy-1,1'-biphényl-4-yl)-1*H*-imidazol-2-yl]éthylcarbamate de butyle (ci-après composé 112) ;
- 4-{[4-(3,5-di-*tert*-butyl-4-hydroxyphényl)-1,3-thiazol-2-yl]méthyl}pipérazine-1-carboxylate de méthyle (ci-après composé 113) ;
- [4-(3,5-di-*tert*-butyl-4-hydroxyphényl)-1,3-thiazol-2-yl]méthylcarbamate de méthyle (ci-après composé 114) ;
- *N*-{[4-(3,5-di-*tert*-butyl-4-hydroxyphényl)-1,3-thiazol-2-yl]méthyl}benzamide (ci-après composé 115) ;
- *N*-{[4-(3,5-di-*tert*-butyl-4-hydroxyphényl)-1,3-thiazol-2-yl]méthyl}-2-phénylacétamide (ci-après composé 116) ;
- *N*-{[4-(3,5-di-*tert*-butyl-4-hydroxyphényl)-ï,3-thiazol-2-yl]méthyl}propanamide (ci-après composé 117) ;
- acétate de 1-{[4-(3,5-di-*tert*-butyl-4-hydroxyphényl)-1,3-thiazol-2-yl]méthyl}pipéridin-4-yle (ci-après composé 118) ;
- 1-{[4-(3,5-di-tert-butyl-4-hydroxyphényl)-1,3-thiazol-2-yl]méthyl}pyrrolidine-3,4-diol (ci-après composé 119) ;
et les sels de ces derniers.

En particulier, l'invention concernera les composés 1 à 112 et leurs sels, et notamment les composés 1 à 49 et leurs sels.

Selon des variantes préférées de l'invention, ces composés possèderont au moins deux des activités citées ci-dessus. En particulier, ils inhiberont à la fois les MAO et piégeront les ROS ou ils auront à la fois une activité antagoniste vis-à-vis des canaux sodiques et une activité piégeuse des ROS. Dans certains cas, les composés de formule générale **(I)** combineront même les trois activités.

Selon une variante particulière de l'invention, les composés de l'invention seront plus spécialement destinés à avoir une activité inhibitrice des MAO et/ou des ROS et ils seront alors de préférence choisis parmi les composés 1 à 28, 50 à 93 et 113 à 119 et les sels de ces composés (notamment parmi les composés 1 à 28 et 50 à 93 et les sels de ces composés, et en particulier parmi les composés 1 à 28 et les sels de ces composés).

Plus préférentiellement, les composés de l'invention, lorsqu'ils seront destinés à avoir une activité inhibitrice des MAO et/ou des ROS, seront choisis parmi les composés parmi les composés 1, 3, 6 à 8, 12, 13, 15, 16, 18 à 20, 22 à 28, 50 à 62, 64 à 71, 73 à 86, 89, 91 à 93 et 119 et les sels de ces composés (notamment parmi les composés 1, 3, 6 à 8, 12, 13, 15, 16, 18 à 20, 22 à 28, 50 à 62, 64 à 71, 73 à 86, 89 et 91 à 93 et les sels de ces composés, et en particulier parmi les composés 1, 3, 6 à 8, 12, 13, 15, 16, 18 à 20 et 22 à 28 et les sels de ces composés).

Encore plus préférentiellement, les composés de l'invention, lorsqu'ils seront destinés à avoir une activité inhibitrice des MAO et/ou des ROS, seront choisis parmi les composés parmi les composés 1, 3, 6 à 8, 12, 13, 15, 16, 18 à 20, 22 à 24, 26, 28, 50 à 52, 54, 55, 57, 58, 61, 62, 65 à 69, 73, 75, 77 à 82, 86, 89, 91, 92 et 119 et les sels de ces composés (notamment parmi les composés 1, 3, 6 à 8, 12, 13, 15, 16, 18 à 20, 22 à 24, 26, 28, 50 à 52, 54, 55, 57, 58, 61, 62, 65 à 69, 73, 75, 77 à 82, 86, 89, 91 et 92 et les sels de ces composés, et en particulier parmi les composés 1, 3, 6 à 8, 12, 13, 15, 16, 18 à 20, 22 à 24, 26 et 28 et les sels de ces composés).

En particulier, les composés de l'invention, lorsqu'ils seront destinés à avoir une activité inhibitrice des MAO et/ou des ROS, seront choisis parmi les composés parmi les composés 1, 3, 6, 15, 16, 18, 20, 23, 24, 26, 28, 50, 52, 55, 61, 65 à 69, 77, 78, 79, 81, 86, 89, 91, 92 et 119 et les sels de ces composés (notamment parmi les composés 1, 3, 6, 15, 16, 18, 20, 23, 24, 26, 28, 50, 52, 55, 61, 65 à 69, 77, 78, 79, 81, 86, 89, 91 et 92 et les sels de ces composés, et en particulier parmi les composés 1, 3, 6, 15, 16, 18, 20, 23, 24, 26 et 28 et les sels de ces composés).

Plus particulièrement, les composés de l'invention, lorsqu'ils seront destinés à avoir une activité inhibitrice des MAO et/ou des ROS, seront choisis parmi les composés parmi les composés 3, 15, 16, 20, 23, 26, 28, 50, 55, 61, 65 à 68, 78, 79, 91 et 92 et les sels de ces composés (notamment parmi les composés 3, 15, 16, 20, 23, 26 et 28 et les sels de ces composés).

Encore plus particulièrement, les composés de l'invention, lorsqu'ils seront destinés à avoir une activité inhibitrice des MAO et/ou des ROS, seront choisis parmi les composés parmi les composés 3, 15, 16, 28, 55, 61, 65, 66 et 79 et les sels de ces composés (notamment parmi les composés 3, 15, 16 et 28 et les sels de ces composés).

Selon une autre variante de l'invention, les composés de l'invention seront plus spécialement destinés à avoir une activité modulatrice des canaux sodiques et ils seront alors de préférence choisis parmi les composés 1, 3, 5, 12, 15, 16, 29 à 35, 37 à 47, 49, 94 à 102 et 104 à 112 et les sels de ces composés (notamment parmi les composés 1, 3, 5, 12, 15, 16, 29 à 35, 37 à 47 et 49 et les sels de ces composés).

Plus préférentiellement, les composés de l'invention destinés à avoir une activité modulatrice des canaux sodiques seront choisis parmi les composés 3, 15, 16, 29 à 35, 37 à 47, 49, 94 à 102 et 104 à 112 et les sels de ces composés (notamment parmi les composés 1, 3, 5, 12, 15, 16, 29 à 35, 37 à 47 et 49 et les sels de ces composés).

Encore plus préférentiellement, les composés de formule générale **(I)** destinés à avoir une activité modulatrice des canaux sodiques seront choisis parmi les composés 30, 37, 42, 44 à 46, 48, 49, 106, 108, 109 et 112 et les sels de ces composés (notamment parmi les composés 30, 37, 42, 44 à 46, 48 et 49 et les sels de ces composés).

Par ailleurs, les composés plus spécialement destinés à avoir une activité inhibitrice de la peroxydation lipidique seront de préférence choisis parmi les composés 1 à 28, 37, 38, 40, 50 à 93 et 113 à 119 et les sels de ces composés (notamment parmi les composés 1 à 28, 37, 38, 40 et 50 à 93 et les sels de ces composés, et en particulier parmi les composés 1 à 28, 37, 38 et 40 et les sels de ces composés).

Plus préférentiellement, les composés plus spécialement destinés à avoir une activité inhibitrice de la peroxydation lipidique seront choisis parmi les composés 1 à 28, 50 à 62, 64 à 93 et 113 à 119 et les sels de ces composés (notamment parmi les composés 1 à 28, 50 à 62 et 64 à 93 et les sels de ces composés, et en particulier parmi les composés 1 à 28 et les sels de ces composés).

Encore plus préférentiellement, les composés plus spécialement destinés à avoir une activité inhibitrice de la peroxydation lipidique seront choisis parmi les composés 13, 18, 19, 22 à 27, 51 à 53, 55 à 60, 62, 64, 69, 73 à 76, 79, 81 à 86 et 91 et les sels de ces composés (notamment parmi les composés 13, 18, 19 et 22 à 27 et les sels de ces composés).

En particulier, les composés plus spécialement destinés à avoir une activité inhibitrice de la peroxydation lipidique seront choisis parmi les composés 13, 23, 58, 64, 81, 82 et 91 et les sels de ces composés (notamment parmi les composés 13 et 23 et les sels de ces composés).

L'invention concerne de plus, en tant que médicaments, les composés sélectionnés mentionnés précédemment et leurs sels pharmaceutiquement acceptables. L'invention concerne également des compositions contenant, à titre de principe actif, au moins l'un des composés sélectionnés mentionnés précédemment ou un sel pharmaceutiquement acceptable d'un de ces composés.

L'invention a encore pour objet l'utilisation d'un des composés sélectionnés mentionnés précédemment ou d'un sel pharmaceutiquement acceptable d'un de ces composés pour préparer un médicament destiné à avoir au moins l'une des trois activités suivantes :
- inhiber les monoamine oxydases, en particulier la monoamine oxydase B,
- inhiber la peroxydation lipidique,
- avoir une activité modulatrice vis-à-vis des canaux sodiques.

En particulier, l'invention concerne l'utilisation d'un des composés sélectionnés mentionnés précédemment ou d'un sel pharmaceutiquement acceptable d'un de ces composés pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses, la migraine ou les douleurs et en particulier les douleurs neuropathiques.

L'invention concerne par ailleurs les composés de formule générale **(I'),** formule générale identique à la formule générale **(I)** excepté le fait que :
(a) soit A est remplacé par un radical A' 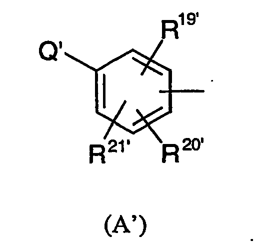 dans lequel Q' représente un radical phényle éventuellement substitué par un ou des substituants choisis indépendamment parmi un atome halogène, un radical OH, cyano, nitro, alkyle, haloalkyle, alkoxy, alkylthio ou -NR^{10'}R^{11'} et un groupe de deux substituants représentant ensemble un radical méthylène dioxy ou éthylènedioxy,
   R^{10'} et R^{11'} représentant, indépendamment, un atome d'hydrogène, un radical alkyle ou un groupe -COR^{12'}, ou bien R^{10'} et R^{11'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   R^{12'} représentant un atome d'hydrogène, un radical alkyle ou alkoxy ou NR^{13'}R^{14'}, R^{13'} et R^{14'} représentant, indépendamment, un atome d'hydrogène ou un radical alkyle, ou bien R^{13'} et R^{14'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   et R^{19'}, R^{20'} et R^{21'} représentent, indépendamment, un hydrogène, un halogène, le groupe OH ou SR^{26'}, ou un radical alkyle, cycloalkyle, alkényle, alkoxy, alkylthio, cyano, nitro, -SO₂NHR⁴⁹', -CONHR⁵⁵', -S(O)_{q}R⁵⁶', -NH(CO)R⁵⁷', -CF₃, -OCF₃ ou NR^{27'}R²⁸',
   R^{26'} représentant un atome d'hydrogène ou un radical alkyle,
   R^{27'} et R^{28'} représentant, indépendamment, un atome d'hydrogène, un radical alkyle ou un groupe -COR^{29'}, ou bien R^{27'} et R^{28'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   R⁴⁹' et R⁵⁵' représentant, indépendamment à chaque fois qu'ils interviennent, un atome d'hydrogène ou un radical alkyle ou alkylcarbonyle,
   q représentant un entier de 0 à 2,
   R⁵⁶' et R⁵⁷' représentant, indépendamment à chaque fois qu'ils interviennent, un atome d'hydrogène ou un radical alkyle ou alkoxy,
   R^{29'} représentant un atome d'hydrogène, un radical alkyle, alkoxy ou -NR^{30'}R^{31'},
   R^{30'} et R^{31'} représentant, indépendamment, un atome d'hydrogène ou un radical alkyle, ou bien R^{30'} et R^{31'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   R⁵¹ étant en outre remplacé par un radical R⁵¹', ledit radical R⁵¹' représentant l'un des radicaux de la définition de R⁵¹ dans la formule générale **(I)** ou un radical haloalkyle, étant entendu que soit Q' représente un radical phényle substitué par au moins un radical haloalkyle, soit l'un au moins de Q', R^{19'}, R^{20'} et R^{21'} représente un radical alkylthio ;
(b) soit Ω est remplacé par un radical Ω', ledit radical Ω' représentant un radical NR⁴⁶R⁴⁷ dans lequel l'un de R⁴⁶' et R⁴⁷' représente un radical -COOR⁵¹' et l'autre représente un atome d'hydrogène, R⁵¹' représentant un radical haloalkyle ;
et les sels desdits composés.

En particulier, cet aspect de l'invention concerne les composés de formule générale **(I'),** formule générale identique à la formule générale **(I)** excepté le fait que :
(a) soit A est remplacé par un radical A' 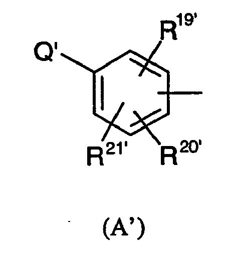 dans lequel Q' représente un radical phényle éventuellement substitué par un ou des substituants choisis indépendamment parmi un atome halogène, un radical OH, cyano, nitro, alkyle, alkoxy, alkylthio ou -NR^{10'}R^{11'} et un groupe de deux substituants représentant ensemble un radical méthylène dioxy ou éthylènedioxy, R^{10'} et R^{11'} représentant, indépendamment, un atome d'hydrogène, un radical alkyle ou un groupe -COR^{12'}, ou bien R^{10'} et R^{11'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   R^{12'} représentant un atome d'hydrogène, un radical alkyle ou alkoxy ou NR^{13'}R^{14'},
   R^{13'} et R^{14'} représentant, indépendamment, un atome d'hydrogène ou un radical alkyle, ou bien R^{13'} et R^{14'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   et R^{19'}, R^{20'} et R^{21'} représentent, indépendamment, un hydrogène, un halogène, le groupe OH ou SR^{26'}, ou un radical alkyle, cycloalkyle, alkényle, alkoxy, alkylthio, cyano, nitro, -SO₂NHR⁴⁹', -CONHR⁵⁵', -S(O)_{q}R⁵⁶', -NH(CO)R⁵⁷', -CF₃, -OCF₃ ou NR^{27'}R^{28'},
   R^{26'} représentant un atome d'hydrogène ou un radical alkyle,
   R^{27'} et R^{28'} représentant, indépendamment, un atome d'hydrogène, un radical alkyle ou un groupe -COR^{29'}, ou bien R^{27'} et R^{28'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine ou thiomorpholine,
   R⁴⁹' et R⁵⁵' représentant, indépendamment à chaque fois qu'ils interviennent, un atome d'hydrogène ou un radical alkyle ou alkylcarbonyle,
   q représentant un entier de 0 à 2,
   R⁵⁶' et R⁵⁷' représentant, indépendamment à chaque fois qu'ils interviennent, un atome d'hydrogène ou un radical alkyle ou alkoxy,
   R^{29'} représentant un atome d'hydrogène, un radical alkyle, alkoxy ou -NR^{30'}R^{31'},
   R^{30'} et R^{31'} représentant, indépendamment, un atome d'hydrogène ou un radical alkyle, ou bien R^{30'} et R^{31'} formant ensemble avec l'atome d'azote un hétérocycle éventuellement substitué comptant de 4 à 7 chaînons et de 1 à 3 hétéroatomes incluant l'atome d'azote déjà présent, les hétéroatomes supplémentaires étant choisis indépendamment dans le groupe constitué des atomes O, N et S, ledit hétérocycle pouvant être par exemple azétidine, pyrrolidine, pipéridine, pipérazine, morpholine
   ou thiomorpholine,
   R⁵¹ étant en outre remplacé par un radical R⁵¹', ledit radical R⁵¹' représentant l'un des radicaux de la définition de R⁵¹ dans la formule générale **(I)** ou un radical haloalkyle, étant entendu que l'un au moins de Q', R^{19'}, R^{20'} et R^{21'} représente un radical alkylthio ;
(b) soit Ω est remplacé par un radical Ω', ledit radical Ω' représentant un radical NR⁴⁶R⁴⁷ dans lequel l'un de R⁴⁶' et R⁴⁷' représente un radical -COOR⁵¹' et l'autre représente un atome d'hydrogène, R⁵¹' représentant un radical haloalkyle ;
   et les sels desdits composés.

Dans le cas (a), les composés de formule générale **(I')** seront de préférence tels que n représente 0 ou 1 et Ω représente un radical NR⁴⁶R⁴⁷ (l'un de R⁴⁶ et R⁴⁷ représentant de préférence un radical COOR⁵¹ quand n = 1). De même, R¹ et R² seront de préférence choisis indépendamment parmi le groupe constitué d'un atome d'hydrogène et un radical alkyle ou cycloalkyle (et de préférence un radical méthyle). Toujours de préférence pour le cas (a), les composés de formule générale **(I')** correspondront à l'une des sous-formules générales **(I)₁** ou **(I)₂,** X représentant de préférence S ou NH, et plus préférentiellement NH. En outre, le radical alkylthio sera de préférence un radical éthylthio ou méthylthio, plus préférentiellement un radical méthylthio.

Dans le cas (b), les composés de formule générale **(I')** seront de préférence tels que n représente 0 ou 1 (et de préférence 1). De même, R¹ et R² seront de préférence des atomes d'hydrogène. En outre, toujours dans le cas (b), le radical haloalkyle sera de préférence un radical substitué exclusivement par un ou des atomes de fluor (par exemple le radical 4,4,4-trifluorobutyle). Toujours de préférence pour le cas (b), les composés de formule générale **(I')** correspondront à l'une des sous-formules générales **(I)₁** ou **(I)₂,** X représentant de préférence S ou NH, et plus préférentiellement NH.

L'invention concerne donc également en particulier les composés de formule générale **(I')** suivants :
- le 2-{4-[4'-(méthylthio)-1,1'-biphényl-4-yl]-1H-imidazol-2-yl}éthylcarbamate de butyle ;
- le 2-[4-(4-pyrrolidin-1-ylphényl)-1*H*-imidazol-2-yl]éthylcarbamate de 4,4,4-trifluorobutyle ;
- le 2-{4-[4'-(trifluorométhyl)-1,1'-biphényl-4-yl]-1H-imidazol-2-yl}éthylcarbamate de butyle ;
et les sels desdits composés ;
et en particulier :
- le 2-{4-[4'-(méthylthio)-1,1'-biphényl-4-yl]-1H-imidazol-2-yl}éthylcarbamate de butyle ;
- le 2-[4-(4-pyrrolidin-1-ylphényl)-1*H*-imidazol-2-yl]éthylcarbamate de 4,4,4-trifluorobutyle ;
et les sels desdits composés ;

L'invention concerne de plus, en tant que médicaments, les composés de formule générale **(I')** définie précédemment et leurs sels pharmaceutiquement acceptables. L'invention concerne également des compositions contenant, à titre de principe actif, au moins l'un des composés de formule générale **(I')** définie précédemment ou un sel pharmaceutiquement acceptable d'un de ces composés.

L'invention a encore pour objet l'utilisation d'un des composés de formule générale **(I')** définie précédemment ou d'un sel pharmaceutiquement acceptable d'un de ces composés pour préparer un médicament destiné à avoir au moins l'une des trois activités suivantes :
- inhiber les monoamine oxydases, en particulier la monoamine oxydase B,
- inhiber la peroxydation lipidique,
- avoir une activité modulatrice vis-à-vis des canaux sodiques.

En particulier, l'invention concerne l'utilisation d'un des composés de formule générale **(I')** définie précédemment ou d'un sel pharmaceutiquement acceptable d'un de ces composés pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses, la migraine ou les douleurs et en particulier les douleurs neuropathiques.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure ou les noms des composés, il faut comprendre que les deux formes énantiomères et leurs mélanges sont représentés.

L'invention concerne également, à titre de médicaments, les composés cités précédemment ou leurs sels pharmaceutiquement acceptables. Elle concerne de même des compositions pharmaceutiques contenant, à titre de principe actif, lesdits composés ou leurs sels pharmaceutiquement acceptables ainsi que l'utilisation de ces mêmes composés ou de leurs sels pharmaceutiquement acceptables pour préparer un médicament destiné à inhiber les monoamine oxydases, en particulier la monoamine oxydase B, inhiber la peroxydation lipidique, avoir une activité modulatrice vis-à-vis des canaux sodiques ou à posséder deux des trois ou les trois activités précitées.

En particulier, les composés de l'invention peuvent être utilisés pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses, la migraine ou les douleurs et en particulier les douleurs neuropathiques. Plus particulièrement, les composés présentés comme inhibant les MAO et/ou les ROS pourront être utilisés pour traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions et les psychoses ; et les composés présentés cmme ayant une activité modulatrice des canaux sodiques pourront être utilisés pour traiter l'un des désordres ou l'une des maladies suivantes : la maladie de Parkinson, la maladie d'Alzheimer, la sclérose latérale amyotrophique, la migraine ou les douleurs et en particulier les douleurs neuropathiques.

Par sel, on entend notamment dans la présente demande les sels d'addition d'acides inorganiques ou organiques ainsi que les sels formés à partir de bases.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-dessous.

### PREPARATION DES COMPOSES DE L'INVENTION :

### Généralités

Les préparations des composés de l'invention dans lesquels Ω représente OH sont effectuées de façon analogue à celles décrites dans la demande de brevet PCT WO 99/09829 et la demande de brevet européen EP 432 740.

En ce qui concerne les composés de l'invention dans lesquels Het est un cycle imidazole, l'homme du métier pourra aussi utilement consulter la demande de brevet PCT WO 99/64401.

Les préparations des autres composés de l'invention sont effectuées de façon analogue à celles décrites dans la demande de brevet PCT WO 98/58934 (*cf*. *en particulier en pages 39 à 45 de ce document les synthèses des intermédiaires de formules générales (XXV) et (XXVIII)*) ou selon les procédures décrites ci-après.

Par ailleurs, les composés de formule générale **(I')** se préparent de façon analogue à celle des composés de formule générale **(I) ;** l'enseignement de l'exposé qui suit pour les composés de formule générale **(I)** pourra d'une façon générale être étendu à la synthèse des composés de formule générale **(I').**

### Préparation des composés de formule générale (I)

Les composés de formule générale **(I)** peuvent être préparés par les 8 voies synthétiques illustrées ci-dessous (schéma 1) à partir des intermédiaires de formule générale **(IV), (V), (VI), (VII), (VIII), (IX), (X)** et **(*I*)*bis*** dans lesquels A, B, Ω, R¹, R², Het et n sont tels que définis ci-dessus, L est un groupe partant comme par exemple un halogène, Alk est un radical alkyle, Gp est un groupe protecteur pour une fonction amine, par exemple un groupement 2-(triméthylsilyl)éthoxyméthyle (SEM), et Gp' un groupe protecteur pour une fonction alcool, par exemple un groupe de type benzyle, acétate ou encore silyle comme le *tert*-butyldiméthylsilyle, et enfin A représente une liaison ou un radical -(CH₂)ₓ-, -CO-(CH₂)ₓ-, -(CH₂)_{y}-O- ou -C(=NH)-. Bien entendu, l'homme du métier pourra choisir d'utiliser d'autres groupes protecteurs Gp et Gp' parmi ceux qu'il connaît, et notamment ceux cités dans : Protective groups in organic synthesis, 2nd ed., (John Wiley & Sons Inc., 1991).

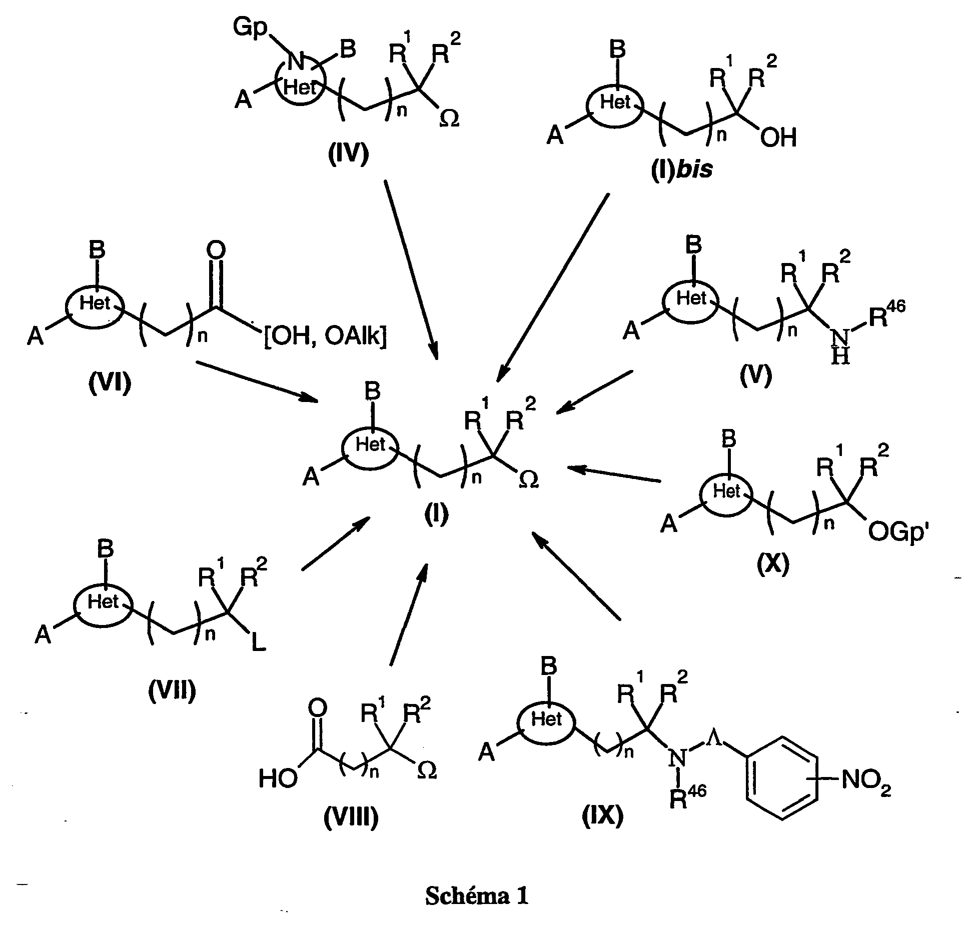

### Voie 1 : Het est imidazole et Ω est NR⁴⁶R⁴⁷ mais pas un radical de type carbamate

Les amines et les carboxamides de formule générale **(I),** schéma 2, dans laquelle A, B, R¹, R², R⁴⁶, R⁴⁷, Het et n sont tels que définis ci-dessus, sont préparés par déprotection par exemple, dans le cas où Gp représente SEM, avec du fluorure de *tert*-butylammonium (TBAF) dans du THF, de l'amine de formule générale **(IV)** pour libérer l'amine de l'hétérocycle du composé de formule générale **(I).** Les amines protégées de formule générale **(IV)** sont accessibles par une voie générale de synthèse décrite dans Biorg. and Med. Chem. Lett., 1993, 3, 915 et Tetrahedron Lett., 1993. 34, 1901 et plus particulièrement dans la demande de brevet PCT WO 98/58934.

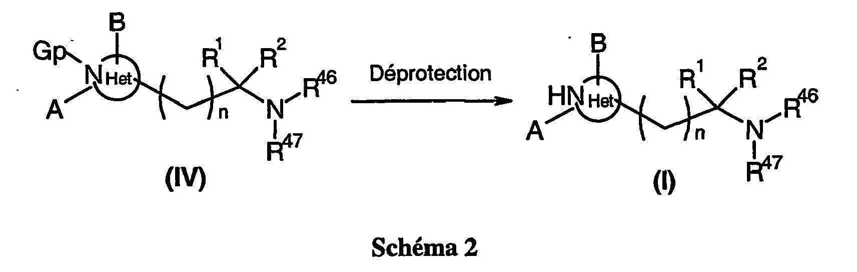

### Voie 2 : Het est imidazole, oxazole ou thiazole et Ω est NR⁴⁶R⁴⁷

Les amines et les carboxamides de formule générale **(I),** schéma 3, dans lesquelles A, B, R¹, R², R⁴⁶, Het, g, k et n sont tels que définis ci-dessus, Δ représente un radical alkyle, cycloalkylalkyle, arylalkyle, aryle, allényle, allénylalkyle, alkényle, alkynyle, cyanoalkyle ou hydroxyalkyle et Δ' représente un radical alkyle, cycloalkylalkyle, arylalkyle ou aryle lorsque g ou k ne représente pas 0, ou Δ' représente un radical alkyle, cycloalkylalkyle, arylalkyle ou un radical aryle de préférence désactivé (c'est-à-dire un radical aryle substitué par un groupe attracteur d'électrons comme par exemple un groupe nitro ou cyano) lorsque g ou k représente 0, sont préparés par condensation des amines de formule générale **(V)** avec les acides carboxyliques (ou les chlorures d'acide correspondants) de formule générale **(XIII)** dans les conditions classiques de la synthèse peptidique, avec les aldéhydes de formule générale **(XII)** en présence d'un agent réducteur comme le triacétoxyborohydrure de sodium ou le borohydrure de sodium, dans un alcool aliphatique inférieur comme le méthanol et éventuellement en présence de tamis moléculaires, ou avec les dérivés halogénés (Hal = atome halogène) de formule générale **(XI).** En particulier, lorsque Δ représente un radical allényle, allénylalkyle, alkényle, alkynyle, cyanoalkyle ou hydroxyalkyle, les composés de formule générale **(V)** sont convertis en les composés de formule générale **(I)** correspondants par réaction avec les dérivés halogénés de formule générale **(XI)** dans un solvant comme l'acétonitrile, le dichlorométhane ou l'acétone et en présence d'une base comme par exemple la triéthylamine ou le carbonate de potassium à une température comprise entre la température ambiante et la température de reflux du solvant.

Les dérivés de formule générale **(V)** sont notamment accessibles par une voie générale de synthèse décrite dans Biorg. and Med. Chem. Lett., 1993, 3, 915 et Tetrahedron Lett., 1993. 34, 1901, et plus particulièrement dans la demande de brevet WO 98/58934. Lorsque R⁴⁶ = H, les composés de formule générale **(V)** peuvent être préparés, par exemple, selon un protocole décrit dans la demande de brevet WO 98/58934 (en utilisant l'acide aminé adéquat à la place du N-Boc-sarcosinamide).

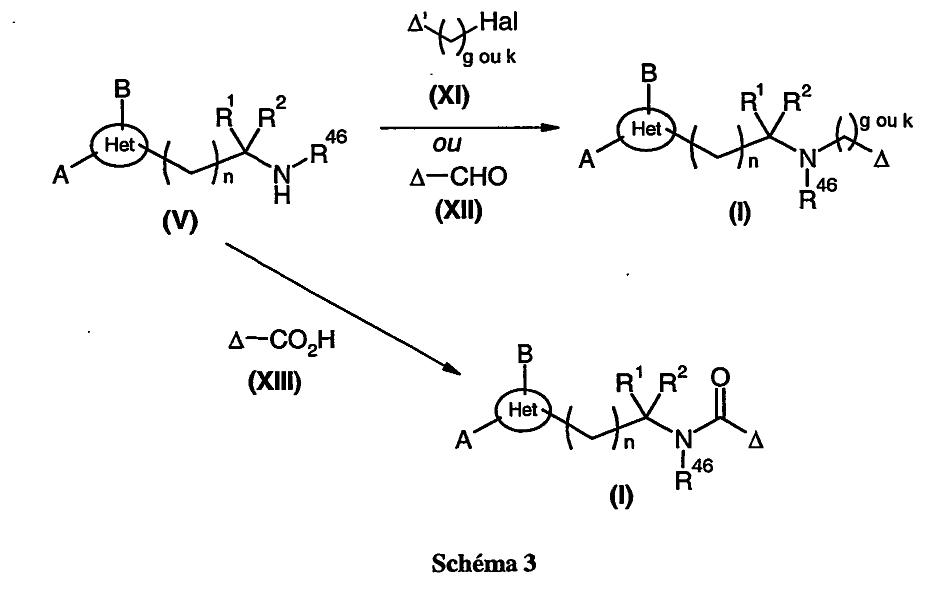

Dans le cas particulier où R⁴⁷ représente un radical cycloalkyle, les amines de formule générale **(I),** schéma 3*bis*, dans laquelle A, B, R¹, R², R⁴⁶, Het et n sont tels que définis ci-dessus et i représente un entier de 0 à 4 sont préparés par condensation des amines de formule générale **(V)** avec les cycloalkylcétones de formule générale **(XIV)** en présence d'un agent réducteur comme le triacétoxyborohydrure de sodium ou le borohydrure de sodium dans un alcool aliphatique inférieur comme le méthanol et éventuellement en présence de tamis moléculaires à température ambiante.

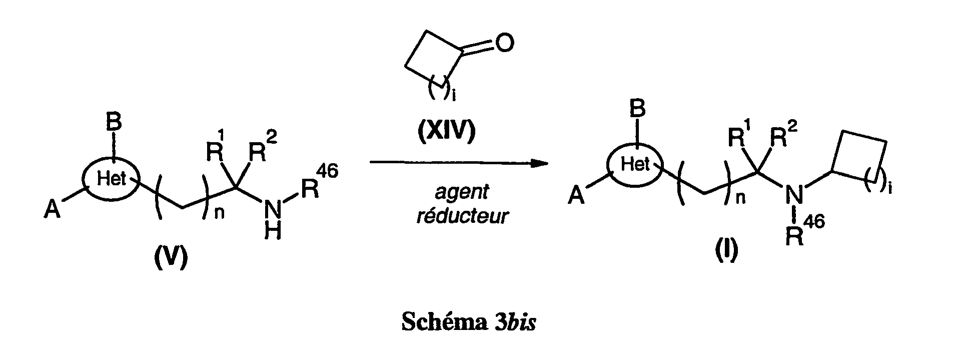

Les sulfonamides de formule générale **(I),** schéma 3*ter*, dans laquelle A, B, R¹, R², R⁴⁶, Het et n sont tels que définis ci-dessus, R⁴⁷ représente un radical -SO₂-Δ et Δ représente un radical alkyle, cycloalkyle, cycloalkylalkyle ou arylalkyle, sont préparés par condensation des amines de formule générale **(V)** avec les sulfochlorures de formule générale **(XV)** dans des conditions classiques, par exemple dans un solvant comme le diméthylformamide à température ambiante.

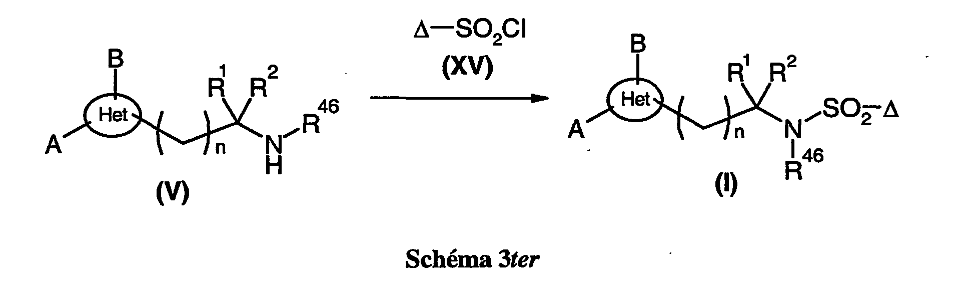

Les urées de formule générale **(I),** schéma 3*quater*, dans laquelle A, B, R¹, R², R⁴⁶, Het et n sont tels que définis ci-dessus, R⁴⁷ représente un radical -CO-NH-Δ et Δ représente un radical alkyle, cycloalkyle, cycloalkylalkyle ou arylalkyle, sont préparés par réaction des amines de formule générale **(V)** avec les isocyanates de formule générale **(XVI)** dans un solvant inerte comme le dichlorométhane ou le 1,2-dichloroéthane.

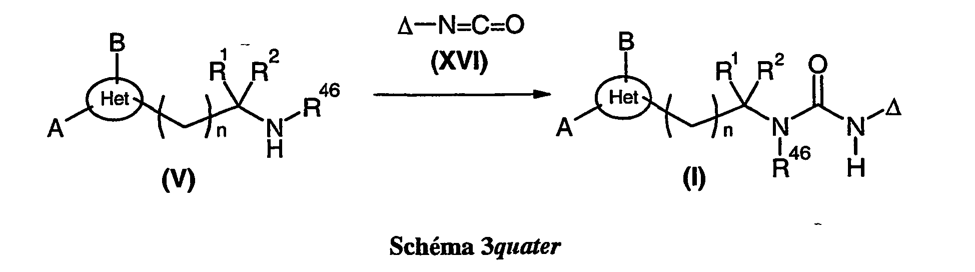

### Voie 3 : Het est oxazole ou thiazole, R¹ et R² sont tous deux H et Ω est OH.

Les dérivés alcooliques de formule générale **(I),** schéma 4, dans lesquels A, B, Het et n sont tels que définis ci-dessus et R¹ et R² sont des atomes d'hydrogène sont obtenus par réduction des acides ou esters de formule générale **(VI)** (accessibles par une voie générale de synthèse décrite dans J.Med Chem., 1996, 39, 237-245 et la demande de brevet PCT WO 99/09829). Cette réduction peut, par exemple, être effectuée par action d'hydrure de bore ou de lithium aluminium ou encore d'hydrure de diisobutylaluminium dans un solvant polaire aprotique comme le tétrahydrofuranne.

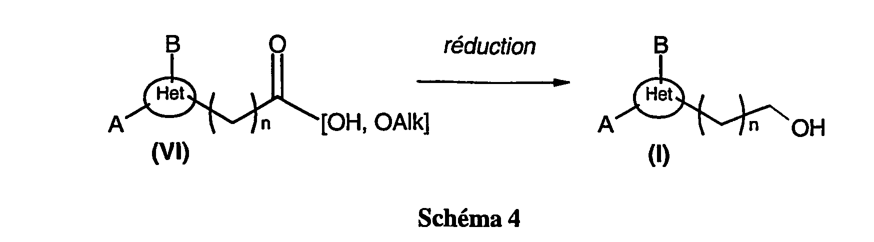

### Voie 4 : Het est oxazole ou thiazole et Ω est NR⁴⁶R⁴⁷

Les amines de formule générale **(I),** schéma 5, dans laquelles A, B, R¹, R², R⁴⁶, R⁴⁷, Het, et n sont tels que définis ci-dessus, sont préparées par condensation des amines primaires ou secondaires de formule générale R⁴⁶-NHR⁴⁷ avec les composés de formule générale **(VII)** (dans lesquels L représente de préférence un atome halogène Hal, mais peut aussi représenter un groupe mésylate ou tosylate) selon une voie générale de synthèse décrite dans J. Med. Chem., 1996, 39, 237-245 et la demande de brevet PCT WO 99/09829 ou le brevet US 4,123,529. Cette voie de synthèse peut en particulier être employée lorsque R⁴⁶ et R⁴⁷ pris ensemble forment avec l'atome d'azote qui les porte un hétérocycle non aromatique de 4 à 8 chaînons. La réaction a typiquement lieu dans un solvant anhydre (par exemple le diméthylformamide, le dichlorométhane, le tétrahydrofuranne ou l'acétone) en présence d'une base (par exemple Na₂CO₃ ou K₂CO₃ en présence de triéthylamine), et de préférence en chauffant.

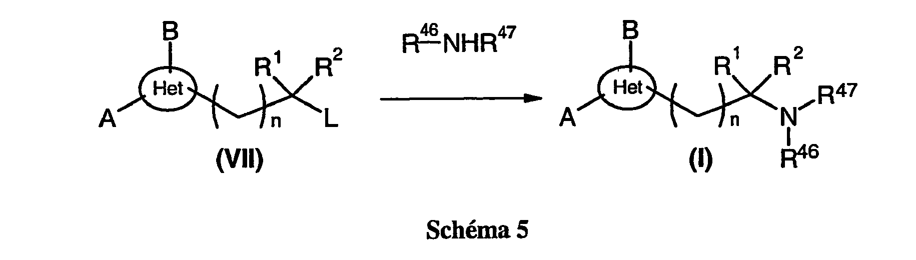

### Voie 5 : Het est imidazole et Ω est un radical de type carbamate

Lorsque Ω est un radical de type carbamate, les acides de formule générale **(VIII)** peuvent être cyclisés sous forme de dérivés d'imidazoles de formule générale **(I),** schéma 6, par ajout de carbonate de césium suivi d'une condensation avec une α-halogénocétone de formule A-CO-CH(B)-[Br, Cl] suivie de l'addition d'un large excès d'acétate d'ammonium (par exemple 15 ou 20 équivalents par équivalent d'acide de formule générale **(VIII)).** Cette réaction s'effectue de préférence dans un mélange de xylènes et en chauffant (on peut aussi, le cas échéant, éliminer simultanément l'eau formée au cours de la réaction).

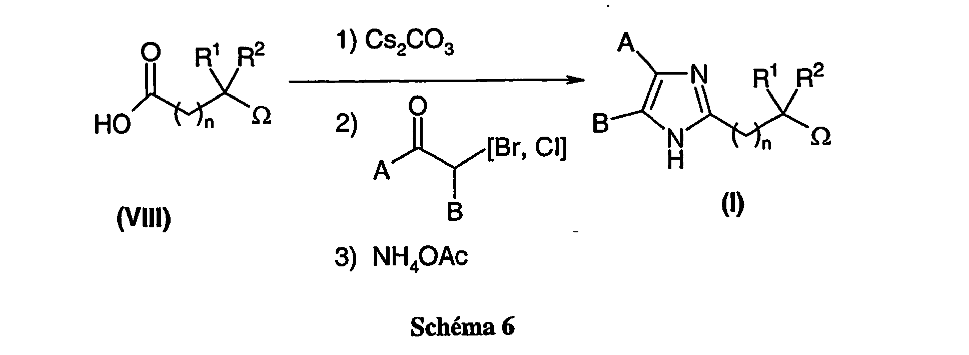

### Voie 6 : Het est imidazole, oxazole ou thiazole et Ω est NR⁴⁶R⁴⁷

Lorsque Ω est un radical -NR⁴⁶R⁴⁷ dans lequel R⁴⁷ est un radical comprenant une terminaison du type aminophénylène, alkylaminophénylène ou dialkylaminophénylène, les composés de formule générale **(I),** dans laquelle A, B, Het, n, R¹, R² et R⁴⁶ sont tels que définis ci-dessus et A représente une liaison ou un radical -(CH₂)ₓ-, -CO-(CH₂)ₓ-, -(CH₂)_{y}-O- ou -C(=NH)-, x et y étant des entiers de 0 à 6, peuvent être obtenus, schéma 7, par réduction du composé de formule générale **(IX),** par exemple par action d'hydrogène en présence d'un catalyseur du type palladium sur charbon dans un solvant comme par exemple le méthanol, l'éthanol, le dichlorométhane ou le tétrahydrofuranne. La réduction de la fonction nitro peut aussi être effectuée, par exemple, en chauffant le produit dans un solvant approprié tel que l'acétate d'éthyle avec un peu d'éthanol en présence de SnCl₂ (J. Heterocyclic Chem. (1987), 24, 927-930 ; Tetrahedron Letters (1984), 25 (8), 839-842) ou en présence de SnCl₂ / Zn (Synthesis (1996),9,1076-1078), à l'aide de NaBH₄-BiCl₃ (Synth. Com. (1995) 25 (23), 3799-3803) dans un solvant tel que l'éthanol, ou alors en utilisant du Ni Raney additionné d'hydrate d'hydrazine (Monatshefte für Chemie, (1995), 126, 725-732), ou encore à l'aide d'indium dans un mélange d'éthanol et de chlorure d'ammonium à reflux (Synlett (1998) 9, 1028).

Lorsque R⁴⁷ est un radical du type aminophénylène, alkylaminophénylène ou dialkylaminophénylène (Alk et Alk' sont des radicaux alkyle identiques ou différents), le composé de formule générale **(IX)** est réduit pour conduire au dérivé aniline de formule générale **(I)** et éventuellement mono- ou di-alkylé selon des réactions classiques connues de l'homme du métier. La mono-alkylation est réalisée par amination réductrice avec un aldéhyde ou par une substitution nucléophile par réaction avec un équivalent d'halogénoalkyle Alk-Hal. Une deuxième alkylation peut ensuite être réalisée le cas échéant au moyen d'un halogénoalkyle Alk'-Hal.

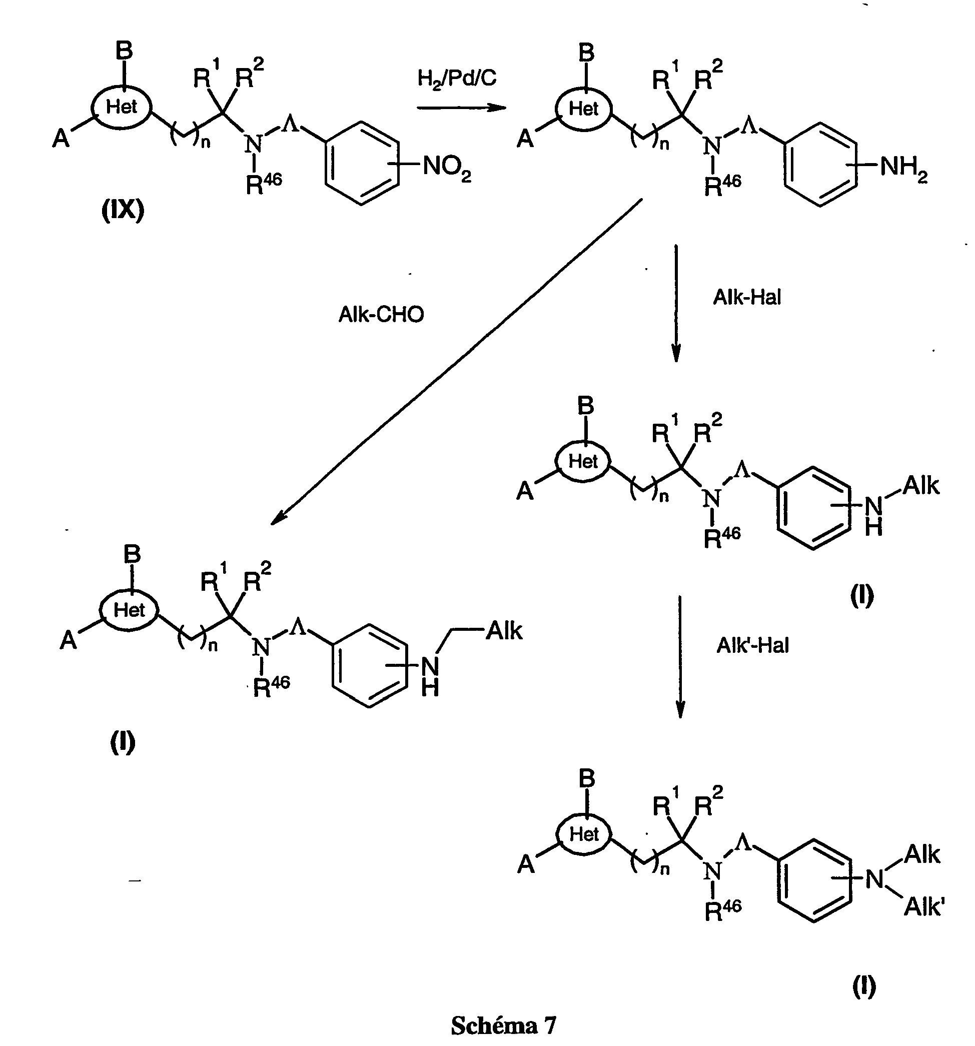

Dans le cas particulier où Alk = Alk' = -CH₃ et où A ne représente pas -CH₂-, le dérivé nitro de formule générale **(IX)** sera traité par des quantités adéquates de paraformaldéhyde sous un flux d'hydrogène dans un solvant comme l'éthanol et en présence d'un catalyseur du type palladium sur charbon (schéma 7*bis*).

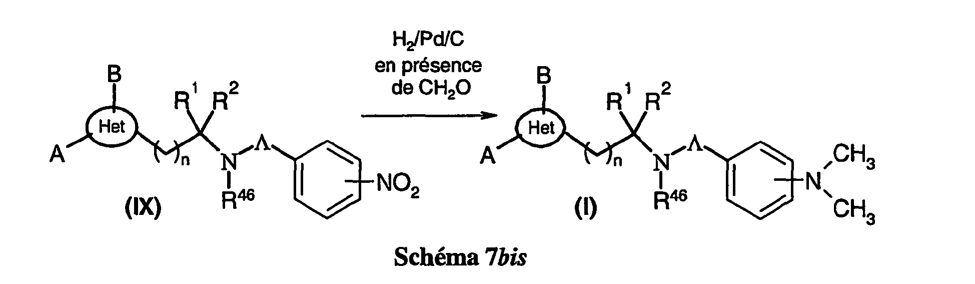

### Voie 7 : Het est imidazole, oxazole ou thiazole et Ω est OH

Cette voie peut être utilisée lorsque Ω est OH. Contrairement à la voie 3, R¹ et R² peuvent ne pas être des atomes d'hydrogène. Dans ce cas, les composés de formule générale **(I)** peuvent être obtenus, schéma 8, par déprotection de l'alcool protégé de formule générale **(X).**

Dans le cas où Gp' est un groupe protecteur de type silyle, la déprotection pourra être faite, par exemple, par addition de fluorure de tétra-*tert*-butylammonium dans un solvant comme le tétrahydrofuranne. Dans le cas où Gp' est un groupe protecteur de type benzyle, la déprotection sera faite par hydrogénation dans un solvant comme par exemple le méthanol, l'éthanol, le dichlorométhane ou le tétrahydrofuranne. Dans le cas où Gp' est un groupe protecteur de type acétate, la déprotection pourra être effectuée, par exemple, à l'aide de carbonate de sodium ou de potassium dans un solvant alcoolique comme le méthanol. Pour les autres cas, l'homme du métier consultera utilement le document suivant : Protective groups in organic synthesis, 2nd ed., (John Wiley & Sons Inc., 1991).

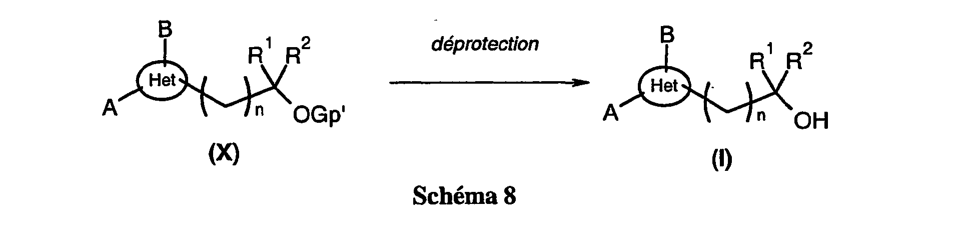

### Voie 8 : Het est imidazole, oxazole ou thiazole et Ω est OR⁴⁸ avec R⁴⁸ • H

Les composés de formule générale **(I)** dans lesquels Ω est un radical OR⁴⁸ avec R⁴⁸ • H sont obtenus, par exemple, schéma 9, à partir des alcools de formule générale **(I)*bis*** (qui sont des composés de formule générale **(I)** telle que définie précédemment dans laquelle Ω représente OH) par réaction de ces derniers avec un halogénure de formule générale R⁴⁸-Hal (Hal = Br, Cl ou I) dans un solvant comme le dichlorométhane, l'acétonitrile, le tétrahydrofuranne anhydre ou l'éther anhydre et en présence d'une base comme le carbonate de potassium ou de sodium, l'hydrure de sodium ou la triéthylamine.

Dans le cas où les radicaux A, B, R¹ et R² comportent des fonctions alcool, phénol, amine ou aniline, il peut être nécessaire d'ajouter des étapes de protection / déprotection de ces fonctions selon des méthodes classiques connues de l'homme du métier (étapes non représentées dans le schéma 9).

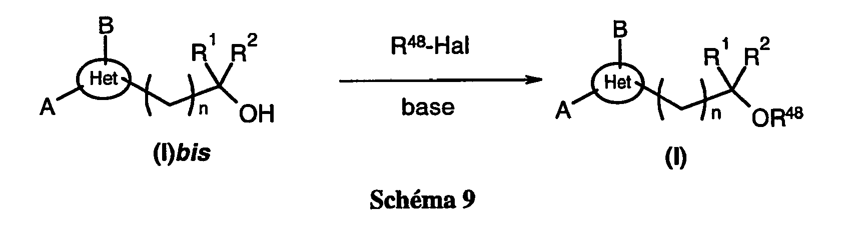

### Préparation des intermédiaires de synthèse

### Préparation des imidazoles et thiazoles de formule générale (V)

### Schéma général

Le dérivé cétonique non commercial de formule générale **(V.i)** ou **(V.i)₂** dans laquelle A et B sont tels que définis dans la formule générale **(I)** est converti, schéma 3.1, en l'α-bromo-cétone correspondante de formule générale **(V.ii)** ou **(V.ii)₂** par réaction avec un agent de bromation tel que CuBr₂ (J. Org. Chem. (1964), 29, 3459), du brome (J. Het. Chem. (1988), 25, 337), du N-bromosuccinimide (J. Amer. Chem. Soc. (1980), 102, 2838) en présence d'acide acétique dans un solvant comme l'acétate d'éthyle ou le dichlorométhane, HBr ou Br₂ dans de l'éther, de l'éthanol ou de l'acide acétique (Biorg. Med. Chem. Lett. (1996), 6(3), 253-258 ; J. Med. Chem. (1988), 31(10), 1910-1918 ; J. Am. Chem. Soc. (1999), 121, 24) ou encore à l'aide d'une résine de bromation (J. Macromol. Sci. Chem. (1977), A11, (3) 507-514). Dans le cas particulier où A est un radical p-diméthylaminophényle, il est possible d'utiliser le mode opératoire figurant dans la publication Tetrahedron Lett., 1998, 39 (28), 4987. L'amine de formule générale **(V)** est ensuite obtenue selon les procédures représentées dans les schémas 3.2 (imidazoles) et 3.3 (thiazoles) ci-après.

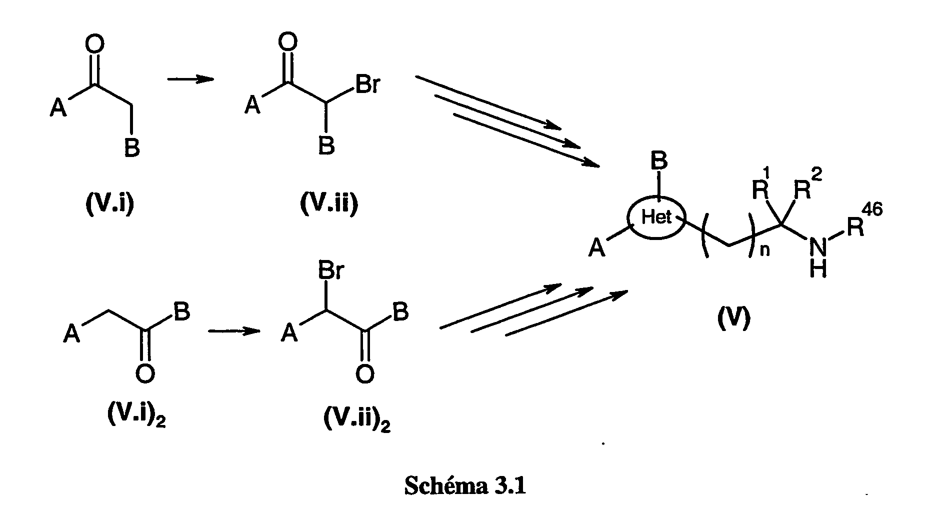

Alternativement à la synthèse présentée dans le schéma 3.1, l'homme du métier pourra, le cas échéant, utiliser une α-chloro-cétone au lieu d'une α-bromo-cétone.

### Obtention des imidazoles de formule générale (V)

L'acide de formule générale **(V.iii),** dans laquelle Gp représente un groupe protecteur pour une fonction amine, par exemple un groupe protecteur de type carbamate, est traité, schéma 3.2, avec Cs₂CO₃ dans un solvant tel que le méthanol ou l'éthanol. Au sel de césium récupéré est ajoutée l'α-halogéno-cétone de formule générale **(V.ii)** dans un solvant inerte tel que le diméthylformamide. Le cétoester intermédiaire cyclise par chauffage à reflux dans du xylène (mélange d'isomères) en présence d'un large excès d'acétate d'ammonium (15 ou 20 équivalents par exemple) pour donner le dérivé d'imidazole de formule générale **(V.iv)** (l'eau formée étant éventuellement éliminée en cours de réaction).

Dans le cas où R³⁸ n'est pas H, la fonction amine du cycle imidazole du composé de formule générale **(V.iv)** est substituée par réaction avec le dérivé halogéné R³⁸-Hal (Hal = atome halogène) ; la fonction amine protégée est ensuite déprotégée dans des conditions classiques (par exemple : acide trifluoroacétique ou HCl dans un solvant organique lorsqu'il s'agit d'un groupe protecteur de type carbamate, ou encore hydrogénation en présence de palladium sur charbon lorsque le groupe protecteur est un carbamate de benzyle).

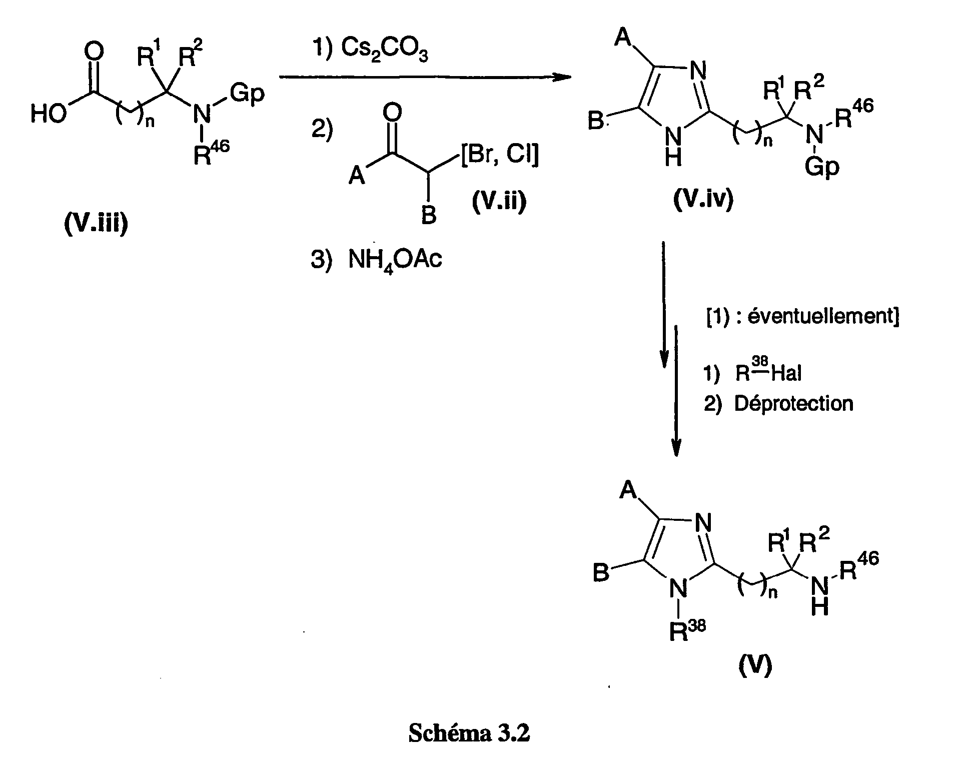

### Obtention des thiazoles de formule générale (V) destinés à la préparation de composés de formule générale (I)₁ ou (I)₂ :

Le thiocarboxamide de formule générale **(V.v),** dans laquelle Gp représente un groupe protecteur pour une fonction amine, par exemple un groupe protecteur de type carbamate, obtenu par exemple par réaction du carboxamide correspondant avec le réactif de Lawesson ou avec (P₂S₅)₂, est mis à réagir, schéma 3.3, avec l'α-bromo-cétone de formule générale **(V.ii)** ou **(V.ii)₂** selon un protocole expérimental décrit dans la littérature (J. Org. Chem., (1995), 60, 5638-5642). La fonction amine protégée est ensuite déprotégée dans des conditions classiques en milieu acide fort (par exemple : acide trifluoroacétique ou HCl dans un solvant organique lorsqu'il s'agit d'un groupe protecteur de type carbamate), libérant l'amine de formule générale (V).

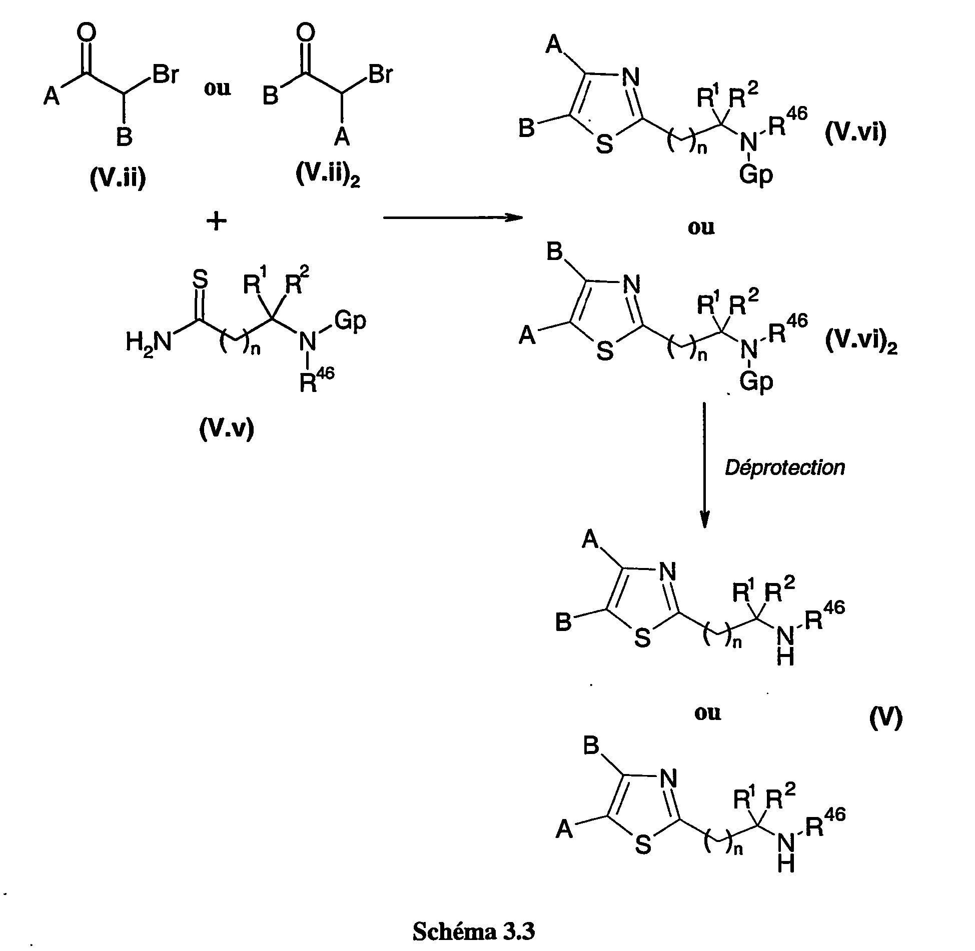

### Obtention des thiazoles de formule générale (V) destinés à la préparation de composés de formule générale (I)₃ :

Ces composés sont obtenus selon une méthode résumée dans le schéma 3.4 ci-dessous. Le carboxamide de formule générale **(VII.ii)** est d'abord traité, par exemple, par le réactif de Lawesson ou avec (P₂S₅)₂ puis le thiocarboxamide de formule générale **(VII.iii)** obtenu est mis à réagir avec le dérivé halogéné de formule générale **(V.vii)** (cf. Biorg. Med. Chem. Lett. (1996), 6(3), 253-258 ; J. Med. Chem. (1988), 31(10), 1910-1918 ; Tetrahedron Lett., (1993), 34 (28), 4481-4484 ; ou J. Med. Chem. (1974), 17, 369-371 ; ou encore Bull. Acd. Sci. USSR Div. Chem. Sci. (Engl Transl) (1980) 29, 1830-1833). L'amine protégée de formule générale **(V.viii)** ainsi obtenue est ensuite déprotégée dans des conditions classiques pour l'homme du métier (par exemple : acide trifluoroacétique ou HCl dans un solvant organique lorsque Gp est un groupe protecteur de type carbamate).

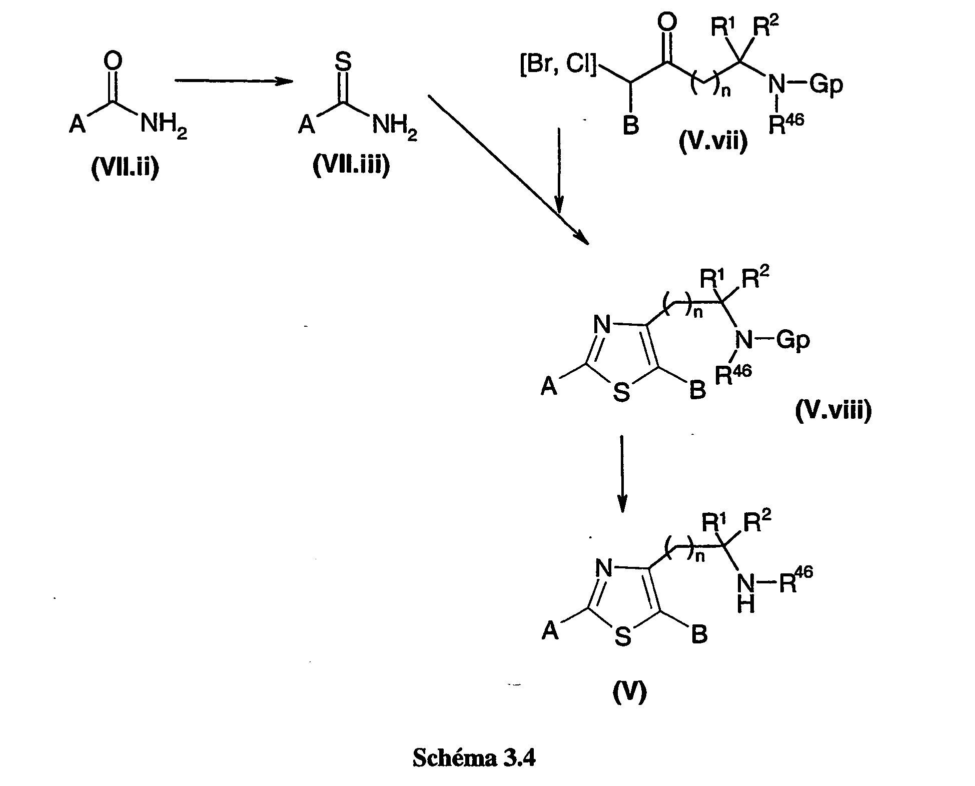

### Obtention des oxazoles de formule générale (V) destinés à la préparation de composés de formule générale (I)₃ :

Ces composés sont obtenus selon une méthode résumée dans le schéma 3.5 ci-dessous. Le carboxamide de formule générale **(VII.ii)** est mis à réagir avec le dérivé halogéné de formule générale **(V.vii).** L'amine protégée de formule générale **(V.ix)** ainsi obtenue est ensuite déprotégée dans des conditions classiques pour l'homme du métier pour donner le composé de formule générale **(V)** (par exemple : acide trifluoroacétique ou HCl dans un solvant organique lorsque Gp est un groupe protecteur de type carbamate).

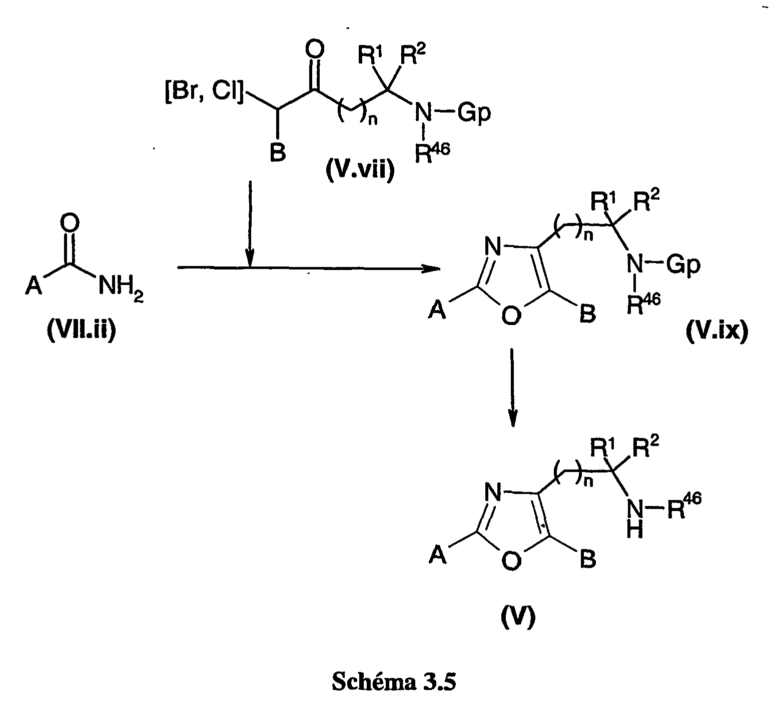

### Préparation des dérivés cétoniques de formule générale (V.i) et de certains dérivés α-bromocétoniques de formule générale (V.ii), (v.ii)₂ ou (V.vii)

Les dérivés cétoniques de formule générale (V.i) non commerciaux ou leurs homologues α-bromocétoniques sont accessibles à partir de méthodes de la littérature ou de méthodes similaires que l'homme du métier aura adaptées. En particulier :
◆ lorsque A représente un radical indolinyle ou tétrahydroquinolyle, les composés de formule générale (V.i) sont accessibles à partir de méthodes de la littérature comme, par exemple, J. Med. Chem. (1986), 29, (6), 1009-1015 ou J. Chem. Soc., Perkin Trans. 1 (1992), 24, 3401-3406.
   Alternativement, les composés de formule générale **(V.ii)** dans lesquels A représente un radical indolinyle ou tétrahydroquinolyle dans lequel R³³ représente H peuvent être synthétisés selon un protocole légèrement modifié par rapport à celui décrit dans J. Chem. Soc., Perkin Trans 1 (1992), 24, 3401-3406. Ce protocole est résumé dans le schéma 3.6 ci-après. 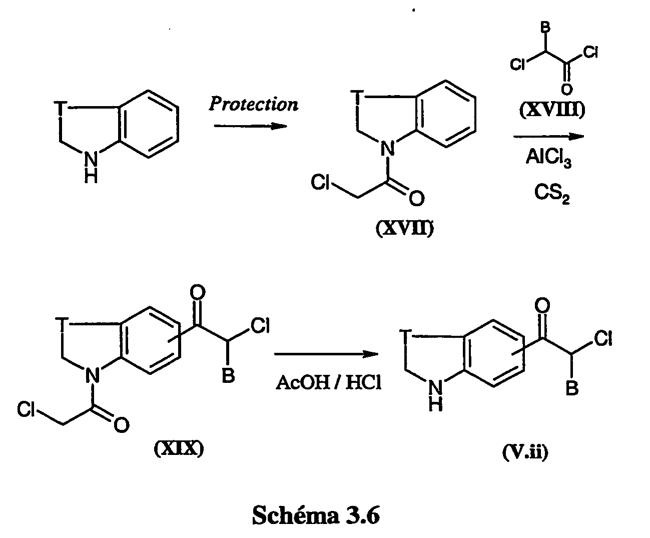
   L'indoline ou la tétrahydroquinoléine (T représente -CH₂- ou -(CH₂)₂-) est protégée en utilisant le chlorure de chloroacétyle pour donner le composé de formule générale **(XVII)** qui est soumis à une réaction de Friedel-Crafts (chlorure de chloroacétyle substitué de formule générale **(XVIII),** dans laquelle B a la signification indiquée précédemment, dans un solvant comme le disulfure de carbone et en présence de chlorure d'aluminium) pour conduire au composé de formule générale **(XIX).** Ensuite le composé de formule générale **(XIX)** est hydrolysé en présence d'acide, par exemple un mélange acide acétique/HCl, pour conduire aux composés de formule générale **(V.ii)** sous forme d'un mélange des isomères méta et para. Ces isomères peuvent être séparés par cristallisation fractionnée dans un solvant comme l'acide acétique glacial.
   L'homme du métier saura adapter les synthèses décrites précédemment aux cas où A représente un radical indolinyle ou tétrahydroquinolyle dans lequel R³³ ne représente pas H. Par exemple, lorsque R³³ représentera un radical alkyle ou aralkyle, les étapes de protection et de déprotection seront inutiles.
◆ lorsque A représente un radical du type 4-(4-hydroxyphényl)-phényle, les composés de formule générale (V.i) sont accessibles à partir de méthodes de la littérature comme par exemple J. Org. Chem., (1994), 59(16), 4482-4489.
   Alternativement, les composés de formule générale **(V.i)** et **(V.ii)** dans lesquels A représente un radical du type 4-(4-hydroxyphényl)-phényle sont accessibles par exemple par la méthode illustrée dans le schéma 3.7 ci après. 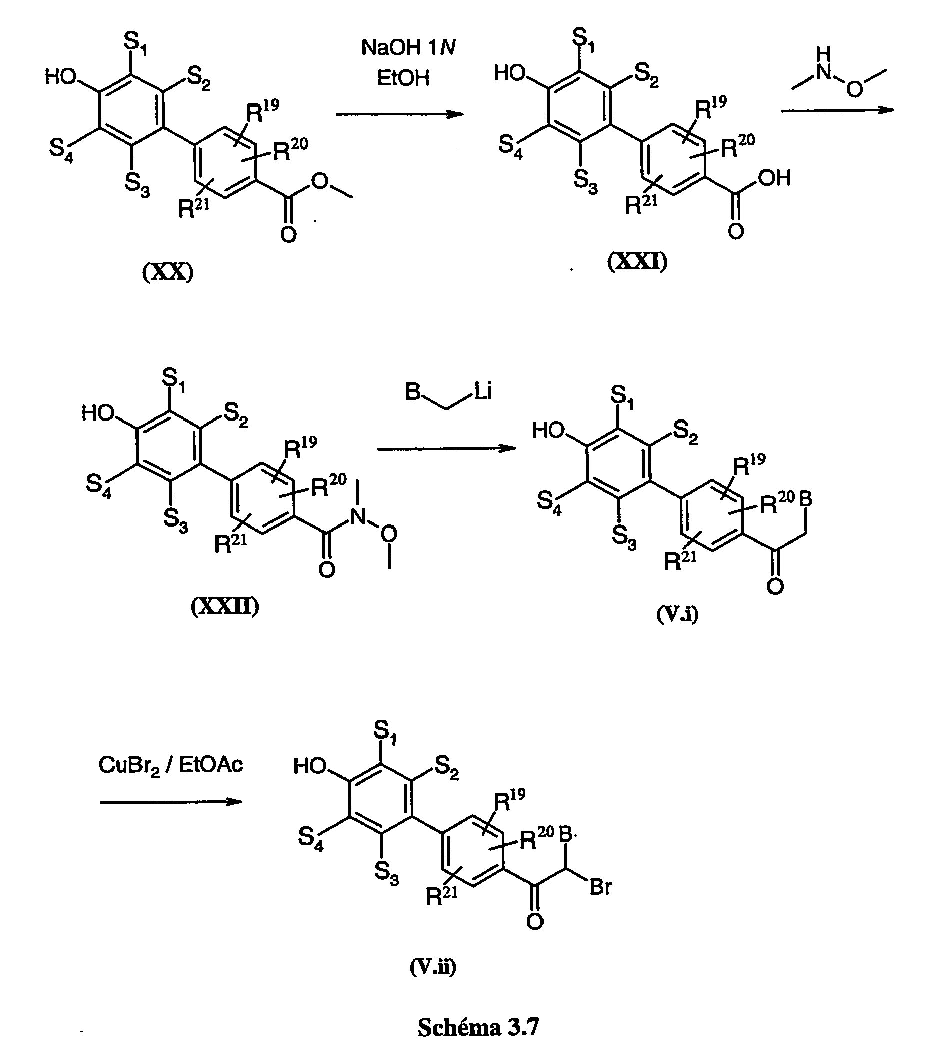
   Les composés de formule générale **(V.i)** ou **(V.ii),** dans lesquels S₁, S₂, S₃ et S₄ sont choisis indépendamment parmi un atome d'hydrogène et OH, cyano, nitro, alkyle, alkoxy ou -NR¹⁰R¹¹ tel que défini dans la formule générale **(I),** sont préparés, schéma 3.7, à partir des esters de formule générale **(XX)** (cf. notamment Chem. Lett. (1998), 9, 931-932 et Synthesis (1993), 8, 788-790). Bien entendu, les fonctions phénol ou aniline résultant de la nature des substituants R¹⁹, R²⁰, R²¹, S₁, S₂, S₃ et S₄ peuvent conduire l'homme du métier à ajouter aux étapes représentées dans le schéma 3.7 des étapes de protection (et, ultérieurement dans la synthèse des composés de formule générale **(I),** de déprotection) de ces fonctions afin qu'elles n'interfèrent pas avec le reste de la synthèse chimique. Les esters de formule générale **(XX)** sont hydrolysés pour donner les acides de formule générale **(XXI).** Ces derniers sont ensuite soumis à un couplage avec la N,O-diméthylhydroxylamine (Syn. Commun. (1995), 25(8), 1255 ; Tetrahedron Lett. (1999), 40(3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXII).** Les composés de formule générale (V.i) sont préparés à partir des composés de formule générale **(XXII)** par une réaction de substitution avec MeLi (J. Med. Chem. (1992), 35(13), 2392). Les bromoacétophénones de formule générale **(V.ii)** sont maintenant accessibles à partir de l'acétophénone de formule générale **(V.i)** dans des conditions précédemment décrites.
◆ lorsque A représente un radical carbazolyle, les composés de formule générale **(V.i)** sont accessibles à partir de méthodes de la littérature comme par exemple J. Org. Chem., (1951), 16, 1198 ou Tetrahedron (1980), 36, 3017.
   Alternativement, les composés de formule générale **(V.ii)** dans lesquels A représente un radical carbazolyle dans lequel R⁹ représente H peuvent être synthétisés selon un protocole légèrement modifié par rapport à celui décrit pour A = carbazolyle dans Tetrahedron (1980), 36, 3017. Cette méthode est résumée dans le schéma 3.8 ci-après : 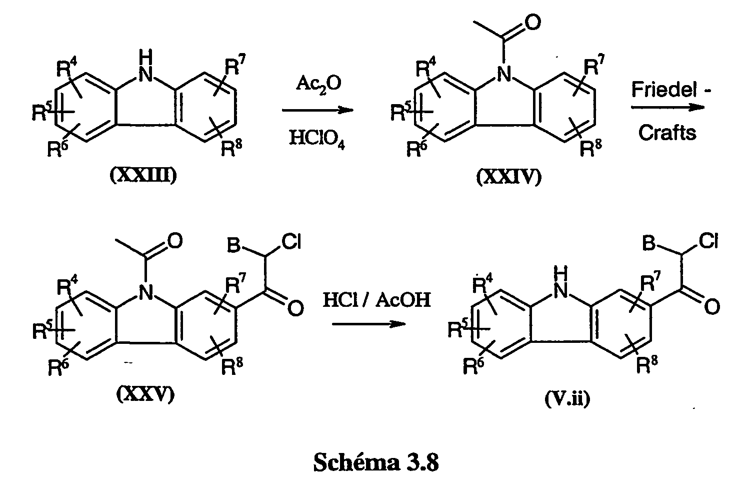
   Le carbazole de formule générale **(XXIII)** est protégé en utilisant l'anhydride acétique pour donner le composé de formule générale **(XXIV),** lequel est soumis à une réaction de Friedel-Crafts (chlorure de chloroacétyle substitué de formule générale **(XVIII)** tel que défini précédemment dans un solvant comme le disulfure de carbone et en présence de chlorure d'aluminium) pour conduire au composé de formule générale **(XXV).** Ensuite le groupe acyle protégeant la fonction amine est hydrolysé en présence d'acide, par exemple un mélange AcOH/HCl, pour conduire au composé de formule générale **(V.ii).** Lorsque A représente un radical carbazolyle dans lequel R⁹ représente alkyle ou un groupe -COR¹⁵ (cas non représenté dans le schéma 3.8), l'étape d'acylation initiale est inutile et les deux dernières étapes du schéma 3.8 permettent d'obtenir les composés de formule générale **(V.ii).** Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.8 des étapes de protection (et, ultérieurement dans la synthèse des composés de formule générale **(I),** de déprotection) de ces fonctions afin qu'elles n'interfèrent pas avec le reste de la synthèse chimique.
◆ lorsque A représente un radical phénothiazinyle, les intermédiaires de formule générale **(V.i)** et **(V.ii)** sont accessibles à partir de méthodes de la littérature : J. Heterocyclic. Chem. (1978), 15, 175-176 et Arzneimittel Forschung (1962), 12, 48.
   Alternativement, les intermédiaires de formule générale **(V.ii)** dans lesquels A représente un radical phénothiazinyle peuvent être préparés selon un protocole légèrement modifié par rapport à celui décrit pour le radical phénothiazinyle dans Arzneimittel Forschung (1962), 12, 48, lequel est résumé dans le schéma 3.9 ci-après (voir aussi les exemples). La phénothiazine de formule générale **(XXVI)** est protégée en utilisant le chlorure de chloroacétyle pour donner le composé de formule générale **(XXVII),** lequel est ensuite soumis à une réaction de Friedel-Crafts (composé de formule générale **(XVIII)** dans un solvant comme le disulfure de carbone en présence de chlorure d'aluminium) pour conduire au composé de formule générale **(XXVIII).** Lors de la dernière étape du procédé, l'hydrolyse avec HCl/acide acétique est accompagnée d'un échange d'halogène et permet d'obtenir la chlorocétone de formule générale **(V.ii).** Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.9 des étapes de protection (et, ultérieurement dans la synthèse des composés de formule générale **(I),** de déprotection) de ces fonctions afin qu'elles n'interfèrent pas avec le reste de la synthèse chimique. 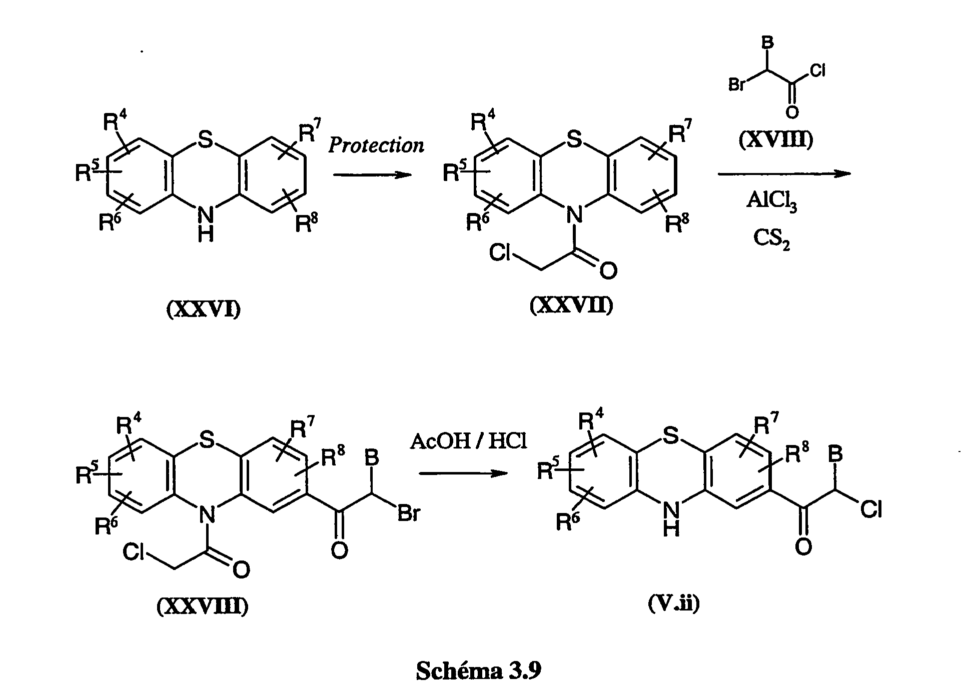
◆ lorsque A représente un radical phénylaminophényle, les composés de formule générale **(V.i)** sont accessibles à partir de méthodes de la littérature comme par exemple Chem. Commun., (1998), 15, (6) 1509-1510 ou Chem Ber., (1986), 119, 3165-3197, ou de méthodes similaires que l'homme du métier aura adaptées.
   Par exemple, les intermédiaires de formule générale **(V.i)*bis*** et **(V.ii)*bis*** dans lesquels A représente un radical phénylaminophényle (qui correspondent aux composés de formule générale **(V.i)** et **(V.ii)** correspondants dont la fonction aniline a été acétylée), peuvent être préparés selon un protocole légèrement modifié par rapport à celui décrit pour le radical phénylaminophényle dans Chem Ber. (1986), 119, 3165-3197. Ce protocole est résumé dans le schéma 3.10 ci-après. 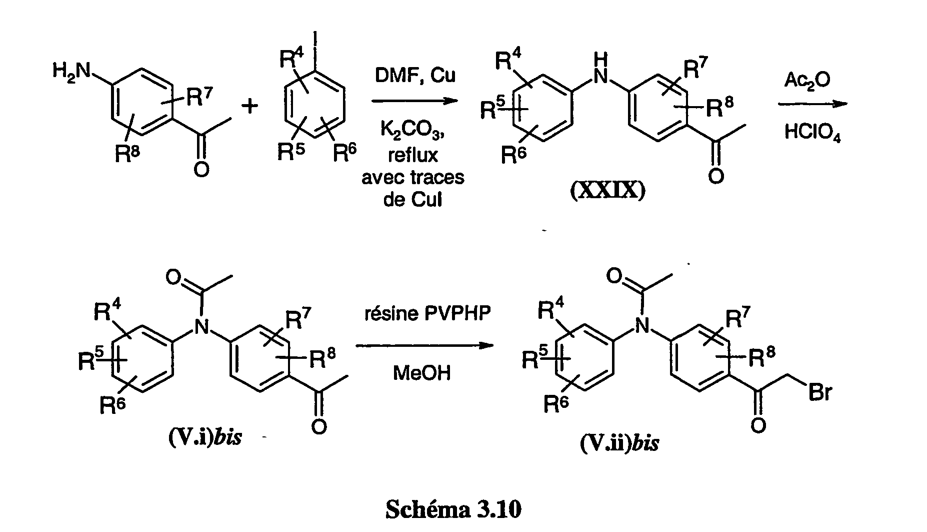
   Dans le cas (représenté sur le schéma 3.10) où le radical R⁹ du composé de formule générale **(I)** à synthétiser est un atome d'hydrogène ou un groupe acétyle, la diphénylamine de formule générale **(XXIX)** formée après la réaction de couplage en présence de CuI est protégée par acétylation en utilisant, par exemple, l'anhydride acétique pour donner le composé de formule générale **(V.i)*bis*.** Dans le cas (non représenté sur le schéma 3.10) où le radical R⁹ du composé de formule générale **(I)** à synthétiser n'est pas un atome d'hydrogène ou un radical acétyle, l'étape d'acétylation est remplacée par une étape de substitution sur l'aniline selon des méthodes classiques connues de l'homme du métier pour donner le composé de formule générale (V.i) correspondant. Le composé de formule générale **(V.i)*bis*** (ou **(V.i),** dans le cas non représenté dans le schéma 3.10) est ensuite soumis à une réaction de bromation à l'aide d'une résine de bromation, la résine PVPHP (*Poly(VinylPyridinium Hydrobromide Perbromide)* ou poly(perbromure d'hydrobromure de vinylpyridinium)), décrite dans J. Macromol. Sci. Chem. (1977), A11, (3), 507-514, pour conduire au composé de formule générale **(V.ii)*bis*** (ou **(V.ii),** dans le cas non représenté dans le schéma 3.10). Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.10 des étapes de protection (et, ultérieurement dans la synthèse des composés de formule générale **(I),** de déprotection) de ces fonctions afin qu'elles n'interfèrent pas avec le reste de la synthèse chimique. La déprotection de la fonction aniline acétylée sera effectuée en principe lors de la dernière étape de la synthèse des composés de formule générale **(I).**
◆ lorsque A représente un radical benzopyrane ou benzofuranne tel que défini dans la formule générale **(I)** avec R³² représentant un atome d'hydrogène, les intermédiaires de formule générale **(V.i)** et **(V.ii)** sont accessibles par les méthodes illustrées dans le schéma 3.11 ci après. 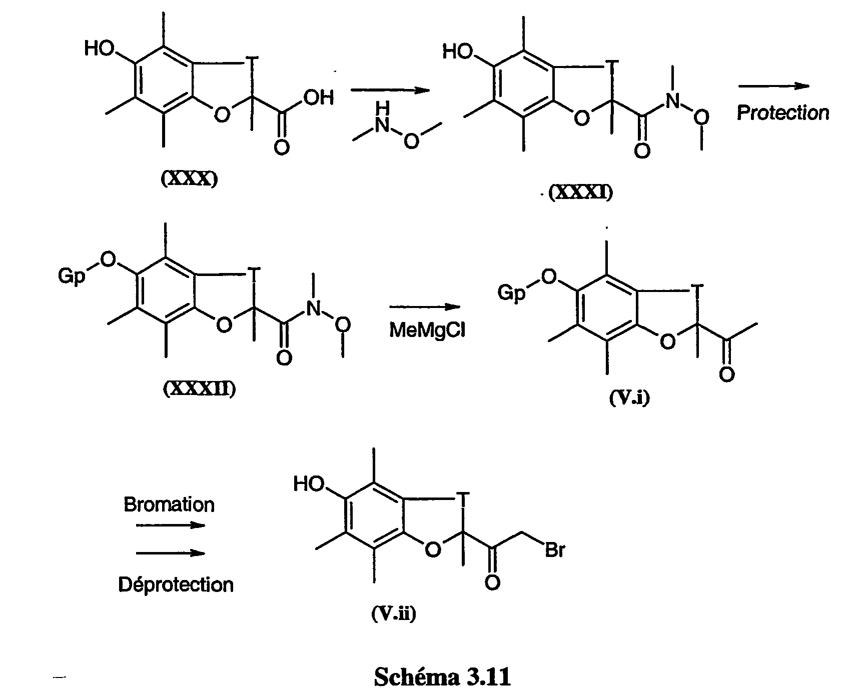
   Les composés de formules générales **(V.i)** et **(V.ii),** schéma 3.11, dans laquelle T est tel que défini ci-dessus et Gp = groupe protecteur, sont préparés à partir des acides de formule générale **(XXX).** Les acides de formule générale **(XXX)** sont soumis à un couplage avec la N,O-diméthylhydroxylamine (Syn. Commun. (1995), 25, (8), 1255 ; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXI).** La protection de la fonction phénol sous forme de dérivé benzylé ou *tert*-butyldiméthylsilylé ou par d'autres groupes protecteurs (Gp) connus de l'homme du métier est alors effectuée pour conduire aux composés de formule générale **(XXXII).** Les composés de formule générale (V.i) sont préparés à partir des composés de formule générale **(XXXII)** par une réaction de substitution avec un réactif de Grignard, MeMgCl (J. Het. Chem. (1990), 27, 1709-1712) ou avec MeLi (J. Med. Chem. (1992), 35, 13). Les bromoacétophénones de formule générale **(V.ii)** sont maintenant accessibles à partir de l'acétophénone de formule générale **(V.i)** dans des conditions précédemment décrites.
   Alternativement, le composé de formule générale **(V.ii)** dans lequel R³² représente un atome d'hydrogène ou un radical alkyle peut être préparé selon un procédé en 3 étapes seulement (cf. schéma 3.12 - voir aussi les exemples). Dans ce procédé, la bromation dans la dernière étape du composé de formule générale **(V.i)** pour donner le composé de formule générale **(V.ii)** sera de préférence effectuée selon J. Am. Chem. Soc. (1999), 121, 24. 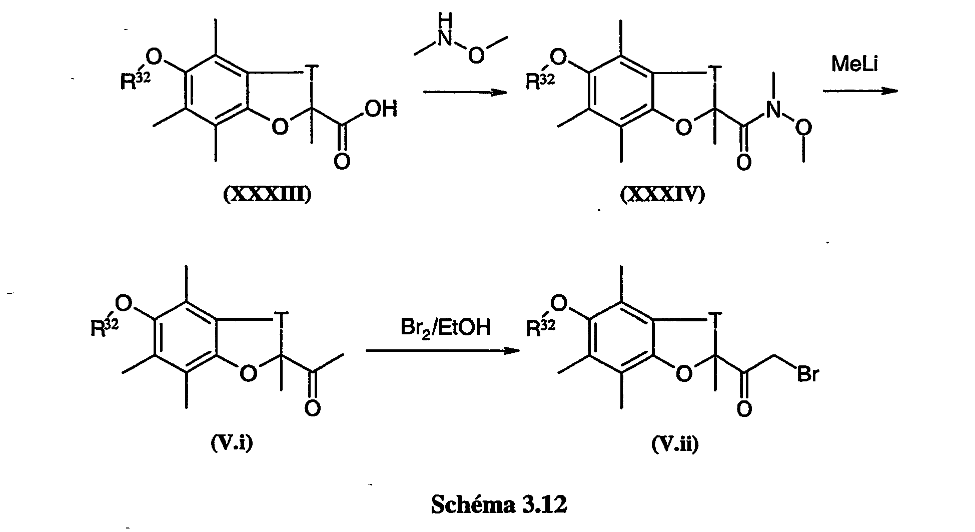
◆ lorsque A représente un radical biphényle substitué, les cétones intermédiaires de formule générale **(V.i)** sont notamment accessibles à l'aide d'une synthèse de Suzuki (cf. Baroni et coll., J.Org. Chem. 1997, 62, 7170-7173 ; cf. aussi l'exemple 44 de la présente demande, étape 44.1).
   Lorsque A représente un radical phénol substitué, il peut être nécessaire de d'utiliser des intermédiaires de formule générale **(V.ii)** telle que définie précédemment dont la fonction phénol a été acétylée (ci-après désignés comme composés de formule générale **(V.ii)*ter*).** En particulier :
◆ lorsque A représente un radical 4-hydroxy-3,5-diisopropylphényle, les dérivés α-bromocétoniques homologues du composé de formule **(V.ii)** dont la fonction phénol est protégée par un radical acétyle peuvent être préparés comme résumé dans le schéma 3.13 ci-après. 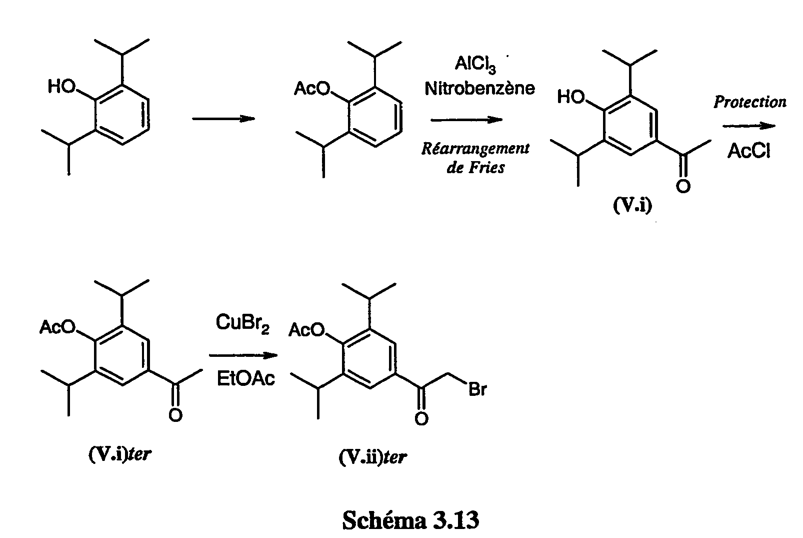
   Le 2,6-diisopropylphénol est acétylé selon des méthodes connues de l'homme du métier, par exemple en le faisant réagir avec de l'acide acétique en présence d'anhydride d'acide trifluoroacétique ou avec du chlorure d'acétyle en présence d'une base comme par exemple K₂CO₃. L'homologue acétylé du 2,6-diisopropylphénol est alors soumis à un réarrangement de Fries en présence de chlorure d'aluminium dans un solvant comme le nitrobenzène pour conduire au composé de formule **(V.i).** Ensuite le composé de formule **(V.i)** est acétylé pour conduire au composé de formule **(V.i)*ter*.** Une bromation est alors effectuée avec CuBr₂ comme précédemment décrit pour conduire au composé de formule **(V.ii)*ter*.** L'étape de déprotection pour libérer la fonction phénol interviendra ultérieurement dans la synthèse des composés de formule générale **(I)** (au moment jugé le plus adapté par l'homme du métier).
◆ lorsque A représente un radical de type diméthoxyphénol, les composés de formule générale **(V.ii)*ter*** peuvent être préparés de façon analogue à la synthèse décrite pour le composé de formule **(V.ii)*ter*** dérivé du 2,6-diisopropylphénol, avec éventuellement quelques modifications mineures à la portée de l'homme du métier. Par exemple, lorsque A représente le radical 3,5-diméthoxy-4-hydroxyphényle, le dérivé α-bromocétonique de formule **(V.ii)*ter*** correspondant peut être préparé, par exemple, comme indiqué dans le schéma 3.13 à partir du composé commercial de formule **(XXXV) :** 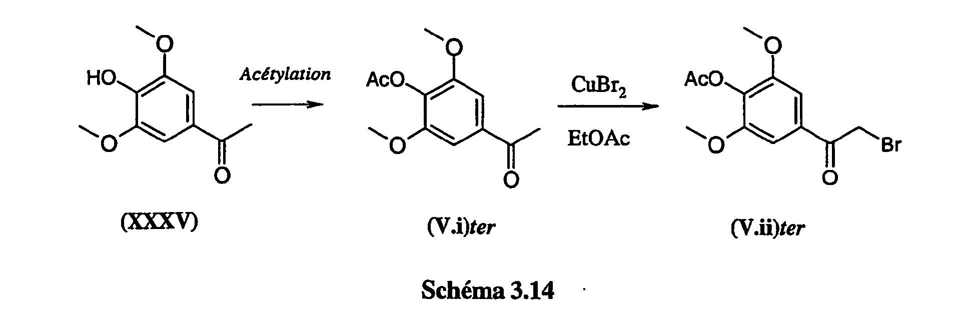
   Les composés de formule générale **(V.ii)₂** dans laquelle A et B sont tels que définis précédemment peuvent être préparés selon la méthode résumée dans le schéma 3.15 ci-après. 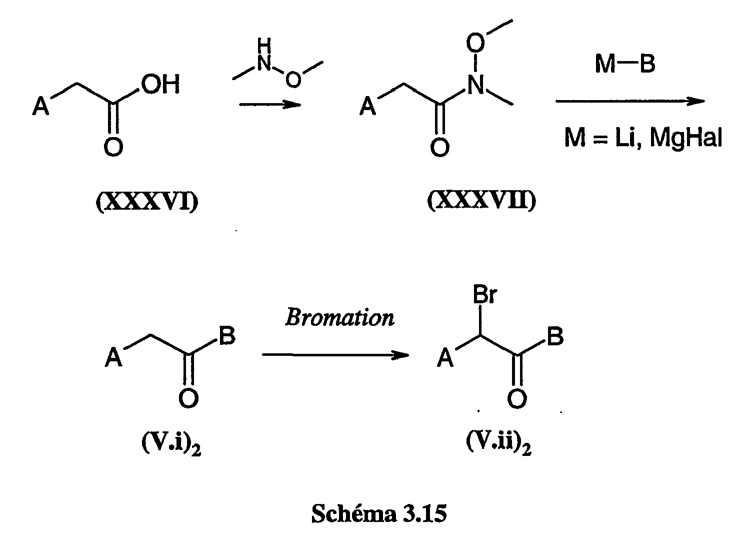
   Les acides de formule générale **(XXXVI)** sont soumis à un couplage avec la N,O-diméthylhydroxylamine (Syn. Commun. (1995), 25, (8), 1255 ; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXVII).** Les composés de formule générale **(V.i)₂** sont préparés à partir des composés de formule générale **(XXXVII)** par une réaction de substitution avec des dérivés lithiens ou magnésiens de formule générale B-M dans laquelle M représente Li ou MgHal (Hal = I, Br ou Cl) dans des solvants comme l'éther ou le tétrahydrofuranne anhydre. Les α-bromo- ou α-chlorocétones de formule générale **(V.ii)₂** sont maintenant accessibles à partir des cétones de formule générale **(V.i)₂** dans des conditions précédemment décrites.

Par ailleurs, les dérivés α-halogénocétoniques de formule générale **(V.vii)** non commerciaux sont accessibles à partir de méthodes de la littérature. En particulier, ils peuvent être obtenus selon une procédure résumée dans le schéma 3.16.

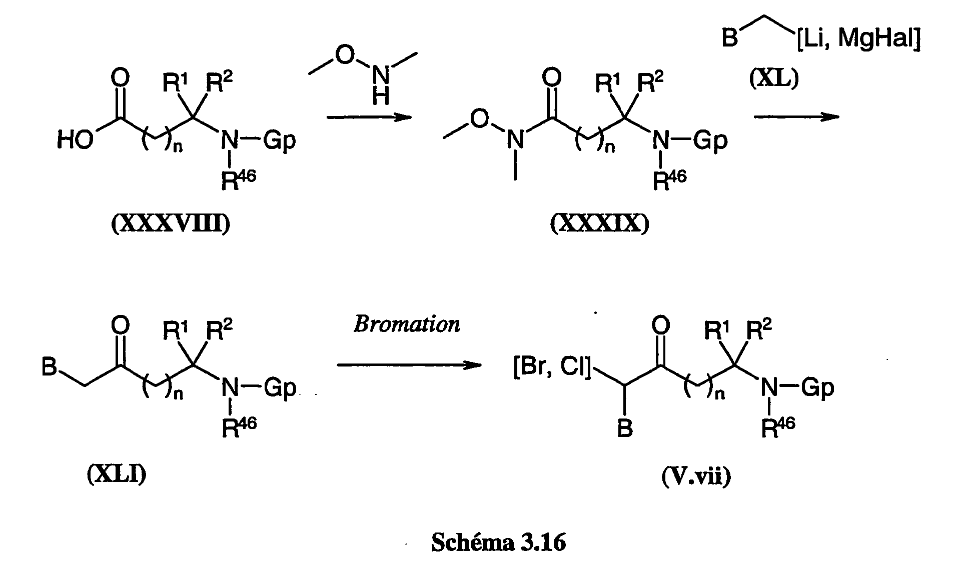

Les aminoacides protégés de formule générale **(XXXVIII)** sont obtenus par la protection des aminoacides correspondants par un groupe de type carbamate selon des méthodes connues de l'homme du métier. Les acides de formule générale **(XXXVIII)** sont ensuite soumis à un couplage avec la N,O-diméthylhydroxylamine (Syn. Commun. (1995), 25, (8), 1255 ; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXIX).** Les composés de formule générale **(XLI)** sont préparés à partir des composés de formule générale **(XXXIX)** par une réaction de substitution avec des dérivés lithiens ou magnésiens de formule générale **(XL)** (dans laquelle Hal = I, Br ou Cl) dans des solvants comme l'éther ou le tétrahydrofuranne anhydre. Les bromo ou chloroacétophénones de formule générale **(V.vii)** sont maintenant accessibles à partir de l'acétophénone de formule générale **(XLI)** dans des conditions précédemment décrites.

Alternativement, l'homme du métier pourra aussi utiliser ou adapter les synthèses décrites dans Angew. Chem. Int. (1998), 37 (10), 411-414, Liebigs Ann. Chem. (1995), 1217 ou Chemin. Pharm. Bull. (1981), 29(11), 3249-3255.

### Préparation des dérivés acides de formule générale (V.iii)

Les dérivés acides de formule générale **(V.iii)** peuvent être obtenus, schéma 3.17, directement par réaction de l'aminoacide commercial de formule générale **(V.vi)** avec des composés de type (ar)alkylchloroformates ou di(ar)alkylcarbonates (Δ représente un radical alkyle ou benzyle) dans des conditions classiques connues de l'homme du métier.

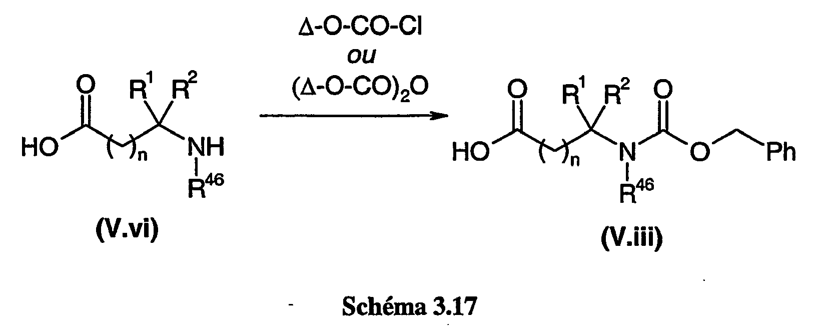

### Préparation des composés de formule générale (V.v)

Les thiocarboxamides de formule générale **(V.v)** peuvent être obtenus en trois étapes à partir des composés de formule générale **(V.vi)** comme indiqué dans le schéma 3.18 ci-dessous. La fonction amine de l'aminoacide de formule générale **(V.vi)** est d'abord protégée dans des conditions classiques avec tBu-O-CO-Cl ou (tBu-O-CO)₂O (ou d'autres groupes protecteurs connus de l'homme du métier), puis l'intermédiaire obtenu est converti en son amide correspondant par des méthodes décrites dans la littérature (cf. par exemple, J. Chem. Soc., Perkin Trans. 1, (1998), 20 , 3479-3484 ou la demande de brevet PCT WO 99/09829). Enfin, le carboxamide est converti en thiocarboxamide de formule générale **(V.v),** par exemple par réaction avec le réactif de Lawesson dans un solvant comme le dioxane ou le tétrahydrofuranne à une température de préférence comprise entre la température ambiante et celle du reflux du mélange, ou encore à l'aide de (P₂S₅)₂ dans des conditions classiques pour l'homme du métier.

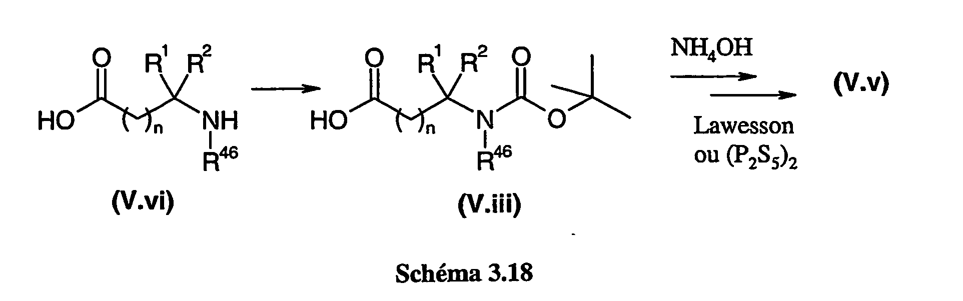

Alternativement, les thiocarboxamides de formule générale (V.v) peuvent également être obtenus, schéma 3.19, par addition de H₂S sur les dérivés cyano de formule générale **(V.x)** correspondants dans des conditions classiques connues de l'homme du métier.

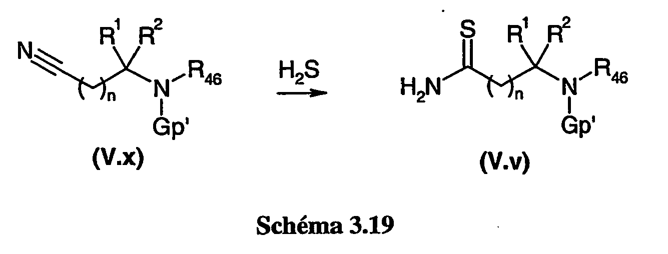

### Préparation des acides de formule générale (VI)

### Préparation des acides dérivés de thiazoles de formule générale (VI)

Les acides de formule générale **(VI)** dérivés de thiazoles peuvent être préparés selon des procédures représentées dans le schéma 4.1 ci-dessous.

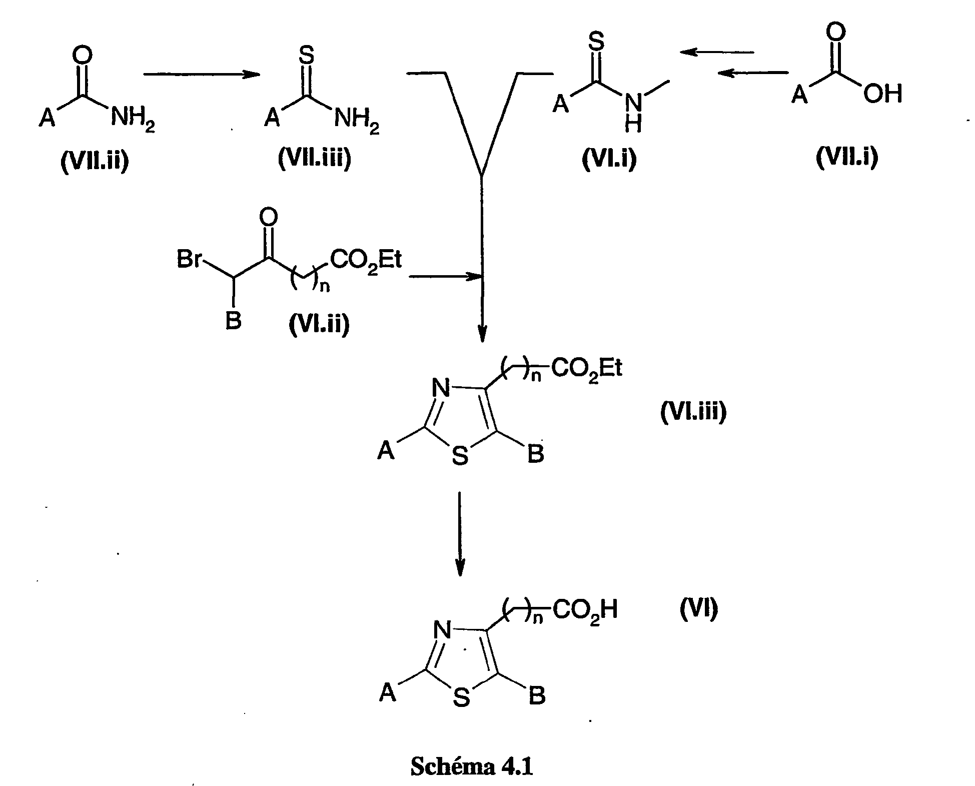

Les carboxamides de formule générale **(VII.ii)** sont traités dans des conditions classiques pour donner le thiocarboxamide de formule générale **(VII.iii),** par exemple par le réactif de Lawesson ou encore à l'aide de (P₂S₅)₂ dans des conditions classiques pour l'homme du métier. Alternativement, l'acide de formule générale **(VII.i)** est activé par action de 1,1'-earbonyldiimidazole puis traité par de la méthylamine dans un solvant polaire aprotique comme par exemple le tétrahydrofuranne. Le carboxamide intermédiaire obtenu est converti en le thiocarboxamide de formule générale **(VI.i)** dans des conditions classiques, par exemple à l'aide du réactif de Lawesson ou encore à l'aide de (P₂S₅)₂ dans des conditions classiques pour l'homme du métier. Le thiocarboxamide de formule générale **(VII.iii)** ou **(VI.i)** est ensuite mis à réagir avec le composé de formule générale **(VI.ii),** par exemple en chauffant à reflux dans un solvant comme le benzène, le dioxane ou le diméthylformamide. L'ester de formule générale **(VI.iii)** obtenu peut ensuite être saponifié par action d'une base comme par exemple la potasse en milieu alcoolique ou LiOH dans du tétrahydrofuranne pour donner l'acide de formule générale **(VI).**

### Préparation des acides dérivés d'oxazoles de formule générale (VI)

Les acides de formule générale **(VI)** dérivés d'oxazoles peuvent être préparés selon une procédure représentée dans le schéma 4.2 ci-dessous.

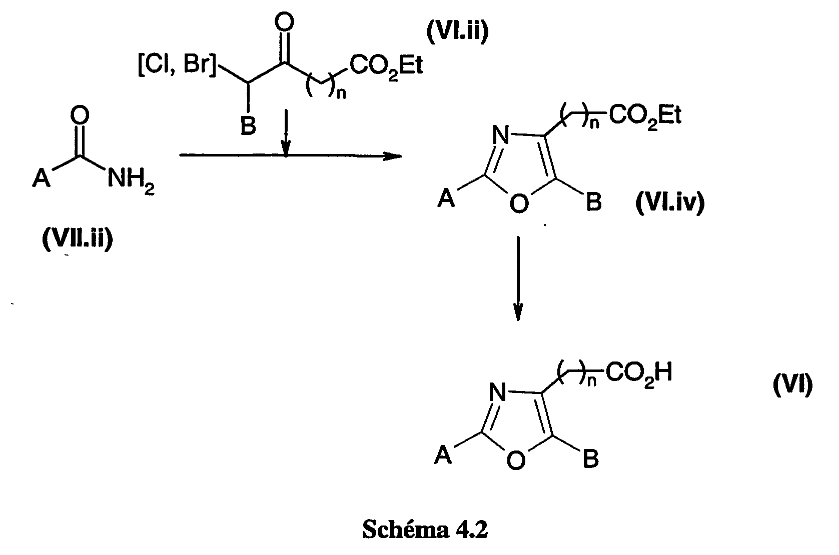

Les carboxamides de formule générale **(VII.ii)** sont mis à réagir avec le composé de formule générale **(VI.ii)** en chauffant, par exemple à reflux, en l'absence ou en la présence d'un solvant comme le diméthylformamide. L'ester de formule générale **(VI.iv)** obtenu peut ensuite être saponifié par action d'une base comme par exemple la potasse en milieu alcoolique ou LiOH dans du tétrahydrofuranne pour donner l'acide de formule générale **(VI).**

### Préparation des acides dérivés d'isoxazolines de formule générale (VI)

Les acides dérivés d'isoxazolines de formule générale **(VI),** utiles à la préparation de composés de formule générale **(I)₄,** peuvent être préparés selon une procédure représentée dans le schéma 4.3 ci-après.

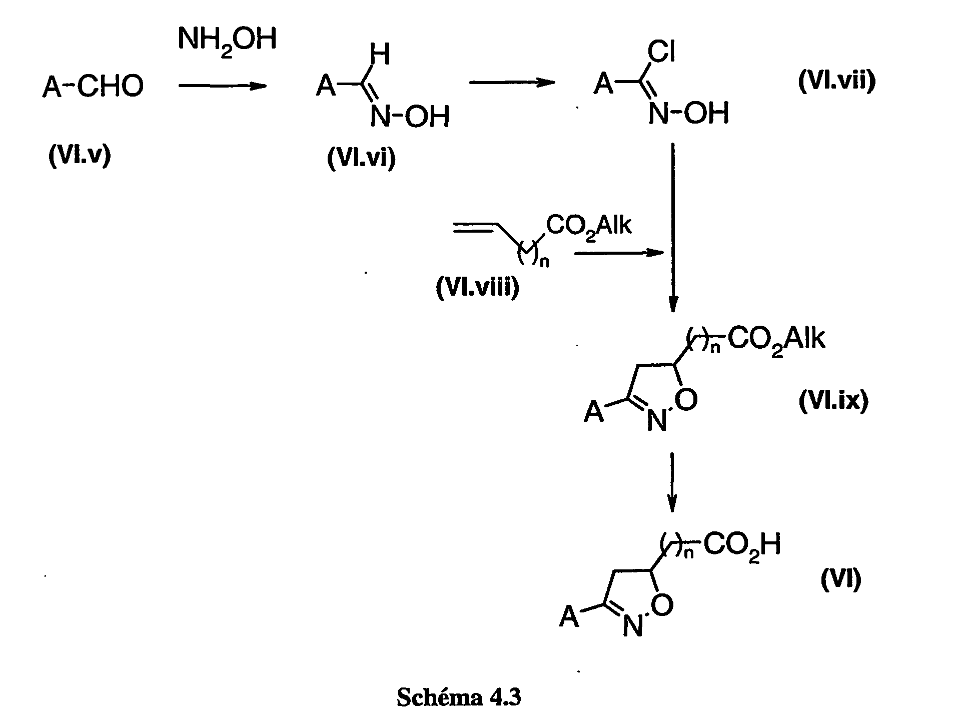

Les acides de formule générale **(VI)** dérivés d'isoxazolines peuvent être préparés comme suit : les aldéhydes commerciaux de formule générale **(VI.v)** sont mis à réagir avec le chlorhydrate d'hydroxylamine. L'oxime de formule générale **(VI.vi)** ainsi obtenu est activé sous forme de chlorure d'oxime, de formule générale **(VI.vii),** par réaction avec le N-chlorosuccinimide dans le DMF avant de réagir avec les esters de formule générale **(VI.viii)** (dans laquelle Alk représente un radical alkyle) pour conduire aux dérivés isoxazolines selon un protocole expérimental décrit dans la littérature (Tetrahedron Lett., 1996, 37 (26), 4455 ; J. Med. Chem., 1997, 40, 50-60 et 2064-2084). La saponification des isoxazolines de formule générale **(VI.ix)** est ensuite effectuée classiquement (par exemple par action de KOH dans un solvant alcoolique ou LiOH dans un solvant comme le tétrahydrofuranne) pour donner le dérivé acide de formule générale **(VI).**

Les esters insaturés non commerciaux de formule générale **(VI.x)** peuvent être préparés selon des méthodes décrites dans la littérature (J. Med. Chem., 1987, 30, 193 ; J. Org. Chem., 1980, 45, 5017).

### Préparation des thiazoles et oxazoles de formule générale (VII)

### Schéma général

Les acides de formule générale **(VII.i),** schéma 5.1, sont convertis en les carboxamides correspondants de formule générale **(VII.ii)** par des méthodes décrites dans la littérature (cf. par exemple, J. Chem. Soc., Perkin Trans. 1, (1998), 20 , 3479-3484 ou la demande de brevet PCT WO 99/09829). Les composés de formule générale **(VII)** peuvent ensuite obtenus classiquement selon les procédures représentées dans les schémas 5.2 et 5.3 (thiazoles) et le schéma 5.4 (oxazoles) ci-après.

Cette voie de synthèse est utile pour préparer ensuite des composés répondant aux sous-formules générales **(I)₁** et **(I)₃.**

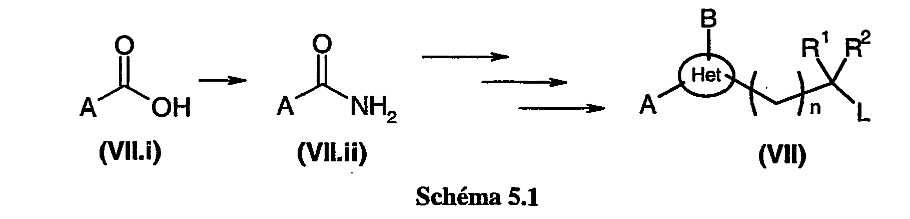

### Obtention des thiazoles de formule générale (VII)

Lorsque R¹ et R² représentent tous deux H, les thiazoles de formule générale **(VII)** destinés à la préparation de composés de formule générale **(I)₃** peuvent être préparés selon la méthode résumée dans le schéma 5.2. Le carboxamide de formule générale **(VII.ii)** est converti en le thiocarboxamide correspondant de formule générale **(VII.iii)** en présence de réactif de Lawesson dans un solvant comme le dioxane ou le benzène à une température de préférence comprise entre la température ambiante et celle du reflux du mélange. Le thiocarboxamide de formule générale **(VII.iii)** est ensuite traité avec l'α-halogénocétoester de formule générale **(VII.iv)** dans laquelle Alk représente un radical alkyle (par exemple méthyle, éthyle ou *tert*-butyle), pour donner l'ester de formule générale **(VII.v),** lequel est réduit en l'alcool correspondant de formule générale **(VII.vi),** par exemple par action d'hydrure de lithium aluminium ou d'hydrure de diisobutylaluminium dans un solvant comme le tétrahydrofuranne. Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

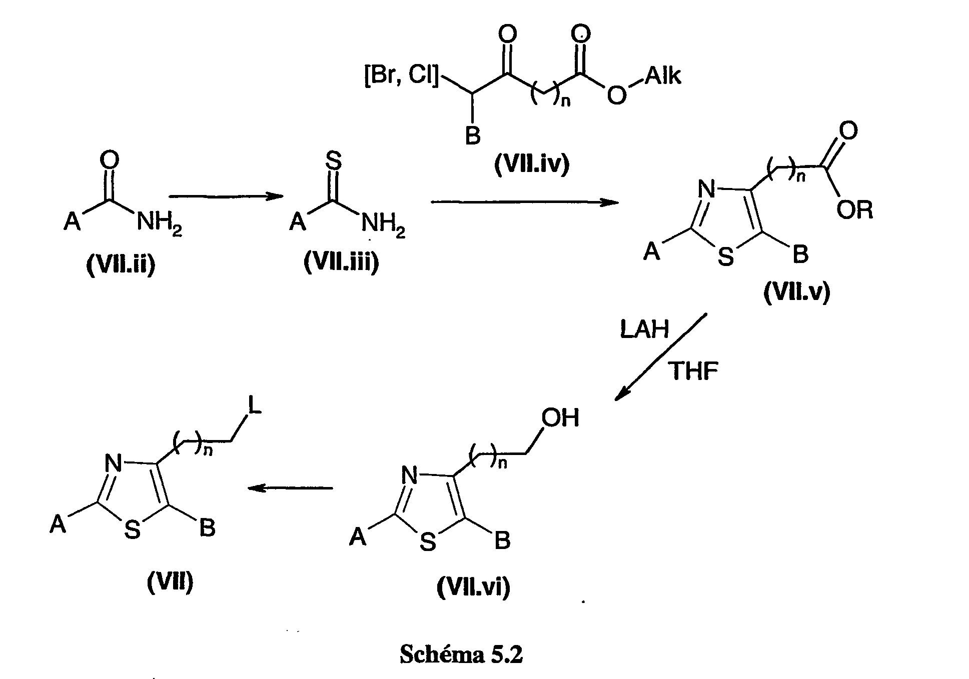

Les thiazoles de formule générale **(VII)** destinés à la préparation de composés de formule générale **(I)₁** peuvent être préparés selon la méthode résumée dans le schéma 5.3. Le dérivé cyano de formule générale **(VII.vii)** dans laquelle Gp' est un groupe protecteur pour une fonction alcool (par exemple un groupe benzyle ou -CO-p dans lequel p représente alkyle, par exemple méthyle ou *tert*-butyle) est converti en le thiocarboxamide correspondant de formule générale **(VII.viii)** par action de H₂S dans un solvant comme l'éthanol en présence de triéthanolamine à une température de préférence comprise entre la température ambiante et celle du reflux du mélange. Le thiocarboxamide de formule générale **(VII.viii)** est ensuite traité avec l'α-halogénocétone de formule générale **(VII.ix)** pour donner le composé de formule générale **(VII.x),** lequel est déprotégé pour donner l'alcool correspondant de formule générale **(VII.xi)** selon des méthodes connues de l'homme du métier (par exemple lorsque Gp' est un groupe protecteur de type acétate, celui-ci est retiré *in situ* par action d'une solution de carbonate de sodium aqueuse). Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

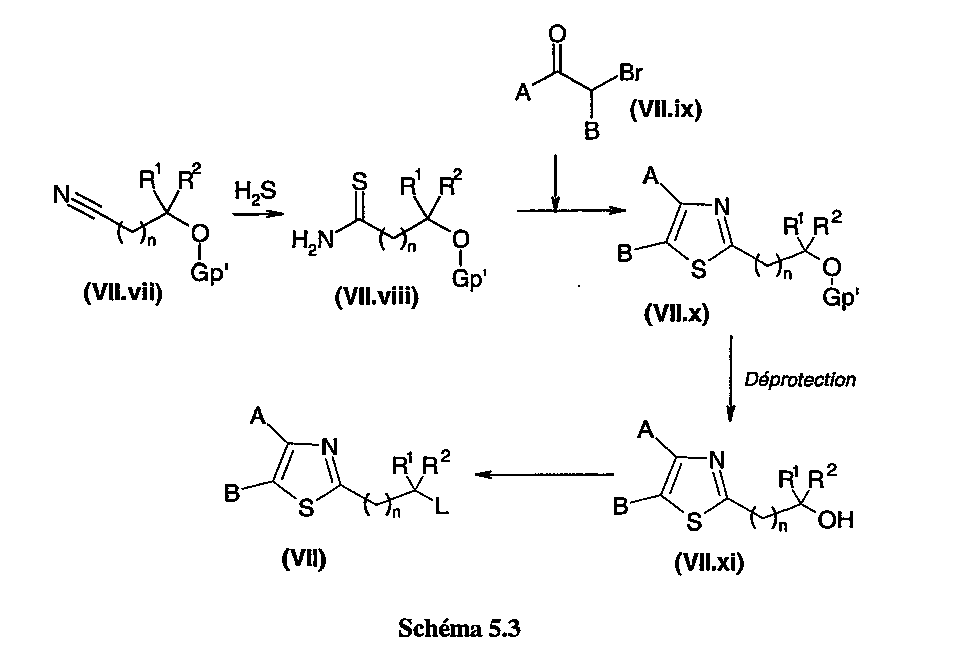

### Obtention des oxazoles de formule générale (VII)

Lorsque R¹ et R² représentent tous deux H, les oxazoles de formule générale **(VII)** destinés à la préparation de composés de formule générale **(I)₃** peuvent être préparés selon la méthode résumée dans le schéma 5.4. Le carboxamide de formule générale **(VII.ii)** est traité avec l'α-halogénocétoester de formule générale **(VII.iv)** dans laquelle Alk représente un radical alkyle (par exemple méthyle, éthyle ou *tert*-butyle), pour donner l'ester/ l'acide de formule générale **(VII.xii).** Ce dernier est réduit en l'alcool correspondant de formule générale **(VII.xiii),** par exemple par action d'hydrure de lithium et d'aluminium ou d'hydrure de diisobutylaluminium dans un solvant comme le tétrahydrofuranne lorsque l'on part de l'ester ou par action de diborane dans le tétrahydrofuranne lorsque l'on part de l'acide. Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

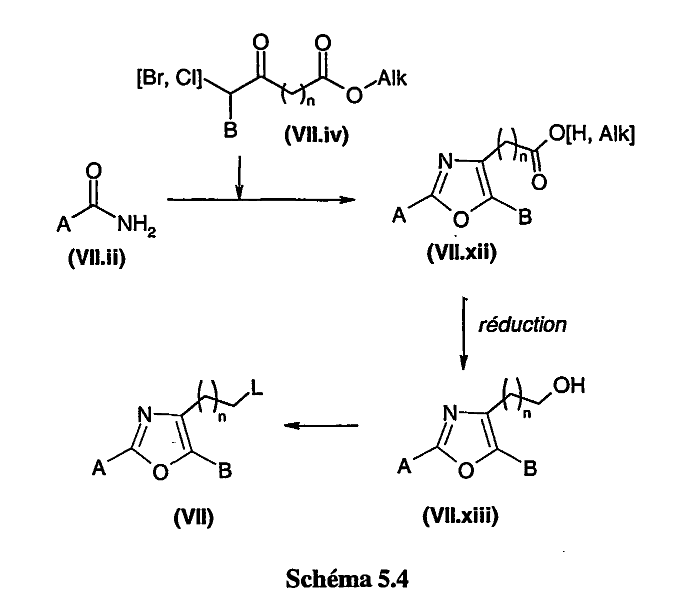

### Préparation des acides de formule générale (VII.i)

Les acides de formule générale **(VII.i)** non commerciaux sont accessibles à partir de méthodes de la littérature. En particulier :
- lorsque A représente un radical phénothiazinyle, les acides de formule générale **(VII.i)** sont accessibles à partir de méthodes de la littérature comme par exemple J. Med. Chem. (1992), 35, 716-724, J. Med. Chem. (1998), 41, 148 -156; Synthesis (1988) 215-217 ; ou J. Chem. Soc. Perkin. Trans. 1 (1998), 351-354 ;
- lorsque A représente un radical indolinyle, les acides de formule générale **(VII.i)** sont accessibles à partir de méthodes de la littérature comme par exemple J. Het. Chem. (1993), 30, 1133-1136 ou Tetrahedron (1967), 23, 3823 ;
- lorsque A représente un radical phénylaminophényle, les acides de formule générale **(VII.i)** sont accessibles à partir de méthodes de la littérature comme par exemple J. Amer. Chem Soc. (1940), 62, 3208 ; Zh. Obshch. Khim. (1953), 23, 121-122 ou J. Org. Chem. (1974), 1239-1243 ;
- lorsque A représente un radical carbazolyle, les acides de formule générale **(VII.i)** sont accessibles à partir de méthodes de la littérature comme par exemple J. Amer. Chem Soc., (1941), 63, 1553-1555 ; J. Chem. Soc. (1934), 1142-1144 ; J. Chem. Soc. (1945), 945-956 ; ou Can. J. Chem. Soc. (1982), 945-956 ; et
- lorsque A représente un radical du type 4-(4-hydroxyphényl)-phényle, on se référera par exemple à la publication suivante : Synthesis, (1993) 788-790.

### Préparation des composés de formule générale (VIII)

Lorsque R¹ et R² représentent tous deux H, les aminoacides protégés de formule générale **(VIII)** sont soit commerciaux, soit obtenus par protection d'aminoacides commerciaux par un groupe de type carbamate selon des méthodes connues de l'homme du métier.

Lorsque au moins l'un de R¹ et R² n'est pas H, et n = 0, les aminoacides protégés de formule générale **(VIII)** sont obtenus en une étape, schéma 6.1, par alkylation, dans un solvant comme le tétrahydrofuranne et à basse température, du composé commercial de formule générale **(VIII.i)** à l'aide de 3 équivalents de butyllithium et d'environ un équivalent de dérivé halogéné de formule générale **(VIII.ii)** dans laquelle R¹ représente un radical de type alkyle, cycloalkyle, cycloalkylalkyle ou arylalkyle et Hal un atome halogène. Selon le cas, une deuxième alkylation (non représentée dans le schéma 6.1) peut être effectuée de façon similaire, permettant ainsi d'obtenir les composés de formule générale **(VIII)** dans lesquels ni R¹ ni R² ne représente H.

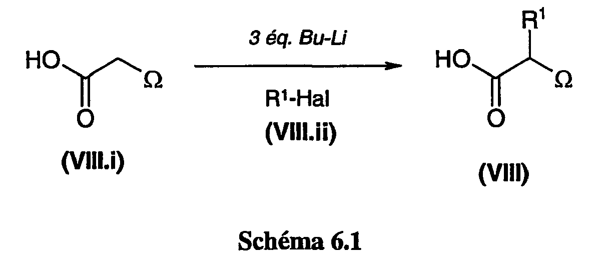

### Préparation des imidazoles, thiazoles et oxazoles de formule générale (IX)

La préparation des intermédiaires de formule générale **(IX)** est décrite dans la demande de brevet WO 98/58934 (cf. en particulier pages 10 à 50 et les exemples de ce document) ou effectuée par analogie à partir de produits de départ commerciaux.

### Préparation des alcools protégés de formule générale (X)

### Préparation des composés de formule générale (X) dérivés d'imidazoles

L'acide de formule générale **(X.i)** est successivement traité, schéma 8.1, par Cs₂CO₃, le composé de formule générale **(V.ii)** et par NH₄OAc, pour donner le composé de formule générale **(X).** Les conditions réactionnelles sont analogues à celles décrites plus haut pour ce type de synthèse.

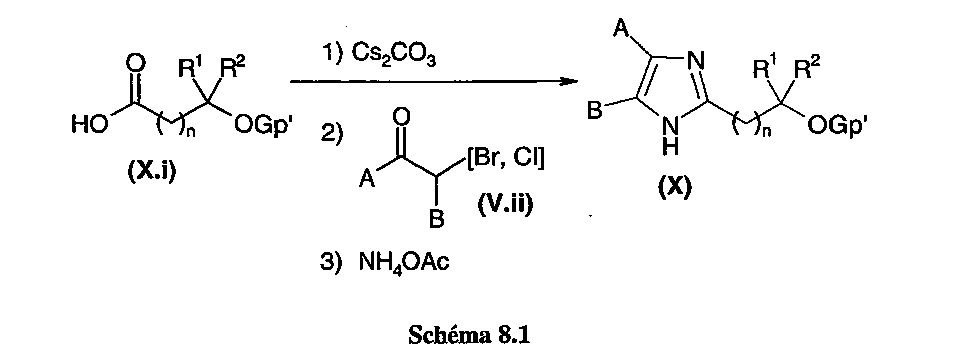

### Préparation des composés de formule générale (X) dérivés de thiazoles

Le dérivé cyano de formule générale **(X.ii)** est traité, schéma 8.2, par H₂S pour donner le thiocarboxamide de formule générale **(X.iii),** lequel, condensé sur le composé de formule générale **(V.ii),** permet d'obtenir le composé de formule générale **(X).** Les conditions réactionnelles sont analogues à celles décrites plus haut (schéma 5.3) pour ce type de synthèse.

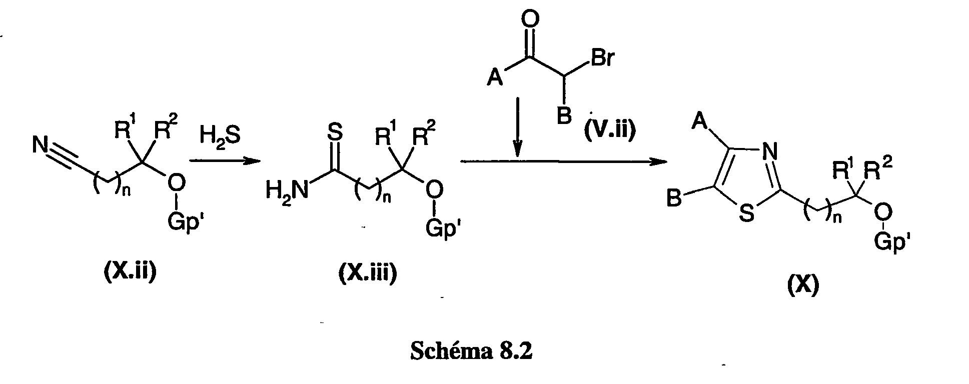

### Préparation des acides de formule générale (XXXVI)

Les acides de formule générale **(XXXVI)** non commerciaux sont accessibles à partir de méthodes de la littérature ou de méthodes similaires que l'homme du métier aura adaptées. En particulier :
◆ lorsque A représente un radical phénothiazinyle, les acides de formule générale **(XXXVI)** sont accessibles à partir de méthodes de la littérature : J. Org. Chem., (1956), 21, 1006 ; Chem. Abstr., 89, 180029 et Arzneimittel Forschung (1969), 19, 1193.
◆ lorsque A représente un radical diphénylamine, les acides de formule générale **(XXXVI)** sont accessibles à partir de méthodes de la littérature : Chem Ber., (1986), 119, 3165-3197 ; J. Heterocyclic. Chem. (1982), 15, 1557-1559 ; Chem. Abstr., (1968), 68, 68730x ; ou par adaptation de ces méthodes par l'homme du métier ;
◆ lorsque A représente un radical du type 4-(4-hydroxyphényl)-phényle, les acides de formule générale **(XXXVI)** sont accessibles à partir de méthodes de la littérature comme par exemple Tetrahedron Lett. (1968), 4739 ou J. Chem. Soc. (1961), 2898.
◆ lorsque A représente un radical carbazolyle, les acides de formule générale **(XXXVI)** sont accessibles à partir de méthodes de la littérature comme par exemple J. Amer. Chem., (1946), 68, 2104 ou J. Het. Chem (1975), 12, 547-549.
◆ lorsque A représente un radical de type benzopyrane ou benzofuranne, les acides de formule générale **(XXXVI)** sont accessibles par des méthodes de la littérature comme par exemple Syn. Commun. (1982), 12(8), 57-66 ; J. Med. Chem. (1995), 38(15), 2880-2886 ; ou Helv. Chim. Acta. (1978), 61, 837-843.
◆ lorsque A représente un radical indolinyle ou tétrahydroquinolyle, les acides de formule générale **(XXXVI)** sont accessibles à partir de méthodes de la littérature comme, par exemple, J. Med. Chem. (1997), 40, (7), 1049-1062 ; Bioorg. Med. Chem. Lett. (1997), 1519-1524 ; Chem. Abstr. (1968), 69, 43814k ; ou Chem. Abstr. (1966), 66, 17538c.

Bien entendu, les fonctions phénol, amine ou aniline résultant de la nature des substituants sur le radical A des composés de formule générale **(XXXVI)** peuvent conduire l'homme du métier à ajouter aux étapes décrites des étapes de protection / déprotection de ces fonctions afin qu'elles n'interfèrent pas avec le reste de la synthèse chimique.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Exemple 1 : chlorhydrate de 2,6-ditert-butyl-4-{2-[2-(méthylamino)éthyl]-1,3-thiazol-4-yl}phénol :

### 1.1) 2-cyanoéthyl(méthyl)carbamate de tert-butyle :

0,1 mol de N-méthyl-β-alaninenitrile est mise en solution dans du dichlorométhane (100 ml) contenant 20,9 ml (0,12 mol) de diisopropyléthylamine. Le mélange est refroidi à 0 °C puis on ajoute par fractions Boc-O-Boc (26,2 g ; 0,12 mol) et on laisse agiter le mélange une nuit à température ambiante. Le milieu réactionnel est alors versé sur de l'eau glacée et extrait au dichlorométhane. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. L'huile marron-rouge obtenue est utilisée telle quelle dans l'étape suivante.

### 1.2) 3-amino-3-thioxopropyl(méthyl)carbamate de tert-butyle :

43,4 mmol de l'intermédiaire 1.1 sont dissoutes dans de l'éthanol (40 ml) contenant de la triéthylamine (6,1 ml). On fait ensuite barboter H₂S dans le mélange pendant 3 h avant d'évaporer les solvants à sec. Le produit attendu est obtenu après chromatographie sur une colonne de silice (éluant : 50% d'acetate d'éthyle dans de l'heptane) sous forme d'huile orange clair. La cristallisation de cette huile dans de l'éther diisopropylique donne un solide blanc avec un rendement de 15% Point de fusion : 104 °C.

### 1.3) 4-[3,5-bis(1,1-diméthyléthyl)-4-hydroxyphényl]-N-[(1,1-diméthyléthoxy)-carbonyl]-N-méthyl-2-thiazoleéthanamine :

L'intermédiaire 1.2 (2,11 mmol) et de la bromo-1-(3,5-di*tert*-butyl-4-hydroxyphényl)éthanone (6,9 g ; 2,11 mmol) sont dissous dans du toluène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est cristallisée sous forme d'un solide blanc. Point de fusion : 204 °C.

### 1.4) Chlorhydrate de 2,6-ditert-butyl-4-{2-[2-(méthylamino)éthyl]-1,3-thiazol-4-yl}phénol :

1,95 mmol de l'intermédiaire 1.3 sont dissous dans de l'acétate d'éthyle (20 ml). La solution est refroidie à 0 °C puis on fait barboter HCl gaz pendant 10 minutes. On laisse le mélange revenir à température ambiante tout en le maintenant agité. Après filtration et séchage sous vide, le produit attendu est récupéré sous la forme de cristaux blancs que l'on lave avec de l'éther. Rendement quantitatif. Point de fusion : 206-208 °C.

### Exemple 2 : 2,6-ditert-butyl-4-[4-(hydroxyméthyl)-1,3-oxazol-2-yl]phénol :

Ce composé peut être obtenu selon une procédure analogue à celle décrite pour l'intermédiaire 1.C de la demande PCT WO 99/09829 dans laquelle le bromopyruvate d'éthyle remplace le 4-chloroacétoacétate et l'ester intermédiaire isolé est ensuite réduit à l'aide de DIBAL dans du dichlorométhane à 0 °C. Le mélange réactionnel est ensuite traité par une solution aqueuse de NH₄Cl et filtré sur célite. On extrait avec un mélange 50/50 de dichlorométhane et d'acétate d'éthyle. Après décantation, séchage sur sulfate de magnésium, filtration et évaporation du filtrat, une cristallisation dans l'éthanol permet d'obtenir le produit attendu sous la forme d'une poudre blanche. Point de fusion : 167-168 °C.

### Exemple 3 : chlorhydrate de 2,6-ditert-butyl-4-{2-[1-(méthylamino)éthyl]-1,3-thiazol-4-yl}phénol :

### 3.1) N'-(tert-butoxycarbonyl)-N'-méthylalaninamide :

12 mmol de Boc-N-Me-DL-Ala-OH sont dissoutes dans du diméthoxyéthane. De la N-méthylmorpholine est ajoutée goutte à goutte, puis du chloroformiate d'*iso*-butyle. Après 15 minutes d'agitation du mélange à -15 °C, on fait barboter de l'ammoniac (NH₃) puis on continue à maintenir le mélange agité à cette température pendant une nuit. On filtre le précipité obtenu. Le produit, une fois séché, est engagé tel quel dans l'étape suivante.

### 3.2) 2-amino-1-méthyl-2-thioxoéthyl(méthyl)carbamate de tert-butyle :

Ce composé est obtenu par réaction avec P₂S₅ dans les conditions décrites dans l'exemple 12, étape 12.2.

### 3.3) 1-[4-(3,5-ditert-butyl-4-hydroxyphényl)-1,3-thiazol-2-yl]éthyl(méthyl)carbamate de tert-butyle :

L'intermédiaire 3.2 et la bromo-1-(3,5-di*tert*-butyl-4-hydroxyphényl)éthanone sont condensés selon un protocole analogue à celui décrit dans l'étape 1.3.

### 3.4) chlorhydrate de 2,6-ditert-butyl-4-{2-[1-(métlrylamino)éthyl]-1,3-thiazol-4-yl}phénol :

Le protocole expérimental utilisé est le même que celui décrit pour l'étape 1.4 de l'exemple 1, l'intermédiaire 3.3 remplaçant l'intermédiaire 1.3. On obtient le produit attendu sous la forme d'une poudre blanche. Point de fusion : 236-237 °C.

### Exemple 4 : 2,6-ditert-butyl-4-[2-(méthoxyméthyl)-1,3-thiazol-4-yl]phénol :

### 4.1) pivalate de [4-(3,5-ditert-butyl-4-hydroxyphényl)-1,3-thiazol-2-yl]méthyle :

L'intermédiaire 4.1 est préparé selon un protocole identique à celui décrit pour l'exemple 1, étape 1.3, en utilisant le 2-(*tert*-butylcarbonyloxy)thioacétamide à la place de l'intermédiaire 1.2 et le toluène remplaçant le benzène. Le composé attendu est obtenu sous forme d'un solide blanc avec un rendement de 100%. Point de fusion : 114,6-116,0 °C.

### 4.2) 2,6-ditert-butyl-4-[2-(hydroxyméthyl)-1,3-thiazol-4-yl]phénol :

L'intermédiaire 4.1 (1,25 mmol) est dissous dans du méthanol (20 ml). La solution est refroidie à l'aide d'un bain de glace puis une solution de NaOH 1*N* est ajoutée goutte à goutte. On laisse le mélange revenir à température ambiante tout en l'agitant. Après évaporation à sec et dilution à l'eau du résidu, la solution est neutralisée à l'aide d'acide citrique et extraite avec du dichlorométhane. La phase organique est lavée avec du chlorure de sodium en solution aqueuse saturée avant d'être séchée sur sulfate de magnésium, filtrée et concentrée sous vide. On obtient un solide blanc avec un rendement de 88%. Point de fusion : 126,4-127,4 °C.

### 4.3) 2,6-ditert-butyl-4-[2-(méthoxyméthyl)-1,3-thiazol-4-yl]phénol :

L'intermédiaire 4.2 (1 équivalent) est méthylé par réaction avec 1,1 équivalent d'iodométhyle en présence de 2 équivalents de triéthylamine, la réaction s'effectuant dans du tétrahydrofuranne. On obtient une poudre crème foncé. Point de fusion : 115,8-117 °C.

### Exemple 5 : chlorhydrate de 2,6-ditert-butyl-4-{4-[(méthylamino)méthyl]-1,3-oxazol-2-yl}phénol :

### 5.1) 2,6-ditert-butyl-4-[4-(bromométhyl)-1,3-oxazol-2-yl]phénol:

On dissout du composé de l'exemple 2 (4,70 mmol) dans du dichlorométhane (30 ml). Après ajout de CBr₄ (2,02 g ; 6,10 mmol), on refroidit le milieu réactionnel à 0 °C. PPh₃ (1,48 g ; 5,63 mmol) est ajouté par fractions puis le mélange est laissé revenir à température ambiante. Le milieu réactionnel est alors versé sur de l'eau glacée avant d'être extrait au dichlorométhane. La phase organique est lavée à l'eau salée avant d'être séchée sur sulfate de magnésium, filtrée et concentrée sous vide. L'huile brute obtenue est suffisamment pure pour pouvoir être utilisé directement dans l'étape suivante.

### 5.2) Chlorhydrate de 2,6-ditert-butyl-4-{4-[(méthylamino)méthyl]-1,3-oxazol-2-yl}phénol :

33 mmol de méthylamine (solution 2M dans THF) sont dissous dans de l'acétonitrile (50 ml). 5,48 mmol de l'intermédiaire 5.1 dissous dans de l'acétonitrile (50 ml) sont ajoutées à 0 °C puis on laisse le mélange agité à température ambiante pendant 3 heures. Les solvants sont évaporés puis le résidu est partagé entre de l'acétate d'éthyle et une solution aqueuse à 10% de NaHCO₃. La phase organique est lavée à l'eau salée avant d'être séchée sur sulfate de magnésium, filtrée et concentrée sous vide. Le chlorhydrate est alors obtenu en solubilisant la base dans de l'éther et en ajoutant 1,2 ml d'une solution 1*N* de HCl dans de l'éther. Après filtration et lavage du solide formé avec de l'éther, on obtient une poudre orange foncé. Point de fusion : se décompose à 150 °C.

### Exemple 6 : N-{[4-(3,5-ditert-butyl-4-hydroxyphényl)-1,3-thiazol-2-yl]méthyl}acétamide :

### 6.1) {4-[3,5-di(tert-butyl)-4-hydroxyphényl]-1,3-thiazol-2-yl}méthylcarbamate de benzyle :

Ce composé est préparé selon un protocole expérimental décrit dans la demande de brevet WO 98/58934 (voir préparation des intermédiaires 26.1 et 26.2), en utilisant Z-Gly-NH₂ à la place du N-Boc sarcosinamide. Le composé attendu est obtenu sous forme d'une huile jaune pâle avec un rendement de 99%. MH+ = 453,20

### 6.2) 4-[2-(aminométhyl)-1,3-thiazol-4-yl]-2,6-di(tert-butyl)phénol :

A une solution de 0,106 g (1,1 mmol) de l'intermédiaire 6.1 dans 10 ml de méthanol on ajoute goutte à goutte 0,1 ml d'une solution d'hydroxyde de potassium à 40%. Après une nuit d'agitation à reflux, le mélange réactionnel est concentré sous vide et le résidu est dilué avec du dichlorométhane et lavé avec une solution HCl 1*N* puis 50 ml d'une solution saturée de NaCl. La phase organique est séparée et séchée sur sulfate de magnésium, filtrée et concentrée sous vide. Le produit attendu est obtenu après chromatographie sur une colonne de silice (éluant : 5% d'éthanol dans du dichlorométhane) sous forme d'une mousse marron avec un rendement de 76%. MH+ = 319,29.

### 6.3) N-{[4-(3,5-ditert-butyl-4-hydroxyphényl)-1,3-thiazol-2-yl]méthyl}acétamide :

L'intermédiaire 6.2 (2 mmol) est dissous dans du dichlorométhane (20 ml). De la triéthylamine (3 mmol) est ajoutée et le mélange refroidi à 0 °C. Du chlorure d'acétyle (3 mmol) est ensuite ajouté goutte à goutte. Une fois l'addition terminée, le mélange est ramené à température ambiante et agité une nuit à cette température avant d'être versé sur de l'eau glacée. La phase aqueuse est extraite avec du dichlorométhane, et la phase organique obtenue lavée avec de l'eau sale avant d'être séchée sur sulfate de magnésium. Après filtration et évaporation des solvants, le produit attendu est obtenu, après chromatographie sur une colonne de silice (éluant : 3% d'éthanol dans du dichlorométhane), avec un rendement de 79%. Mousse crème foncé. MH+ = 361,2.

### Exemple 7 : [4-(3,5-ditert-butyl-4-hydroxyphényl)-1,3-thiazol-2-yl]méthylcarbamate d'éthyle :

Une solution contenant l'intermédiaire 6.2 décrit ci-dessus (5 mmol) et 5 ml d'une solution d'hydroxyde de sodium 1N est refroidie à 10 °C. Du chloroformiate d'éthyle (5 mmol) et 2,5 ml d'une solution d'hydroxyde de sodium 2 N sont ajoutés simultanément. Après 16 heures d'agitation à 23 °C, on ajoute environ 0,5 ml d'une solution d'acide chlorhydrique concentré (environ 11 N) pour ajuster le pH à 4-5. L'huile obtenue est extraite à l'acétate d'éthyle (2 x 5 ml), lavée avec de l'eau puis séchée sur sulfate de magnésium. Les solvants sont évaporés et le produit attendu est récupéré sous la forme de cristaux blancs. MH+ =391,2.

### Exemple 8 : 2,6-ditert-butyl-4-[2-(morpholin-4-ylméthyl)-1,3-thiazol-4-yl]phénol :

### 8.1) 4-[2-(bromométhyl)-1,3-thiazol-4-yl]-2,6-ditert-butylphénol :

On dissout 1,5 g (4,70 mmol) de l'intermédiaire 4.2, le (2,6-ditert-butyl-4-[2-(hydroxyméthyl)-1,3-thiazol-4-yl]phénol, dans du dichlorométhane (30 ml). Après ajout de CBr₄ (2,02 g ; 6,10 mmol), on refroidit le milieu réactionnel à 0 °C. PPh₃ (1,48 g ; 5,63 mmol) est ajouté par fractions puis le mélange est laissé revenir à température ambiante. Le milieu réactionnel est alors versé sur de l'eau glacée avant d'être extrait au dichlorométhane. La phase organique est lavée à l'eau salée avant d'être séchée sur sulfate de magnésium, filtrée et concentrée sous vide. Le produit attendu est obtenu après chromatographie sur une colonne de silice (éluant : 30% d'acétate d'éthyle dans de l'heptane), pour conduire à une huile marron avec un rendement de 92%. Ce produit est suffisamment pur pour pouvoir être utilisé directement dans l'étape suivante. MH+ = 382,20.

### 8.2) 2,6-ditert-butyl-4-[2-(morpholin-4-ylméthyl)-1,3-thiazol-4-yl]phénol :

1,57 mmol de morpholine et 0,4 ml (2,62 mmol) de triéthylamine sont dissous dans du diméthylformamide (15 ml). 0,400 g (1,05 mmol) de l'intermédiaire 8.1 dissous dans du diméthylformamide (5 ml) sont ajoutés puis on laisse le mélange agité à température ambiante pendant 18 heures. Le milieu réactionnel est ensuite versé sur de l'eau glacée et extrait avec de l'acétate d'éthyle. La phase organique est lavée à l'eau salée avant d'être séchée sur sulfate de magnésium, filtrée et concentrée sous vide. Le produit attendu est obtenu après chromatographie sur une colonne de silice (éluant : 50% d'acétate d'éthyle dans de l'heptane), pour conduire à une huile orange avec un rendement de 92%. On obtient des cristaux crème clair. Point de fusion : 136,7-137,2 °C.

### Exemple 9 : 2,6-ditert-butyl-4-[2-(thiomorpholin-4-ylméthyl)-1,3-thiazol-4-yl]phénol :

Le protocole expérimental utilisé est le même que celui décrit pour l'exemple 8, la thiomorpholine remplaçant la morpholine dans la deuxième étape. On obtient le produit attendu sous la forme d'un solide orange clair. Point de fusion : 153,4-154,6 °C.

### Exemple 10 : 4-[2-(anilinométhyl)-1,3-thiazol-4-yl]-2,6-ditert-butylphénol :

Le protocole expérimental utilisé est le même que celui décrit pour l'exemple 8, l'aniline remplaçant la morpholine dans la deuxième étape. On obtient le produit attendu sous la forme de cristaux bruns. Point de fusion : 147,2-148,0 °C.

### Exemple 11 : 2,6-ditert-butyl-4-(2-{[[2-(diméthylamino)éthyl]-(méthyl)amino]méthyl}-1,3-thiazol-4-yl)phénol :

### 11.1) Chlorhydrate de 4-[3,5-bis(1,1-diméthyléthyl)-4-hydroxyphényl]-N-méthyl-2-thiazoleméthanamine :

Ce composé est obtenu en utilisant un protocole expérimental identique à celui des étapes 14.1 à 14.4 de l'exemple 14 (voir plus loin).

### 11.2) 2,6-ditert-butyl-4-(2-{[[2-(diméthylamino)éthyl]-(méthyl)amino]méthyl}-1,3-thiazol-4-yl)phénol :

A une solution de 1 mmol de l'intermédiaire 11.1 dans 20 ml de diméthylformamide, on ajoute goutte-à-goutte, à température ambiante sous atmosphère d'argon, 5 mmol de triéthylamine et un léger excès (1,2 mmol) de N-diméthyl-N-(2-chloroéthyl)amine. Après 24 heures d'agitation à 80 °C, le mélange réactionnel est versé sur de l'eau glacée. On extrait avec de l'acétate d'éthyle, lave avec une solution saturée de NaCl, sèche sur sulfate de magnésium et concentre la solution. Le produit attendu est obtenu après chromatographie sur une colonne de silice (éluant : dichlorométhane contenant de 5% d'éthanol avec des traces d'ammoniaque à dichlorométhane contenant de 5% d'éthanol avec des traces d'ammoniaque). Après évaporation, les fractions pures donnent une huile marron visqueuse. MH+ = 404,26.

### Exemple 12 : chlorhydrate de 2,6-ditert-butyl-4-{5-méthyl-2-[(méthylamino)méthyl]-1,3-thiazol-4-yl}phénol :

### 12.1) N-Boc-sarcosinamide :

15,0 g (0,120 mol) de chlorhydrate de sarcosinamide (N-Me-Gly-NH₂.HCl) sont mis en solution dans du dichlorométhane contenant 46,2 ml (0,265 mol) de diisopropyléthylamine. Le mélange est refroidi à 0 °C puis on ajoute par fractions Boc-O-Boc (28,8 g ; 0,132 mol) et on laisse agiter le mélange une nuit à température ambiante. Le milieu réactionnel est alors versé sur de l'eau glacée et extrait au dichlorométhane. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther diisopropylique pour conduire à un solide blanc avec un rendement de 72%. Point de fusion : 103 °C.

### 12.2) 2-{[(1,1-diméthyléthoxy)carbonyl]méthyl}amino-éthanethioamide :

16,0 g (0,085 mol) de l'intermédiaire 12.1 sont dissous dans du diméthoxyéthane (500 ml) et la solution obtenue est refroidie à 5 °C. Du bicarbonate de sodium (28,5 g ; 0,34 mol) puis, par petites portions, (P₂S₅)₂ (38,76 g ; 0,17 mol) sont ajoutés. Le milieu réactionnel est laissé revenir à température ambiante tout en étant agité durant 24 heures. Après évaporation sous vide des solvants, on ajoute au résidu une solution aqueuse de bicarbonate de sodium à 10% et la solution est extraite à l'aide d'acétate d'éthyle. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther pour conduire à un solide de couleur blanche avec un rendement de 65%. Point de fusion : 150-151 °C.

### 12.3) bromo-1-(3,5-ditert-bulyl-4-hydroxyphényl)propan-1-one :

Ce composé est obtenu de façon simple par réaction de 1-(3,5-ditert-butyl-4-hydroxyphényl)propan-1-one (préparée à partir du 2,6-ditertbutylphénol selon Russ. J. Org. Chem. (1997), 33, 1409-1416) avec du brome dans de l'acide acétique-ou encore selon un protocole décrit dans l'une des références suivantes : Biorg. Med. Chem. Lett. (1996), 6(3), 253-258 ; J. Med. Chem. (1988), 31(10), 1910-1918 ; J. Am. Chem. Soc. (1999), 121, 24.

### 12.4) 5-méthyl-4-[3,5-bis(1,1-diméthyléthyl)-4-hydroxyphényl]-N-[(1,1-diméthyléthoxy)-carbonyl]-N-méthyl-2-thiazoleméthanamine :

L'intermédiaire 12.2 (4,3 g ; 2,11 mmol) et l'intermédiare 12.3 (2,11 mmol) sont dissous dans du toluène (75 ml) sous atmosphère d'argon puis le mélange est agité à température ambiante durant 12 heures. Le milieu réactionnel est porté au reflux pendant 4 heures. Après évaporation des solvants, le résidu est dilué avec de l'acétate d'éthyle et lavé avec une solution NaHCO₃ à 10% puis avec une solution saturée en NaCl. La phase organique est séparée, séchée sur sulfate de magnésium, filtrée et concentrée sous vide. Le produit attendu est obtenu après chromatographie sur une colonne de silice (éluant : 30% d'acétate d'éthyle dans de l'heptane). L'huile récupérée est utilisée telle quelle dans l'étape qui suit.

### 12.5) chlorhydrate de 2,6-ditert-butyl-4-{5-méthyl-2-[(méthylamino)méthyl]-1,3-thiazol-4-yl}phénol :

Ce composé est obtenu sous la forme d'une poudre blanche par un protocole expérimental analogue à celui de l'étape 1.4 de l'exemple 1. Point de fusion : 140-142 °C.

### Exemple 13 : chlorhydrate de 1-[4-(10H-phénothiazin-2-yl)-1,3-thiazol-2-yl]méthanamine :

### 13.1) 2-chloro-1-(10H-phénothiazin-2-yl)éthanone :

La 2-chloro-l-[10-(chloroacétyl)-10*H*-phénothiazin-2-yl)éthanone est préparée à partir de la phénothiazine d'après un protocole décrit dans la littérature (J. Heterocyclic. Chem. (1978), 15, 175 et Arzneimittel Forschung, (1962), 12, 48), lequel est suivi par une réaction de déprotection en milieu acide (acide acétique et acide chlorhydrique) du groupe chloroacétyle (qui a servi à protéger la position 10 de la phénothiazine lors de la réaction de Friedel-Crafts).

### 13.2) 2-amino-2-thioxoéthylcarbamate de benzyle :

85 mmol de Z-Gly-NH₂ sont dissoutes dans du diméthoxyéthane (500 ml) et la solution obtenue est refroidie à 5 °C. Du bicarbonate de sodium (28,5 g ; 0,34 mol) puis, par petites portions, (P₂S₅)₂ (38,76 g ; 0,17 mol) sont ajoutés. Le milieu réactionnel est laissé revenir à température ambiante tout en étant agité durant 24 heures. Après évaporation sous vide des solvants, on ajoute au résidu une solution aqueuse de bicarbonate de sodium à 10% et la solution est extraite à l'aide d'acétate d'éthyle. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est ensuite purifié par cristallisation dans de l'éther.

### 13.3) [4-(10H-phénothiazin-2-yl)-1,3-thiazol-2-yl]méthylcarbantate de benzyle :

Les intermédiaires 13.1 et 13.2 sont couplés selon un protocole analogue à celui décrit dans l'étape 1.3 de l'exemple 1.

### 13.4) chlorhydrate de 1-[4-(10H-phénothiazin-2-yl)-1,3-thiazol-2-yl]méthanamine

Le protocole expérimental utilisé est le même que celui décrit pour l'étape 1.4 de l'exemple 1, l'intermédiaire 13.3 remplaçant l'intermédiaire 1.3. Après séchage sous vide, on obtient le produit attendu sous forme d'une poudre vert foncé. Point de fusion : > 275 °C.

### Exemple 14 : N-{[4-(3,5-ditert-butyl-4-hydroxyphényl)-1,3-thiazol-2-yl]méthyl}-N-méthylacétamide :

### 14.1) N-Boc-sarcosinamide :

La préparation de ce composé a déjà été décrite à l'étape 12.1 de l'exemple 12.

### 14.2) 2-{[(1,1-diméthyléthoxy)carbonyl]méthyl}amino-éthanethioamide :

La préparation de ce composé a déjà été décrite à l'étape 12.2 de l'exemple 12.

### 14.3) 4-[3,5-bis(1,1-diméthyléthyl)-4-hydroxyphényl]-N-[(1,1-diméthyléthoxy)-carbonyl]-N-méthyl-2-thiazoleméthanamine :

L'intermédiaire 14.2 (4,3 g; 2,11 mmol) et de la bromo-1-(3,5-di*tert*-butyl-4-hydroxyphényl)éthanone (6,9g ; 2,11 mmol) sont dissous dans du benzène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est obtenu après chromatographie sur une colonne de silice (éluant : 20% d'acétate d'éthyle dans de l'heptane) sous forme d'une huile qui cristallise très lentement au réfrigérateur avec un rendement de 28%.
Point de fusion : 126,5-127,3 °C.

### 14.4) chlorhydrate de 4-[3,5-bis(1,1-diméthyléthyl)-4-hydroxyphényl]-N-méthyl-2-thiazoleméthanamine :

1,95 mmol de l'intermédiaire 14.3 sont dissous dans de l'acétate d'éthyle (20 ml). La solution est refroidie à 0 °C puis on fait barboter HCl gaz pendant 10 minutes. On laisse le mélange revenir à température ambiante tout en le maintenant agité. Après filtration et séchage sous vide, le produit attendu est récupéré (rendement quantitatif).

### 14.5) N-{[4-(3,5-ditert-butyl-4-hydroxyphényl)-1,3-thiazol-2-yl]méthyl}-N-méthylacétamide :

Ce composé est obtenu selon un protocole identqiue à celui décrit pour l'étape 6.3 de l'exemple 6, l'intermédiaire 14.5 remplaçant l'intermédiaire 6.2. Cristaux blancs. Point de fusion: 132,3-133,1 °C.

### Exemple 15 : chlorhydrate de 1-[4-(3,5-ditert-butyl-4-méthoxyphényl)-1,3-thiazol-2-yl]-N-méthylméthanamine :

### 15.1) 4-[3,5-bis(1,1-diméthyléthyl)-4-méthoxyphényl]-N-[(1,1-diméthyléthoxy)-carbonyl]-N-méthyl-2-thiazoleméthanamine :

L'intermédiaire 14.3 est méthylé par action d'iodure de méthyle en présence de NaH dans du tétrahydrofuranne pour donner le produit attendu. L'huile marron qui est obtenue est engagée telle quelle dans l'étape suivante.

### 15.2) chlorhydrate de 1-[4-(3,5-ditert-butyl-4-méthoxyphényl)-1,3-thiazol-2-yl]-N-méthylméthanamine

Le mode opératoire est analogue à celui de l'étape 14.4 de l'exemple 14, l'intermédiaire 15.1 remplaçant l'intermédiaire 14.3 et l'acétate d'éthyle étant remplacé par un mélange d'acétate d'éthyle et d'éther. Le produit attendu est récupéré sous la forme de cristaux crème clair. Point de fusion : 218,4-219,6 °C.

### Exemple 16 : chlorhydrate de 2,6-ditert-butyl-4-{2-[(éthylamino)méthyl]-1,3-thiazol-4-yl}phénol :

Le protocole expérimental est identique à celui utilisé dans les étapes 14.1 à 14.4 de l'exemple 14, le N-éthylglycineamide (J. Med. Chem. (1995), 38(21), 4244-4256) remplaçant le N-sarcosinamide dans la première étape. Cristaux blancs. Point de fusion : 232,4-234,6 °C.

### Exemple 17 : chlorhydrate de 2,6-ditert-butyl-4-{2-[(4-phénylpipérazin-1-yl)méthyl]-1,3-thiazol-4-yl}phénol :

Le protocole expérimental utilisé est le même que celui décrit pour l'exemple 8, la 4-phénylpipérazine remplaçant la morpholine dans la deuxième étape. Cristaux crème clair. Point de fusion : 225,3-226,9 °C.

### Exemple 18 : chlorhydrate de 2,6-ditert-butyl-4-{2-[(4-méthyl-1,4-diazépan-1-yl)méthyl]-1,3-thiazol-4-yl}phénol :

Le protocole expérimental utilisé est le même que celui décrit pour l'exemple 8, la N-méthylhomopipérazine remplaçant la morpholine dans la deuxième étape. Cristaux blancs. Point de fusion : 222,1-225,4 °C.

### Exemple 19 : chlorhydrate de N-{1-[4-(4-anilinophényl)-1,3-thiazol-2-yl]éthyl}-N-méthylamine :

### 19.1) 1-(4-anilinophényl)éthanone

De la 4-amino-acétophénone (4,87 g ; 36,0 mmol) est dissoute dans du diméthylformamide (75 ml). On ajoute 15 g (0,108 mol) de carbonate de potassium (préalablement séché à 170 °C sous atmosphère d'argon), 7,236 g (36,0 mmol) d'iodobenzène, 0,4 g de cuivre en poudre et une quantité catalytique d'iodure de cuivre. Le mélange réactionnel est porté à reflux pendant 12 heures. Après avoir laissé le milieu réactionnel revenir à température ambiante, on filtre celui-ci sur célite et le verse sur de l'eau glacée. Après extraction avec de l'acétate d'éthyle, la phase organique est lavée avec de l'eau avant d'être séchée sur sulfate de magnésium, filtrée et concentrée sous vide. Le produit obtenu est purifié par cristallisation dans de l'heptane pour conduire à un solide jaune avec un rendement de 53,4%. Point de fusion : 105 °C.

### 19.2) N-(4-acétylphényl)-N-phénylacétamide :

Ce composé est obtenu selon une méthode inspirée de Tetrahedron (1980), 36, 3017-3019. L'intermédiaire 19.1 (60 mmol) est mis en suspension dans 150 ml d'anhydride acétique. De l'acide perchlorique à 70% (0,5 ml) est ajouté. Après chauffage pendant 15 minutes à 70 °C, le mélange est versé sur de la glace et le précipité formé est filtré. Après séchage sous vide, redissolution dans du -dichlorométhane et traitement au noir animal, la suspension est filtrée sur célite et les solvants sont évaporés. Après cristallisation dans de l'heptane, on obtient un solide jaune avec un rendement de 54,2%. Point de fusion: 118-120 °C (valeur dans la littérature : 122-123 °C).

### 19.3) N-[4-(bromoacétyl)phényl]-N-phénylacétamide

L'intermédiaire 19.2 (0,633 g ; 2,5 mmol) est dissous dans du méthanol (20 ml) et on ajoute 1 g (2,0 mmol) de résine de bromation PVPHP (J. Macromol. Sci. Chem. (1977), A11, (3), 507-514). Après agitation sous atmosphère d'argon pendant 4 heures, on filtre et rince les résines au méthanol. Après évaporation des solvants du filtrat et cristallisation dans du méthanol, on obtient un solide blanc avec un rendement de 59%. Point de fusion : 152-153 °C.

### 19.4) (4-{4-[acétyl(phényl)amino]phényl}-1,3-thiazol-2-yl)méthyl(méthyl)carbamate de tert-butyle :

De l'intermédiaire 19.3 (2,11 mmol) et de l'intermédiaire 3.2 (2,11 mmol) sont dissous dans du toluène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est obtenu et engagé tel quel dans l'étape suivante.

### 19.5) chlorhydrate de N-{1-[4-(4-anilinophényl)-1,3-thiazol-2-yl]éthyl}-N-méthylamine

L'intermédiaire 19.4 (1,5 mmol) est traité avec du HCl concentré (15 ml) et de l'acide acétique (30 ml). Après une mise à reflux pendant 24 h et évaporation des solvants, le résidu est repris dans toluène, les solvants à nouveau évaporés puis le produit cristallisé dans un peu d'eau. On obtient une poudre grise. Point de fusion : > 250 °C.

### Exemple 20 : chlorhydrate de 2,6-ditert-butyl-4-{2-[(isopropylamino)méthyl]-1,3-thiazol-4-yl}phénol :

L'intermédiaire 6.2 (2 mmol), en solution dans du méthanol (20 ml), est mis à réagir avec de l'acétone (2,2 mmol), NaBH₄ (2,2 mmol) en présence de tamis moléculaires. Le produit de la réaction est ensuite converti en chlorhydrate selon un mode opératoire analogue à celui de l'étape 1.4 de l'exemple 1. Cristaux blancs. Point de fusion : 197,1-198,8 °C.

### Exemple 21 : chlorhydrate de 2,6-ditert-butyl-4-{2-[(cyclohexylamino)méthyl]-1,3-thiazol-4-yl}phénol :

Le protocole expérimental utilisé est le même que celui décrit pour l'exemple 20, la cyclohexanone remplaçant l'acétone. Cristaux blancs. Point de fusion : 202,1-203,4 °C.

### Exemple 22 : chlorhydrate de 2,6-ditert-butyl-4-{2-[(4-isopropylpipérazin-1-yl)méthyl]-1,3-thiazol-4-yl}phénol :

Le protocole expérimental utilisé est le même que celui décrit pour l'exemple 8, la N-isopropylpipérazine remplaçant la morpholine dans la deuxième étape. Cristaux blancs. Point de fusion : 238,4-239,7 °C.

### Exemple 23 : chlorhydrate de N-méthyl-1-[4-(10H-phénothiazin-2-yl)-1,3-thiazol-2-yl]éthanamine :

Le protocole expérimental utilisé est le même que celui décrit pour l'étape 19.4 de l'exemple 19, l'intermédiaire 13.1 remplaçant l'intermédiaire 19.3, cette étape étant suivie d'une étape analogue à celle de l'étape 1.4 de l'exemple 1 afin d'obtenir le chlorhydrate. Poudre vert foncé. Point de fusion : > 250 °C.

### Exemple 24 : chlorhydrate de 2,6-ditert-butyl-4-{2-[(4-éthylpipérazin-1-yl)méthyl]-1,3-thiazol-4-yl}phénol :

Le protocole expérimental utilisé est le même que celui décrit pour l'exemple 8, la N-éthylpipérazine remplaçant la morpholine dans la deuxième étape. Cristaux blancs. Point de fusion : 247,0-248,8 °C.

### Exemple 25 : chlorhydrate de N-{[4-(4-anilinophényl)-1,3-thiazol-2-yl]méthyl}-N-éthylamine :

Le protocole expérimental utilisé est le même que celui décrit pour les étapes 14.1 à 14.4 de l'exemple 14, le N-éthyl-glycineamide (J. Med. Chem. (1995), 38(21), 4244-56) remplaçant le sarcosinamide et l'intermédiaire 19.3 remplaçant la bromo-1-(3,5-di*tert-*butyl-4-hydroxyphényl)éthanone. Poudre vert foncé. Point de fusion : > 250 °C.

### Exemple 26 : chlorhydrate de N-{[4-(10H-phénothinin-2-yl)-1,3-thiazol-2-yl]méthyl}éthanamine :

Le protocole expérimental utilisé est le même que celui décrit pour les étapes 14.1 à 14.4 de l'exemple 14, le N-éthyl-glycineamide (J. Med. Chem. (1995), 38(21), 4244-56) remplaçant le sarcosinamide et l'intermédiaire 13.1 remplaçant la bromo-1-(3,5-di*tert-*butyl-4-hydroxyphényl)éthanone. Poudre vert foncé. Point de fusion : > 250 °C.

### Exemple 27 : chlorhydrate de 2,6-ditert-butyl-4-(2-{[4-(diméthylamino)pipéridin-1-yl]méthyl}-1,3-thiazol-4-yl)phénol :

Le protocole expérimental utilisé est le même que celui décrit pour l'exemple 8, la 4-diméthylaminopipéridine (J. Med. Chem. (1983), 26, 1218-1223 ou J. Chem. Soc. (1957), 3165-3172) remplaçant la morpholine dans la deuxième étape. Poudre vert foncé. Point de fusion : 113,0-113,4 °C.

### Exemple 28 : chlorhydrate de 1-{[4-(3,5-ditert-butyl-4-hydroxyphényl)-1,3-thiazol-2-yl]méthyl}pipéridin-4-ol :

### 28.1) 1-{[4-(3,5-ditert-butyl-4-hydroxyphényl)-1,3-thiazol-2-yl]méthyl}pipéridin-4-ol

Le protocole expérimental utilisé est le même que celui décrit pour l'exemple 8, le chlorhydrate de pipéridin-4-one (J. Org. Chem. (1949), 14, 530-535) remplaçant la morpholine et 2 équivalents de triéthylamine supplémentaires étant engagés dans la deuxième étape. Le produit obtenu est engagé tel quel dans l'étape suivante.

### 28.2) chlorhydrate de 1-{[4-(3,5-ditert-butyl-4-hydroxyphényl)-1,3-thiazol-2-yl]méthyl}pipéridin-4-ol :

L'intermédiaire 28.1 est réduit en alcool par action de NaBH₄ dans du méthanol. Une fois la réaction terminée, du dichlorométhane et de l'eau salée sont ajoutés au milieu réactionnel. La phase aqueuse est extraite avec du dichlorométhane et lavée avec de l'eau salée. Les phases organiques réunies sont séchées sur sulfate de magnésium et les solvants évaporés.
Le produit obtenu précédemment est mis en solution dans de l'acétate d'éthyle et la solution refroidie à 0 °C. Une solution 1*N* de HCl dans l'éther (3 équivalents) est ajoutée lentement, le mélange étant maintenu à la température de 0 °C pendant l'addition puis laissé revenir à température ambiante, l'agitation étant maintenue ainsi pendant 12 heures. Le produit attendu est récupéré sous la forme d'un solide blanc. Point de fusion : 215,4-218,2 °C.

### Exemple 29 : 2-[4-(1,1'-biphényl-4-yl)-1H-imidazol-2-yl]éthylcarbamate de 4-méthylpentyle :

### 29.1) N-{[(4-méthylpentyl)oxy]carbonyl}-β-alanine :

Du triphosgène à 23 °C (5,3 g ; 0,019 mol) est ajouté à une solution contenant du 4-méthyl-1-pentanol (5 g ; 0,049 mol) dans 80 ml de dichlorométhane. Le mélange est refroidi à 0 °C puis on ajoute goutte à goutte de la pyridine (3,8 g ; 0,049 mol). Le mélange est ramené à 23 °C et l'agitation maintenue pendant 2 h. Les solvants sont évaporés à l'aide d'un évaporateur rotatif. Le solide blanc récupéré est filtré sur fritté après l'avoir trituré dans de l'éther. L'éther du filtrat est évaporé.
Un mélange contenant de la β-alanine (4,4 g, 0,049 mol) et 50 ml d'une solution d'hydroxyde de sodium 1*N* est refroidi à 10 °C. Du chlorure de 4-méthylpentylcarbonate préparé fraîchement précédemment et 50 ml d'une solution d'hydroxyde de sodium 1*N* à 5 °C sont ajoutés simultanément au mélange de β-alanine et d'hydroxyde de sodium préparé ci-dessus. Après 16 heures d'agitation à 23 °C, on ajoute environ 80 ml d'une solution d'acide chlorhydrique (environ 1*N*) pour ajuster le pH à 4-5.jusqu'à obtention d'un léger précipité blanc. Le mélange réactionnel est extrait à l'acétate d'éthyle (2 x 50 ml) et l'extrait lavé avec de l'eau puis séché sur sulfate de magnésium. On obtient une huile incolore (7,2 g ; rendement de 68%).
RMN H¹ (δ ppm, DMSO) : 0,85 (dq, 6H) ; 1,15 (m, 2H) ; 1,49-1,53 (m, 3H) ; 2,35 (t, 2H) ; 3,14-3,19 (m, 2H) ; 3,88-3,91 (m, 2H) ; 7,04 (se, 1H). ; 12 (sé, 1H)

### 29.2) 2-[4-(1,1'-biphényl-4-yl)-1H-imidazol-2-yl]éthylcarbamate de 4-méthylpentyle :

Un mélange de l'intermédiaire 29.1 (4,52 g ; 0,021 mol) et de carbonate de césium (3,4 g ; 0,0105 mol) dans 35 ml de méthanol est agité à 23 °C pendant 1 heure. Le méthanol est éliminé par évaporation sous pression réduite dans un évaporateur rotatif. Le mélange obtenu est dissous dans 70 ml de diméthylformamide puis de la 2-bromo-4-phénylacétophénone (5,7 g ; 0,021 mol) est ajoutée. Après 16 heures d'agitation, le solvant est évaporé sous pression réduite. Le mélange obtenu est repris dans de l'acétate d'éthyle puis le bromure de césium est filtré. L'acétate d'éthyle du filtrat est évaporé et l'huile réactionnelle est reprise dans un mélange de xylènes (300 ml) et d'acétate d'ammonium (32 g ; 0,42 mol). On chauffe à reflux environ une heure et demie en évacuant l'eau à l'aide d'un Dean-Stark puis, après refroidissement, un mélange d'eau glacée et d'acétate d'éthyle est versé dans le milieu réactionnel. Après décantation, la phase organique est lavée avec une solution saturée en bicarbonate de sodium, séchée sur sulfate de magnésium puis évaporée sous vide. Après purification sur colonne de silice (éluant : acétate d'éthyle-heptane / 5-5 à 10-0), on obtient une poudre de couleur blanche (rendement de 10%). Point de fusion : 128,3 °C. MH+ = 392,3.

*Les composés des exemples 30 à 43 sont obtenus selon des procédures analogues à celle décrite pour l'exemple 29 ou ci-dessus dans la partie intitulée « Préparation des composés de formule générale (I) »*.

### Exemple 30 : 2-[4-(4-pyrrolidin-1-ylphényl)-1H-imidazol-2-yl]éthylcarbamate de 3,3-diméthylbutyle :

Point de fusion : 119,2 °C. MH+ = 385,3.

### Exemple 31 : 2-[4-(1,1'-biphényl-4-yl)-1H-imidazol-2-yl]éthylcarbamate d'isopentyle :

Point de fusion : 128-130 °C. MH+ = 378,3.

### Exemple 32 : 2-[4-(4-bromo-1,1'-biphényl-4-yl)-1H-imidazol-2-yl]éthylcarbamate d'hexyle :

Point de fusion : 138-140 °C. MH+ = 470,2.

### Exemple 33 : 2-[4-(4-tert-butylphényl)-1H-imidazol-2-yl]éthylcarbamate de benzyle :

Point de fusion : 173 °C. MH+ = 378,2.

### Exemple 34 : 2-[4-(1,1'-biphényl-4-yl)-1H-imidazol-2-yl]éthylcarbamate de 3,3-diméthylbutyle :

Point de fusion : 98,4 °C. MH+ = 392,15.

### Exemple 35 : 2-[4-(4-pyrrolidin-1-ylphényl)-1H-imidazol-2-yl]éthylcarbamate d'hexyle :

Point de fusion : 110-114 °C. MH+ = 385,3.

### Exemple 36 : 2-[4-(4-pyrrolidin-1-ylphényl)-1H-imidazol-2-yl]éthylcarbamate de 4,4,4-trifluorobutyle :

Point de fusion : 148,3 °C. MH+ = 411,3.

### Exemple 37 : 2-[4-(3,5-ditert-butyl-4-hydroxyphényl)-1H-imidazol-2-yl]éthylcarbamate d'hexyle :

Point de fusion : 197,4 °C. MH+ = 444,4.

### Exemple 38 : 2-[4-(3,5-ditert-butyl-4-hydroxyphényl)-1H-imidazol-2-yl]éthylcarbamate de 3,3-diméthylbutyle :

Point de fusion : 118-120 °C. MH+ = 441,3.

### Exemple 39 : 2-[4-(4-méthoxyphényl)-1H-imidazol-2-yl]éthylcarbamate de 3,3-diméthylbutyle :

Point de fusion : 116,8 °C. MH+ = 346,2.

### Exemple 40 : 2-[4-(3,5-ditert-butyl-4-hydroxyphényl)-1H-imidazol-2-yl]éthylcarbamate de benzyle :

Point de fusion : 177,5 °C. MH+ = 450,3.

### Exemple 41 : 2-[4-(4-pyrrolidin-1-ylphényl)-1H-imidazol-2-yl]éthylcarbamate de benzyle :

Point de fusion : 122,4 °C. MH+ = 391,2.

### Exemple 42 : 2-[4-(1,1'-biphényl-4-yl)-1H-imidazol-2-yl]éthylcarbamate de 2-phényléthyle :

Point de fusion : 142-143 °C. MH+ = 412,2.

### Exemple 43 : 2-[4-(4'-fluoro-1,1'-biphényl-4-yl)-1H-imidazol-2-yl]éthylcarbamate de butyle :

Point de fusion : 149,3 °C. MH+ = 382,2.

### Exemple 44 : 2-[4-(1,1'-biphényl-4-yl)-5-méthyl-1H-imidazol-2-yl]éthylcarbamate de butyle :

### 44.1) 1-(1,1'-biphényl-4-yl)propan-1-one :

On chauffe à reflux pendant 4 h un mélange contenant de l'acide phénylborique (6,1 g ; 50 mmol), de la 4'-bromopropiophénone (10,65 g ; 50 mmol), du carbonate de sodium (5,3 g ; 50 mmol) et du chlorure de palladium (500 mg, 2,8 mmol) dans 300 ml d'eau.

De l'acide borique (1 g ; 0,8 mmol) est ensuite ajouté puis on chauffe encore pendant 30 minutes. Au mélange revenu à 23 °C, on rajouter 250 ml d'acétate d'éthyle puis on filtre sur fritté puis sur papier GFA. Le filtrat est décanté et la phase organique lavée avec une solution saturée en NaCl avant d'être séchée sur MgSO₄ et concentrée à l'aide d'un évaporateur rotatif. Le précipité est agité pendant 30 minutes dans 100 ml d'isopentane et 5 ml de dichlorométhane. Après filtration sur fritté, le solide est rincé à l'isopentane. On obtient une poudre de couleur crème (8,7 g ; 83%). Point de fusion : 98-99 °C. MH+ = 211,1

### 44.2) 1-(1,1'-biphényl-4-yl)-2-bromopropan-1-one :

L'intermédiaire 43.1 préparé précédemment est agité avec une résine de bromation PVPHP (30 g ; 2 mmol Br₃/g) dans 120 ml de toluène pendant 3 h à une température d'environ 5 °C. On rajoute environ 15 g de résine de bromation puis on agite encore pendant 3 h à 23 °C. On ajoute à nouveau environ 15 g de résine puis on laisse le mélange agité pendant 16 h. La résine est récupérée par filtration sur fritté et rincée au toluène puis au dichlorométhane. Le filtrat est concentré à sec et le précipité obtenu est agité dans de l'acétate d'isopropyle pendant 30 minutes. On filtre sur fritté et rince avec de l'isopentane. On obtient une poudre de couleur crème (9,58 g ; 87%). Point de fusion : 102-104 °C. MH+ = 398,2.

### 44.3) N-(butoxycarbonyl)-β-alanine :

Une solution contenant de la β-alanine (8,9 g ; 0,1 mol) et 100 ml d'une solution d'hydroxyde de sodium 1N est refroidie à 10 °C. Du chloroformiate de *n*-butyle (13,66 g ; 0,1 mol) et 50 ml d'une solution d'hydroxyde de sodium 2*N* sont ajoutés simultanément. Après 16 heures d'agitation à 23 °C, on ajoute environ 10 ml d'une solution d'acide chlorhydrique concentré (environ 11 N) pour ajuster le pH à 4-5. L'huile obtenue est extraite à l'acétate d'éthyle (2 x 50 ml), lavée avec de l'eau puis séchée sur sulfate de magnésium. Le produit cristallise dans l'isopentane sous forme d'une poudre blanche (rendement de 68%). Point de fusion : 50,5 °C.

### 44.4) 2-[4-(1,1'-biphényl-4-yl)-5-méthyl-1H-imidazol-2-yl]éthylcarbamate de butyle :

Un mélange de N-(butoxycarbonyl)-β-alanine (préparée à l'étape 44.3 ; 3,27 g ; 0,0173 mol) et de carbonate de césium (2,81 g ; 0,0087 mol) dans 50 ml de méthanol est agité à 23 °C pendant 1 heure. Le méthanol est éliminé par évaporation sous pression réduite dans un évaporateur rotatif. Le mélange obtenu est dissous dans 50 ml de diméthylformamide puis de l'intermédiaire 44.2 (5 g ; 0,0173 mol) est ajouté. Après -16 heures d'agitation, le solvant est évaporé sous pression réduite. Le mélange obtenu est repris dans de l'acétate d'éthyle puis le bromure de césium est filtré. L'acétate d'éthyle du filtrat est évaporé et l'huile réactionnelle est reprise dans un mélange de xylène (80 ml) et d'acétate d'ammonium (26,6 g ; 0,35 mol). On chauffe à reflux environ une heure et demie en évacuant l'eau à l'aide d'un Dean-Stark puis, après refroidissement, un mélange d'eau glacée et d'acétate d'éthyle est versé dans le milieu réactionnel. Après décantation, la phase organique est lavée avec une solution saturée en bicarbonate de sodium, séchée sur sulfate de magnésium puis évaporée sous vide. Après purification sur colonne de silice (éluant : CH₂Cl₂-éthanol / 9-1), on obtient une huile incolore qui cristallise dans un mélange d'isopentane et d'éther isopropylique. Après filtration et séchage on obtient une poudre de couleur blanche (3,31 g, rendement de 50%). Point de fusion : 143-144 °C. MH+ = 378,2.

*Les composés des exemples 45 à 49 sont obtenus selon des procédures analogues à celle décrite pour l'exemple 44 ou ci-dessus dans la partie intitulée « Préparation des composés de formule générale (I)* ».

### Exemple 45 : 2-[4-(4'-méthyl-1,1'-biphényl-4-yl)-1H-imidazol-2-yl]éthylcarbamate de butyle :

Point de fusion : 168,4 °C. MH+ = 378,2.

### Exemple 46 : 2-[4-(4-chloro-1,1'-biphényl-4-yl)-1H-imidazol-2-yl]éthylcarbamate de butyle :

Point de fusion : 164,2 °C. MH+ = 398,2.

### Exemple 47 : 2-[4-(2'-fluoro-1,1'-biphényl-4-yl)-1H-imidazol-2-yl]éthylcarbamate de butyle :

Point de fusion : 113,8 °C. MH+ = 382,2.

### Exemple 48 : 2-{4-[4'-(méthylthio)-1,1'-biphényl-4-yl]-1H-imidazol-2-yl}éthylcarbamate de butyle :

Point de fusion : 167,9 °C. MH+ = 410,3.

### Exemple 49 : 2-[4-(2',4'-difluoro-1,1'-biphényl-4-yl)-1H-imidazol-2-yl]éthylcarbamate de butyle :

Point de fusion : 105,7 °C. MH+ = 430,2.

### Exemple 50 : chlorhydrate de 2,6-di-tert-butyl-4-{2-[(propylamino)méthyl]-1,3-thiazol-4-yl}phénol :

### 50.1) 2,6-di-tert-butyl-4-{2-[(propylamino)yéthyl]-1,3-thiazol-4-yl}phénol:

Dans un ballon contenant 20 ml de MeOH anhydre, sous atmosphère inerte, on ajoute successivement 0,636 g (2,0 mmol) de l'intermédiaire 6.2, 0,16 ml (2,2 mmol) de propionaldéhyde et 1 g de tamis moléculaire 4 Å pulvérulent préalablement activé. Le mélange réactionnel est agité vigoureusement pendant 18 heures avant l'addition, par portions, de 0,083 g (2,2 mmol) de NaBH₄. L'agitation est maintenue 4 heures supplémentaires puis 5 ml d'eau sont ajoutés. Un quart d'heure après, le tamis est filtré et le mélange réactionnel est extrait par 2 fois 100 ml de Cl₂Cl₂. La phase organique est lavée successivement avec 50 ml d'eau et 50 ml de saumure avant d'être séchée sur sulfate de sodium, filtrée et concentrée sous vide. Le résidu est purifié sur une colonne de silice (éluant : 30% d'acétate d'éthyle dans de l'heptane). On obtient une huile jaune engagée telle quelle dans l'étape suivante.

### 50.2) Chlorhydrate de 2,6-di-tert-butyl-4-{2-[(propylamino)méthyl]-1,3-thiazol-4-yl}phénol :

L'intermédiaire 50.1 est dissous dans de l'éther anhydre (15 ml). La solution est refroidie à 0 °C puis on ajoute goutte à goutte un excès d'une solution de HCl 1*N* dans de l'éther (0,6 ml). On laisse le mélange revenir à température ambiante tout en le maintenant agité. Après filtration, lavage avec de l'éther puis de l'isopentane et séchage sous vide, un solide blanc-gris est récupéré avec un rendement de 4%. MH+ = 361,2.

### Exemple 51 : chlorhydrate de N-{[4-(10H-phénothiazin-2-yl)-1,3-thiazol-2-yl]méthyl}-N-propylamine :

Le protocole expérimental utilisé est le même que celui décrit pour l'étape 50.1 de l'exemple 50, le composé de l'exemple 13 remplaçant l'intermédiaire 6.2. On obtient un solide jaune-vert avec un rendement de 32%. MH+ = 354,2.

### Exemple 52 : N-{[4-(10H-phénothiazin-2-yl)-1,3-thiazol-2-yl]méthyl}butan-1-amine :

Le protocole expérimental utilisé est le même que celui décrit pour l'exemple 8, la butylamine remplaçant la morpholine dans l'étape 8.2. On obtient un solide jaune avec un rendement de 25,6%. Point de fusion : 139,0-141,0 °C.

### Exemple 53 : chlorhydrate de N-{[4-(10H-phénothiazin-2-yl)-1,3-thiazol-2-yl]méthyl}pentan-1-amine :

Le protocole expérimental utilisé est le même que celui décrit pour l'étape 50.1 de l'exemple 50, le composé de l'exemple 13 et le valéraldéhyde remplaçant respectivement l'intermédiaire 6.2 et le propionaldéhyde. On obtient un solide de couleur foncée avec un rendement de 38%. MH+ = 382,2.

### Exemple 54 : chlorhydrate de (R,S)-1-{[4-(3,5-di-tert-butyl-4-hydroxyphényl)-1,3-thiazol-2-yl]méthyl}pipéridin-3-ol :

Le protocole expérimental utilisé est le même que celui décrit pour l'exemple 8, la (*R*,*S*)-3-hydroxypipéridine remplaçant la morpholine dans l'étape 8.2. Le produit obtenu sous forme de base est salifié selon le protocole décrit pour l'étape 50.2 pour conduire à un solide crème clair avec un rendement de 81 %. Point de fusion : 126,9-130,1 °C.

### Exemple 55 : chlorhydrate de (R,S)-1-{[4-(3,5-di-tert-butyl-4-hydroxyphényl)-1,3-thiazol-2yl]méthyl}pyrrolidin-3-ol :

Le protocole expérimental utilisé est le même que celui décrit pour l'exemple 8, la (*R*,*S*)-3-hydroxypyrrolidine remplaçant la morpholine dans l'étape 8.2. Le produit obtenu sous forme de base est salifié selon le protocole décrit pour l'étape 50.2 pour conduire à un solide crème clair avec un rendement de 93%. Point de fusion : 79,8-83,3 °C.

### Exemple 56 : [4-(10H-phénothiazin-2-yl)-1,3-thiazol-2-yl]méthanol :

### 56.1) Pivalate de [4-(10H-phénothiazin-2-yl)-1,3-thiazol-2-yl]méthyle :

Ce composé est préparé selon un protocole identique à celui décrit pour l'étape 1.3 de l'exemple 1, en utilisant respectivement le 2-(tert-butylcarbonyloxy)thioacétamide et la 2-bromo-1-[10-(chloroacétyl)-10H-phénothiazin-2-yl)éthanone à la place de l'intermédiaire 1.2 et de la bromo-1-(3,5-ditert-butyl-4-hydroxyphényl)éthanone. Le composé attendu est obtenu sous forme d'un solide verdâtre avec un rendement de 63,2%. Point de fusion : 120,0-122,0 °C.

### 56.2) [4-(10H-phénothiazin-2-yl)-1,3-thiazol-2-yl]méthanol :

Ce composé est préparé à partir de l'intermédiaire 56.1 selon un protocole identique à celui décrit pour l'étape 4.2 de l'exemple 4. Le composé attendu est obtenu sous forme d'un solide verdâtre avec un rendement de 61%. Point de fusion : 145,0-147,0 °C.

### Exemple 57 : N,N-diméthyl-N-{[4-(10H-phénothiazin-2-yl)-1,3-thiazol-2-yl]méthyl}amine :

### 57.1) 2-[2-(bromométhyl)-1,3-thiazol-4-yl]-10H-phénothiazine :

Ce composé est préparé selon un protocole identique à celui décrit pour l'étape 8.1 de l'exemple 8, en utilisant l'intermédiaire 56.2 à la place de l'intermédiaire 4.2. Le composé attendu est obtenu sous forme de cristaux vert-jaune doré brillant avec un rendement de 42%. Point de fusion : 165-170 °C (décomp.).

### 57.2) N,N-diméthyl-N-{[4-(10H-phénothiazin-2-yl)-1,3-thiazol-2-yl]méthyl}amine :

Ce composé est préparé selon un protocole identique à celui décrit pour l'étape 8.2 de l'exemple 8 en utilisant respectivement l'intermédiaire 57.1 et la N,N-diméthylamine à la place de l'intermédiaire 8.1 et de la morpholine. Le composé attendu est obtenu sous forme d'un solide jaune avec un rendement de 41,8%. Point de fusion : 155,0-157,0 °C.

### Exemple 58 : chlorhydrate de 2-{2-[(4-méthylpipérazin-1-yl)méthyl]-1,3-thiazol-4-yl}-10H-phénothiazine :

Le protocole expérimental utilisé est le même que celui décrit pour l'exemple 8, la l'intermédiaire 57.1 et la N-méthylpipérazine remplaçant respectivement l'intermédiaire 8.1 et la morpholine dans l'étape 8.2. Le produit obtenu sous forme de base est salifié selon le protocole décrit pour l'étape 50.2 pour conduire à un solide gris avec un rendement de 67%. Point de fusion : 210,0-212,0 °C.

### Exemple 59 : 2-[2-(pipéridin-1-ylméthyl)-1,3-thiazol-4-yl]-10H-phénothiazine :

Le protocole expérimental utilisé est le même que celui décrit pour l'exemple 8, la l'intermédiaire 57.1 et la pipéridine remplaçant respectivement l'intermédiaire 8.1 et la morpholine dans l'étape 8.2. Le produit obtenu sous forme de base est salifié selon le protocole décrit pour l'étape 50.2 pour conduire à un solide gris-jaune avec un rendement de 67%. Point de fusion : 186,0-188,0 °C.

### Exemple 60 : chlorhydrate de 2-[2-(pipérazin-1-ylméthyl)-1,3-thiazol-4-yl]-10H-phénothiazine :

### 60.1) 4-{[4-(10H-phénothiazin-2-yl)-1,3-thiazol-2-yl]méthyl}pipérazine-1-carboxylate de tert-butyle :

Ce composé est préparé selon un protocole identique à celui décrit pour l'exemple 8, en utilisant respectivement l'intermédiaire 57.1 et la N-*tert*-butoxycarbonylpipérazine à la place de l'intermédiaire 8.1 et de la morpholine. Le composé attendu est obtenu avec un rendement de 81,2%. MH+ = 481,2.

### 60.2) Chlorhydrate de 2-[2-(pipérazin-1-ylméthyl)-1,3-thiazol-4-yl]-10H-phénothiazine :

Ce composé est préparé selon un protocole identique à celui décrit pour l'étape 1.4 de l'exemple 1, l'intermédiaire 60.1 remplaçant l'intermédiaire 1.3. Le composé attendu est obtenu sous forme d'un solide gris-vert avec un rendement de 78,9%. Point de fusion : 210,0-215,0 °C.

### Exemple 61 : chlorhydrate de 1-{[4-(3,5-di-tert-butyl-4-hydroxyphényl)-1,3-thiazol-2-yl]méthyl}azétidin-3-ol :

### 61.1) Chlorhydrate de 1-(diphénylméthyl)-3-hydroxyazétidine :

De l'aminodiphénylméthane (55 g ; 0,3 mol) et de l'épichlorhydrine (23,5 ml ; 0,3 mol) sont mélangés dans du méthanol (200 ml). Le mélange est porté à reflux pendant 5 jours. Le méthanol est ensuite évaporé pour donner un solide beige. Ce dernier est filtré et lavé avec de l'éther pour conduire à un solide blanc avec un rendement de 45%. Point de fusion : 186,0-186,4 °C.

### 61.2) Azétidin-3-ol :

L'intermédiaire 61.1 est mis en solution dans un mélange éthanol / THF (7:3) auquel est ajouté de l'eau pour obtenir une bonne solubilisation. L'atmosphère est purgée avec de l'argon puis de l'hydrogéne avant de placer le mélange à température ambiante sous une pression de 3 bars d'hydrogène. Après filtration et lavage avec de l'éthanol, les solvants sont évaporés et la pâte résiduelle reprise dans de l'éther. Le solide formé est filtré et rincé avec de l'éther pour conduire à un solide blanc (rendement de 86%). Point de fusion : 74,0-76,8 °C.

### 61.3) Chlorhydrate de 1-{[4-(3,5-di-tert-butyl-4-hydroxyphényl)-1,3-thiazol-2-yl]méthyl}azétidin-3-ol :

Ce composé est préparé selon un protocole identique à celui décrit pour l'exemple 8, l'intermédiaire 61.2 remplaçant la morpholine dans l'étape 8.2. Le produit obtenu sous forme de base est salifié selon le protocole décrit pour l'étape 50.2 pour conduire à un solide crème clair avec un rendement de 74%. Point de fusion : 124,2-126,5 °C.

### Exemple 62 : 2-[2-(morpholin-4-ylméthyl)-1,3-thiazol-4-yl]-10H-phénothiazine :

Ce composé est préparé selon un protocole identique à celui décrit pour l'exemple 8, l'intermédiaire 57.1 remplaçant l'intermédiaire 8.1 dans l'étape 8.2 pour conduire à un solide blanc cassé avec un rendement de 86,0%. Point de fusion : 203,0-205,0 °C.

### Exemple 63 : 2-[2-(thiomorpholin-4-ylméthyl)-1,3-thiazol-4-yl]-10H-phénothiazine :

Ce composé est préparé selon un protocole identique à celui décrit pour l'exemple 8, l'intermédiaire 57.1 et la thiomorpholine remplaçant respectivement l'intermédiaire 8.1 et la morpholine dans l'étape 8.2. Le produit attendu est obtenu sous forme d'un solide jaune avec un rendement de 80,8%. Point de fusion : 229,0-231,0 °C.

### Exemple 64 : 2-{2-[(4-méthyl-1,4-diazépan-1-yl)méthyl]-1,3-thiazol-4-yl}-10H-phénothiazine :

Ce composé est préparé selon un protocole identique à celui décrit pour l'exemple 8, l'intermédiaire 57.1 et l'homopipérazine remplaçant respectivement l'intermédiaire 8.1 et la morpholine dans l'étape 8.2. Le produit attendu est obtenu sous forme d'un solide jaune avec un rendement de 27,0%. Point de fusion : 135-137 °C.

### Exemple 65 : chlorhydrate de (3R)-1-{[4-(3,5-di-tert-butyl-4-hydroxyphényl)-1,3-thiazol-2-yl]méthyl}pyrrolidin-3-ol :

Ce composé est préparé selon un protocole identique à celui décrit pour l'exemple 8, le (*R*)-3-pyrrolidinol remplaçant la morpholine dans l'étape 8.2. Le produit obtenu sous forme de base est salifié selon le protocole décrit pour l'étape 50.2 pour conduire à un solide blanc avec un rendement de 93%. Point de fusion : 162,0-164,6 °C.

### Exemple 66 : chlorhydrate de (3S)-1-{[4-(3,5-di-tert-butyl-4-hydroxyphényl)-1,3-thiazol-2-yl]méthyl}pyrrolidin-3-ol :

Ce composé est préparé selon un protocole identique à celui décrit pour l'exemple 8, le (*S*)-3-pyrrolidinol remplaçant la morpholine dans l'étape 8.2. Le produit obtenu sous forme de base est salifié selon le protocole décrit pour l'étape 50.2 pour conduire à un solide blanc avec un rendement de 93%. Point de fusion : 162,8-165,9 °C.

### Exemple 67 : chlorhydrate de 2,6-di-tert-butyl-4-[2-(pyrrolidin-1-ylméthyl)-1,3-thiazol-4-yl]phénol :

Ce composé est préparé selon un protocole identique à celui décrit pour l'exemple 8, la pyrrolidine remplaçant la morpholine dans l'étape 8.2. Le produit obtenu sous forme de base est salifié selon le protocole décrit pour l'étape 50.2 pour conduire à un solide blanc cassé avec un rendement de 73%. Point de fusion : 188,0-195,0 °C.

### Exemple 68 : chlorhydrate de 2,6-di-tert-butyl-4-{2-[(butylamino)méthyl]-1,3-thiazol-4-yl}phénol :

Ce composé est préparé selon un protocole identique à celui décrit pour l'exemple 8, la butylamine remplaçant la morpholine dans l'étape 8.2. Le produit obtenu sous forme de base est salifié selon le protocole décrit pour l'étape 50.2 pour conduire à un solide blanc cassé avec un rendement de 72%. Point de fusion : 179,7-180,2 °C.

### Exemple 69 : 2-{2-[(4-éthylpipérazin-1-yl)méthyl]-1,3-thiazol-4-yl}-10H-phénothiazine :

Ce composé est préparé selon un protocole identique à celui décrit pour l'exemple 8, l'intermédiaire 57.1 et la N-éthylpipérazine remplaçant respectivement l'intermédiaire 8.1 et la morpholine dans l'étape 8.2. Le produit attendu est obtenu sous forme d'un solide blanc avec un rendement de 57,7%. Point de fusion : 182,0-184,0 °C.

### Exemple 70 : chlorhydrate de N-méthyl-N-{[4-(10H-phénothiazin-2-yl)-1H-imidazol-2-yl]méthyl}amine :

### 70.1) Méthyl{[4-(10H-phénothiazin-2-yl)-1H-imidazol-2-yl]méthyl}carbamate de tert-butyle :

Ce composé est préparé selon un protocole identique à celui décrit pour l'étape 44.4 de l'exemple 44, Boc-Sar-OH et la 2-chloro-1-[10-(chloroacétyl)-10*H*-phénothiazin-2-yl)éthanone (cf. étape 13.1 de l'exemple 13) remplaçant respectivement la *N*-(butoxycarbonyl)-β-alanine et l'intermédiaire 44.2 tandis que l'éthanol remplace le méthanol dans l'étape 44.4. Le produit attendu est obtenu avec un rendement de 81,6% et engagé dans tel quel dans l'étape suivante.

### 70.2) Chlorhydrate de N-méthyl-N-{[4-(10H-phénothiazin-2-yl)-1H-imidazol-2-yl]méthyl}amine :

L'intermédiaire 70.1 est déprotégé avant d'être converti en chlorhydrate selon un mode opératoire analogue à celui de l'étape 1.4 de l'exemple 1. Le produit attendu est obtenu sous forme d'une poudre marron avec un rendement de 53,7%. Point de fusion : 190,0-195,0 °C.

### Exemple 71 : [4-(10H-phénothiazin-2-yl)-1,3-thiazol-2-yl]méthylcarbamate de méthyle :

Du composé de l'exemple 13 (0,622 g ; 2,0 mmol) est mis en solution dans du dioxanne (100 ml) refroidi à 0 °C. De la triéthylamine est ajoutée, puis, goutte à goutte, du méthylchloroformate (2,5 mmol). Le milieu réactionnel est ensuite laissé agité durant 3 heures à température ambiante avant d'être versé sur de l'eau glacée et extrait avec de l'acétate d'éthyle. La phase organique est séchée sur sulfate de magnésium, filtrée et concentrée sous vide. Le produit attendu est purifié sur colonne de silice (éluant : 10% d'acétone dans du dichlorométhane). Les fractions pures sont rassemblées et les solvants évaporés pour donner un solide blanc cassé avec un rendement de 46,0%. Point de fusion: 151-153 °C.

### Exemple 72 : [4-(10H-phénothiazin-2-yl)-1,3-thiazol-2-yl]méthylcarbamate de butyle :

Ce composé est préparé selon un protocole identique à celui décrit pour l'exemple 71, en utilisant du *n*-butylchloroformate à la place du méthylchloroformate. Le produit attendu est obtenu sous forme d'un solide jaune avec un rendement de 61,0%. Point de fusion : 186,0-188,0 °C.

### Exemple 73 : N-néopentyl-N-{[4-(10H-phénothiazin-2-yl)-1,3-thiazol-2-yl]méthyl}amine :

Ce composé est préparé selon un protocole identique à celui décrit pour l'étape 50.1 de l'exemple 50, le composé de l'exemple 13 et le pivaldéhyde remplaçant respectivement l'intermédiaire 6.2 et le propionaldéhyde. Le produit attendu est obtenu sous forme d'un solide blanc cassé avec un rendement de 40,6%. Point de fusion : 172,0-174,0 °C.

### Exemple 74 : 1-{[4-(10H-phénothiazin-2-yl)-1,3-thiazol-2-yl]méthyl}pipéridin-4-ol :

Ce composé est préparé selon un protocole identique à celui décrit pour l'exemple 8, l'intermédiaire 57.1 et la 4-hydroxy-pipéridine remplaçant respectivement l'intermédiaire 8.1 et la morpholine dans l'étape 8.2. Le produit attendu est obtenu sous forme d'un solide blanc avec un rendement de 52,5%. Point de fusion : 205,0-207,0 °C.

### Exemple 75 : N-{[4-(10H-phénothiazin-2-yl)-1,3-thiazol-2-yl]méthyl}acétamide :

Ce composé est préparé selon un protocole identique à celui décrit pour l'exemple 6, le composé de l'exemple 13 remplaçant l'intermédiaire 6.2 dans l'étape 6.3. Le produit attendu est obtenu sous forme d'un solide jaune avec un rendement de 25,0%. Point de fusion : 219,0-221,0 °C.

### Exemple 76 : N-{[4-(10H-phénothiazin-2-yl)-1,3-thiazol-2-yl]méthyl}butanamide :

Ce composé est préparé selon un protocole identique à celui décrit pour l'exemple 6, le composé de l'exemple 13 et le chlorure de butanoyle remplaçant respectivement l'intermédiaire 6.2 et le chlorure d'acétyle dans l'étape 6.3. Le produit attendu est obtenu sous forme d'un solide jaune avec un rendement de 47,2%. Point de fusion : 218,0-220,0 °C.

### Exemple 77 : 2,6-di-tert-butyl-4-{2-[(4-propylpipérazin-1-yl)méthyl]-1,3-thiazol-4-yl}phénol :

### 77.1) Chlorhydrate de 4-{[4-(3,5-ditert-butyl-4-hydroxyphényl)-1,3-thiazol-2-yl]méthyl}pipérazine-1-carboxylate de tert-butyle :

Ce composé est préparé selon un protocole identique à celui décrit pour l'exemple 8, la *N*-Boc-pipérazine remplaçant la morpholine dans l'étape 8.2. On obtient un solide orange pâle avec un rendement de 64%. Point de fusion : 108-109 °C.

### 77.2) Chlorhydrate de 2,6-ditert-butyl-4-[2-(pipérazin-1-ylméthyl)-1,3-thiazol-4-yl]phénol :

Ce composé est préparé selon un protocole identique à celui décrit pour l'étape 1.4, l'intermédiaire 77.1 remplaçant l'intermédiaire 1.3. Un solide blanc est obtenu avec un rendement de 86%. Point de fusion : 255,4-257,7 °C.

### 77.3) 2,6-di-tert-butyl-4-{2-[(4-propylpipérazin-1-yl)méthyl]-1,3-thiazol-4-yl}phénol :

Ce composé est préparé selon un protocole identique à celui décrit pour l'étape 50.1 de l'exemple 50, l'intermédiaire 77.2 remplaçant l'intermédiaire 6.2 et un excès de triéthylamine étant ajouté initialement pour convertir l'intermédiaire 77.2 en la base correspondante. Le produit attendu est obtenu sous forme d'un solide blanc cassé avec un rendement de 45%. Point de fusion : 236,5-238,2 °C.

### Exemple 78 : chlorhydrate de 2,6-di-tert-butyl-4-{2-[2-méthyl-1-(méthylamino)propyl]-1,3-thiazol-4-yl}phénol :

### 78.1) N-(tert-butoxycarbonyl)-N-méthylvaline :

De la N-méthyl-*D*,*L*-valine (10,0 g ; 0,0763 mol) est mise en solution dans un mélange dioxanne-eau (9 :1) (100 ml) contenant de la triéthylamine (13 ml). Le mélange est refroidi à 0 °C puis on ajoute, par portions, Boc-O-Boc (18,32 g ; 0,0763 mol) et on laisse agiter le mélange une nuit à température ambiante. Le milieu réactionnel est alors versé sur de l'eau glacée et extrait avec de l'acétate d'éthyle. La phase organique est lavée successivement avec une solution aqueuse de bicarbonate de sodium à 10% et de l'eau, puis enfin avec une solution de chlorure de sodium saturée. La phase organique est ensuite séchée sur sulfate de magnésium, filtrée et concentrée sous vide pour donner un produit huileux qui cristallise dans de l'éther de pétrole.Le produit attendu est récupéré avec un rendement de 67% avant d'être engagé tel quel dans l'étape suivante. Point de fusion : 83-85 °C.

### 78.2) N²-(tert-butoxycarbonyl)-N²-méthylvalinamide :

A l'intermédiaire 78.1 (11,8 g ; 0,051 mol) dans du dichlorométhane (200 ml), on ajoute successivement du chlorhydrate de 1-(3-diméthylaminopropyl)-3-éthyl-carbodiimide (9,777 g ; 0,051 mol) et de l'hydroxybenzotriazole (7,8 g ; 0,051 mol). De la triéthylamine (13 ml) est alors ajoutée goutte à goutte puis on laisse le mélange agité pendant 12 heures à température ambiante. Le milieu réactionnel est alors versé sur une solution aqueuse de bicarbonate de sodium à 10%. Après décantation, la phase organique est lavée à l'eau puis avec une solution de chlorure de sodium saturée. La phase organique est alors séchée sur sulfate de magnésium, filtrée et concentrée sous vide. Le résidu est repris dans du méthanol préablablement saturé avec du gaz ammoniac (150 ml). Le mélange est placé dans un four autoclave à 50 °C et agité pendant 4 jours à cette température. Le méthanol est évaporé et le produit est repris dans du dichlorométhane avant d'être lavé avec une solution de chlorure de sodium saturée.

La phase organique est séchée sur sulfate de magnésium, filtrée et concentrée sous vide. Le produit est purifié par trituration dans l'éther pour donner un solide blanc avec un rendement de 23,5%. Point de fusion : 181-183 °C.

### 78.3) 1-(aminocarbonothioyl)-2-méthylpropyl(méthyl)carbamate de tert-butyle :

Ce composé est préparé par réaction de l'intermédiaire 78.2 avec P₂S₅ dans les conditions décrites dans l'exemple 12, étape 12.2. Le produit attendu est purifié par chromatographie sur colonne de silice (éluant = 5% méthanol dans du dichlorométhane) pour donner un solide blanc cassé avec un rendement de 32,5%. Point de fusion : 199,0-201,0 °C.

### 78.4) Chlorhydrate de 2,6-di-tert-butyl-4-{2-[2-méthyl-1-(méthylamino)propyl]-1,3-thiazol-4-yl}phénol :

Ce composé est préparé selon un protocole identique à celui décrit pour l'étape 1.3 de l'exemple 1, l'intermédiaire 78.3 remplaçant l'intermédiaire 1.2. Le composé intermédiaire obtenu est déprotégé par l'acide bromhydrique libéré *in situ* pour donner le produit attendu sous forme de base libre, lequel est purifié par chromatographie sur colonne de silice (éluant : 30% d'acétate d'éthyle dans de l'heptane). La base libre est ensuite salifiée par dissolution dans de l'acétate d'éthyle au sein duquel on fait passer un courant de HCl gaz pendant 10 minutes. Après une heure d'agitation du mélange, ce dernier est évaporé à sec et le résidu repris dans de l'éther. Après filtration, on récupère un solide rose pâle avec un rendement de 92%. Point de fusion : 248,6-250,0 °C.

### Exemple 79 : chlorhydrate de N,2-diméthyl-1-[4-(10H-phénothiazin-2-yl)-1,3-thiazol-2-yl]propan-1-amine :

### 79.1) Méthyl{2-méthyl-1-[4-(10H-phénothiazin-2-yl)-1,3-thiazol-2-yl]propyl}carbamate de tert-butyle :

Les intermédiaires 78.3 et 13.1 sont couplés selon un protocole analogue à celui décrit dans l'étape 1.3 de l'exemple 1. On obtient le composé attendu sous forme d'une huile qui est purifiée par chromatographie sur colonne de silice (éluant : dichlorométhane pur). Le produit attendu est obtenu sous la forme d'un solide blanc avec un rendement de 72,4%. Celui-ci est engagé tel quel dans l'étape suivante.

### 79.2) Chlorhydrate de N,2-diméthyl-1-[4-(10H-phénothiazin-2-yl)-1,3-thiazol-2-yl]propan-1-amine :

Ce composé est préparé selon un protocole identique à celui décrit pour l'étape 1.4 de l'exemple 1, l'intermédiaire 79.1 remplaçant l'intermédiaire 1.3. Après lavage avec de l'éther et de l'isopentane puis séchage, le composé attendu est obtenu sous forme d'une poudre vert foncé avec un rendement de 62%. MH+ = 368,1.

### Exemple 80 : N-{[4-(10H-phénothiazin-2-yl)-1,3-thiazol-2-yl]méthyl}heganamide :

Ce composé est préparé selon un protocole identique à celui décrit pour l'exemple 6, le composé de l'exemple 13 et le chlorure d'hexanoyle remplaçant respectivement l'intermédiaire 6.2 et le chlorure d'acétyle dans l'étape 6.3. Le produit attendu est obtenu sous forme d'un solide marron avec un rendement de 40,7%. Point de fusion : 192.0-194,0 °C.

### Exemple 81 : (3R)-1-{[4-(10H-phénothiazin-2-yl)-1,3-thiazol-2-yl]méthyl}pyrrolidin-3-ol :

Ce composé est préparé selon un protocole identique à celui décrit pour l'exemple 8, l'intermédiaire 57.1 et le (*R*)-3-pyrrolidinol remplaçant respectivement l'intermédiaire 8.1 et la morpholine dans l'étape 8.2. Le produit attendu est obtenu sous forme d'un solide blanc avec un rendement de 49,5%. Point de fusion : 180,0-182,0 °C.

### Exemple 82 : (3S)-1-{[4-(10H-phénothiazin-2-yl)-1,3-thiazol-2-yl]méthyl}pyrrolidin-3-ol :

Ce composé est préparé selon un protocole identique à celui décrit pour l'exemple 8, l'intermédiaire 57.1 et le (*S*)-3-pyrrolidinol remplaçant respectivement l'intermédiaire 8.1 et la morpholine dans l'étape 8.2. Le produit attendu est obtenu sous forme d'un solide blanc avec un rendement de 49,5%. Point de fusion : 178,0-180,0 °C.

### Exemple 83 : 1-{[4-(10H-phénothiazin-2-yl)-1,3-thiazol-2-yl]méthyl}azétidin-3-ol :

Ce composé est préparé selon un protocole identique à celui décrit pour l'exemple 8, l'intermédiaire 57.1 et l'azétidine-3-ol (intermédiaire 61.2) remplaçant respectivement l'intermédiaire 8.1 et la morpholine dans l'étape 8.2. Le produit attendu est obtenu sous forme d'un solide blanc-cassé avec un rendement de 20,4%. Point de fusion : 240,0-242,0 °C.

### Exemple 84 : 2-{2-[(4-propylpipérazin-1-yl)méthyl]-1,3-thiazol-4-yl}-10H-phénothiazine :

Ce composé est préparé selon un protocole identique à celui décrit pour l'exemple 8, l'intermédiaire 57.1 et la *N*-propylpipérazine remplaçant respectivement l'intermédiaire 8.1 et la morpholine dans l'étape 8.2. Le produit attendu est obtenu sous forme d'un solide blanc avec un rendement de 42,6%. Point de fusion : 189,0-190,0 °C.

### Exemple 85 : 2-{2-[(4-acétylpipérazin-1-yl)méthyl]-1,3-thiazol-4-yl}-10H-phénothiazine :

Ce composé est préparé selon un protocole identique à celui décrit pour l'exemple 8, l'intermédiaire 57.1 et la N-acétyl-pipérazine remplaçant respectivement l'intermédiaire 8.1 et la morpholine dans l'étape 8.2. Le produit attendu est obtenu sous forme d'un solide blanc-cassé avec un rendement de 53,5%. Point de fusion : 218,0-220,0 °C.

### Exemple 86 : 2-{2-[(4-butylpipérazin-1-yl)méthyl]-1,3-thiazol-4-yl}-10H-phénothiazine :

Ce composé est préparé selon un protocole identique à celui décrit pour l'exemple 8, l'intermédiaire 57.1 et la *N*-butylpipérazine remplaçant respectivement l'intermédiaire 8.1 et la morpholine dans l'étape 8.2. Le produit attendu est obtenu sous forme d'un solide blanc avec un rendement de 69,3%. Point de fusion : 188,0-190,0 °C.

### Exemple 87 : 4-{[4-(10H-phénothiazin-2-yl)-1,3-thiazol-2-yl]méthyl}pipérazine-1-carboxylate de méthyle :

De l'intermédiaire 60.2 (0,380 g ; 1 mmol) est dissous dans du THF. On ajoute à la solution ainsi obtenue de la triéthylamine (1 ml) puis, goutte à goutte, du méthylchloroformate (0,1 ml). Une fois la réaction achevée, on verse le mélange réactionnel sur de l'eau glacée et extrait avec de l'acétate d'éthyle. La phase organique récupérée est filtrée et les solvants évaporés. Après cristallisation dans de l'iso-propanol, le produit attendu est obtenu sous forme d'un solide blanc avec un rendement de 66,1%. Point de fusion : 180,0-182,0 °C.

### Exemple 88 : chlorhydrate de 4-[2-(aminométhyl)-1H-imidazol-4-yl]-2,6-di-tert-butylphénol :

### 88.1) [4-(3,5-di-tert-butyl-4-hydroxyphényl)-1H-imidazol-2-yl]méthylcarbamate de benzyle :

Ce composé est préparé selon un protocole similaire à celui décrit pour l'étape 44.4 de l'exemple 44, la carbobenzyloxyglycine et la bromo-1-(3,5-di*tert*-butyl-4-hydroxyphényl)éthanone remplaçant respectivement la N-(butoxycarbonyl)-β-alanine et l'intermédiaire 44.2. Le produit attendu est obtenu avec un rendement de 55%. Point de fusion : 212,1-213,4 °C.

### 88.2) Chlorhydrate de 4-[2-(aminométhyl)-1H-imidazol-4-yl]-2,6-di-tert-butylphénol :

De l'intermédiaire 88.1 (2,2 g ; 5,05 mmol) est dissous dans un mélange 50/50 d'éthanol et de THF (70 ml). On ajoute 0,7 g de palladium sur charbon (10 %) et on place l'ensemble sous atmosphère d'hydrogène (3,5 bars de pression). Le catalyseur est filtré puis le solvant évaporé sous pression réduite. La base obtenue est solubilisée dans de l'éther et le chlororhydrate est préparé par ajout d'une solution de HCl 1*N* dans de l'éther (20 ml). Après filtration et séchage sous vide, le produit attendu est récupéré sous la forme d'un solide blanc à légèrement gris que l'on lave avec de l'éther puis avec de l'*iso*-pentane (rendement de 56%). Point de fusion : 225-228,3 °C.

### Exemple 89 : chlorhydrate de 4-{2-[(benzylamino)méthyl]-1,3-thiazol-4-yl}-2,6-di-tert-butylphénol :

Ce composé est préparé selon un protocole identique à celui décrit pour l'exemple 8, la benzylamine remplaçant la morpholine dans l'étape 8.2. Le produit attendu est obtenu sous forme d'un solide blanc avec un rendement de 62%. Point de fusion : 166,4-167,8 °C.

### Exemple 90 : chlorhydrate de 4-{2-[(4-acétylpipérazin-1-yl)méthyl]-1,3-thiazol-4-yl}-2,6-di-tert-butylphénol :

Ce composé est préparé selon un protocole identique à celui décrit pour l'exemple 8, la *N*-acétyl-pipérazine remplaçant la morpholine dans l'étape 8.2. Le produit attendu est obtenu sous forme d'un solide blanc avec un rendement de 64%. Point de fusion : 199,0-200,4 °C.

### Exemple 91 : chlorhydrate de N-méthyl-N-{[4-(10H-phénoxazin-2-yl)-1,3-thiazol-2-yl]méthyl}amine :

Ce composé est préparé selon un protocole identique à celui décrit pour l'exemple 12, la 2-chloro-1-(10*H*-phénoxazin-2-yl)éthanone remplaçant la bromo-1-(3,5-di*tert*-butyl-4-hydroxyphényl)propan-1-one (la 2-chloro-1-(10*H*-phénoxazine-2-yl)éthanone étant préparée de façon analogue à celle utilisée pour l'intermédiaire 13.1 - cf. J. Org. Chem. (1960), 25, 747-753). Le produit attendu est obtenu après couplage et déprotection et salification sous forme d'un solide vert. Point de fusion : 218-220 °C.

### Exemple 92 : chlorhydrate de 4-[2-(azétidin-1-ylméthyl)-1,3-thiazol-4-yl]-2,6-di-tert-butylphénol :

Ce composé est préparé selon un protocole identique à celui décrit pour l'exemple 8, l'azétidine remplaçant la morpholine dans l'étape 8.2. Le produit attendu est obtenu sous forme d'un solide blanc avec un rendement de 90%. Point de fusion : 141,7-144,2 °C.

### Exemple 93 : chlorhydrate de 2,6-di-tert-butyl-4-{2-[(4-butylpipérazin-1-yl)méthyl]-1,3-thiazol-4-yl}phénol :

Ce composé est préparé selon un protocole identique à celui décrit pour l'exemple 8, le *N*-butyl-pipérazine remplaçant la morpholine dans l'étape 8.2. Le produit attendu est obtenu sous forme d'un solide blanc cassé avec un rendement de 68%. Point de fusion : 229,9-230,5 °C.

*Les composés des exemples 94 à 112 sont obtenus selon des procédures analogues à celle décrite pour l'exemple* 29 *ou ci-dessus dans la partie intitulée « Préparation des composés de formule générale (I) ».*

### Exemple 94 : 2-[4-(3-chloro-1,1'-biphényl-4-yl)-1H-imidazol-2-yl]éthylcarbamate de butyle :

Point de fusion : 142,6 °C. MH+ = 398,3.

### Exemple 95 : 2-[4-(3'-fluoro-1,1'-biphényl-4-yl)-1H-imidazol-2-yl]éthylcarbamate de butyle :

Point de fusion : 141,5 °C. MH+ = 381,2.

### Exemple 96 : 2-[4-(4-isobutylphényl)-1H-imidazol-2-yl]éthylcarbamate de butyle :

Point de fusion : 95,5 °C. MH+ = 344,2.

### Exemple 97 : 2-[4-(4-isobutylphényl)-1H-imidazol-2-yl]éthylcarbamate de benzyle :

Point de fusion : 125,2 °C. MH+ = 378,4.

### Exemple 98 : 2-[4-(3'-chloro-4'-fluoro-1,1'-biphényl-4-yl)-1H-imidazol-2-yl]éthylcarbamate de butyle :

Point de fusion : 132,4 °C. MH+ = 416,3.

### Exemple 99 : 2-[4-(3',4'-dichloro-1,1'-biphényl-4-yl)-1H-imidazol-2-yl]éthylcarbamate de butyle :

Point de fusion : 137,5 °C. MH+ = 432,2.

### Exemple 100 : 2-[4-(4-propylphényl)-1H-imidazol-2-yl]éthylcarbamate de butyle :

Point de fusion : 83,2 °C. MH+ = 330,4.

### Exemple 101 : 2-[4-(4-éthylphényl)-1H-imidazol-2-yl]éthylcarbamate de butyle :

Point de fusion : 92,4 °C. MH+ = 316,3.

### Exemple 102: 2-[4-(4'-cyano-1,1'-biphényl-4-yl)-1H-imidazol-2-yl]éthylcarbamate de butyle :

Point de fusion : 147 °C. MH+ = 389,2.

### Exemple 103 : 2-{4-[4'-(trifluorométhyl)-1,1'-biphényl-4-yl]-1H-imidazol-2-yl}éthylcarbamate de butyle :

Point de fusion : 168,5 °C. MH+ = 432,3.

### Exemple 104 : 2-[4-(1,1'-biphényl-4-yl)-5-éthyl-1H-imidazol-2-yl]éthylcarbamate de butyle :

Point de fusion : 127-128 °C. MH+ = 392,2.

### Exemple 105 : 2-[4-(2'-chloro-1,1'-biphényl-4-yl)-1H-imidazol-2-yl]éthylcarbamate de butyle :

Point de fusion : 99,7 °C. MH+ = 398,1.

### Exemple 106 : 2-[4-(2',3'-difluoro-1,1'-biphényl-4-yl)-1H-imidazol-2-yl]éthylcarbamate de butyle :

Point de fusion : 90 °C. MH+ = 400,1.

### Exemple 107 : 2-[4-(2'-bromo-1,1'-biphényl-4-yl)-1H-imidazol-2-yl]éthylcarbamate de butyle :

Point de fusion : 109,6 °C. MH+ = 442,1.

### Exemple 108 : 2-[4-(3',5'-difluoro-1,1'-biphényl-4-yl)-1H-imidazol-2-yl]éthylcarbamate de butyle :

Point de fusion : 111,1 °C. MH+ = 400,2.

### Exemple 109 : 2-[4-(2'-méthoxy-1,1'-biphényl-4-yl)-1H-imidazol-2-yl]éthylcarbamate de butyle :

Point de fusion : 116-121 °C. MH+ = 394,3.

### Exemple 110 : 2-[4-(3'-nitro-1,1'-biphényl-4-yl)-1H-imidazol-2-yl]éthylcarbamate de butyle :

Point de fusion : 100,5-101,5 °C. MH+ = 409,2.

### Exemple 111 : 2-[4-(2',5'-difluoro-1,1'-biphényl-4-yl)-1H-imidazol-2-yl]éthylcarbamate de butyle :

Point de fusion : 109,5 °C. MH+ = 400,2.

### Exemple 112 : 2-[4-(3'-méthoxy-1,1'-biphényl-4-yl)-1H-imidazol-2-yl]éthylcarbamate de butyle :

Point de fusion : 112-113 °C. MH+ = 394,2.

### Exemple 113 : chlorhydrate de 4-{[4-(3,5-di-tert-butyl-4-hydroxyphényl)-1,3-thiazol-2-yl]méthyl}pipérazine-1-carboxylate de méthyle :

Ce composé est préparé selon un protocole identique à celui décrit pour l'exemple 8, l'ester méthylique de l'acide pipérazine-1-carboxylique remplaçant la morpholine dans l'étape 8.2. Le produit attendu est obtenu sous forme de cristaux blancs avec un rendement de 51%. Point de fusion : 240,6-241,4 °C.

### Exemple 114 : [4-(3,5-di-tert-butyl-4-hydroxyphényl)-1,3-thiazol-2-yl]méthylcarbamate de méthyle :

Ce composé est préparé selon un protocole identique à celui décrit pour l'exemple 71, l'intermédiaire 6.2 remplaçant le composé de l'exemple 13. Le produit attendu est obtenu sous forme d'un solide blanc cristallin avec un rendement de 18%. Point de fusion : 94,0-95,9 °C.

### Exemple 115 : N-{[4-(3,5-di-tert-butyl-4-hydroxyphényl)-1,3-thiazol-2-yl]méthyl}benzamide :

Ce composé est préparé selon un protocole identique à celui décrit pour l'exemple 71, l'intermédiaire 6.2 remplaçant le composé de l'exemple 13 et le chlorure de benzoyle remplaçant le méthylchloroformate. Le produit attendu est obtenu sous forme d'un solide blanc cristallin avec un rendement de 84%. Point de fusion : 200,4-201,2 °C.

### Exemple 116 : N-{[4-(3,5-di-tert-butyl-4-hydroxyphényl)-1,3-thiazol-2-yl]méthyl}-2-phénylacétamide :

Ce composé est préparé selon un protocole identique à celui décrit pour l'exemple 71, l'intermédiaire 6.2 remplaçant le composé de l'exemple 13 et le chlorure de phénylacétyle remplaçant le méthylchloroformate. Le produit attendu est obtenu sous forme d'un solide blanc cristallin avec un rendement de 45%. Point de fusion : 123,5-125,4 °C.

### Exemple 117 : N-{[4-(3,5-di-tert-butyl-4-hydroxyphényl)-1,3-thiazol-2-yl]méthyl}propanamide :

Ce composé est préparé selon un protocole identique à celui décrit pour l'exemple 71, l'intermédiaire 6.2 remplaçant le composé de l'exemple 13 et le chlorure de propionyle remplaçant le méthylchloroformate. Le produit attendu est obtenu sous forme d'un solide blanc cristallin avec un rendement de 45%. Point de fusion : 82,0-83,5 °C.

### Exemple 118 : acétate de 1-{[4-(3,5-di-tert-butyl-4-hydroxyphényl)-1,3-thiazol-2-yl]méthyl}pipéridin-4-yle :

Ce composé est préparé selon un protocole identique à celui décrit pour l'exemple 8, la 1-acétyl-pipérazine remplaçant la morpholine dans l'étape 8.2. Le produit attendu est obtenu sous forme d'un solide orange cristallin avec un rendement de 50%. Point de fusion : 160,3-160,6 °C.

### Exemple 119 : 1-{[4-(3,5-di-tert-butyl-4-hydroxyphényl)-1,3-thiazol-2-yl]méthyl}pyrrolidine-3,4-diol :

Ce composé est préparé selon un protocole identique à celui décrit pour l'exemple 8, la 3,4-dihydroxypyrrolidine remplaçant la morpholine dans l'étape 8.2. Le produit attendu est obtenu sous forme d'une mousse marron avec un rendement de 29%. MH+ = 405,20.

### Etude pharmacologique des produits de l'invention

### Etude des effets sur la liaison d'un ligand spécifique de la MAO-B, le [³H]Ro 19-6327

L'activité inhibitrice des produits de l'invention est déterminée par la mesure de leurs effets sur la liaison d'un ligand spécifique de la MAO-B, le [³H]Ro 19-6327.

### a) Préparation mitochondriale de cortex de rats

La préparation mitochondriale de cortex de rats est réalisée selon la méthode décrite dans Cesura A M, Galva M D, Imhof R et Da Prada M, J. Neurochem. 48 (1987), 170-176. Les rats sont décapités et leurs cortex prélevés, homogénéisés dans 9 volumes d'un tampon sucrose 0,32 M tamponné à pH 7,4 avec 5 mM d'HEPES, puis centrifugés à 800 g pendant 20 minutes. Les surnageants sont récupérés et les culots lavés 2 fois avec le tampon sucrose 0,32 M comme précédemment. Les surnageants récoltés sont centrifugés à 10000 g pendant 20 minutes. Les culots obtenus sont mis en suspension dans un tampon Tris (50 mM Tris, 130 mM NaCl, 5 mM KCl, 0,5 mM EGTA, 1 mM MgCl₂, pH 7,4) et centrifugés à 10000 g pendant 20 minutes. Cette étape est répétée 2 fois, et le culot final, correspondant à la fraction mitochondriale, est conservé à -80 °C dans le tampon Tris. Le contenu protéique de la préparation est déterminé par la méthode de Lowry.

### b) Liaison du [³H]Ro 19-6327

Dans un tube Eppendorf, 100 µl de la préparation mitochondriale (2 mg protéine/ml) sont incubés pendant 1 heure à 37 °C en présence de 100 µl de [³H] Ro 19-6327 (33 nM, concentration finale) et 100 µl de tampon Tris contenant ou non les inhibiteurs. La réaction est arrêtée par l'addition de 1 ml de tampon Tris froid dans chaque tube, puis les échantillons sont centrifugés 2 minutes à 12000 g. Les surnageants sont aspirés et les culots lavés avec 1 ml de tampon Tris. Les culots sont ensuite solubilisés dans 200 µl de sodium dodécyl sulfate (20% poids/volume) pendant 2 heures à 70 °C. La radioactivité est déterminée par comptage des échantillons en scintillation liquide.

### c) Résultats

Les composés des exemples 1, 3, 5, 11 et 18 décrits ci-dessus présentent une CI₅₀ inférieure ou égale à 10 µM.

### Etude des effets sur la peroxydation lipidique du cortex cérébral de rat

L'activité inhibitrice des produits de l'invention est déterminée par la mesure de leurs effets sur le degré de peroxydation lipidique, déterminée par la concentration en malondialdéhyde (MDA). Le MDA produit par la peroxydation des acides gras insaturés est un bon indice de la peroxydation lipidique (H Esterbauer and KH Cheeseman, Meth. Enzymol. (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g / 15 ml et centrifugé à 515 g pendant 10 minutes à 4 °C. Le surnageant est utilisé immédiatement pour la détermination de la peroxidation lipidique. L'homogénat de cortex cérébral de rat (500 µl) est incubé à 37 °C pendant 15 minutes en présence des composés à tester ou du solvant (10 µl). La réaction de peroxydation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. European J. Phannacol. (1995), 285, 203-206). Les composés des exemples 1, 3 à 28, 50 à 62, 64 à 86, 88 à 93 et 114 à 118 décrits ci-dessus présentent une CI₅₀ inférieure ou égale à 10 µM.

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (J. Neurosci. (1986), 6, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M ; K₂HPO₄ 5 mM ; pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM ; KCl 5,4 mM ; MgSO₄ 0,8 mM ; glucose 5,5 mM ; chlorure de choline 130 mM pH 7,4) puis aliquoté et conservé à -80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de veratridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM ; CaCl₂ 1,8 mM ; MgSO₄ 0,8 mM ; chlorure de choline 130 mM ; BSA 0,01 % ; pH 7,4). Après avoir ajouté 20 µl de Microscint 0 ^{®}, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1, 3, 5, 12, 15, 16, 18, 20, 28 à 47, 49, 52, 61, 65 à 69, 89 et 94 à 112 décrits ci-dessus présentent tous une CI₅₀ inférieure ou égale à 1 µM.

## Revendications

1. Composé **caractérisé en ce qu'**il est choisi parmi les composés suivants :
- 2,6-di*tert*-butyl-4-{2-[2-(méthylamino)éthyl]-1,3-thiazol-4-yl}phénol ;
- 2,6-di*tert*-butyl-4-[4-(hydroxyméthyl)-1,3-oxazol-2-yl]phénol ;
- 2,6-di*tert*-butyl-4-{2-[1-(méthylamino)éthyl]-1,3-thiazol-4-yl}phénol ;
- 2,6-di*tert*-butyl-4-[2-(méthoxyméthyl)-1,3-thiazol-4-yl]phénol ;
- 2,6-di*tert*-butyl-4-{4-[(méthylamino)méthyl]-1,3-oxazol-2-yl}phénol ;
- N-{[4-(3,5-di*tert*-butyl-4-hydroxyphényl)-1,3-thiazol-2-yl]méthyl}acétamide;
- [4-(3,5-di*tert*-butyl-4-hydroxyphényl)-1,3-thiazol-2-yl]méthylcarbamate d'éthyle ;
- 2,6-di*tert*-butyl-4-[2-(morpholin-4-ylméthyl)-1,3-thiazol-4-yl]phénol ;
- 2,6-di*tert*-butyl-4-[2-(thiomorpholin-4-ylméthyl)-1,3-thiazol-4-yl]phénol ;
- 4-[2-(anilinométhyl)-1,3-thiazol-4-yl]-2,6-di*tert*-butylphénol ;
- 2,6-di*tert*-butyl-4-(2-{[[2-(diméthylamino)éthyl](méthyl)amino]méthyl}-1,3-thiazol-4-yl)phénol ;
- 2,6-di*tert*-butyl-4-{5-méthyl-2-[(méthylamino)méthyl]-1,3-thiazol-4-yl}phénol ;
- 1-[4-(10H-phénothiazin-2-yl)-1,3-thiazol-2-yl]méthanamine ;
- *N*-{[4-(3,5-di*tert*-butyl-4-hydroxyphényl)-1,3-thiazol-2-yl]méthyl}-*N*-méthylacétamide ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thiazol-4-yl}phénol ;
- 2,6-di*tert*-butyl-4-{2-[(4-phénylpipérazin-1-yl)méthyl]-1,3-thiazol-4-yl}phénol ;
- 2,6-di*tert*-butyl-4-{2-[(4-méthyl-1,4-diazépan-1-yl)méthyl]-1,3-thiazol-4-yl}phénol ;
- N-{1-[4-(4-anilinophényl)-1,3-thiazol-2-yl]éthyl}-N-méthylamine ;
- 2,6-di*tert*-butyl-4-{2-[(isopropylamino)méthyl]-1,3-thiazol-4-yl}phénol ;
- 2,6-di*tert*-butyl-4-{2-[(cyclohexylamino)méthyl]-1,3-thiazol-4-yl}phénol :
- 2,6-di*tert*-butyl-4-{2-[(4-isopropylpipérazin-1-yl)méthyl]-1,3-thiazol-4-yl}phénol ;
- N-méthyl-1-[4-(10*H*-phénothiazin-2-yl)-1,3-thiazol-2-yl]éthanamine ;
- 2,6-di*tert*-butyl-4-{2-[(4-éthylpipérazin-1-yl)méthyl]-1,3-thiazol-4-yl}phénol ;
- *N*-{[4-(4-anilinophényl)-1,3-thiazol-2-yl]méthyl}-*N*-éthylamjne ;
- *N*-{[4-(10*H*-phénothiazin-2-yl)-1,3-thiazol-2-yl]méthyl}éthanamine ;
- 2,6-di*tert*-butyl-4-(2-{[4-(diméthylamino)pipéridin-1-yl]méthyl}-1,3-thiazol-4-yl)phénol ;
- 1-{[4-(3,5-di*tert*-butyl-4-hydroxyphényl)-1,3-thiazol-2-yl]méthyl}pipéridin-4-ol ;
- 2-[4-(1,1'-biphényl-4-yl)-1*H*-imidazol-2-yl]éthylcarbamate de 4-méthylpentyle ;
- 2-[4-(4-pyrrolidin-1-ylphényl)-1*H*-imidazol-2-yl]éthylcarbamate de 3,3-di néthylbutyle ;
- 2-[4-(1,1'-biphényl-4-yl)-1*H*-imidazol-2-yl]éthylcarbamate d'isopentyle ;
- 2-[4-(4'-bromo-1,1'-biphényl-4-yl)-1*H*-imidazol-2-yl]éthylcarbamate d'hexyle ;
- 2-[4-(4-*tert*-butylphényl)-1*H*-imidazol-2-yl]éthylcarbamate de benzyle ;
- 2-[4-(1,1'-biphényl-4-yl)-1*H*-imidazol-2-yl]éthylcarbamate de 3,3-diméthylbutyle ;
- 2-[4-(4-pyrrolidin-1-ylphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate de 3,3-di néthylbutyle ;
- 2-[4-(4-méthoxyphényl)-1*H*-imidazol-2-yl]éthylcarbamate de 3,3-diméthylbutyle ;
- 2-[4-(3,5-ditert-butyl-4-hydroxyphényl)-1*H*-imidazol-2-yl]éthylcarbamate de benzyle ;
- 2-[4-(4-pyrrolidin-1-ylphényl)-1*H*-imidazol-2-yl]éthylcarbamate de benzyle ;
- 2-[4-(1,1'-biphényl-4-yl)-1*H*-imidazol-2-yl]éthylcarbamate de 2-phényléthyle ;
- 2-[4-(4'-fluoro-1,1'-biphényl-4-yl)-1*H*-imidazol-2-yl]éthylcarbamate de butyle ;
- 2-[4-(1,1'-biphényl-4-yl)-5-méthyl-1*H*-imidazol-2-yl]éthylcarbamate de butyle ;
- 2-[4-(4'-méthyl-1,1'-biphényl-4-yl)-1*H*-imidazol-2-yl]éthylcarbamate de butyle ;
- 2-[4-(4'-chloro-1,1'-biphényl-4-yl)-1*H*-imidazol-2-yl]éthylcarbamate de butyle ;
- 2-[4-(2'-fluoro-1,1'-biphényl-4-yl)-1*H*-imidazol-2-yl]éthylcarbamate de butyle ;
- 2-[4-(2',4'-difluoro-1,1'-biphényl-4-yl)-1H-imidazol-2-yl]éthylcarbamate de butyle ;
- 2,6-di-*tert*-butyl-4-{2-[(propylamino)méthyl]-1,3-thiazol-4-yl}phénol ;
- *N*-{[4-(10*H*-phénothiazin-2-yl)-1,3-thiazol-2-yl]méthyl}-*N*-propylamine ;
- *N*-{[4-(10*H*-phénothiazin-2-yl)-1,3-thiazol-2-yl]méthyl}butan-1-amine ;
- *N*-{[4-(10*H*-phénothiazin-2-yl)-1,3-thiazol-2-yl]méthyl}pentan-1-amine ;
- 1-{[4-(3,5-di-*tert*-butyl-4-hydroxyphényl)-1,3-thiazol-2-yl]méthyl}pipéridin-3-ol ;
- 1-{[4-(3,5-di-*tert*-butyl-4-hydroxyphényl)-1,3-thiazol-2-yl]méthyl}pyrrolidin-3-ol ;
- [4-(10*H*-phénothiazin-2-yl)-1,3-thiazol-2-yl]méthanol ;
- *N*,*N*-diméthyl-*N*-{[4-(10*H*-phénothiazin-2-yl)-1,3-thiazol-2-yl]méthyl}amine ;
- 2-{2-[(4-méthylpipérazin-1-yl)méthyl]-1,3-thinol-4-yl}-10*H*-phénothiazine ;
- 2-[2-(pipéridin-1-ylméthyl)-1,3-thiazol-4-yl]-10*H*-phénothiazine ;
- 2-[2-(pipérazin-1-ylméthyl)-1,3-thiazol-4-yl]-10*H*-phénothiazine ;
- 1-{[4-(3,5-di-*tert*-butyl-4-hydroxyphényl)-1,3-thiazol-2-yl]méthyl}azétidin-3-ol ;
- 2-[2-(morpholin-4-ylméthyl)-1,3-thiazol-4-yl]-10*H*-phénothiazine ;
- 2-[2-(thiomorpholin-4-ylméthyl)-1,3-thiazol-4-yl]-10*H*-phénothiazine ;
- 2-{2-[(4-méthyl-1,4-diazépan-1-yl)méthyl]-1,3-thiazol-4-yl}-10*H*-phénothiazine ;
- (3R)-1-{[4-(3,5-di-tert-butyl-4-hydroxyphényl)-1,3-thiazol-2-yl]méthyl}pyrrolidin-3-ol ;
- (3S)-1-{[4-(3,5-di-tert-butyl-4-hydroxyphényl)-1,3-thiazol-2-yl]méthyl}pyrrolidin-3-ol ;
- 2,6-di-*tert*-butyl-4-[2-(pyrrolidin-1-ylméthyl)-1,3-thiazol-4-yl]phénol ;
- 2,6-di-tert-butyl-4-{2-[(butylamino)méthyl]-1,3-thiazol-4-yl}phénol ;
- 2-{2-[(4-éthylpipérazin-1-yl)méthyl]-1,3-thiazol-4-yl}-10*H*-phénothiaxine ;
- *N*-méthyl-*N*-{[4-(10*H*-phénothiazin-2-yl)-1*H*-imidazol-2-yl]méthyl}amine ;
- [4-(10*H*-phénothiazin-2-yl)-1,3-thiazol-2-yl]méthylcarbamate de méthyle ;
- [4-(10*H*-phénothiazin-2-yl)-1,3 -thiazol-2-yl]méthylcarbamate de butyle ;
- *N*-néopentyl-N-{[4-(10*H*-phénothiazin-2-yl)-1,3-thiazol-2-yl]méthyl}amine ;
- 1-{[4-(10*H*-phénothiazin-2-yl)-1,3-thiazol-2-yl]méthyl}pipéridin-4-ol ;
- *N*-{[4-(10*H*-phénothiazin-2-yl)-1,3-thiazol-2-yl]méthyl}acétamide ;
- *N*-{[4-(10*H*-phénothiazin-2-yl)-1,3-thiazol-2-yl]méthyl}butanamide ;
- 2,6-*tert*-butyl-4-{2-[(4-propylpipérazin-1-yl)méthyl]-1,3-thiazol-4-yl}phénol ;
- 2,6-di-*tert*-butyl-4-{2-[2-méthyl-1-(méthylamino)propyl]-1,3-thiazol-4-yl}phénol ;
- *N*,2-diméthyl-1-[4-(10*H*-phénothiazin-2-yl)-1,3-thiazol-2-yl]propan-1-amine ;
- *N*-{[4-(10*H*-phénothiazin-2-yl)-1,3-thiazol-2-yl]méthyl}hexanamide ;
- (3*R*)-1-{[4-(10*H*-phénothiazin-2-yl)-1,3-thiazol-2-yl]méthyl}pyrrolidin-3-ol ;
- (3*S*)-1-{[4-(10*H*-phénothiazin-2-yl)-1,3-thiazol-2-yl]méthyl}pyrrolidin-3-ol ;
- 1-{[4-(10*H*-phénothiazin-2-yl)-1,3-thiazol-2-yl]méthyl}azétidin-3-ol;
- 2-{2-[(4-propylpipérazin-1-yl)méthyl]-1,3-thiazol-4-yl}-10*H*-phénothiazine ;
- 2-{2-[(4-acétylpipérazin-1-yl)méthyl]-1,3-thiazol-4-yl}-10*H*-phénothiazine ;
- 2-{2-[(4-butylpipérazin-1-yl)méthyl]-1,3-thiazol-4-yl}-10*H*-phénothiazine ;
- 4-{[4-(10*H*-phénothiazin-2-yl)-1,3-thiazol-2-yl]méthyl}pipérazine-1-carboxylate de méthyle ;
- 4-[2-(aminométhyl)-1H-imidazol-4-yl]-2,6-di-*tert*-butylphénol ;
- 4-{2-[(benzylammo)méthyl]-1,3-thiazol-4-yl}-2,6-di-*tert*-butylphénol ;
- 4-{2-[(4-acétylpipérazin-1-yl)méthyl]-1,3-thiazol-4-yl}-2,6-di-*tert*-butylphénol ;
- *N*-méthyl-N-{[4-(10*H*-phénoxazin-2-yl)-1,3-thiazol-2-yl]méthyl}amine ;
- 4-[2-(azétidin-1-ylméthyl)-1,3-thiazol,4-yl]-2,6-di-*tert*-butylphénol ;
- 2,6-di-*tert*-butyl-4-{2-[(4-butylpipérazin-1-yl)méthyl]-1,3-thiazol-4-yl}phénol ;
- 2-[4-(3'-chloro-1,1'-biphényl-4-yl)-1*H*-imidazol-2-yl]éthylcarbamate de butyle ;
- 2-[4-(3'-fluoro-1,1'-biphényl-4-yl)-1*H*-imidazol-2-yl]éthylcarbamate de butyle ;
- 2-[4-(4-isobutylphényl)-1*H*-imidazol-2-yl]éthylcarbamate de butyle ;
- 2-[4-(4-isobutylphényl)-1*H*-imidazol-2-yl]éthylcarbamate de benzyle ;
- 2-[4-(3'-chloro-4'-fluoro-1,1'-biphényl-4-yl)-1*H*-imidazol-2-yl]éthylcarbamate de butyle ;
- 2-[4-(3',4'-dichloro-1,1'-biphényl-4-yl)-1*H*-imidazol-2-yl]éthylcarbainate de butyle ;
- 2-[4-(4-propylphényl)-1*H*-imidazol-2-yl]éthylcarbamate de butyle ;
- 2-[4-(4-éthylphényl)-1*H*-imidazol-2-yl]éthylcarbamale de butyle ;
- 2-[4-(4'-cyano-1,1'-biphényl-4-yl)-1*H*-imidazol-2-yl]éthylcarbamate de butyle ;
- 2-[4-(1,1'-biphényl-4-yl)-5-éthyl-1*H*-imidazol-2-yl]éthylcarbamate de butyle ;
- 2-[4-(2'-chloro-1,1'-biphényl-4-yl)-1*H*-imidazol-2-yl]éthylcarbamate de butyle ;
- 2-[4-(2',3'-difluoro-1,1'-biphényl-4-yl)-1*H*-imidazol-2-yl]éthylcarbamate de butyle ;
- 2-[4-(2'-bromo-1,1'-biphényl-4-yl)-1*H*-imidazol-2-yl]éthylcarbamate de butyle ;
- 2-[4-(3',5'-difluoro-1,1'-biphényl-4-yl)-1*H*-imidazol-2-yl]éthylcarbamate de butyle ;
- 2-[4-(2'-méthoxy-1,1'-biphényl-4-yl)-1*H*-imidazol-2-yl]éthylcarbamate de butyle ;
- 2-[4-(3'-nitro-1,1'-biphényl-4-yl)-1*H*-imidazol-2-yl]éthylcarbamate de butyle ;
- 2-[4-(2',5'-difluoro-1,1'-biphényl-4-yl)-1*H*-imidazol-2-yl]éthylcarbamate de butyle ;
- 2-[4-(3'-méthoxy-1,1'-biphényl-4-yl)-1*H*-imidazol-2-yl]éthylcarbamate de butyle ;
- 4-{[4-(3,5-di-*tert*-butyl-4-hydroxyphényl)-1,3-thiazol-2-yl]méthyl}pipérazine-1-carboxylate de méthyle ;
- [4-(3,5-di-*tert*-butyl-4-hydroxyphényl)-1,3-thiazol-2-yl]méthylcarbamate de méthyle ;
- *N*-{[4-(3,5-di-*tert*-butyl-4-hydroxyphényl)-1,3-thiazol-2-yl]méthyl}benzamide ;
- *N*-{[4-(3,5-di-*tert*-butyl-4-hydroxyphényl)-1,3-thiazol-2-yl]méthyl}-2-phénylacétamide ;
- *N*-{[4-(3,5-di-*tert*-butyl-4-bydroxyphényl)-1,3-thiazol-2-yl]méthyl}propanamide ;
- acétate de 1-{[4-(3,5-di-*tert*-butyl-4-hydroxyphényl)-1,3-thiazol-2-yl]méthyl}pipéridin-4-yle ;
- 1-{[4-(3',5-di-tert-butyl-4-hydoxyphényl)-1,3-thiazol-2-yl]méthyl}pyrrolidine-3,4-diol ;
ou sel d'un tel composé.

2. A titre de médicament, un composé **caractérisé en ce qu'**il est choisi parmi les composés suivants :
- 2,6-di*tert*-butyl-4-{2-[2-(méthylamino)éthyl]-1,3-thiazol-4-yl}phénol ;
- 2,6-di*tert*-butyl-4-[4-(hydroxyméthyl)-1,3-oxazol-2-yl]phénol ;
- 2,6-di*tert*-butyl-4-{2-[1-(méthylamino)éthyl]-1,3-thiazol-4-yl}phénol ;
- 2,6-di*tert*-butyl-4-[2-(méthoxyméthyl)-1,3-thiazol-4-yl]phénol ;
- 2,6-di*tert*-butyl-4-{4-[(méthylamino)méthyl]-1,3-oxazol-2-yl}phénol ;
- N-{[4-(3,5-di*tert*-butyl-4-hydroxyphényl)-1,3-thiazol-2-yl]méthyl}acétamide ;
- [4-(3,5-di*tert*-butyl-4-hydroxyphényl)-1,3-thiazol-2-yl]méthylcarbanate d'éthyle ;
- 2,6-di*tert*-butyl-4-[2-(morpholin-4-ylméthyl)-1,3-thiazol-4-yl]phénol ;
- 2,6-di*tert*-butyl-4-[2-(thiomorpholin-4-ylméthyl)-1,3-thiazol-4-yl]phénol ;
- 4-[2-(anilinométhyl)-1,3-thiazol-4-yl]-2,6-di*tert*-butylphénol ;
- 2,6-di*tert*-butyl-4-(2-{[[2-(diméthylamino)éthyl](méthyl)amino]méthyl}-1,3-thiazol-4-yl)phénol ;
- 2,6-di*tert*-butyl-4-{5-méthyl-2-[(méthylamino)méthyl]-1,3-thinol-4-yl}phénol ;
- 1-[4-(10H-phénothiazin-2-yl)-1,3-thiazol-2-yl]méthanamine ;
- *N*-{[4-(3,5-di*tert*-butyl-4-hydroxyphényl)-1,3-thiazol-2-yl]méthyl}-*N*-méthylacétamide ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thiazol-4-yl}phénol ;
- 2,6-di*tert*-butyl-4-{2-[(4-phénylpipérazin-1-yl)méthyl]-1,3-thiazol-4-yl}phénol ;
- 2,6-di*tert*-butyl-4-{2-[(4-méthyl-1,4-diazépan-1-yl)méthyl]-1,3-thiazol-4-yl}phénol ;
- N-{1-[4-(4-anilinophényl)-1,3-thia2ol-2-yl]éthyl}-N-méthylamine ;
- 2,6-di*tert*-butyl-4-{2-[(isopropylamino)méthyl]-1,3-thiazol-4-yl}phénol ;
- 2,6-di*tert*-butyl-4-{2-[(cyclohexylamino)méthyl]-1,3-thiazol-4-yl}phénol ;
- 2,6-di*tert*-butyl-4-{2-[(4-isopropylpipérain-1-yl)méthyl]-1,3-thiazol-4-yl}phénol ;
- *N*-méthyl-1-[4-(10*H*-phénothiazin-2-yl)-1,3-thiazol-2-yl]éthanamine ;
- 2,6-di*tert*-butyl-4-{2-[(4-éthylpipérazin-1-yl)méthyl]-1,3-thiazol-4-yl}phénol ;
- *N*-{[4-(4-anilinophényl)-1,3-thiazol-2-yl]méthyl}-*N*-éthylamine ;
- *N*-{[1-(10*H*-phénothinin-2-yl)-1,3-thiazol-2-yl]méthyl}éthanamine ;
- 2,6-di*tert*-butyl-4-(2-{[4-(diméthylamino)pipéridin-1-yl]méthyl}-1,3-thiazol-4-yl)phénol ;
- 1-{[4-(3,5-di*tert*-butyl-4-hydroxyphényl)-1,3-thiazol-2-yl]méthyl}pipéridin-4-ol ;
- 2-[4-(1,1'-biphényl-4-yl)-1*H*-imidazol-2-yl]éthylcarbamate de 4-méthylpentyle ;
- 2-[4-(4-pyrrolidin-1-ylphényl)-1*H*-imidazol-2-yl]éthylcarbamate de 3,3-diméthylbutyle ;
- 2-[4-(1,1'-biphényl-4-yl)-1*H*-imidazol-2-yl]éthylcarbamate d'isopentyle ;
- 2-[4-(4'-bromo-1,1'-biphényl-4-yl)-1*H*-imidazol-2-yl]éthylcarbamate d'hexyle ;
- 2-[4-(4-*tert*-butylphényl)-1*H*-imidazol-2-yl]éthylcarbamate de benzyle ;
- 2-[4-(1,1'-biphényl-4-yl)-1*H*-imidazol-2-yl]éthylcarbamate de 3,3-diméthylbutyle ;
- 2-[4-(4-pyrrolidin-1-ylphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate de 3,3-diméthylbutyle ;
- 2-[4-(4-méthoxyphényl)-1*H*-imidazol-2-yl]éthylcarbamate de 3,3-diméthylbutyle ;
- 2-[4-(3,5-di*tert*-butyl-4-hydroxyphényl)-1*H*-imidazol-2-yl]éthylcarbamate de benzyle ;
- 2-[4-(4-pyrrolidin-1-ylphényl)-1*H*-imidazol-2-yl]éthylcarbamate de benzyle ;
- 2-[4-(1,1'-biphényl-4-yl)-1*H*-imidazol-2-yl]éthylcarbamate de 2-phényléthyle ;
- 2-[4-(4'-fluoro-1,1'-biphényl-4-yl)-1*H*-imidazol-2-yl]éthylcarbamate de butyle ;
- 2-[4-(1,1'-biphényl-4-yl)-5-méthyl-1*H*-imidazol-2-yl]éthylcarbamate de butyle ;
- 2-[4-(4'-méthyl-1,1'-biphényl-4-yl)-1*H*-imidazol-2-yl]éthylcarbamate de butyle ;
- 2-[4-(4'-chloro-1,1'-biphényl-4-yl)-1*H*-imidazol-2-yl]éthylcarbamate de butyle ;
- 2-[4-(2'-fluoro-1,1'-biphényl-4-yl)-1*H*-imidazol-2-yl]éthylcarbamate de butyle ;
- 2-[4-(2',4'-difluoro-1,1'-biphényl-4-yl)-1H-imidazol-2-yl]éthylcarbamate de butyle ;
- 2,6-di-*tert*-butyl-4-{2-[(propylamino)méthyl]-1,3-thiazol-4-yl}phénol ;
- *N*-{[4-(10*H*-phénothiazin-2-yl)-1,3-thiazol-2-yl]méthyl}-*N*-propylamine ;
- *N*-{[4-(10*H*-phénothiazin-2-yl)-1,3-thiazol-2-yl]méthyl}butan-1-amine;
- *N*-{[4-(10*H*-phénothiazin-2-yl)-1,3-thiazol-2-yl]méthyl}pentan-1-amine;
- 1-{[4-(3,5-di-*tert*-butyl-4-hydroxyphényl)-1,3-thiazol-2-yl]méthyl}pipéridin-3-ol ;
- 1-{[4-(3,5-di-*tert*-butyl-4-hydroxyphényl)-1,3-thiazol-2-yl]méthyl}pyrrolidin-3-ol ;
- [4-(10*H*-phénothiazin-2-yl)-1,3-thiazol-2-yl]méthanol ;
- *N*,*N*-diméthyl-*N*-{[4-(10*H*-phénothiazin-2-yl)-1,3-thiazol-2-yl]méthyl}amine ;
- 2-{2-[(4-méthylpipérazin-1-yl)méthyl]-1,3-thiazol-4-yl}-10*H*-phénothiazine ;
- 2-[2-(pipéridin-1-ylméthyl)-1,3-thiazol-4-yl]-10*H*-phénothiazine ;
- 2-[2-(pipérazin-1-ylméthyl)-1,3-thiazol-4-yl]-10*H*-phénothiazine ;
- 1-{[4-(3,5-di-*tert*-butyl-4-hydroxyphényl)-1,3-thiazol-2-yl]méthyl}azétidin-3-ol ;
- 2-[2-(moipholin-4-ylméthyl)-1,3-thiazol-4-yl]-14*H*-phénothiazine ;
- 2-[2-(thiomorpholin-4-ylméthyl)-1,3-thiazol-4-yl]-10*H*-phénothiazine ;
- 2-{2-[(4-méthyl-1,4-diazépan-1-yl)méthyl]-1,3-thiazol-4-yl}-10*H*-phénothiazine ;
- (3R)-1-{[4-(3,5-di-tert-butyl-4-hydroxyphényl)-1,3-thiazol-2-yl]méthyl}pyrrolidin-3-ol ;
- (3S)-1-{[4-(3,5-di-tert-butyl-4-hydroxyphényl)-1,3-thiazol-2-yl]méthyl}pyrrolidin-3-ol ;
- 2,6-di-*tert*-butyl-4-[2-(pyrrolidin-1-ylméthyl)-1,3-thiazol-4-yl]phénol ;
- 2,6-di-tert-butyl-4-{2-[(butylamino)méthyl]-1,3-thiazol-4-yl}phénol ;
- 2-{2-[(4-éthylpipérazin-1-yl)méthyl]-1,3-thiazol-4-yl}-10*H*-phénothiazine ;
- *N*-méthyl-*N*-{[4-(10*H*-phénothiazin-2-yl)-1*H*-imidazol-2-yl]méthyl}amine ;
- [4-(10*H*-phénothiazin-2-yl)-1,3-thiazol-2-yl]méthylcarbamate de méthyle ;
- [4-(10*H*-phénothiazin-2-yl)-1,3-thiazol-2-yl]méthylcarbamate de butyle ;
- *N*-néopentyl-N-{[4-(10*H*-phénothiazin-2-yl)-1,3-thiazol-2-yl]méthyl}amine ;
- 1-{[4-(10*H*-phénothiazin-2-yl)-1,3-thiazol-2-yl]méthyl}pipéridin-4-ol ;
- *N*-{[4-(10*H*-phénothiazin-2-yl)-1,3-thiazol-2-yl]méthyl}acétamide ;
- *N*-{[4-(10*H*-phénothiazin-2-yl)-1,3-thiazol-2-yl]méthyl}butanamide ;
- 2,6-di-*tert*-butyl-4-{2-[(4-propylpipérazin-1-yl)méthyl]-1,3-thiazol-4-yl}phénol ;
- 2,6-di-*tert*-butyl-4-{2-[2-méthyl-1-(méthylamino)propyl]-1,3-thiazol-4-yl}phénol ;
- *N*,2-diméthyl-1-[4-(10*H*-phénothiazin-2-yl)-1,3-thiazol-2-yl]propan-1-amine ;
- *N*-{[4-(10*H*-phénothiazin-2-yl)-1,3-thiazol-2-yl]méthyl}hexanamide ;
- (3*R*)-1-{[4-(10*H*-phénothiazin-2-yl)-1,3-thiazol-2-yl]méthyl}pyrrolidin-3-ol ;
- (3*S*)-1-{[4-(10*H*-phénothiazin-2-yl)-1,3-thiazol-2-yl]méthyl}pyrrolidin-3-ol ;
- 1-{[4-(10*H*-phénothiazin-2-yl)-1,3-thiazol-2-yl]méthyl}azétidin-3-ol ;
- 2-{2-[(4-propylpipérazin-1-yl)méthyl]-1,3-thiazol-4-yl}-10*H*-phénothiazine ;
- 2-{2-[(4-acétylpipérazin-1-yl)méthyl]-1,3-thiazol-4-yl}-10*H*-phénothiazine ;
- 2-{2-[(4-butylpipérazin-1-yl)méthyl]-1,3-thiazol-4-yl}-10*H*-phénothiazine ;
- 4-{[4-(10*H*-phénothiazin-2-yl)-1,3-thiazol-2-yl]méthyl}pipérazine-1-carboxylate de méthyle ;
- 4-[2-(aminométhyl)-1H-imidazol-4-yl]-2,6-di-*tert*-butylphénol ;
- 4-{2-[(benzylamino)méthyl]-1,3-thiazol-4-yl}-2,6-di-*tert*-butylphénol ;
- 4-{2-[(4-acétylpipérazin-1-yl)méthyl]-1,3-thiazol-4-yl}-2,6-di-*tert*-butylphénol ;
- *N*-méthyl-N-{[4-(10*H*-phénoxazin-2-yl)-1,3-thiazol-2-yl]méthyl}amine ;
- 4-[2-(azétidin-1-ylméthyl)-1,3-thiazol-4-yl]-2,6-di-*tert*-butylphénol ;
- 2,6-di-*tert*-butyl-4-{2-[(4-butylpipérazin-1-yl)méthyl]-1,3-thiazol-4-yl}phénol ;
- 2-[4-(3'-chloro-1,1'-biphényl-4-yl)-1*H*-imidazol-2-yl]éthylcarbamate de butyle ;
- 2-[4-(3'-fluoro-1,1'-biphényl-4-yl)-1*H*-imidazol-2-yl]éthylcarbamate de butyle ;
- 2-[4-(4-isobutylphényl)-1*H*-imidazol-2-yl]éthylcarbamate de butyle ;
- 2-[4-(4-isobutylphényl)-1*H*-imidazol-2-yl]éthylcarbamate de benzyle ;
- 2-[4-(3'-chloro-4'-fluorro-1,1'-biphényl-4-yl)-1*H*-imidazol-2-yl]éthylcarbamate de butyle ;
- 2-[4-(3',4'-dichloro-1,1'-biphényl-4-yl)-1*H*-imidazol-2-yl]éthylcarbamate de butyle ;
- 2-[4-(4-propylphényl)-1*H*-imidazol-2-yl]éthylcarbamate de butyle ;
- 2-[4-(4-éthylphényl)-1*H*-imidazol-2-yl]éthylcarbamate de butyle ;
- 2-[4-(4'-cyano-1,1'-biphényl-4-yl)-1*H*-imidazol-2-yl]éthylcarbamate de butyle ;
- 2-[4-(1,1'-biphényl-4-yl)-5-éthyl-1*H*-imidnol-2-yl]éthylcarbamate de butyle ;
- 2-[4-(2'-chloro-1,1'-biphényl-4-yl)-1*H*-imidazol-2-yl]éthylcarbamate de butyle ;
- 2-[4-(2',3'-difluoro-1,1'-biphényl-4-yl)-1*H*-imidazol-2-yl]éthylcarbamate de butyle ;
- 2-[4-(2'-bromo-1,1'-biphényl-4-yl)-1*H*-imidazol-2-yl]éthylcarbamate de butyle ;
- 2-[4-(3',5'-difluoro-1,1'-biphényl-4-yl)-1*H*-imidazol-2-yl]éthylcarbamate de butyle ;
- 2-[4-(2'-méthoxy-1,1'-biphényl-4-yl)-1*H*-imidazol-2-yl]éthylcarbamate de butyle ;
- 2-[4-(3'-nitro-1,1'-biphényl-4-yl)-1*H*-imidazol-2-yl]éthylcarbamate de butyle ;
- 2-[4-(2',5'-difluoro-1,1'-biphényl-4-yl)-1*H*-imidazol-2-yl]éthylcarbamate de butyle ;
- 2-[4-(3'-méthoxy-1,1'-biphényl-4-yl)-1*H*-imidazol-2-yl]éthylcarbamate de butyle ;
- 4-{[4-(3,5-di-*tert*-butyl-4-hydroxyphényl)-1,3-thiazol-2-yl]méthyl}pipérazine-1-carboxylate de méthyle ;
- [4-(3,5-di-*tert*-butyl-4-hydroxyphényl)-1,3-thiazol-2-yl]méthylcarbamate de méthyle ;
- *N*-{[4-(3,5-di-*tert*-butyl-4-hydroxyphényl)-1,3-thiazol-2-yl]méthyl}benzamide;
- *N*-{[4-(3,5-di-*tert*-butyl-4-hydroxyphényl)-1,3-thiazol-2-yl]méthyl}-2-phénylacétamide ;
- *N*-{[1-(3,5-di-*tert*-butyl-4-hydroxyphényl)-1,3-thiazol-2-yl]méthyl}propanamide ;
- acétate de 1-{[4-(3,5-di-*tert*-butyl-4-hydroxyphényl)-1,3-thiazol-2-yl]méthyl}pipéridin-4-yle ;
- 1-{[4-(3,5-di-*tert*-butyl-4-hydroxyphényl)-1,3-thiazol-2-yl]méthyl}pyrrolidine-3,4-diol ;
ou sel pharmaceutiquement acceptable d'un tel composé.

3. Composition pharmaceutique contenant, à titre de principe actif, au moins l'un des composés selon la revendication 2.

4. Utilisation d'un composé **caractérisé en ce qu'**il est choisi parmi les composés suivantes :
- 2,6-di*tert*-butyl-4-{2-[2-(méthylamino)éthyl]-1,3-thiazol-4-yl}phénol ;
- 2,6-di*tert*-butyl-4-[4-(hydroxyméthyl)-1,3-oxazol-2-yl]phénol ;
- 2,6-di*tert*-butyl-4-{2-[1-(méthylamino)éthyl]-1,3-thiazol-4-yl}phénol ;
- 2,6-di*tert*-butyl-4-[2-(méthoxyméthyl)-1,3-thiazol-4-yl]phénol ;
- 2,6-di*tert*-butyl-4-{4-[(méthylaminio)méthyl]-1,3-oxazol-2-yl}phénol ;
- N-{[4-(3,5-di*tert*-butyl-4-hydroxyphényl)-1,3-thiazol-2-yl]méthyl}acétamide ;
- [4-(3,5-di*tert*-butyl-4-hydroxyphényl)-1,3-thiazol-2-yl]méthylcarbamate d'éthyle;
- 2,6-di*tert*-butyl-4-[2-(morpholin-4-ylméthyl)-1,3-thiazol-4-yl]phénol ;
- 2,6-di*tert*-butyl-4-[2-(thiomorpholin-4-ylméthyl)-1,3-thiazol-4-yl]phénol ;
- 4-[2-(anilinométhyl)-1,3-thiazol-4-yl]-2,6-di*tert*-butylphénol ;
- 2,6-di*tert*-butyl-4-(2-{[[2-(diméthylamino)éthyl](méthyl)amino]méthyl}-1,3-thiazol-4-yl)phénol ;
- 2,6-di*tert*-butyl-4-{5-méthyl-2-[(méthylmnino)méthyl]-1,3-thinol-4-yl}phénol ;
- 1-[4-(10H-phénothiazin-2-yl)-1,3-thiazol-2-yl]méthanamine ;
- *N*-{[4-(3,5-di*tert*-butyl-4-hydroxyphényl)-1,3-thiazol-2-yl]méthyl}-*N*-méthylacétamide ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thizol-4-yl}phénol ;
- 2,6-di*tert*-butyl-4-{2-[(4-phénylpipérazin-1-yl)méthyl]-1,3-thiazol-4-yl}phénol ;
- 2,6-di*tert*-butyl-4-{2-[(4-méthyl-1,4-diazépan-1-yl)méthyl]-1,3-thiazol-4-yl}phénol ;
- N-{1-[4-(4-anilinophényl)-1,3-thiazol-2-yl]éthyl}-N-méthylamine ;
- 2,6-di*tert*-butyl-4-{2-[(isopropylamino)méthyl]-1,3-thiazol-4-yl}phénol ;
- 2,6-ditert-butyl-4-{2-[(cyclohexylamino)méthyl]-1,3-thiazol-4-yl}phénol ;
- 2,6-di*tert*-butyl-4-{2-[(4-isopropylpipérazin-1-yl)méthyl]-1,3-thiazol-4-yl}phénol ;
- *N*-méthyl-1-[4-(10*H*-phénothiazin-2-yl)-1,3-thiazol-2-yl]éthanamine ;
- 2,6-di*tert*-butyl-4-{2-[(4-éthylpipérazin-1-yl)méthyl]-1,3-thiazol-4-yl}phénol ;
- N-{[4-(4-anilinophényl)-1,3-thiazol-2-yl]méthyl}-*N*-éthylamine ;
- *N*-{[4-(10*H*-phénothiazin-2-yl)-1,3-thiazol-2-yl]méthyl}éthanamine;
- 2,6-di*tert*-butyl-4-(2-{[4-(diméthylanino)pipéridin-1-yl]méthyl}-1,3-thiazol-4-yl)phénol ;
- 1-{[4-(3,5-di*tert*-butyl-4-hydroxyphényl)-1,3-thiazol-2-yl]méthyl}pipéridin-4-ol ;
- 2-[4-(1,1'-biphényl-4-yl)-1*H*-imidazol-2-yl]éthylcarbamate de 4-méthylpentyle ;
- 2-[4-(4-pyrrolidin-1-ylphényl)-1*H*-imidazol-2-yl]éthylcarbamate de 3,3-diméthylbutyle ;
- 2-[4-(1,1'-biphényl-4-yl)-1*H*-imidazol-2-yl]éthylcarbamate d'isopentyle ;
- 2-[4-(4'-bromo-1,1'-biphényl-4-yl)-1*H*-imidazol-2-yl]éthylcarbamate d'hexyle ;
- 2-[4-(4-*tert*-butylphényl)-1*H*-imidazol-2-yl]éthylcarbamate de benzyle ;
- 2-[4-(1,1'-biphényl-4-yl)-1*H*-imidazol-2-yl]éthylcarbamate de 3,3-diméthylbutyle ;
- 2-[4-(4-pyrrolidin-1-ylphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate de 3,3-dimethylbutyle ;
- 2-[4-(4-méthoxyphényl)-1*H*-imidazol-2-yl]éthylcarbamate de 3,3-diméthylbutyle ;
- 2-[4-(3,5-di*tert*-butyl-4-hydroxyphényl)-1*H*-imidazol-2-yl]éthylcarbamate de benzyle ;
- 2-[4-(4-pyrrolidin-1-ylphényl)-1*H*-imidazol-2-yl]éthylcarbamate de benzyle ;
- 2-[4-(1,1'-biphényl-4-yl)-1*H*-imidazol-2-yl]éthylcarbamate de 2-phényléthyle ;
- 2-[4-(4'-fluoro-1,1'-biphényl-4-yl)-1*H*-imidazol-2-yl]éthylcarbamate de butyle ;
- 2-[4-(1,1'-biphényl-4-yl)-5-méthyl-1*H*-imidazol-2-yl]éthylcarbamate de butyle ;
- 2-[4-(4'-méthyl-1,1'-biphényl-4-yl)-1*H*-imidazol-2-yl]éthylcarbamate de butyle ;
- 2-[4-(4'-chloro-1,1'-biphényl-4-yl)-1*H*-imidazol-2-yl]éthylcarbamate de butyle ;
- 2-[4-(2'-fluoro-1,1'-biphényl-4-yl)-1*H*-imidazol-2-yl]éthylcarbamate de butyle ;
- 2-[4-(2',4'-difluoro-1,1'-biphényl-4-yl)-1H-imidazol-2-yl]éthylcarbamate de butyle ;
- 2,6-di-*tert*-butyl-4-{2-[(propylamino)méthyl]-1,3-thiazol-4-yl}phénol ;
- *N*-{[4-(10*H*-phénothiazin-2-yl)-1,3-thiazol-2-yl]méthyl}-*N*-propylamine ;
- *N*-{[4-(10*H*-phénothiazin-2-yl)-1,3-thiazol-2-yl]méthyl}butan-1-amine ;
- *N*-{[4-(10*H*-phénothiazin-2-yl)-1,3-thiazol-2-yl]méthyl}pentan-1-amine ;
- 1-{[4-(3,5-di-*tert*-butyl-4-hydroxyphényl)-1,3-thiazol-2-yl]méthyl}pipéridin-3-ol ;
- 1-{[4-(3,5-di-*tert*-butyl-4-hydroxyphényl)-1,3-thiazol-2-yl]méthyl}pyrrolidin-3-ol ;
- [4-(10*H*-phénothiazin-2-yl)-1,3-thiazol-2-yl]méthanol ;
- *N*,*N*-diméthyl-*N*-{[4-(10*H*-phénothiazin-2-yl)-1,3-thiazol-2-yl]méthyl}amine ;
- 2-{2-[(4-méthylpipérazin-1-yl)méthyl]-1,3-thiazol-4-yl}-10*H*-phénothiazine ;
- 2-[2-(pipéridin-1-ylméthyl)-1,3-thiazol-4-yl]-10*H*-phénothiazine ;
- 2-[2-(pipérazin-1-ylméthyl)-1,3-thiazol-4-yl]-10*H*-phénothiazine ;
- 1-{[4-(3,5-di-*tert*-butyl-4-hydxoxyphényl)-1,3-thiazol-2-yl]méthyl}azétidin-3-ol ;
- 2-[2-(morpholin-4-ylméthyl)-1,3-thiazol-4-yl]-10*H*-phénothiazine ;
- 2-[2-(thiomorpholin-4-ylméthyl)-1,3-thiazol-4-yl]-10*H*-phénothiazine ;
- 2-{2-[(4-méthyl-1,4-diazépan-1-yl)méthyl]-1,3-thiazol-4-yl}-10*H*-phénothiaine ;
- (3R)-1-{[4-(3,5-di-*tert*-butyl-4-hydroxyphényl)-1,3-thiazol-2-yl]méthyl}pyrrolidin-3-ol ;
- (3S)-1-{[4-(3,5-di-tert-butyl-4-hydroxyphényl)-1,3-thiazol-2-yl]méthyl}pyrrolidin-3-ol ;
- 2,6-di-*tert*-butyl-4-[2-(pyrrolidin-1-ylméthyl)-1,3-thiazol-4-yl]phénol ;
- 2,6-di-*tert*-butyl-4-{2-[(butylamino)méthyl]-1,3-thiazol-4-yl}phénol ;
- 2-{2-[(4-éthylpipérazin-1-yl)méthyl]-1,3-thiazol-4-yl}-10*H*-phénothiazine ;
- *N*-methyl-*N*-{[4-(10*H*-phénothiazin-2-yl)-1*H*-imidazol-2-yl]méthyl}amine ;
- [4-(10*H*-phénothiazin-2-yl)-1,3-thiazol-2-yl]méthylcarbamate de méthyle ;
- [4-(10*H*-phénothiazin-2-yl)-1,3-thiazol-2-yl]méthylcarbamate de butyle ;
- *N*-néopentyl-N-{[4-(10*H*-phénothiazin-2-yl)-1,3-thiazol-2-yl]méthyl}amine ;
- 1-{[4-(10*H*-phénothiazin-2-yl)-1,3-thiazol-2-yl]méthyl}pipéridin-4-ol ;
- *N*-{[4-(10*H*-phénothiazin-2-yl)-1,3-thiazol-2-yl]méthyl}acétamide ;
- *N*-{[4-(10*H*-phénothiazin-2-yl)-1,3-thiazol-2-yl]méthyl}butanamide ;
- 2,6-di-*tert*-butyl-4-{2-[(4-propylpipérazin-1-yl)méthyl]-1,3-thiazol-4-yl}phénol ;
- 2,6-di-*tert*-butyl-4-{2-[2-méthyl-1-(méthylamino)propyl]-1,3-thiazol-4-yl}phénol ;
- *N*,2-diméthyl-1-[4-(10*H*-phénothiazin-2-yl)-1,3-thiazol-2-yl]propan-1-amine ;
- *N*-{[4-(10*H*-phénothiazin-2-yl)-1,3-thiazol-2-yl]méthyl}hexanamide ;
- (3*R*)-1-{[4-(10*H*-phénothiazin-2-yl)-1,3-thiazol-2-yl]méthyl}pyrrolidin-3-ol ;
- (3*S*)-1-{[4-(10*H*-phénothitazin-2-yl)-1,3-thiazol-2-yl]méthyl}pyrrolidin-3-ol ;
- 1-{[4-(10*H*-phénothiazin-2-yl)-1,3-thiazol-2-yl]méthyl}azétidin-3-ol ;
- 2-{2-[(4-propylpipérazin-1-yl)méthyl]-1,3-thiazol-4-yl}-10*H*-phénothiazine ;
- 2-{2-[(4-acétylpipérazin-1-yl)méthyl]-1,3-thiazol-4-yl}-10*H*-phénothiazine ;
- 2-{2-[(4-butylpipérazin-1-yl)méthyl]-1,3-thiazol-4-yl}-10*H*-phénothiazine ;
- 4-{[4-(10*H*-phénothiazin-2-yl)-1,3-thiazol-2-yl]méthyl}pipérazine-1-carboxylate de méthyle ;
- 4-[2(-aminométhyl)-1H-imidazol-4-yl]-2,6-di-*tert*-butylphénol ;
- 4-{2-[(benzylamino)méthyl]-1,3-thiazol-4-yl}-2,4-di-*tert*-butylphénol ;
- 4-{2-[(4-acétylpipérazin-1-yl)méthyl]-1,3-thiazol-4-yl}-2,6-di-*tert*-butylphénol ;
- *N*-méthyl-N-{[4-(10*H*-phénoxazin-2-yl)-1,3-thiazol-2-yl]méthyl}amine ;
- 4-[2-(azétidin-1-ylméthyl)-1,3-thiazol-4-yl]-2,6-di-*tert*-butylphénol ;
- 2,6-di-*tert*-butyl-4-{2-[(4-butylpipérazin-1-yl)méthyl]-1,3-thiazol-4-yl}phénol ;
- 2-[4-(3'-chloro-1,1'-biphényl-4-yl)-1*H*-imidazol-2-yl]éthylcarbamate de butyle ;
- 2-[4-(3'-fluoro-1,1'-biphényl-4-yl)-1*H*-imidazol-2-yl]éthylcarbamate de butyle ;
- 2-[4-(4-isobutylphényl)-1*H*-imidazol-2-yl]éthylcarbamate de butyle ;
- 2-[4-(4-isobutylphényl)-1*H*-imidaxol-2-yl]éthylcarbamate de benzyle ;
- 2-[4-(3'-chloro-4'-fluoro-1,1'-biphényl-4-yl)-1*H*-imidazol-2-yl]éthylcarbamate de butyle ;
- 2-[4-(3',4'-dichloro-1,1'-biphényl-4-yl)-1*H*-imidazol-2-yl]éthylcarbamate de butyle ;
- 2-[4-(4-propylphényl)-1*H*-imidazol-2-yl]éthylcarbamate de butyle ;
- 2-[4-(4-éthylphényl)-1*H*-imidazol-2-yl]éthylcarbamate de butyle ;
- 2-[4-(4'-cyano-1,1'-biphényl-4-yl)-1*H*-imidazol-2-yl]éthylcarbamate de butyle ;
- 2-[4-(1,1'-biphényl-4-yl)-5-éthyl-1*H*-imidazol-2-yl]éthylcarbarmate de butyle ;
- 2-[4-(2'-chloro-1,1'-biphényl-4-yl)-1*H*-imidaxol-2-yl]éthylcarbamate de butyle ;
- 2-[4-(2',3'-difluoro-1,1'-biphényl-4-yl)-1*H*-imidiol-2-yl]éthylcarbamate de butyle ;
- 2-[4-(2'-bromo-1,1'-biphényl-4-yl)-1*H*-imidazol-2-yl]éthylcarbamate de butyle ;
- 2-[4-(3',5'-difluoro-1,1'-biphényl-4-yl)-1*H*-imidazol-2-yl]éthylcarbamate de butyle ;
- 2-[4-(2'-méthoxy-1,1'-biphényl-4-yl)-1*H*-imidazol-2-yl]éthylcarbamate de butyle ;
- 2-[4-(3'-nitro-1,1'-biphényl-4-yl)-1*H*-imidazol-2-yl]éthylcarbamate de butyle ;
- 2-[4-(2',5'-difluoro-1,1'-biphéthyl-4-yl)-1*H*-imidazol-2-yl]éthylcarbamate de butyle ;
- 2-[4-(3'-méthoxy-1,1'-biphényl-4-yl)-1*H*-imidazol-2-yl]éthylcarbamate de butyle ;
- 4-{[4-(3,5-di-*tert*-butyl-4-hydroxyphényl)-1,3-thiazol-2-yl]méthyl}pipérazine-1-carboxylate de méthyle ;
- [4-(2,5-di-*tert*-butyl-4-hydroxyphényl)-1,3-thiazol-2-yl]méthylcarbamate de méthyle ;
- *N*-{[4-(3,5-di-*tert*-butyl-4-hydroxyphényl)-1,3-thiazol-2-yl]méthyl}benzamide ;
- *N*-{[4-(3,5-di-*tert*-butyl-4-hydroxyphényl)-1,3-thiazol-2-yl]méthyl}-2-phénylacétamide ;
- *N*-{[4-(3,5-di-*tert*-butyl-4-hydroxyphényl)-1,3-thiazol-2-yl]méthyl}propanamide ;
- acétate de 1-{[4-(3,5-di-*tert*-butyl-4-hydroxyphényl)-1,3-thiazol-2-yl]méthyl}pipéridin-4-yle ;
- 1-{[4-(3,5-di-tert-butyl-4-hydroxyphényl)-1,3-thiazol-2-yl]méthyl}pyrrolidine-3,4-diol ;
ou d'un sel pharmaceutiquement acceptable d'un de ces derniers
pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses, la migraine ou les douleurs et en particulier les douleurs neuropathiques.

5. Utilisation d'un des composés suivants :
- 2,6-di*tert*-butyl-4-{2-[2-(méthylamino)éthyl]-1,3-thiazol-4-yl}phénol ;
- 2,6-di*tert*-butyl-4-[4-(hydroxylméthyl)-1,3-oxazol-2-yl]phénol ;
- 2,6-di*tert*-butyl-4-{2-[1-(méthylamino)éthyl]-1,3-thiazol-4-yl}phénol ;
- 2,6-di*tert*-butyl-4-[2-(méthoxyméthyl)-1,3-thiazol-4-yl]phénol ;
- 2,6-di*tert*-butyl-4-{4-[(méthylamino)méthyl]-1,3-oxazol-2-yl}phénol ;
- N-{[4-(3,5-di*tert*-butyl-4-hydroxyphényl)-1,3-thiazol-2-yl]méthyl}acétamide ;
- [4-(3,5-di*tert*-butyl-4-hydroxyphényl)-1,3-thiazol-2-yl]méthylcarbamate d'éthyle;
- 2,6-di*tert*-butyl-4-[2-(morpholin-4-ylméthyl)-1,3-thiazol-1-yl]phénol ;
- 2,6-di*tert*-butyl-4-[2-(thiomorpholin-4-ylméthyl)-1,3-thiazol-4-yl]phénol ;
- 4-[2-(anilinométhyl)-1,3-thiazol-4-yl]-2,6-di*tert*-butylphénol ;
- 2,6-di*tert*-butyl-4-(2-{[[2-(diméthylamino)éthyl](méthyl)amino]méthyl}-1,3-thiazol-4-yl)phénol ;
- 2,6-di*tert*-butyl-4-{5-méthyl-2-[(méthylamino)méthyl]-1,3-thiazol-4-yl}phénol ;
- 1-[4-(10H-phénothiazin-2-yl)-1,3-thiazol-2-yl]méthanamine ;
- *N*-{[4-(3,5-di*tert*-butyl-4-hydroxyphényl)-1,3-thiazol-2-yl]méthyl}-*N*-méthylacétamide ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thiazol-4-yl}phénol ;
- 2,6-di*tert*-butyl-4-{2-[(4-phénylpipérazin-1-yl)méthyl]-1,3-thiazol-4-yl}phénol ;
- 2,6-di*tert*-butyl-4-{2-[(4-méthyl-1,4-diazépan-1-yl)méthyl]-1,3-thiazol-4-yl}phénol ;
- N-{1-[4-(4-anilinophényl)-1,3-thiazol-2-yl]éthyl}-N-méthylamine ;
- 2,6-di*tert*-butyl-4-{2-[(isopropylamino)méthyl]-1,3-thiazol-4-yl}phénol ;
- 2,6-di*tert*-butyl-4-{2-[(cyclohexylamino)méthyl]-1,3-thiazol-4-yl}phénol ;
- 2,6-di*tert*-butyl-4-{2-[(4-isopropylpipérazin-1-yl)méthyl]-1,3-thiazol-4-yl}phénol ;
- *N*-méthyl-1-[4-(10*H*-phénothiazin-2-yl)-1,3-thiazol-2-yl]éthanamine ;
- 2,6-di*tert*-butyl-4-{2-[(4-éthylpipérazin-1-yl)méthyl]-1,3-thiazol-4-yl}phénol ;
- *N*-{[4-(4-anilinophényl)-1,3-thiazol-2-yl]méthyl}-*N*-éthylamine ;
- *N*-{[4-(10*H*-phénothiazin-2-yl)-1,3-thiazol-2-yl]méthyl}éthazine ;
- 2,6-di*tert*-butyl-4-(2-{[4-(diméthylamino)pipéridin-1-yl]méthyl}-1,3-thiazol-4-yl)phénol ;
- 1-{[4-(3,5-di*tert*-butyl-4-hydroxyphényl)-1,3-thiazol-2-yl]méthyl}pipéridin-4-ol ;
- 2,6-di-*tert*-butyl-4-{2-[(propylamino)méthyl]-1,3-thiazol-4-yl}phénol ;
- *N*-{[4-(10*H*-phénothiazin-2-yl)-1,3-thiazol-2-yl]méthyl}-*N*-propylamine ;
- N-{[4-(10*H*-phénothiazin-2-yl)-1,3-thiazol-2-yl]méthyl}butan-1-amine ;
- *N*-{[4-(10*H*-phénothiazin-2-yl)-1,3-thiazol-2-yl]méthyl}pentan-1-amine ;
- 1-{[4-(3,5-di-*tert*-butyl-4-hydroxyphényl)-1,3-thiazol-2-yl]méthyl}pipéridin-3-ol ;
- 1-{[4-(3,5-di-*tert*-butyl-4-hydroxyphényl)-1,3-thiazol-2-yl]méthyl}pyrrolidin-3-ol ;
- [4-(10*H*-phénothiazin-2-yl)-1,3-thiazol-2-yl]méthanol ;
- *N*,*N*-diméthyl-*N*-{[4-(10*H*-phénothiazin-2-yl)-1,3-thiazol-2-yl]méthyl}amine ;
- 2-{2-[(4-méthylpipérazin-1-yl)méthyl]-1,3-thiazol-4-yl}-10*H*-phénothiazine ;
- 2-[2-(pipéridin-1-ylméthyl)-1,3-thiazol-4-yl]-10*H*-phénothiazine ;
- 2-[2-(piperazin-1-ylméthyl)-1,3-thiazol-4-yl]-10*H*-phénothiazine ;
- 1-{[4-(3,5-di-*tert*-butyl-4-hydroxyphényl)-1,3-thiazol-2-yl]méthyl}azétidin-3-ol ;
- 2-[2-(morpholin-4-ylméthyl)-1,3-thiazol-4-yl]-10*H*-phénothiazine ;
- 2-[2-(thiomorpholin-4-ylméthyl)-1,3-thiazol-4-yl]-10*H*-phénothiazine ;
- 2-{2-[(4-méthyl-1,4-diazépan-1-yl)méthyl]-1,3-thiazol-4-yl}-10*H*-phénothiazine ;
- (3R)-1-{[4-(3,5-di-tert-butyl-4-hydroxyphényl)-1,3-thiazol-2-yl]méthyl}pyrrolidin-3-ol ;
- (3S)-1-{[4-(3,5-di-tert-butyl-4-hydroxyphényl)-1,3-thiazol-2-yl]méthyl}pyrrolidin-3-ol ;
- 2,6-di-*tert*-butyl-4-[2-(pyrirolidin-1-ylméthyl)-1,3-thiazol-4-yl]phénol ;
- 2,6-di-tert-butyl-4-{2-[(butylamino)méthyl]-1,3-thiazol-4-yl}phénol ;
- 2-{2-[(4-éthylpipérazin-1-yl)méthyl]-1,3-thiazol-4-yl}-10*H*-phénothiazine ;
- *N*-méthyl-*N*-{[4-(10*H*-phénothiazin-2-yl)-1*H*-imidazol-2-yl]méthyl}amine ;
- [4-(10*H*-phénothiazin-2-yl)-1,3-thiazol-2-yl]méthylcarbamate de méthyle ;
- [4-(10*H*-phénothiazin-2-yl)-1,3-thiazol-2-yl]méthylearbamate de butyle ;
- *N*-néopentyl-N-{[4-(10*H*-phénothiazin-2-yl)-1,3-thiazol-2-yl]méthyl}amine ;
- 1-{[4-(10*H*-phénothiazin-2-yl)-1,3-thiazol-2-yl]méthyl}pipéridin-4-ol ;
- *N*-{[4-(10*H*-phénothiazin-2-yl)-1,3-thiazol-2-yl]méthyl}acétamide ;
- *N*-{[4-(10*H*-phénothiazin-2-yl)-1,3-thiazol-2-yl]méthyl}butanamide ;
- 2,6-di-*tert*-butyl-4-{2-[(4-propylpipérazin-1-yl)méthyl]-1,3-thiazol-4-yl}phénol ;
- 2,6-di-*tert*-butyl-4-{2-[2-méthyl-1-(méthylamino)propyl]-1,3-thiazol-4-yl}phénol ;
- *N*,2-diméthyl-1-[4-(10*H*-phénothiazin-2-yl)-1,3-thiazol-2-yl]propan-1-amine ;
- *N*-{[4-(10*H*-phénothiazin-2-yl)-1,3-thiazol-2-yl]méthyl}hexanamide ;
- (3*R*)-1-{[4-(10*H*-phénothiazin-2-yl)-1,3-thiazol-2-yl]méthyl}pyrrolidin-3-ol ;
- (3*S*)-1-{[4-(10*H*-phénothiazin-2-yl)-1,3-thiazol-2-yl]méthyl}pyrrolidin-3-ol ;
- 1-{[4-(10*H*-phénothiazin-2-yl)-1,3-thiazol-2-yl]méthyl}azétidin-3-ol ;
- 2-{2-[(4-propylpipérazin-1-yl)méthyl]-1,3-thiazol-4-yl}-10*H*-phénothiazine ;
- 2-{2-[(4-acétylpipérazin-1-yl)méthyl]-1,3-thiazol-4-yl}-10*H*-phénothiazine ;
- 2-{2-[(4-butylpipérazin-1-yl)méthyl]-1,3-thiazol-4-yl}-10*H*-phénothiazine ;
- 4-{[4-(10*H*-phénothiazin-2-yl)-1,3-thiazol-2-yl]méthyl}pipérazine-1-carboxylate de méthyle ;
- 4-[2-(aminométhyl)-1H-imidazol-4-yl]-2,6-di-*tert*-butylphénol ;
- 4-{2-[(benzylamino)méthyl]-1,3-thiazol-4-yl}-2,6-di-*tert*-butylphénol ;
- 4-{2-[(4-acétylpipérazin-1-yl)méthyl]-1,3-thiazol-4-yl}-2,6-di-*tert*-butylphénol ;
- *N*-méthyl-N-{[4-(10*H*-phénoxazin-2-yl)-1,3-thiazol-2-yl]méthyl}amine ;
- 4-[2-(azétidin-1-ylméthyl)-1,3-thiazol-4-yl]-2,6-di-*tert*-butylphénol ;
- 2,6-di-*tert*-butyl-4-{2-[(4-butylpipérazin-1-yl)méthyl]-1,3-thiazol-4-yl}phénol ;
- 4-{[4-(3,5,di-*tert*-butyl-4-hydroxyphényl)-1,3-thiazol-2-yl]méthyl}pipérazine-1-carboxylate de méthyle ;
- [4-(3,5-di-*tert*-butyl-4-hydroxyphényl)-1,3-thiazol-2-yl]méthylcarbamate de méthyle;
- *N*-{[4-(3,5-di-*tert*-butyl-4-hydroxyphényl)-1,3-thiazol-2-yl]méthyl}benzamide ;
- *N*-{[4-(3,5-di-*tert*-butyl-4-hydroxyphényl)-1,3-thiazol-2-yl]méthyl}-2-phénylacétamide ;
- *N*-{[4-(3,5-di-*tert*-butyl-4-hydroxyphényl)-1,3-thiazol-2-yl]méthyl}propanamide ;
- acétate de 1-{[4-(3,5-di-*tert*-butyl-4-hydroxyphényl)-1,3-thiazol-2-yl]méthyl}pipéridin-4-yle ;
- 1-{[4-(3,5-di-*tert*-butyl-4-hydroxyphényl)-1,3-thiazol-2-yl]méthyl}pyrolidine-3,4-diol ;
ou d'un sel pharmaceutiquement acceptable d'un de ces derniers
pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions et les psychoses.

6. Utilisation selon la revendication 5, **caractérisée en ce que** le composé utilisé est choisi parmi l'un des composés suivants :
- 2,6-di*tert*-butyl-4-{2-[1-(méthylamino)éthyl]-1,3-thiazol-4-yl}phénol ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thiazol-4-yl}phénol ;
- 1-{[4-(3,5-di*tert*-butyl-4-hydroxyphényl)-1,3-thiazol-2-yl]méthyl}pipéridin-4-ol ;
- 1-{[4-(3,5-di-*tert*-butyl-4-hydroxyphényl)-1,3-thiazol-2-yl]méthyl}pyrrolidin-3-ol ;
- 1-{[4-(3,5-di-*tert*-butyl-4-hydroxyphényl)-1,3-thiazol-2-yl]méthyl}azétidin-3-ol ;
- (3*R*)-1-{[4-(3,5-di-tert-butyl-4-hydroxyphényl)-1,3-thiazol-2-yl]méthyl}pyrrolidin-3-ol ;
- (3*S*)-1-{[4-(3,5-di-tert-butyl-4-hydroxyphényl)-1,3-thiazol-2-yl]méthyl}pyrrolidin-3-ol ;
- *N*,2-diméthyl-1-[4-(10*H*-phénothiazin-2-yl)-1,3-thiazol-2-yl]propan-1-amine ;
et les sels pharmaceutiquement acceptables de ces derniers.

7. Utilisation d'un des composés suivants
- 2,6-di*tert*-butyl-4-{2-[2-(méthylamino)éthyl]-1,3-thiazol-4-yl}phénol ;
- 2,6-di*tert*-butyl-4-{2-[1-(méthylamino)éthyl]-1,3-thlazol-4-yl}phénol ;
- 2,6-di*tert*-butyl-4-{4-[(méthylamino)méthyl]-1,3-oxazol-2-yl}phénol ;
- 2,6-di*tert*-butyl-4-{5-méthyl-2-[(méthylamino)nléthyl]-1,3-thiazol-4-yl}phénol ;
- 1-[4-(3,5-di*tert*-butyl-4-méthoxyphényl)-1,3-thiazol-2-yl]-N-méthylméthanamine ;
- 2,6-di*tert*-butyl-4-{2-[(éthylamino)méthyl]-1,3-thiazol-4-yl}phénol ;
- 2-[4-(1,1'-biphényl-4-yl)-1*H*-imidazol-2-yl]éthylcarbamate de 4-méthylpentyle ;
- 2-[4-(4-pyrrolidin-1-ylphényl)-1*H*-imidazol-2-yl]éthylcarbamate de 3,3-diméthylbutyle ;
- 2-[4-(1,1'-biphényl-4-yl)-1*H*-imidazol-2-yl]éthylcarbamate d'isopentyle ;
- 2-[4-(4'-bromo-1,1'-biphényl-4-yl)-1*H*-imidazol-2-yl]éthylcarbamate d'hexyle ;
- 2-[4-(4-*tert*-butylphényl)-1*H*-imidazol-2-yl]éthylcarbamate de benzyle ;
- 2-[4-(1,1'-biphényl-4-yl)-1*H*-imidazol-2-yl]éthylcarbamate de 3,3-diméthylbutyle ;
- 2-[4-(4-pyrrolidin-1-ylphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate d'hexyle ;
- 2-[4-(3,5-di*tert*-butyl-4-hydroxyphényl)-1*H*-imidazol-2-yl]éthylcarbamate d'hexyle ;
- 2-[4-(4-méthoxyphényl)-1*H*-imidazol-2-yl]éthylcarbamate de 3,3-diméthylbutyle ;
- 2-[4-(3,5-di*tert*-butyl-4-hydroxyphényl)-1*H*-imidazol-2-yl]éthylcarbamate de benzyle ;
- 2-[4-(4-pyrrolidin-1-ylphényl)-1*H*-imidazol-2-yl]éthylcarbamate de benzoyle ;
- 2-[4-(1,1'-biphényl-4-yl)-1*H*-imidazol-2-yl]éthylcarbamate de 2-phényléthyle ;
- 2-[4-(4'-fluoro-1,1'-biphényl-4-yl)-1*H*-imidazol-2-yl]éthylcarbamate de butyle ;
- 2-[4-(1,1'-biphényl-4-yl)-5-méthyl-1*H*-imidazol-2-yl]éthylcarbamate de butyle ;
- 2-[4-(4'-méthyl-1,1'-biphényl-4-yl)-1*H*-imidazol-2-yl]éthylcarbamate de butyle ;
- 2-[4-(4'-chloro-1,1'-biphényl-4-yl)-1*H*-imidazol-2-yl]éthylcarbamate de butyle ;
- 2-[4-(2'-fluoro-1,1'-biphényl-4-yl)-1*H*-imidazol-2-yl]éthylcarbamate de butyle ;
- 2-[4-(2',4'-difluoro-1,1'-biphényl-4-yl)-1H-imidazol-2-yl]éthylcarbamate de butyle ;
- 2-[4-(3'-chloro-1,1'-biphényl-4-yl)-1*H*-imidazol-2-yl]éthylcarbamate de butyle ;
- 2-[4-(3'-fluoro-1,1'-biphényl-4-yl)-1*H*-imidazol-2-yl]éthylcarbamate de butyle ;
- 2-[4-(4-isobutylphényl)-1*H*-imidazol-2-yl]éthylcarbamate de butyle ;
- 2-[4-(4-isobutylphényl)-1*H*-imidazol-2-yl]éthylcarbamate de benzyle ;
- 2-[4-(3'-chloro-4'-fluoro-1,1'-biphényl-4-yl)-1*H*-imidazol-2-yl]éthylcarbamate de butyle ;
- 2-[4-(3',4'-dichloro-1,1'-biphényl-4-yl)-1*H*-imidazol-2-yl]éthylcarbamate de butyle ;
- 2-[4-(4-propylphényl)-1*H*-imidazol-2-yl]éthylcarbamate de butyle ;
- 2-[4-(4-éthylphényl)-1*H*-imidazol-2-yl]éthylcarbamate de butyle ;
- 2-[4-(4'-cyano-1,1'-biphényl-4-yl)-1*H*-imidazol-2-yl]éthylcarbamate de butyle ;
- 2-[4-(1,1'-biphényl-4-yl)-5-éthyl-1*H*-imidazol-2-yl]éthylcarbamate de butyle ;
- 2-[4'-(2'-chloro-1,1'-biphényl-4-yl)-1*H*-imidazol-2-yl]éthylcarbamate de butyle ;
- 2-[4-(2',3'-difluoro-1,1'-biphényl-4-yl)-1*H*-imidazol-2-yl]éthylcarbamate de butyle ;
- 2-[4-(2'-bromo-1,1'-biphényl-4-yl)-1*H*-imidazol-2-yl]éthylcarbamate de butyle ;
- 2-[4-(3',5'-difluoro-1,1'-biphényl-4-yl)-1*H*-imidazol-2-yl]éthylcarbamate de butyle ;
- 2-[4-(2'-méthoxy-1,1'-biphényl-4-yl)-1*H*-imidazol-2-yl]éthylcarbamate de butyle ;
- 2-[4-(3'-nitro-1,1'-biphényl-4-yl)-1*H*-imidazol-2-yl]éthylcarbamate de butyle ;
- 2-[4-(2',5'-difluoro-1,1'-biphényl-4-yl)-1*H*-imidazol-2-yl]éthylcarbamate de butyle ;
- 2-[4-(3'-méthoxy-1,1'-biphényl-4-yl)-1*H*-imidazol-2-yl]éthylcarbamate de butyle ;
ou d'un sel pharmaceutiquement acceptable d'un de ces derniers,
pour préparer un médicament destiné à traiter l'un des désordres ou l'une des maladies suivantes ; la maladie de Parkinson, la maladie d'Alzheimer, la sclérose latérale amyotrophique, la migraine ou les douleurs et en particulier les douleurs neuropathiques.

## Claims

1. Compound **characterized in that** it is chosen from the following compounds:
- 2,6-di*tert*-butyl-4-{2-[2-(methylamino)ethyl]-1,3-thiazol-4-yl}phenol;
- 2,6-di*tert*-butyl-4-[4-(hydroxymethyl)-1,3-oxazol-2-yl]phenol;
- 2,6-di*tert*-butyl-4-{2-[1-(methylamino)ethyl]-1,3-thiazol-4-yl}phenol;
- 2,6-di*tert*-butyl-4-[2-(methoxymethyl)-1,3-thiazol-4-yl]phenol;
- 2,6-di*tert*-butyl-4-{4-[(methylamino)methyl]-1,3-oxazol-2-yl}phenol;
- N-{[4-(3,5-di*tert*-butyl-4-hydroxyphenyl)-1,3-thiazol-2-yl]methyl}acetamide;
- ethyl [4-(3,5-di*tert*-butyl-4-hydroxyphenyl)-1,3-thiazol-2-yl]methylcarbamate;
- 2,6-ditert-butyl-4-[2-(morpholin-4-ylmethyl)-1,3-thiazol-4-yl]phenol;
- 2,6-di*tert*-butyl-4-[2-(thiomorpholin-4-ylmethyl)-1,3-thiazol-4-yl]phenol;
- 4-[2-(anilinomethyl)-1,3-thiazol-4-yl]-2,6-ditert-butylphenol;
- 2,6-di*tert*-butyl-4-(2-{[[2-(dimethylamino)ethyl](methyl)amino]methyl}-1,3-thiazol-4-yl)phenol;
- 2,6-di*tert*-butyl-4-{5-methyl-2-[(methylamino)methyl]-1,3-thiazol-4-yl}phenol;
- 1-[4-(10*H*-phenothiazin-2-yl)-1,3-thiazol-2-yl]methanamine;
- *N*-{[4-(3,5-di*tert*-butyl-4-hydroxyphenyl)-1,3-thiazol-2-yl]methyl}-*N*-methylacetamide;
- 1-[4-(3,5-ditert-butyl-4-methoxyphenyl)-1,3-thiazol-2-yl]-N-methylmethanamine;
- 2,6-di*tert*-butyl-4-{2-[(ethylamino)methyl]-1,3-thiazol-4-yl}phenol;
- 2,6-di*tert*-butyl-4-{2-[(4-phenylpiperazin-1-yl)methyl]-1,3-thiazol-4-yl}phenol;
- 2,6-di*tert*-butyl-4-{2-[(4-methyl-1,4-diazepan-1-yl)methyl]-1,3-thiazol-4-yl}phenol;
- N-{1-[4-(4-anilinophenyl)-1,3-thiazol-2-yl]ethyl}-N-methylamine;
- 2,6-di*tert*-butyl-4-{2-[(isopropylamino)methyl]-1,3-thiazol-4-yl}phenol;
- 2,6-ditert-butyl-4-{2-[(cyclohexylamino)methyl]-1,3-thiazol-4-yl}phenol;
- 2,6-di*tert*-butyl-4-{2-[(4-isopropylpiperazin-1-yl)methyl]-1,3-thiazol-4-yl}phenol;
- *N*-methyl-1-[4-(10*H*-phenothiazin-2-yl)-1,3-thiazol-2-yl]ethanamine;
- 2,6-di*tert*-butyl-4-{2-[(4-ethylpiperazin-1-yl)methyl]-1,3-thiazol-4-yl}phenol;
- *N*-{[4-(4-anilinophenyl)-1,3-thiazol-2-yl]methyl}-*N*-ethylamine;
- *N*-{[4-(10*H*-phenothiazin-2-yl)-1,3-thiazol-2-yl]methyl}ethanamine;
- 2,6-di*tert*-butyl-4-(2-{[4-(dimethylamino)piperidin-1-yl]methyl}-1,3-thiazol-4-yl)phenol;
- 1-{[4-(3,5-di*tert*-butyl-4-hydroxyphenyl)-1,3-thiazol-2-yl]methyl}piperidin-4-ol;
- 4-methylpentyl 2-[4-(1,1'-biphenyl-4-yl)-1*H*-imidazol-2-yl]ethylcarbamate;
- 3,3-dimethylbutyl 2-[4-(4-pyrrolidin-1-ylphenyl)-1*H*-imidazol-2-yl]ethylcarbamate;
- isopentyl 2-[4-(1,1'-biphenyl-4-yl)-1*H*-imidazol-2-yl]ethylcarbamate;
- hexyl 2-[4-(4'-bromo-1,1'-biphenyl-4-yl)-1*H*-imidazol-2-yl]ethylcarbamate;
- benzyl 2-[4-(4-*tert*-butylphenyl)-1*H*-imidazol-2-yl]ethylcarbamate;
- 3,3-dimethylbutyl 2-[4-(1,1'-biphenyl-4-yl)-1*H*-imidazol-2-yl]ethylcarbamate;
- hexyl 2-[4-(4-pyrrolidin-1-ylphenyl)-1*H*-imidazol-2-yl]ethylcarbamate;
- hexyl 2-[4-(3,5-di*tert*-butyl-4-hydroxyphenyl)-1*H*-imidazol-2-yl]ethylcarbamate;
- 3,3-dimethylbutyl 2-[4-(3,5-di*tert*-butyl-4-hydroxyphenyl)-1*H*-imidazol-2-yl] ethylcarbamate;
- 3,3-dimethylbutyl 2-[4-(4-methoxyphenyl)-1*H*-imidazol-2-yl]ethylcarbamate;
- benzyl 2-[4-(3,5-di*tert*-butyl-4-hydroxyphenyl)-1*H*-imidazol-2-yl]ethylcarbamate;
- benzyl 2-[4-(4-pyrrolidin-1-ylphenyl)-1*H*-imidazol-2-yl]ethylcarbamate;
- 2-phenylethyl 2-[4-(1,1'-biphenyl-4-yl)-1*H*-imidazol-2-yl]ethylcarbamate;
- butyl 2-[4-(4'-fluoro-1,1'-biphenyl-4-yl)-1*H*-imidazol-2-yl]ethylcarbamate;
- butyl 2-[4-(1,1'-biphenyl-4-yl)-5-methyl-1*H*-imidazol-2-yl]ethylcarbamate;
- butyl 2-[4-(4'-methyl-1,1'-biphenyl-4-yl)-1*H*-imidazol-2-yl]ethylcarbamate;
- butyl 2-[4-(4'-chloro-1,1'-biphenyl-4-yl)-1*H*-imidazol-2-yl]ethylcarbamate;
- butyl 2-[4-(2'-fluoro-1,1'-biphenyl-4-yl)-1*H*-imidazol-2-yl]ethylcarbamate;
- butyl 2-[4-(2',4'-difluoro-1,1'-biphenyl-4-yl)-1H-imidazol-2-yl]ethylcarbamate;
- 2,6-di-*tert*-butyl-4-{2-[(propylamino)methyl]-1,3-thiazol-4-yl}phenol;
- *N*-{[4-(10*H*-phenothiazin-2-yl)-1,3-thiazol-2-yl]methyl}-*N*-propylamine;
- *N*-{[4-(10*H*-phenothiazin-2-yl)-1,3-thiazol-2-yl]methyl}butan-1-amine;
- *N*-{[4-(10*H*-phenothiazin-2-yl)-1,3-thiazol-2-yl]methyl}pentan-1-amine;
- 1-{[4-(3,5-di-*tert*-butyl-4-hydroxyphenyl)-1,3-thiazol-2-yl]methyl}piperidin-3-ol;
- 1-{[4-(3,5-di-*tert*-butyl-4-hydroxyphenyl)-1,3-thiazol-2-yl]methyl}pyrrolidin-3-ol;
- [4-(10*H*-phenothiazin-2-yl)-1,3-thiazol-2-yl]methanol;
- *N*,*N*-dimethyl-*N*-{[4-(10*H*-phenothiazin-2-yl)-1,3-thiazol-2-yl]methyl}amine;
- 2-{2-[(4-methylpiperazin-1-yl)methyl]-1,3-thiazol-4-yl}-10*H*-phenothiazine;
- 2-[2-(piperidin-1-ylmethyl)-1,3-thiazol-4-yl]-10*H*-phenothiazine;
- 2-[2-(piperazin-1-ylmethyl)-1,3-thiazol-4-yl]-10*H*-phenothiazine;
- 1-{[4-(3,5-di-*tert*-butyl-4-hydroxyphenyl)-1,3-thiazol-2-yl]methyl}azetidin-3-ol;
- 2-[2-(morpholin-4-ylmethyl)-1,3-thiazol-4-yl]-10*H*-phenothiazine;
- 2-[2-(thiomorpholin-4-ylmethyl)-1,3-thiazol-4-yl]-10*H*-phenothiazine;
- 2-{2-[(4-methyl-1,4-diazepan-1-yl)methyl]-1,3-thiazol-4-yl}-10*H*-phenothiazine;
- (3*R*)-1-{[4-(3,5-di-*tert*-butyl-4-hydroxyphenyl)-1,3-thiazol-2-yl]methyl}pyrrolidin-3-ol;
- (3*S*)-1-{[4-(3,5-di-tert-butyl-4-hydroxyphenyl)-1,3-thiazol-2-yl]methyl}pyrrolidin-3-ol;
- 2,6-di-*tert*-butyl-4-[2-(pyrrolidin-1-ylmethyl)-1,3-thiazol-4-yl]phenol;
- 2,6-di-tert-butyl-4-{2-[(butylamino)methyl]-1,3-thiazol-4-yl}phenol;
- 2-{2-[(4-ethylpiperazin-1-yl)methyl]-1,3-thiazol-4-yl}-10*H*-phenothiazine;
- *N*-methyl-*N*-{[4-(10*H*-phenothiazin-2-yl)-1*H*-imidazol-2-yl]methyl}amine;
- methyl [4-(10*H*-phenothiazin-2-yl)-1,3-thiazol-2-yl]methylcarbamate;
- butyl [4-(10*H*-phenothiazin-2-yl)-1,3-thiazol-2-yl]methylcarbamate;
- *N*-neopentyl-N-{[4-(10*H*-phenothiazin-2-yl)-1,3-thiazol-2-yl]methyl}amine;
- 1-{[4-(10*H*-phenothiazin-2-yl)-1,3-thiazol-2-yl]methyl}piperidin-4-ol;
- *N*-{[4-(10*H*-phenothiazin-2-yl)-1,3-thiazol-2-yl]methyl}acetamide;
- *N*-{[4-(10*H*-phenothiazin-2-yl)-1,3-thiazol-2-yl]methyl}butanamide;
- 2,6-di-*tert*-butyl-4-{2-[(4-propylpiperazin-1-yl)methyl]-1,3-thiazol-4-yl}phenol;
- 2,6-di-*tert*-butyl-4-{2-[2-methyl-1-(methylamino)propyl]-1,3-thiazol-4-yl}phenol;
- *N*,2-dimethyl-1-[4-(10*H*-phenothiazin-2-yl)-1,3-thiazol-2-yl]propan-1-amine;
- *N*-{[4-(10*H*-phenothiazin-2-yl)-1,3-thiazol-2-yl]methyl}hexanamide;
- (3*R*)-1-{[4-(10*H*-phenothiazin-2-yl)-1,3-thiazol-2-yl]methyl}pyrrolidin-3-ol;
- (3*S*)-1-{[4-(10*H*-phenothiazin-2-yl)-1,3-thiazol-2-yl]methyl}pyrrolidin-3-ol;
- 1-{[4-(10*H*-phenothiazin-2-yl)-1,3-thiazol-2-yl]methyl}azetidin-3-ol;
- 2-{2-[(4-propylpiperazin-1-yl)methyl]-1,3-thiazol-4-yl}-10*H*-phenothiazine;
- 2-{2-[(4-acetylpiperazin-1-yl)methyl]-1,3-thiazol-4-yl}-10*H*-phenothiazine;
- 2-{2-[(4-butylpiperazin-1-yl)methyl]-1,3-thiazol-4-yl}-10*H*-phenothiazine;
- methyl 4-{[4-(10*H*-phenothiazin-2-yl)-1,3-thiazol-2-yl]methyl}piperazine-1-carboxylate;
- 4-[2-(aminomethyl)-1H-imidazol-4-yl]-2,6-di-*tert*-butylphenol;
- 4-{2-[(benzylamino)methyl]-1,3-thiazol-4-yl}-2,6-di-*tert*-butylphenol;
- 4-{2-[(4-acetylpiperazin-1-yl)methyl]-1,3-thiazol-4-yl}-2,6-di-*tert*-butylphenol;
- *N*-methyl-*N*-{[4-(10*H*-phenoxazin-2-yl)-1,3-thiazol-2-yl]methyl}amine;
- 4-[2-(azetidin-1-ylmethyl)-1,3-thiazol-4-yl]-2,6-di-*tert*-butylphenol;
- 2,6-di-*tert*-butyl-4-{2-[(4-butylpiperazin-1-yl)methyl]-1,3-thiazol-4-yl}phenol;
- butyl 2-[4-(3'-chloro-1,1'-biphenyl-4-yl)-1*H*-imidazol-2-yl]ethylcarbamate;
- butyl 2-[4-(3'-fluoro-1,1'-biphenyl-4-yl)-1*H*-imidazol-2-yl]ethylcarbamate;
- butyl 2-[4-(4-isobutylphenyl)-1*H*-imidazol-2-yl]ethylcarbamate;
- benzyl 2-[4-(4-isobutylphenyl)-1*H*-imidazol-2-yl]ethylcarbamate;
- butyl 2-[4-(3'-chloro-4'-fluoro-1,1'-biphenyl-4-yl)-1*H*-imidazol-2-yl]ethylcarbamate;
- butyl 2-[4-(3',4'-dichloro-1,1'-biphenyl-4-yl)-1*H*-imidazol-2-yl]ethylcarbamate;
- butyl 2-[4-(4-propylphenyl)-1*H*-imidazol-2-yl]ethylcarbamate;
- butyl 2-[4-(4-ethylphenyl)-1*H*-imidazol-2-yl]ethylcarbamate;
- butyl 2-[4-(4'-cyano-1,1'-biphenyl-4-yl)-1*H*-imidazol-2-yl]ethylcarbamate;
- butyl 2-[4-(1,1'-biphenyl-4-yl)-5-ethyl-1*H*-imidazol-2-yl]ethylcarbamate;
- butyl 2-[4-(2'-chloro-1,1'-biphenyl-4-yl)-1*H*-imidazol-2-yl]ethylcarbamate;
- butyl 2-[4-(2',3'-difluoro-1,1'-biphenyl-4-yl)-1*H*-imidazol-2-yl]ethylcarbamate;
- butyl 2-[4-(2'-bromo-1,1'-biphenyl-4-yl)-1*H*-imidazol-2-yl]ethylcarbamate;
- butyl 2-[4-(3',5'-difluoro-1,1'-biphenyl-4-yl)-1*H*-imidazol-2-yl]ethylcarbamate;
- butyl 2-[4-(2'-methoxy-1,1'-biphenyl-4-yl)-1*H*-imidazol-2-yl]ethylcarbamate;
- butyl 2-[4-(3'-nitro-1,1'-biphenyl-4-yl)-1*H*-imidazol-2-yl]ethylcarbamate;
- butyl 2-[4-(2',5'-difluoro-1,1'-biphenyl-4-yl)-1*H*-imidazol-2-yl]ethylcarbamate;
- butyl 2-[4-(3'-methoxy-1,1'-biphenyl-4-yl)-1*H*-imidazol-2-yl]ethylearbainate;
- methyl 4-{[4-(3,5-di-tert-butyl-4-hydroxyphenyl)-1,3-thiazol-2-yl]methyl}piperazine-1-carboxylate;
- methyl [4-(3,5-di-tert-butyl-4-hydroxyphenyl)-1,3-thiazol-2-yl]methylcarbamate;
- *N*-{[4-(3,5-di-*tert*-butyl-4-hydroxyphenyl)-1,3-thiazol-2-yl]methyl}benzamide;
- *N*-{[4-(3,5-di-*tert*-butyl-4-hydroxyphenyl)-1,3-thiazol-2-yl]methyl}-2-phenylacetamide;
- *N*-{[4-(3,5-di-*tert*-butyl-4-hydroxyphenyl)-1,3-thiazol-2-yl]methyl}propanamide;
- 1-{[4-(3,5-di-tert-butyl-4-hydroxyphenyl)-1,3-thiazol-2-yl]methyl}piperidin-4-yl acetate;
- 1-{[4-(3,5-di-tert-butyl-4-hydroxyphenyl)-1,3-thiazol-2-yl]methyl}pyrrolidine-3,4-diol;
or salt of such a compound.

2. As a medicament, a compound **characterized in that** it is chosen from the following compounds:
- 2,6-di*tert*-butyl-4-{2-[2-(methylamino)ethyl]-1,3-thiazol-4-yl}phenol;
- 2,6-ditert-butyl-4-[4-(hydroxymethyl)-1,3-oxazol-2-yl]phenol;
- 2,6-di*tert*-butyl-4-{2-[1-(methylamino)ethyl]-1,3-thiazol-4-yl}phenol;
- 2,6-di*tert*-butyl-4-[2-(methoxymethyl)-1,3-thiazol-4-yl]phenol;
- 2,6-di*tert*-butyl-4-{4-[(methylamino)methyl]-1,3-oxazol-2-yl}phenol;
- N-{[4-(3,5-di*tert*-butyl-4-hydroxyphenyl)-1,3-thiazol-2-yl]methyl}acetamide;
- ethyl [4-(3,5-di*tert*-butyl-4-hydroxyphenyl)-1,3-thiazol-2-yl]methylcarbamate;
- 2,6-ditert-butyl-4-[2-(morpholin-4-ylmethyl)-1,3-thiazol-4-yl]phenol;
- 2,6-ditert-butyl-4-[2-(thiomorpholin-4-ylmethyl)-1,3-thiazol-4-yl]phenol;
- 4-[2-(anilinomethyl)-1,3-thiazol-4-yl]-2,6-di*tert*-butylphenol;
- 2,6-di*tert*-butyl-4-(2-{[[2-(dimethylamino)ethyl](methyl)amino]methyl}-1,3-thiazol-4-yl)phenol;
- 2,6-di*tert*-butyl-4-{5-methyl-2-[(methylamino)methyl]-1,3-thiazol-4-yl}phenol;
- 1-[4-(10*H*-phenothiazin-2-yl)-1,3-thiazol-2-yl]methanamine;
- *N*-{[4-(3,5-di*tert*-butyl-4-hydroxyphenyl)-1,3-thiazol-2-yl]methyl}-N-methylacetamide;
- 1-[4-(3,5-di*tert*-butyl-4-methoxyphenyl)-1,3-thiazol-2-yl]-N-methylmethanamine;
- 2,6-di*tert*-butyl-4-{2-[(ethylamino)methyl]-1,3-thiazol-4-yl}phenol;
- 2,6-di*tert*-butyl-4-{2-[(4-phenylpiperazin-1-yl)methyl]-1,3-thiazol-4-yl}phenol;
- 2,6-di*tert*-butyl-4-{2-[(4-methyl-1,4-diazepan-1-yl)methyl]-1,3-thiazol-4-yl}phenol;
- N-{1-[4-(4-anilinophenyl)-1,3-thiazol-2-yl]ethyl}-N-methylamine;
- 2,6-di*tert*-butyl-4-{2-[(isopropylamino)methyl]-1,3-thiazol-4-yl}phenol;
- 2,6-di*tert*-butyl-4-{2-[(cyclohexylamino)methyl]-1,3-thiazol-4-yl}phenol;
- 2,6-di*tert*-butyl-4-{2-[(4-isopropylpiperazin-1-yl)methyl]-1,3-thiazol-4-yl}phenol;
- *N*-methyl-1-[4-(10*H*-phenothiazin-2-yl)-1,3-thiazol-2-yl]ethanamine;
- 2,6-di*tert*-butyl-4-{2-[(4-ethylpiperazin-1-yl)methyl]-1,3-thiazol-4-yl}phenol;
- *N*-{[4-(4-anilinophenyl)-1,3-thiazol-2-yl]methyl}-*N*-ethylamine;
- *N*-{[4-(10*H*-phenothiazin-2-yl)-1,3-thiazol-2-yl]methyl}ethanamine;
- 2,6-di*tert*-butyl-4-(2-{[4-(dimethylamino)piperidin-1-yl]methyl}-1,3-thiazol-4-yl)phenol;
- 1-{[4-(3,5-di*tert*-butyl-4-hydroxyphenyl)-1,3-thiazol-2-yl]methyl}piperidin-4-ol;
- 4-methylpentyl 2-[4-(1,1'-biphenyl-4-yl)-1*H*-imidazol-2-yl]ethylcarbamate;
- 3,3-dimethylbutyl 2-[4-(4-pyrrolidin-1-ylphenyl)-1*H*-imidazol-2-yl]ethylcarbamate;
- isopentyl 2-[4-(1,1'-biphenyl-4-yl)-1*H*-imidazol-2-yl]ethylcarbamate;
- hexyl 2-[4-(4'-bromo-1,1'-biphenyl-4-yl)-1*H*-imidazol-2-yl]ethylcarbamate;
- benzyl 2-[4-(4-*tert*-butylphenyl)-1*H*-imidazol-2-yl]ethylcarbamate;
- 3,3-dimethylbutyl 2-[4-(1,1'-biphenyl-4-yl)-1*H*-imidazol-2-yl]ethylcarbamate;
- hexyl 2-[4-(4-pyrrolidin-1-ylphenyl)-1*H*-imidazol-2-yl]ethylcarbamate;
- hexyl 2-[4-(3,5-di*tert*-butyl-4-hydroxyphenyl)-1*H*-imidazol-2-yl]ethylcarbamate;
- 3,3-dimethylbutyl 2-[4-(3,5-di*tert*-butyl-4-hydroxyphenyl)-1*H*-imidazol-2-yl]ethylcarbamate;
- 3,3-dimethylbutyl 2-[4-(4-methoxyphenyl)-1*H*-imidazol-2-yl]ethylcarbamate;
- benzyl 2-[4-(3,5-di*tert*-butyl-4-hydroxyphenyl)-1*H*-imidazol-2-yl]ethylcarbamate;
- benzyl 2-[4-(4-pyrrolidin-1-ylphenyl)-1*H*-imidazol-2-yl]ethylcarbamate;
- 2-phenylethyl 2-[4-(1,1'-biphenyl-4-yl)-1*H*-imidazol-2-yl]ethylcarbamate;
- butyl 2-[4-(4'-fluoro-1,1'-biphenyl-4-yl)-1*H*-imidazol-2-yl]ethylcarbamate;
- butyl 2-[4-(1,1'-biphenyl-4-yl)-5-methyl-1*H*-imidazol-2-yl]ethylcarbamate;
- butyl 2-[4-(4'-methyl-1,1'-biphenyl-4-yl)-1*H*-imidazol-2-yl]ethylcarbamate;
- butyl 2-[4-(4'-chloro-1,1'-biphenyl-4-yl)-1*H*-imidazol-2-yl]ethylcarbamate;
- butyl 2-[4-(2'-fluoro-1,1'-biphenyl-4-yl)-1*H*-imidazol-2-yl]ethylcarbamate;
- butyl 2-[4-(2',4'-difluoro-1,1'-biphenyl-4-yl)-1H-imidazol-2-yl]ethylcarbamate;
- 2,6-di-*tert*-butyl-4-{2-[(propylamino)methyl]-1,3-thiazol-4-yl}phenol;
- *N*-{[4-(10*H*-phenothiazin-2-yl)-1,3-thiazol-2-yl]methyl}-*N*-propylamine;
- *N*-{[4-(10*H*-phenothiazin-2-yl)-1,3-thiazol-2-yl]methyl}butan-1-amine;
- *N*-{[4-(10*H*-phenothiazin-2-yl)-1,3-thiazol-2-yl]methyl}pentan-1-amine;
- 1-{[4-(3,5-di-*tert*-butyl-4-hydroxyphenyl)-1,3-thiazol-2-yl]methyl}piperidin-3-ol;
- 1-{[4-(3,5-di-*tert*-butyl-4-hydroxyphenyl)-1,3-thiazol-2-yl]methyl}pyrrolidin-3-ol;
- [4-(10*H*-phenothiazin-2-yl)-1,3-thiazol-2-yl]methanol;
- *N,N*-dimethyl-*N*-{[4-(10*H*-phenothiazin-2-yl)-1,3-thiazol-2-yl]methyl}amine;
- 2-{2-[(4-methylpiperazin-1-yl)methyl]-1,3-thiazol-4-yl}-10*H*-phenothiazine;
- 2-[2-(piperidin-1-ylmethyl)-1,3-thiazol-4-yl]-10*H*-phenothiazine;
- 2-[2-(piperazin-1-ylmethyl)-1,3-thiazol-4-yl]-10*H*-phenothiazine;
- 1-{[4-(3,5-di-*tert*-butyl-4-hydroxyphenyl)-1,3-thiazol-2-yl]methyl}azetidin-3-ol;
- 2-[2-(morpholin-4-ylmethyl)-1,3-thiazol-4-yl]-10*H*-phenothiazine;
- 2-[2-(thiomorpholin-4-ylmethyl)-1,3-thiazol-4-yl]-10*H*-phenothiazine;
- 2-{2-[(4-methyl-1,4-diazepan-1-yl)methyl]-1,3-thiazol-4-yl}-10*H*-phenothiazine;
- (3*R*)-1-{[4-(3,5-di-tert-butyl-4-hydroxyphenyl)-1,3-thiazol-2-yl]methyl}pyrrolidin-3-ol;
- (3*S*)-1-{[4-(3,5-di-tert-butyl-4-hydroxyphenyl)-1,3-thiazol-2-yl]methyl}pyrrolidin-3-ol;
- 2,6-di-*tert*-butyl-4-[2-(pyrrolidin-1-ylmethyl)-1,3-thiazol-4-yl]phenol;
- 2,6-di-*tert*-butyl-4-{2-[(butylamino)methyl]-1,3-thiazol-4-yl}phenol;
- 2-{2-[(4-ethylpiperazin-1-yl)methyl]-1,3-thiazol-4-yl}-10*H*-phenothiazine;
- *N*-methyl-*N*-{[4-(10*H*-phenothiazin-2-yl)-1*H*-imidazol-2-yl]methyl}amine;
- methyl [4-(10*H*-phenothiazin-2-yl)-1,3-thiazol-2-yl]methylcarbamate;
- butyl [4-(10*H*-phenothiazin-2-yl)-1,3-thiazol-2-yl]methylcarbamate;
- *N*-neopentyl-N-{[4-(10*H*-phenothiazin-2-yl)-1,3-thiazol-2-yl]methyl}amine;
- 1-{[4-(10*H*-phenothiazin-2-yl)-1,3-thiazol-2-yl]methyl}piperidin-4-ol;
- *N*-{[4-(10*H*-phenothiazin-2-yl)-1,3-thiazol-2-yl]methyl}acetamide;
- *N*-{[4-(10*H*-phenothiazin-2-yl)-1,3-thiazol-2-yl]methyl}butanamide;
- 2,6-di-*tert*-butyl-4-{2-[(4-propylpiperazin-l-yl)methyl]-1,3-thiazol-4-yl}phénol;
- 2,6-di-*tert*-butyl-4-{2-[2-methyl-1-(methylamino)propyl]-1,3-thiazol-4-yl}phénol;
- *N*,2-dimethyl-1-[4-(10*H*-phenothiazin-2-yl)-1,3-thiazol-2-yl]propan-1-amine;
- *N*-{[4-(10*H*-phenothiazin-2-yl)-1,3-thiazol-2-yl]methyl}hexanamide;
- (3*R*)-1-{[4-(10*H*-phenothiazin-2-yl)-1,3-thiazol-2-yl]methyl}pyrrolidin-3-ol;
- (3*S*)-1-{[4-(10*H*-phenothiazin-2-yl)-1,3-thiazol-2-yl]methyl}pyrrolidin-3-ol;
- 1-{[4-(10*H*-phenothiazin-2-yl)-1,3-thiazol-2-yl]methyl}azetidin-3-ol;
- 2-{2-[(4-propylpiperazin-1-yl)methyl]-1,3-thiazol-4-yl}-10*H*-phenothiazine;
- 2-{2-[(4-acetylpiperazin-1-yl)methyl]-1,3-thiazol-4-yl}-10*H*-phenothiazine;
- 2-{2-[(4-butylpiperazin-1-yl)methyl]-1,3-thiazol-4-yl}-10*H*-phenothiazine;
- methyl 4-{[4-(10*H*-phenothiazin-2-yl)-1,3-thiazol-2-yl]methyl}piperazine-1-carboxylate;
- 4-[2-(aminomethyl)-1H-imidazol-4-yl]-2,6-di-*tert*-butylphenol;
- 4-{2-[(benzylamino)methyl]-1,3-thiazol-4-yl}-2,6-di-*tert*-butylphenol;
- 4-{2-[(4-acetylpiperazin-1-yl)methyl]-1,3-thiazol-4-yl}-2,6-di-*tert*-butylphenol;
- *N*-methyl-N-{[4-(10*H*-phenoxazin-2-yl)-1,3-thiazol-2-yl]methyl}amine;
- 4-[2-(azetidin-1-ylmethyl)-1,3-thiazol-4-yl]-2,6-di-*tert*-butylphenol;
- 2,6-di-*tert*-butyl-4-{2-[(4-butylpiperazin-1-yl)methyl]-1,3-thiazol-4-yl}phenol;
- butyl 2-[4-(3'-chloro-1,1'-biphenyl-4-yl)-1*H*-imidazol-2-yl]ethylcarbamate;
- butyl 2-[4-(3'-fluoro-1,'1-biphenyl-4-yl)-1*H*-imidazol-2-yl]ethylcarbamate;
- butyl 2-[4-(4-isobutylphenyl)-1*H*-imidazol-2-yl]ethylcarbamate;
- benzyl 2-[4-(4-isobutylphenyl)-1*H*-imidazol-2-yl]ethylcarbamate;
- butyl 2-[4-(3'-chloro-4'-fluoro-1,1'-biphenyl-4-yl)-1*H*-imidazol-2-yl]ethylcarbamate;
- butyl 2-[4-(3',4'-dichloro-1,1'-biphenyl-4-yl)-1*H*-imidazol-2-yl]ethylcarbamate;
- butyl 2-[4-(4-propylphenyl)-1*H*-imidazol-2-yl]ethylcarbamate;
- butyl 2-[4-(4-ethylphenyl)-1*H*-imidazol-2-yl]ethylcarbamate;
- butyl 2-[4-(4'-cyano-1,1'-biphenyl-4-yl)-1*H*-imidazol-2-yl]ethylcarbamate;
- butyl 2-[4-(1,1'-biphenyl-4-yl)-5-ethyl-1*H*-imidazol-2-yl]ethylcarbamate;
- butyl 2-[4-(2'-chloro-1,1'-biphenyl-4-yl)-1*H*-imidazol-2-yl]ethylcarbamate;
- butyl 2-[4-(2',3'difluoro-1,1'-biphenyl-4-yl)-1*H*-imidazol-2-yl]ethylcarbamate;
- butyl 2-[4-(2'-bromo-1,1'-biphenyl-4-yl)-1*H*-imidazol-2-yl]ethylcarbamate;
- butyl 2-[4-(3',5'-difluoro-1,1'-biphenyl-4-yl)-1*H*-imidazol-2-yl]ethylcarbamate;
- butyl 2-[4-(2'-methoxy-1,1'-biphenyl-4-yl)-1*H*-imidazol-2-yl]ethylcarbamate;
- butyl 2-[4-(3'-nitro-1,1'-biphenyl-4-yl)-1*H*-imidazol-2-yl]ethylcarbamate;
- butyl 2-[4-(2',5'-difluoro-1,1'-biphenyl-4-yl)-1*H*-imidazol-2-yl]ethylcarbamate;
- butyl 2-[4-(3'-methoxy-1,1'-biphenyl-4-yl)-1*H*-imidazol-2-yl]ethylcarbamate;
- methyl 4-{[4-(3,5-di-tert-butyl-4-hydroxyphenyl)-1,3-thiazol-2-yl]methyl}piperazine-1-carboxylate;
- methyl [4-(3,5-di-tert-butyl-4-hydroxyphenyl)-1,3-thiazol-2-yl]methylcarbamate;
- *N*-{[4-(3,5-di-*tert*-butyl-4-hydroxyphenyl)-1,3-thiazol-2-yl]methyl}benzamide;
- *N*-{[4-(3,5-di-*tert*-butyl-4-hydroxyphenyl)-1,3-thiazol-2-yl]methyl}-2-phenylacetamide;
- *N*-{[4-(3,5-di-*tert*-butyl-4-hydroxyphenyl)-1,3-thiazol-2-yl]methyl}propanamide;
- 1-{[4-(3,5-di-tert-butyl-4-hydroxyphenyl)-1,3-thiazol-2-yl]methyl}piperidin-4-yl acetate;
- 1-{[4-(3,5-di-tert-butyl-4-hydroxyphenyl)-1,3-thiazol-2-yl]methyl}pyrrolidine-3,4-diol;
or a pharmaceutically acceptable salt of such a compound.

3. Pharmaceutical composition containing, as active ingredient, at least one of the compounds according to claim 2.

4. Use of a compound **characterized in that** it is chosen from the following compounds:
- 2,6-di*tert*-butyl-4-{2-[2-(methylamino)ethyl]-1,3-thiazol-4-yl}phenol;
- 2,6-di*tert*-butyl-4-[4-(hydroxymethyl)-1,3-oxazol-2-yl]phenol;
- 2,6-di*tert*-butyl-4-{2-[1-(methylamino)ethyl]-1,3-thiazol-4-yl}phenol;
- 2,6-di*tert*-butyl-4-[2-(methoxymethyl)-1,3-thiazol-4-yl]phenol;
- 2,6-di*tert*-butyl-4-{4-[(methylamino)methyl]-1,3-oxazol-2-yl}phenol;
- N-{[4-(3,5-di*tert*-butyl-4-hydroxyphenyl)-1,3-thiazol-2-yl]methyl}acetamide;
- ethyl[4-(3,5-di*tert*-butyl-4-hydroxyphenyl)-1,3-thiazol-2-yl]methylcarbamate;
- 2,6-di*tert*-butyl-4-[2-(morpholin-4-ylmethyl)-1,3-thiazol-4-yl]phenol;
- 2,6-di*tert*-butyl-4-[2-(thiomorpholin-4-ylmethyl)-1,3-thiazol-4-yl]phenol;
- 4-[2-(anilinomethyl)-1,3-thiazol-4-yl]-2,6-di*tert*-butylphenol;
- 2,6-di*tert*-butyl-4-(2-{[[2-(dimethylamino)ethyl](methyl)amino]methyl}-1,3-thiazol-4-yl)phenol;
- 2,6-di*tert*-butyl-4-{5-methyl-2-[(methylamino)methyl]-1,3-thiazol-4-yl}phenol;
- 1-[4-(10*H*-phenothiazin-2-yl)-1,3-thiazol-2-yl]methanamine;
- *N*-{[4-(3,5-di*tert*-butyl-4-hydroxyphenyl)-1,3-thiazol-2-yl]methyl}-*N*-methylacetamide;
- 1-[4-(3,5-di*tert*-butyl-4-methoxyphenyl)-1,3-thiazol-2-yl]-N-methylmethanamine;
- 2,6-di*tert*-butyl-4-{2-[(ethylamino)methyl]-1,3-thiazol-4-yl}phenol;
- 2,6-di*tert*-butyl-4-{2-[(4-phenylpiperazin-1-yl)methyl]-1,3-thiazol-4-yl}phenol;
- 2,6-di*tert*-butyl-4-{2-[(4-methyl-1,4-diazepan-1-yl)methyl]-1,3-thiazol-4-yl}phenol;
- N-{1-[4-(4-anilinophenyl)-1,3-thiazol-2-yl]ethyl}-N-methylamine;
- 2,6-d*itert*-butyl-4-{2-[(isopropylamino)methyl]-1,3-thiazol-4-yl}phenol;
- 2,6-di*tert*-butyl-4-{2-[(cyclohexylamino)methyl]-1,3-thiazol-4-yl}phenol;
- 2,6-di*tert*-butyl-4-{2-[(4-isopropylpiperazin-1-yl)methyl]-1,3-thiazol-4-yl}phenol;
- *N*-methyl-1-[4-(10*H*-phenothiazin-2-yl)-1,3-thiazol-2-yl]ethanamine;
- 2,6-di*tert*-butyl-4-{2-[(4-ethylpiperazin-1-yl)methyl]-1,3-thiazol-4-yl}phenol;
- *N*-{[4-(4-anilinophenyl)-1,3-thiazol-2-yl]methyl}-*N*-ethylamine;
- *N*-{[4-(10*H*-phenothiazin-2-yl)-1,3-thiazol-2-yl]methyl}ethanamine;
- 2,6-di*tert*-butyl-4-(2-{[4-(dimethylamino)piperidin-1-yl]methyl}-1,3-thiazol-4-yl)phenol;
- 1-{[4-(3,5-di*tert*-butyl-4-hydroxyphenyl)-1,3-thiazol-2-yl]methyl}piperidin-4-ol;
- 4-methylpentyl 2-[4-(1,1'-biphenyl-4-yl)-1*H*-imidazol-2-yl]ethylcarbamate;
- 3,3-dimethylbutyl 2-[4-(4-pyrrolidin-1-ylphenyl)-1*H*-imidazol-2-yl]ethylcarbamate;
- isopentyl 2-[4-(1,1'-biphenyl-4-yl)-1*H*-imidazol-2-yl]ethylcarbamate;
- hexyl 2-[4-(4'-bromo-1,1'-biphenyl-4-yl)-1*H*-imidazol-2-yl]ethylcarbainate;
- benzyl 2-[4-(4-*tert*-butylphenyl)-1*H*-imidazol-2-yl]ethylcarbamate;
- 3,3-dimethylbutyl 2-[4-(1,1'-biphenyl-4-yl)-1*H*-imidazol-2-yl]ethylcarbamate;
- hexyl 2-[4-(4-pyrrolidin-1-ylphenyl)-1*H*-imidazol-2-yl]ethylcarbamate;
- hexyl 2-[4-(3,5-di*tert*-butyl-4-hydroxyphenyl)-1*H*-imidazol-2-yl]ethylcarbamate;
- 3,3-dimethylbutyl 2-[4-(3,5-di*tert*-butyl-4-hydroxyphenyl)-1*H*-imidazol-2-yl]ethylcarbamate;
- 3,3-dimethylbutyl 2-[4-(4-methoxyphenyl)-1*H*-imidazol-2-yl]ethylcarbamate;
- benzyl 2-[4-(3,5-di*tert*-butyl-4-hydroxyphenyl)-1*H*-imidazol-2-yl]ethylcarbamate;
- benzyl 2-[4-(4-pyrrolidin-1-ylphenyl)-1*H*-imidazol-2-yl]ethylcarbamate;
- 2-phenylethyl 2-[4-(1,1'-biphenyl-4-yl)-1*H*-imidazol-2-yl]ethylcarbamate;
- butyl 2-[4-(4'-fluoro-1,1'-biphenyl-4-yl)-1*H*-imidazol-2-yl]ethylcarbamate;
- butyl 2-[4-(1,1'-biphenyl-4-yl)-5-methyl-1*H*-imidazol-2-yl]ethylcarbamate;
- butyl 2-[4-(4'-methyl-1,1'-biphenyl-4-yl)-1*H*-imidazol-2-yl]ethylcarbamate;
- butyl 2-[4-(4'-chloro-1,1'-biphenyl-4-yl)-1*H*-imidazol-2-yl]ethylcarbamate;
- butyl 2-[4-(2'-fluoro-1,1'-biphenyl-4-yl)-1*H*-imidazol-2-yl]ethylcarbamate;
- butyl 2-[4-(2',4'-difluoro-1,1'-biphenyl-4-yl)-1*H*-imidazol-2-yl]ethylcarbamate;
- 2,6-di-*tert*-butyl-4-{2-[(propylamino)methyl]-1,3-thiazol-4-yl}phenol;
- *N*-{[4-(10*H*-phenothiazin-2-yl)-1,3-thiazol-2-yl]methyl}-*N*-propylamine;
- *N*-{[4-(10*H*-phenothiazin-2-yl)-1,3-thiazol-2-yl]methyl}butan-1-amine;
- *N*-{[4-(10*H*-phenothiazin-2-yl)-1,3-thiazol-2-yl]methyl}pentan-1-amine;
- 1-{[4-(3,5-di-*tert*-butyl-4-hydroxyphenyl)-1,3-thiazol-2-yl]methyl}piperidin-3-ol;
- 1-{[4-(3,5-di-*tert*-butyl-4-hydroxyphenyl)-1,3-thiazol-2-yl]methyl}pyrrolidin-3-ol;
- [4-(10*H*-phenothiazin-2-yl)-1,3-thiazol-2-yl]methanol;
- *N*,*N*-dimethyl-*N*-{[4-(10*H*-phenothiazin-2-yl)-1,3-thiazol-2-yl]methyl}amine;
- 2-{2-[(4-methylpiperazin-1-yl)methyl]-1,3-thiazol-4-yl}-10*H*-phenothiazine;
- 2-[2-(piperidin-1-ylmethyl)-1,3-thiazol-4-yl]-10*H*-phenothiazine;
- 2-[2-(piperazin-1-ylmethyl)-1,3-thiazol-4-yl]-10*H*-phenothiazine;
- 1-{[4-(3,5-di-*tert*-butyl-4-hydroxyphenyl)-1,3-thiazol-2-yl]methyl}azetidin-3-ol;
- 2-[2-(morpholin-4-ylmethyl)-1,3-thiazol-4-yl]-10*H*-phenothiazine;
- 2-[2-(thiomorpholin-4-ylmethyl)-1,3-thiazol-4-yl]-10*H*-phenothiazine;
- 2-{2-[(4-methyl-1,4-diazepan-1-yl)methyl]-1,3-thiazol-4-yl}-10*H*-phenothiazine;
- (3*R*)-1-{[4-(3,5-di-tert-butyl-4-hydroxyphenyl)-1,3-thiazol-2-yl]methyl}pyrrolidin-3-ol;
- (3*S*)-1-{[4-(3,5-di-tert-butyl-4-hydroxyphenyl)-1,3-thiazol-2-yl]methyl}pyrrolidin-3-ol;
- 2,6-di-*tert*-butyl-4-[2-(pyrrolidin-1-ylmethyl)-1,3-thiazol-4-yl]phenol;
- 2,6-di-*tert*-butyl-4-{2-[(butylamino)methyl]-1,3-thiazol-4-yl}phenol;
- 2-{2-[(4-ethylpiperazin-1-yl)methyl]-1,3-thiazol-4-yl}-10*H*-phenothiazine;
- *N*-methyl-*N*-{[4-(10*H*-phenothiazin-2-yl)-1*H*-imidazol-2-yl]methyl}amine;
- methyl [4-(10*H*-phenothiazin-2-yl)-1,3-thiazol-2-yl]methylcarbamate;
- butyl [4-(10*H*-phenothiazin-2-yl)-1,3-thiazol-2-yl]methylcarbamate;
- *N*-neopentyl-N-{[4-(10*H*-phenothiazin-2-yl)-1,3-thiazol-2-yl]methyl}amine;
- 1-{[4-(10*H*-phenothiazin-2-yl)-1,3-thiazol-2-yl]methyl}piperidin-4-ol;
- *N*-{[4-(10*H*-phenothiazin-2-yl)-1,3-thiazol-2-yl]methyl}acetamide;
- *N*-{[4-(10*H*-phenothiazin-2-yl)-1,3-thiazol-2-yl]methyl}butanamide;
- 2,6-di-*tert*-butyl-4-{2-[(4-propylpiperazin-1-yl)methyl]-1,3-thiazol-4-yl}phenol;
- 2,6-di-*tert*-butyl-4-{2-[2-methyl-1-(methylamino)propyl]-1,3-thiazol-4-yl}phenol;
- N,2-dimethyl-1-[4-(10*H*-phenothiazin-2-yl)-1,3-thiazol-2-yl]propan-1-amine;
- *N*-{[4-(10*H*-phenothiazin-2-yl)-1,3-thiazol-2-yl]methyl}hexanamide;
- (3*R*)-1-{[4-(10*H*-phenothiazin-2-yl)-1,3-thiazol-2-yl]methyl}pyrrolidin-3-ol;
- (3*S*)-1-{[4-(10*H*-phenothiazin-2-yl)-1,3-thiazol-2-yl]methyl}pyrrolidin-3-ol;
- 1-{[4-(10*H*-phenothiazin-2-yl)-1,3-thiazol-2-yl]methyl}azetidin-3-ol;
- 2-{2-[(4-propylpiperazin-1-yl)methyl]-1,3-thiazol-4-yl}-10*H*-phenothiazine;
- 2-{2-[(4-acetylpiperazin-1-yl)methyl]-1,3-thiazol-4-yl}-10*H*-phenothiazine;
- 2-{2-[(4-butylpiperazin-1-yl)methyl]-1,3-thiazol-4-yl}-10*H*-phenothiazine;
- methyl 4-{[4-(10*H*-phenothiazin-2-yl)-1,3-thiazol-2-yl]methyl}piperazine-1-carboxylate;
- 4-[2-(aminomethyl)-1*H*-imidazol-4-yl]-2,6-di-*tert*-butylphenol;
- 4-{2-[(benzylamino)methyl]-1,3-thiazol-4-yl}-2,6-di-*tert*-butylphenol;
- 4-{2-[(4-acetylpiperazin-1-yl)methyl]-1,3-thiazol-4-yl}-2,6-di-*tert*-butylphenol;
- *N*-methyl-N-{[4-(10*H*-phenoxazin-2-yl)-1,3-thiazol-2-yl]methyl}amine;
- 4-[2-(azetidin-1-ylmethyl)-1,3-thiazol-4-yl]-2,6-di-tert-butylphenol;
- 2,6-di-*tert*-butyl-4-{2-[(4-butylpiperazin-1-yl)methyl]-1,3-thiazol-4-yl}phenol;
- butyl 2-[4-(3'-chloro-1,1'-biphenyl-4-yl)-1*H*-imidazol-2-yl]ethylcarbamate;
- butyl 2-[4-(3'-fluoro-1,1'-biphenyl-4-yl)-1*H*-imidazol-2-yl]ethylcarbamate;
- butyl 2-[4-(4-isobutylphenyl)-1*H*-imidazol-2-yl]ethylcarbamate;
- benzyl 2-[4-(4-isobutylphenyl)-1*H*-imidazol-2-yl]ethylcarbamate;
- butyl 2-[4-(3'-chloro-4'-fluoro-1,1'-biphenyl-4-yl)-1*H*-imidazol-2-yl]ethylcarbamate;
- butyl 2-[4-(3',4'-dichloro-1,1'-biphenyl-4-yl)-1*H*-imidazol-2-yl]ethylcarbamate;
- butyl 2-[4-(4-propylphenyl)-1*H*-imidazol-2-yl]ethylcarbamate;
- butyl 2-[4-(4-ethylphenyl)-1*H*-imidazol-2-yl]ethylcarbamate;
- butyl 2-[4-(4'-cyano-1,1'-biphenyl-4-yl)-1*H*-imidazol-2-yl]ethylcarbamate;
- butyl 2-[4-(1,1'-biphenyl-4-yl)-5-ethyl-1*H*-imidazol-2-yl]ethylcarbamate;
- butyl 2-[4-(2'-chloro-1,1'-biphenyl-4-yl)-1*H*-imidazol-2-yl]ethylcarbamate;
- butyl 2-[4-(2',3'-difluoro-1,1'-biphenyl-4-yl)-1*H*-imidazol-2-yl]ethylcarbamate;
- butyl 2-[4-(2'-bromo-1,1'-biphenyl-4-yl)-1*H*-imidazol-2-yl]ethylcarbamate;
- butyl 2-[4-(3',5'-difluoro-1,1'-biphenyl-4-yl)-1*H*-imidazol-2-yl]ethylcarbamate;
- butyl 2-[4-(2'-methoxy-1,1'-biphenyl-4-yl)-1*H*-imidazol-2-yl]ethylcarbamate;
- butyl 2-[4-(3'-nitro-1,1'-biphenyl-4-yl)-1*H*-imidazol-2-yl]ethylcarbamate;
- butyl 2-[4-(2',5'-difluoro-1,1'-biphenyl-4-yl)-1*H*-imidazol-2-yl]ethylcarbamate;
- butyl 2-[4-(3'-methoxy-1,1'-biphenyl-4-yl)-1*H*-imidazol-2-yl]ethylcarbamate;
- methyl 4-{[4-(3,5-di-tert-butyl-4-hydroxyphenyl)-1,3-thiazol-2-yl]methyl}piperazine-1-carboxylate;
- methyl [4-(3,5-di-tert-butyl-4-hydroxyphenyl)-1,3-thiazol-2-yl]methylcarbamate;
- *N*-{[4-(3,5-di-*tert*-butyl-4-hydroxyphenyl)-1,3-thiazol-2-yl]methyl}benzamide;
- *N*-{[4-(3,5-di-*tert*-butyl-4-hydroxyphenyl)-1,3-thiazol-2-yl]methyl}-2-phenylacetamide;
- *N*-{[4-(3,5-di-*tert*-butyl-4-hydroxyphenyl)-1,3-thiazol-2-yl]methyl}propanamide;
- 1-{[4-(3,5-di-tert-butyl-4-hydroxyphenyl)-1,3-thiazol-2-yl]methyl}piperidin-4-yl acetate;
- 1-{[4-(3,5-di-tert-butyl-4-hydroxyphenyl)-1,3-thiazol-2-yl]methyl}pyrrolidine-3,4-diol;
or a pharmaceutically acceptable salt of one of the latter for preparing a medicament intended to treat one of the following disorders diseases: Parkinson's disease, senile dementia, Alzheimer's disease, Huntington's chorea, amyotrophic lateral sclerosis, schizophrenia, depressions, psychoses, migraine or pains and in particular neuropathic pains.

5. Use of one of the following compounds:
- 2,6-di*tert*-butyl-4-{2-[2-(methylamino)ethyl]-1,3-thiazol-4-yl}phenol;
- 2,6-di*tert*-butyl-4-[4-(hydroxymethyl)-1,3-oxazol-2-yl]phenol;
- 2,6-di*tert*-butyl-4-{2-[1-(methylamino)ethyl]-1,3-thiazol-4-yl}phenol;
- 2,6-di*tert*-butyl-4-[2-(methoxymethyl)-1,3-thiazol-4-yl]phenol;
- 2,6-di*tert*-butyl-4-{4-[(methylamino)methyl]-1,3-oxazol-2-yl}phenol;
- N-{[4-(3,5-di*tert*-butyl-4-hydroxyphenyl)-1,3-thiazol-2-yl]methyl}acetamide;
- ethyl [4-(3,5-di*tert*-butyl-4-hydroxyphenyl)-1,3-thiazol-2-yl]methylcarbamate;
- 2,6-di*tert*-butyl-4-[2-(morpholin-4-ylmethyl)-1,3-thiazol-4-yl]phenol;
- 2,6-ditert-butyl-4-[2-(thiomorpholin-4-ylmethyl)-1,3-thiazol-4-yl]phenol;
- 4-[2-(anilinomethyl)-1,3-thiazol-4-yl]-2,6-ditert-butylphenol;
- 2,6-di*tert*-butyl-4-(2-{[[2-(dimethylamino)ethyl](methyl)amino]methyl}-1,3-thiazol-4-yl)phenol;
- 2,6-di*tert*-butyl-4-{5-methyl-2-[(methylamino)methyl]-1,3-thiazol-4-yl}phenol;
- 1-[4-(10*H*-phenothiazin-2-yl)-1,3-thiazol-2-yl]methanamine;
- *N*-{[4-(3,5-di*tert*-butyl-4-hydroxyphenyl)-1,3-thiazol-2-yl]methyl}-*N*-methylacetamide;
- 1-[4-(3,5-di*tert*-butyl-4-methoxyphenyl)-1,3-thiazol-2-yl]-N-methylmethanamine;
- 2,6-di*tert*-butyl-4-{2-[(ethylamino)methyl]-1,3-thiazol-4-yl}phenol;
- 2,6-di*tert*-butyl-4-{2-[(4-phenylpiperazin-1-yl)methyl]-1,3-thiazol-4-yl}phenol;
- 2,6-di*tert*-butyl-4-{2-[(4-methyl-1,4-diazepan-1-yl)methyl]-1,3-thiazol-4-yl}phenol;
- N-{1-[4-(4-anilinophenyl)-1,3-thiazol-2-yl]ethyl}-N-methylamine;
- 2,6-di*tert*-butyl-4-{2-[(isopropylamino)methyl]-1,3-thiazol-4-yl}phenol;
- 2,6-di*tert*-butyl-4-{2-[(cyclohexylamino)methyl]-1,3-thiazol-4-yl}phenol;
- 2,6-di*tert*-butyl-4-{2-[(4-isopropylpiperazin-1-yl)methyl]-1,3-thiazol-4-yl}phenol;
- *N*-methyl-1-[4-(10*H*-phenothiazin-2-yl)-1,3-thiazol-2-yl]ethanmline;
- 2,6-di*tert*-butyl-4-{2-[(4-ethylpiperazin-1-yl)methyl]-1,3-thiazol-4-yl}phenol;
- *N*-{[4-(4-anilinophenyl)-1,3-thiazol-2-yl]methyl}-*N*-ethylamine;
- *N*-{[4-(10*H*-phenothiazin-2-yl)-1,3-thiazol-2-yl]methyl}ethanamine;
- 2,6-di*tert*-butyl-4-(2-{[4-(dimethylamino)piperidin-1-yl]methyl}-1,3-thiazol-4-yl)phenol;
- 1-{[4-(3,5-di*tert*-butyl-4-hydroxyphenyl)-1,3-thiazol-2-yl]methyl}piperidin-4-ol;
- 2,6-di-*tert*-butyl-4-{2-[(propylamino)methyl]-1,3-thiazol-4-yl}phenol;
- *N*-{[4-(10*H*-phenothiazin-2-yl)-1,3-thiazol-2-yl]methyl}-*N*-propylamine;
- *N-*{[4-(10*H*-phenothiazin-2-yl)-1,3-thiazol-2-yl]methyl}butan-1-amine;
- *N*-{[4-(10*H*-phenothiazin-2-yl)-1,3-thiazol-2-yl]methyl}pentan-1-amine;
- 1-{[4-(3,5-di-*tert*-butyl-4-hydroxyphenyl)-1,3-thiazol-2-yl]methyl}piperidin-3-ol;
- 1-{[4-(3,5-di-*tert*-butyl-4-hydroxyphenyl)-1,3-thiazol-2-yl]methyl}pyrrolidin-3-ol;
- [4-(10*H*-phenothiazin-2-yl)-1,3-thiazol-2-yl]methanol;
- *N*,*N*-dimethyl-*N*-{[4-(10*H*-phenothiazin-2-yl)-1,3-thiazol-2-yl]methyl}amine;
- 2-{2-[(4-methylpiperazin-1-yl)methyl]-1,3-thiazol-4-yl}-10*H*-phenothiazine;
- 2-[2-(piperidin-1-ylmethyl)-1,3-thiazol-4-yl]-10*H*-phenothiazine;
- 2-[2-(piperazin-1-ylmethyl)-1,3-thiazol-4-yl]-10*H*-phenothiazine;
- 1-{[4-(3,5-di-*tert*-butyl-4-hydroxyphenyl)-1,3-thiazol-2-yl]methyl}azetidin-3-ol;
- 2-[2-(morpholin-4-ylmethyl)-1,3-thiazol-4-yl]-10*H*-phenothiazine;
- 2-[2-(thiomorpholin-4-ylmethyl)-1,3-thiazol-4-yl]-10*H*-phenothiazine;
- 2-{2-[(4-methyl-1,4-diazepan-1-yl)methyl]-1,3-thiazol-4-yl}-10*H*-phenothiazine;
- (3R)-1-{[4-(3,5-di-tert-butyl-4-hydroxyphenyl)-1,3-thiazol-2-yl]methyl}pyrrolidin-3-ol;
- (3S)-1-{[4-(3,5-di-tert-butyl-4-hydroxyphenyl)-1,3-thiazol-2-yl]methyl}pyrrolidin-3-ol;
- 2,6-di-*tert*-butyl-4-[2-(pyrrolidin-1-ylmethyl)-1,3-thiazol-4-yl]phenol;
- 2,6-di-tert-butyl-4-{2-[(butylamino)methyl]-1,3-thiazol-4-yl}phenol;
- 2-{2-[(4-ethylpiperazin-1-yl)methyl]-1,3-thiazol-4-yl}-10*H*-phenothiazine;
- *N*-methyl-*N*-{[4-(10*H*-phenothiazin-2-yl)-1*H*-imidazol-2-yl]methyl}amine;
- methyl [4-(10*H*-phenothiazin-2-yl)-1,3-thiazol-2-yl]methylcarbamate;
- butyl [4-(10*H*-phenothiazin-2-yl)-1,3-thiazol-2-yl]methylcarbamate;
- *N*-neopentyl-N-{[4-(10*H*-phenothiazin-2-yl)-1,3-thiazol-2-yl]methyl}amine;
- 1-{[4-(10*H*-phenothiazin-2-yl)-1,3-thiazol-2-yl]methyl}piperidin-4-ol;
- *N*-{[4-(10*H*-phenothiazin-2-yl)-1,3-thiazol-2-yl]methyl}acetamide;
- *N*-{[4-(10*H*-phenothiazin-2-yl)-1,3-thiazol-2-yl]methyl}butanamide;
- 2,6-di-*tert*-butyl-4-{2-[(4-propylpiperazin-1-yl)methyl]-1,3-thiazol-4-yl}phenol;
- 2,6-di-*tert*-butyl-4-{2-[2-methyl-1-(methylamino)propyl]-1,3-thiazol-4-yl}phenol;
- *N*,2-dimethyl-1-[4-(10*H*-phenothiazin-2-yl)-1,3-thiazol-2-yl]propan-1-amine;
- *N*-{[4-(10*H*-phenothiazin-2-yl)-1,3-thiazol-2-yl]methyl}hexanamide;
- (3*R*)-1-{[4-(10*H*-phenothiazin-2-yl)-1,3-thiazol-2-yl]methyl}pyrrolidin-3-ol;
- (3*S*)-1-{[4-(10*H*-phenothiazin-2-yl)-1,3-thiazol-2-yl]methyl}pyrrolidin-3-ol;
- 1-{[4-(10*H*-phenothiazin-2-yl)-1,3-thiazol-2-yl]methyl}azetidin-3-ol;
- 2-{2-[(4-propylpiperazin-1-yl)methyl]-1,3-thiazol-4-yl}-10*H*-phenothiazine;
- 2-{2-[(4-acetylpiperazin-1-yl)methyl]-1,3-thiazol-4-yl}-10*H*-phenothiazine;
- 2-{2-[(4-butylpiperazin-1-yl)methyl]-1,3-thiazol-4-yl}-10*H*-phenothiazine;
- methyl 4-{[4-(10*H*-phenothiazin-2-yl)-1,3-thiazol-2-yl]methyl}piperazine-1-carboxylate;
- 4-[2-(aminomethyl)-1H-imidazol-4-yl]-2,6-di-*tert*-butylphenol;
- 4-{2-[(benzylamino)methyl]-1,3-thiazol-4-yl}-2,6-di-*tert*-butylphenol;
- 4-{2-[(4-acetylpiperazin-1-yl)methyl]-1,3-thiazol-4-yl}-2,6-di-*tert*-butylphenol;
- *N*-methyl-N-{[4-(10*H*-phenoxazin-2-yl)-1,3-thiazol-2-yl]methyl}amine; naw58270- 4-[2-(azetidin-1-ylmethyl)-1,3-thiazol-4-yl]-2,6-di-*tert*-butylphenol;
- 2,6-di-*tert*-butyl-4-{2-[(4-butylpiperazin-1-yl)methyl]-1,3-thiazol-4-yl}phenol;
- methyl 4-{[4-(3,5-di-tert-butyl-4-hydroxyphenyl)-1,3-thiazol-2-yl]methyl}piperazine-1-carboxylate;
- methyl [4-(3,5-di-tert-butyl-4-hydroxyphenyl)-1,3-thiazol-2-yl]methylcarbamate;
- *N*-{[4-(3,5-di-*tert*-butyl-4-hydroxyphenyl)-1,3-thiazol-2-yl]methyl}benzamide;
- *N*-{[4-(3,5-di-*tert*-butyl-4-hydroxyphenyl)-1,3-thiazol-2-yl]methyl}-2-phenylacetamide;
- *N*-{[4-(3,5-di-*tert*-butyl-4-hydroxyphenyl)-1,3-thiazol-2-yl]methyl}propanamide;
- 1-{[4-(3,5-di-tert-butyl-4-hydroxyphenyl)-1,3-thiazol-2-yl]methyl}piperidin-4-yl acetate;
- 1-{[4-(3,5-di-tert-butyl-4-hydroxyphenyl)-1,3-thiazol-2-yl]methyl}pyrrolidine-3,4-diol;
or of a pharmaceutically acceptable salt of one of the latter
for preparing a medicament intended to treat one of the following disorders or diseases: Parkinson's disease, senile dementia, Alzheimer's disease, Huntington's chorea, amyotrophic lateral sclerosis, schizophrenia, depressions and psychoses.

6. Use according to claim 5, **characterized in that** the compound used is chosen from one of the following compounds:
- 2,6-di*tert*-butyl-4-{2-[1-(methylamino)ethyl]-1,3-thiazol-4-yl}phenol ;
- 1-[4-(3,5-di*tert*-butyl-4-methoxyphenyl)-1,3-thiazol-2-yl]-N-methylmethanamine ;
- 2,6-di*tert*-butyl-4-{2-[(ethylamino)methyl]-1,3-thiazol-4-yl}phenol ;
- 1-{[4-(3,5-di*tert*-butyl-4-hydroxyphenyl)-1,3-thiazol-2-yl]methyl}piperidin-4-ol ;
- 1-{[4-(3,5-di-*tert*-butyl-4-hydroxyphenyl)-1,3-thiazol-2-yl]methyl}pyrrolidin-3-ol ;
- 1-{[4-(3,5-di-*tert*-butyl-4-hydroxyphenyl)-1,3-thiazol-2-yl]methyl}azetidin-3-ol ;
- (3*R*)-1-{[4-(3,5-di-tert-butyl-4-hydroxyphenyl)-1,3-thiazol-2-yl]methyl}pyrrolidin-3-ol ;
- (3*S*)-1-{[4-(3,5-di-tert-butyl-4-hydroxyphenyl)-1,3-thiazol-2-yl]methyl}pyrrolidin-3-ol ;
- *N*,2-dimethyl-1-[4-(10*H*-phenothiazin-2-yl)-1,3-thiazol-2-yl]propan-1-amine ;
and the salts of the latter.

7. Use of one of the following compounds
- 2,6-di*tert*-butyl-4-{2-[2-(methylamino)ethyl]-1,3-thiazol-4-yl}phenol;
- 2,6-di*tert*-butyl-4-{2-[1-(methylamino)ethyl]-1,3-thiazol-4-yl}phenol;
- 2,6-di*tert*-butyl-4-{4-[(methylamino)methyl]-1,3-oxazol-2-yl}phenol;
- 2,6-di*tert*-butyl-4-{5-methyl-2-[(methylamino)methyl]-1,3-thiazol-4-yl}phenol;
- 1-[4-(3,5-di*tert*-butyl-4-methoxyphenyl)-1,3-thiazol-2-yl]-N-methylmethanamine;
- 2,6-di*tert*-butyl-4-{2-[(ethylamino)methyl]-1,3-thiazol-4-yl}phenol;
- 4-methylpentyl 2-[4-(1,1'-biphenyl-4-yl)-1*H*-imidazol-2-yl]ethylcarbamate;
- 3,3-dimethylbutyl 2-[4-(4-pyrrolidin-1-ylphenyl)-1*H*-imidazol-2-yl]ethylcarbamate;
- isopentyl 2-[4-(1,1'-biphenyl-4-yl)-1*H*-imidazol-2-yl]ethylcarbamate;
- hexyl 2-[4-(4'-bromo-1,1'-biphenyl-4-yl)-1*H*-imidazol-2-yl]ethylcarbamate;
- benzyl 2-[4-(4-*tert*-butylphenyl)-1*H*-imidazol-2-yl]ethylcarbamate;
- 3,3-dimethylbutyl 2-[4-(1,1'-biphenyl-4-yl)-1*H*-imidazol-2-yl]ethylcarbamate;
- hexyl 2-[4-(4-pyrrolidin-1-ylphenyl)-1*H*-imidazol-2-yl]ethylcarbamate;
- hexyl 2-[4-(3,5-ditert-butyl-4-hydroxyphenyl)-1*H*-imidazol-2-yl]ethylcarbamate;
- hexyl 2-[4-(3,5-ditert-butyl-4-hydroxyphenyl)-1*H*-imidazol-2-yl]ethylcarbamate;
- 3,3-dimethylbutyl 2-[4-(4-methoxyphenyl)-1*H*-imidazol-2-yl]ethylcarbamate;
- benzyl 2-[4-(3,5-di*tert*-butyl-4-hydroxyphenyl)-1*H*-imidazol-2-yl]ethylcarbamate;
- benzyl 2-[4-(4-pyrrolidin-1-ylphenyl)-1*H*-imidazol-2-yl]ethylcarbamate;
- 2-phenylethyl 2-[4-(1,1'-biphenyl-4-yl)-1*H*-imidazol-2-yl]ethylcarbamate;
- butyl 2-[4-(4'-fluoro-1,1'-biphenyl-4-yl)-1*H*-imidazol-2-yl]ethylcarbamate;
- butyl 2-[4-(1,1'-biphenyl-4-yl)-5-methyl-1*H*-imidazol-2-yl]ethylcarbamate;
- butyl 2-[4-(4'-methyl-1,1'-biphenyl-4-yl)-1*H*-imidazol-2-yl]ethylcarbamate;
- butyl 2-[4-(4'-chloro-1,1'-biphenyl-4-yl)-1*H*-imidazol-2-yl]ethylcarbamate;
- butyl 2-[4-(2'-fluoro-1,1'-biphenyl-4-yl)-1*H*-imidazol-2-yl]ethylcarbamate;
- butyl 2-[4-(2',4'-difluoro-1,1'-biphenyl-4-yl)-1H-imidazol-2-yl]ethylcarbamate;
- butyl 2-[4-(3'-chloro-1,1'-biphenyl-4-yl)-1*H*-imidazol-2-yl]ethylcarbamate;
- butyl 2-[4-(3'-fluoro-1,1'-biphenyl-4-yl)-1*H*-imidazol-2-yl]ethylcarbamate;
- butyl 2-[4-(4-isobutylphenyl)-1*H*-imidazol-2-yl]ethylcarbamate;
- benzyl 2-[4-(4-isobutylphenyl)-1*H*-imidazol-2-yl]ethylcarbamate;
- butyl 2-[4-(3'-chloro-4'-fluoro-1,1'-biphenyl-4-yl)-1*H*-imidazol-2-yl]ethylcarbamate;
- butyl 2-[4-(3',4'-dichloro-1,1'-biphenyl-4-yl)-1*H*-imidazol-2-yl]ethylcarbamate;
- butyl 2-[4-(4-propylphenyl)-1*H*-imidazol-2-yl]ethylcarbamate;
- butyl 2-[4-(4-ethylphenyl)-1*H*-imidazol-2-yl]ethylcarbamate;
- butyl 2-[4-(4'-cyano-1,1'-biphenyl-4-yl)-1*H*-imidazol-2-yl]ethylcarbamate;
- butyl 2-[4-(1,1'-biphenyl-4-yl)-5-ethyl-1*H*-imidazol-2-yl]ethylcarbamate;
- butyl 2-[4-(2'-chloro-l,l'-biphenyl-4-yl)-1*H*-imidazol-2-yl]ethylcarbamate;
- butyl 2-[4-(2',3'-difluoro-1,1'-biphenyl-4-yl)-1*H*-imidazol-2-yl]ethylcarbamate;
- butyl 2-[4-(2'-bromo-1,1'-biphenyl-4-yl)-1*H*-imidazol-2-yl]ethylcarbamate;
- butyl 2-[4-(3',5'-difluoro-1,1'-biphenyl-4-yl)-1*H*-imidazol-2-yl]ethylcarbamate;
- butyl 2-[4-(2'-methoxy-1,1'-biphenyl-4-yl)-1*H*-imidazol-2-yl]ethylcarbamate;
- butyl 2-[4-(3'-nitro-1,1'-biphenyl-4-yl)-1*H*-imidazol-2-yl]ethylcarbamate;
- butyl 2-[4-(2',5'-difluoro-1,1'-biphenyl-4-yl)-1*H*-imidazol-2-yl]ethylcarbamate;
- butyl 2-[4-(3'-methoxy-1,1'-biphenyl-4-yl)-1*H*-imidazol-2-yl]ethylcarbamate;
or a pharmaceutically acceptable salt of one of the latter
for preparing a medicament intended to treat one of the following disorders or diseases: Parkinson's disease, Alzheimer's disease, amyotrophic lateral sclerosis, migraine or pains and in particular neuropathic pains.

## Patentansprüche

1. Verbindung, **dadurch gekennzeichnet, dass** sie aus den folgenden Verbindungen ausgewählt ist:
2,6-Di-*tert*-butyl-4-{2-[2-(methylamino)ethyl]-1,3-thiazol-4-yl}phenol;
2,6-Di-*tert*-butyl-4-[4-(hydroxymethyl)-1,3-oxazol-2-yl]phenot;
2,6-Di-*tert*-butyl-4-{2-[1-(methylamino)ethyl]-1,3-thiazol-4-yl}phenol;
2,6-Di-*tert*-butyl-4-[2-(methoxymethyl)-1,3-thiazol-4-yl]phenol;
2,6-Di-*tert*-butyl-4-{4-[(methylamino)methyl]-1,3-oxazol-2-yl}phenol;
N-{[4-(3,5-Di-*tert*-butyl-4-hydroxyphenyl)-1,3-thiazol-2-yl]methyl}acetamid;
Ethyl [4-(3,5-Di-*tert*-butyl-4-hydroxyphenyl)-1,3-thiazol-2-yl]methylcarbamat;
2,6-Di-*tert*-butyl-4-[2-(morpholin-4-ylmethyl)-1,3-thiazol-4-yl]phenol;
2,6-Di-*tert*-butyl-4-[2-(thiomorpholin-4-ylmethyl)-1,3-thiazol-4-yl]phenol;
4-[2-(Anilinomethyl)-1,3-thiazol-4-yl]-2,6-di-*tert*-butylphenol;
2,6-Di-*tert*-butyl-4-(2-{[[2-(dimethylamino)ethyl](methyl)amino]methyl}-1,3-thiazol-4-yl)phenol;
2,6-Di-*tert*-butyl-4-{5-methyl-2-[(methylamino)methyl]-1,3-thiazol-4-yl}phenol;
1-[4-(10*H*-Phenothiazin-2-yl)-1,3-thiazol-2-yl]methanamin;
*N*-{[4-(3,5-Di-*tert*-butyl-4-hydroxyphenyl)-1,3-thiazol-2-yl]methyl}-*N*-methylacetamid;
1-[4-(3,5-Di-*tert*-butyl-4-methoxyphenyl)-1,3-thiazol-2-yl]-N-methanamin;
2,6-Di-*tert*-butyl-4-{2-[(ethylamino)methyl]-1,3-thiazol-4-yl}phenol;
2,6-Di-*tert*-butyl-4-{2-[(4-phenylpiperazin-1-yl)methyl]-1,3-thiazol-4-yl}phenol;
2,6-Di-*tert*-butyl-4-{2-[(4-methyl-1,4-diazepan-1-yl)methyl]-1,3-thiazol-4-yl}phenol;
N-{1-[4-(4-Anilinophenyl)-1,3-thiazol-2-yl]ethyl}-N-methylamin;
2,6-Di-*tert*-butyl-4-{2-[(isopropylamino)methyl]-1,3-thiazol-4-yl}phenol;
2,6-Di-*tert*-butyl-4-{2-[(cyclohexylamino)methyl]-1,3-thiazol-4-yl}phenol;
2,6-Di-*tert*-butyl-4-{2-[(4-isopropylpiperazin-1-yl)methyl]-1,3-thiazol-4-yl}phenol;
*N*-Methyl-1-[4-(10*H*-phenothiazin-2-yl)-1,3-thiazol-2-yl]ethanamin;
2,6-Di-*tert*-butyl-4-{2-[(4-ethylpiperazin-1-yt)methyl]-1,3-thiazol-4-yl}phenol;
*N*-{[4-(4-Anilinophenyl)-1,3-thiazol-2-yl]methyl}-*N*-ethylamin;
*N*-{[4-(10*H*-Phenothiazin-2-yl)-1,3-thiazol-2-yl]methyl}ethanamin;
2,6-Di-*tert*-butyl-4-(2-{[4-(dimethylamino)piperidin-1-yl]methyl}-1,3-thiazol-4-yl)phenol;
1-{[4-(3,5-Di-*tert*-butyl-4-hydroxyphenyl)-1,3-thiazol-2-yl]methyl}piperidin-4-ol;
4-Methylpentyl 2-[4-(1,1'-Biphenyl-4-yl)-1*H*-imidazol-2-yl]ethylcarbamat;
3,3-Dimethylbutyl 2-[4-(4-Pyrrolidin-1-ylphenyl)-1*H*-imidazol-2-yl]ethylcarbamat;
Isopentyl 2-[4-(1,1'-Biphenyl-4-yl)-1*H*-imidazol-2-yl]ethylcarbamat;
Hexyl 2-[4-(4'-Bromo-1,1'-biphenyl-4-yl)-1*H*-imidazol-2-yl]ethylcarbamat;
Benzyl 2-[4-(4-*tert*-Butylphenyl)-1*H*-imidazol-2-yl]ethylcarbamat;
3,3-Dimethylbutyl 2-[4-(1,1'-Biphenyl-4-yl)-1*H*-imidazol-2-yl]ethylcarbamat;
Hexyl 2-[4-(4-Pyrrolidin-1-ylphenyl)-1*H*-imidazol-2-yl]ethylcarbamat;
Hexyl 2-[4-(3,5-Di-*tert*-butyl-4-hydroxyphenyl)-1*H*-imidazol-2-yl]ethylcarbamat;
3,3-Dimethylbutyl 2-[4-(3,5-Di-*tert*-butyl-4-hydroxyphenyl)-1*H*-imidazol-2-yl]ethylcarbamat;
3,3-Dimethylbutyl 2-[4-(4-Methoxyphenyl)-1*H*-imidazol-2-yl]ethylcarbamat;
Benzyl 2-[4-(3,5-Di-*tert*-butyl-4-hydroxyphenyl)-1*H*-imidazol-2-yl]ethylcarbamat;
Benzyl 2-[4-(4-Pyrrolidin-1-ylphenyl)-1*H*-imidazol-2-yl]ethylcarbamat;
2-Hhenylethyl 2-[4-(1,1'-Biphenyl-4-yl)-1*H*-imidazol-2-yl]ethylcarbamat;
Butyl 2-[4-(4'-Fluoro-1,1'-biphenyl-4-yl)-1*H*-imidazol-2-yl]ethylcarbamat;
Butyl 2-[4-(1,1'-Biphenyl-4-yl)-5-methyl-1*H*-imidazol-2-yl]ethylcarbamat;
Butyl 2-[4-(4'-Methyl-1,1'-biphenyl-4-yl)-1*H*-imidazol-2-yl]ethylcarbamat;
Butyl 2-[4-(4'-Chloro-1,1'-biphenyl-4-yl)-1*H*-imidazol-2-yl]ethylcarbamat;
Butyl 2-[4-(2'-Fluoro-1,1'-biphenyl-4-yl)-1*H*-imidazol-2-yl]ethylcarbamat;
2-[4-(2',4'-Difluoro-1,1'-biphenyl-4-yl)-1*H*-imidazol-2-yl]butylethylcarbamat;
2,6-Di-*tert*-butyl-4-{2-[(propylamino)methyl]-1,3-thiazol-4-yl}phenol;
*N*-{[4-(10*H*-Phenothiazin-2-yl)-1,3-thiazol-2-yl]methyl}-*N*-propylamin;
*N*-{[4-(10*H*-Phenothiazin-2-yl)-1,3-thiazol-2-yl]methyl}butan-1-amin;
*N*-{[4-(10*H*-Phenothiazin-2-yl)-1,3-thiazol-2-yl]methyl}pentan-1-amin;
1-{[4-(3,5-Di-*tert*-butyl-4-hydroxyphenyl)-1,3-thiazol-2-yl]methyl}piperidin-3-ol;
1-{[4-(3,5-Di-*tert*-butyl-4-hydroxyphenyl)-1,3-thiazol-2-yl]methyl}pyrrolidin-3-ol;
[4-(10*H*-phenothiazin-2-yl)-1,3-thiazol-2-yl]methanol;
*N*,*N*-Dimethyl-*N*-{[4-(10*H*-phenothiazin-2-yl)-1,3-thiazol-2-yl]methyl}amin;
2-{2-[(4-Methylpiperazin-1-yl)methyl]-1,3-thiazol-4-yl}-10*H*-phenothiazin;
-[2-(Piperidin-1-ylmethyl)-1,3-thiazol-4-yl]-10*H*-phenothiazin;
2-[2-(Piperazin-1-ylmethyl)-1,3-thiazol-4-yl]-10*H*-phenothiazin;
1-{[4-(3,5-Di-*tert*-butyl-4-hydroxyphenyl)-1,3-thiazol-2-yl]methyl}azetidin-3-ol;
2-[2-(Morpholin-4-ylmethyl)-1,3-thiazol-4-yl]-10*H*-phenothiazin;
2-[2-(Thiomorpholin-4-ylmethyl)-1,3-thiazol-4-yl]-10*H*-phenothiazin;
2-{2-[(4-Methyl-1,4-diazepan-1-yl)methyl]-1,3-thiazol-4-yl}-10*H*-phenothiazin;
(3R)-1-{[4-(3,5-Di-tert-butyl-4-hydroxyphenyl)-1,3-thiazol-2-yl]methyl}pyrrolidin-3-ol;
(3S)-1-{[4-(3,5-Di-tert-butyl-4-hydroxyphenyl)-1,3-thiazol-2-yl]methyl}pyrrolidin-3-ol;
2,6-Di-*tert*-butyl-4-[2-(pyrrolidin-1-ylmethyl)-1,3-thiazol-4-yl]phenol;
2,6-Di-*tert*-butyl-4-{2-[(butylamino)methyl]-1,3-thiazol-4-yl]phenol;
2-{2-[(4-Ethylpiperazin-1-yl)methyl]-1,3-thiazol-4-yl}-10*H*-phenothiazin;
*N*-Methyl-*N*-{[4-(10*H*-phenothiazin-2-yl)-1*H*-imidazol-2-yl]methyl}amin;
Methyl [4-(10*H*-Phenothiazin-2-yl)-1,3-thiazol-2-yl]methylcarbamat;
Butyl [4-(10*H*-Phenothiazin-2-yl)-1,3-thiazol-2-yl]methylcarbamat;
*N*-Neopentyl-N-{[4-(10*H*-phenothiazin-2-yl)-1,3-thiazol-2-yl]methyl}amin;
1-{[4-(10*H*-Phenothiazin-2-yl)-1,3-thiazol-2-yl]methyl}piperidin-4-ol;
*N*-{[4-(10*H*-Phenothiazin-2-yl)-1,3-thiazol-2-yl]methyl}acetamid;
*N*-{[4-(10*H*-Phenothiazin-2-yl)-1,3-thiazol-2-yl]methyl}butanamid;
2,6-Di-*tert*-butyl-4-{2-[(4-propylpiperazin-1-yl)methyl]-1,3-thiazol-4-yl}phenol;
2,6-Di-*tert*-butyl-4-{2-[2-methyl-1-(methylamino)propyl]-1,3-thiazol-4-yl}phenol;
*N*,2-Dimethyl-1-[4-(10*H*-phenothiazin-2-yl)-1,3-thiazol-2-yl]propan-1-amin;
*N*-{[4-(10*H*-Phenothiazin-2-yl)-1,3-thiazol-2-yl]methyl}hexanamid;
(3*R*)-1-{[4-(10*H*-Phenothiazin-2-yl)-1,3-thiazol-2-yl]methyl}pyrrolidin-3-ol;
(3S)-1-{[4-(10*H*-Phenothiazin-2-yl)-1,3-thiazol-2-yl]methyl}pyrrolidin-3-ol;
1-{[4-(10*H*-Phenothiazin-2-yl)-1,3-thiazol-2-yl]methyl}azetidin-3-ol;
2-{2-[(4-Propylpiperazin-1-yl)methyl]-1,3-thiazol-4-yl}-10*H*-phenothiazin;
2-{2-[(4-Acetylpiperazin-1-yl)methyl]-1,3-thiazol-4-yl}-10*H*-phenothiazin;
2-{2-[(4-Butylpiperazin-1-yl)methyl]-1,3-thiazol-4-yl}-10*H*-phenothiazin;
Methyl 4-{[4-(10*H*-Phenothiazin-2-yl)-1,3-thiazol-2-yl]methyl}piperazin-1-carboxylat;
4-[2-(Aminomethyl)-1-imidazol-4-yl]-2,6-di-*tert*-butylphenol;
4-[2-[(Benzylamino)methyl]-1,3-thiazol-4-yl}-2,6-di-*tert*-butylphenol;
4-[2-[(4-Acetylpiperazin-1-yl)methyl]-1,3-thiazol-4-yl}-2,6-di-*tert*-butylphenol;
*N*-Methyl-N-{[4-(10*H*-phenoxazin-2-yl)-1,3-thiazol-2-yl]methyl}amin;
4-[2-(Azeitidin-1-ylmethyl)-1,3-thiazol-4-yl]-2,6-di-*tert*-butylphenol;
2,6-Di-*tert*-butyl-4-{2-[(4-butylpiperazin-1-yl)methyl]-1,3-thiazol-4-yl}phenol;
Butyl 2-[4-(3'-Chloro-1,1'-biphenyl-4-yl)-1*H*-imidazol-2-yl]ethylcarbamat;
Butyl 2-[4-(3'-Fluoro-1,1'-biphenyl-4-yl)-1*H*-imidazol-2-yl]ethylcarbamat;
Butyl 2-[4-(4-Isobutylphenyl)-1*H*-imidazol-2-yl]ethylcarbamat;
Benzyl 2-[4-(4-lsobutylphenyl)-1*H*-imidazol-2-yl]ethylcarbamat;
Butyl 2-[4-(3'-Chloro-4'-fluoro-1,1'-biphenyl-4-yl)-1*H*-imidazol-2-yl]ethylcarbamat;
Butyl 2-[4-(3',4'-Dichloro-1,1'-biphenyl-4-yl)-1*H*-imidazol-2-yl]ethylcarbamat;
Butyl 2-[4-(4-Propylphenyl)-1*H*-imidazol-2-yl]ethylcarbamat;
Butyl 2-[4-(4-Ethylphenyl)-1*H*-imidazol-2-yl]ethylcarbamat;
Butyl 2-[4-(4'-Cyano-1,1'-biphenyl-4-yl)-1*H*-imidazol-2-yl]ethylcarbamat;
Butyl 2-[4-(1,1'-Biphenyl-4-yl)-5-ethyl-1*H*-imidazol-2-yl]ethylcarbamat;
Butyl 2-[4-(2'-Chloro-1,1'-biphenyl-4-yl)-1*H*-imidazol-2-yl]ethylcarbamat;
Butyl 2-[4-(2',3'-Difluoro-1,1'-biphenyl-4-yl)-1*H*-imidazol-2-yl]ethylcarbamat;
Butyl 2-[4-(2'-Bromo-1,1'-biphenyl-4-yl)-1*H*-imidazol-2-yl]ethylcarbamat;
Butyl 2-[4-(3',5'-Difluoro-1,1'-biphenyl-4-yl)-1*H*-imidazol-2-yl]ethylcarbamat;
Butyl 2-[4-(2'-Methoxy-1,1'-biphenyl-4-yl)-1*H*-imidazol-2-yl]ethylcarbamat;
Butzyl 2-[4-(3'-Nitro-1,1'-biphenyl-4-yl)-1*H*-imidazol-2-yl]ethylcarbamat;
Butyl 2-[4-(2',5'-Difluoro-1,1'-biphenyl-4-yl)-1*H*-imidazol-2-yl]ethylcarbamat;
Butyl 2-[4-(3'-Methoxy-1,1'-biphenyl-4-yl)-1*H*-imidazol-2-yl]ethylcarbamat;
Methyl 4-{[4-(3,5-Di-*tert*-butyl-4-hydroxyphenyl)-1,3-thiazol-2-yl]methyl}piperazin-1-carboxylat;
Methyl [4-(3,5-Di-*tert*-butyl-4-hydroxyphenyl)-1,3-thiazol-2-yl]methylcarbamat;
*N*-{[4-(3,5-Di-*tert*-butyl-4-hydroxyphenyl)-1,3-thiazol-2-yl]methyl}benzamid;
*N*-{[4-(3,5-Di-*tert*-butyl-4-hydroxyphenyl)-1,3-thiazol-2-yl]methyl}-2-phenylacetamid;
*N*-{[4-(3,5-Di-*tert*-butyl-4-hydroxyphenyl)-1,3-thiazol-2-yl]methyl}propanamid;
1-{[4-(3,5-Di-*tert*-butyl-4-hydroxyphenyl)-1,3-thiazol-2-yl]methyl}piperidin-4-yl-acetat;
1-{[4-(3,5-Di-*tert*-butyl-4-hydroxyphenyl)-1,3-thiazol-2-yl]methyl}pyrrolidin-3,4-diol;
oder ein Salz einer solchen Verbindung.

2. Eine Verbindung in Form eines Medikaments, **dadurch gekennzeichnet, dass** sie aus den folgenden Verbindungen ausgewählt ist:
2,6-Di-*tert*-butyl-4-{2-[2-(methylamino)ethyl]-1,3-thiazol-4-yl}phenol;
2,6-Di-*tert*-butyl-4-[4-(hydroxymethyl)-1,3-oxazol-2-yl]phenol;
2,6-Di-*tert*-butyl-4-{2-[1-(methylamino)ethyl]-1,3-thiazol-4-yl}phenol;
2,6-Di-*tert*-butyl-4-[2-(methoxymethyl)-1,3-thiazol-4-yl]phenol;
2,6-Di-*tert*-butyl-4-{4-[(methylamino)methyl]-1,3-oxazol-2-yl}phenol;
N-{[4-(3,5-Di-*tert*-butyl-4-hydroxyphenyl)-1,3-thiazol-2-yl]methyl}acetamid;
Ethyl [4-(3,5-Di-*tert*-butyl-4-hydroxyphenyl)-1,3-thiazol-2-yl]methylcarbamat;
2,6-Di-*tert*-butyl-4-[2-(morpholin-4-ylmethyl)-1,3-thiazol-4-yl]phenol;
2,6-Di-*tert*-butyl-4-[2-(thiomorpholin-4-ylmethyl)-1,3-thiazol-4-yl]phenol;
4-[2-(Anilinomethyl)-1,3-thiazol-4-yl]-2,6-di-*tert*-butylphenol;
2,6-Di-*tert*-butyl-4-(2-{[[2-(dimethylamino)ethyl](methyl)amino]methyl}-1,3-thiazol-4-yl)phenol;
2,6-Di-*tert*-butyl-4-{5-methyl-2-[(methylamino)methyl]-1,3-thiazol-4-yl}phenol;
1-[4-(10*H*-Phenothiazin-2-yl)-1,3-thiazol-2-yl]methanamin;
*N*-{[4-(3,5-Di-*tert*-butyl-4-hydroxyphenyl)-1,3-thiazol-2-yl]methyl}-*N*-methylacetamid;
1-[4-(3,5-Di-*tert*-butyl-4-methoxyphenyl)-1,3-thiazol-2-yl]-N-methanamin;
2,6-Di-*tert*-butyl-4-{2-[(ethylamino)methyl]-1,3-thiazol-4-yl}phenol;
2,6-Di-*tert*-butyl-4-{2-[(4-phenylpiperazin-1-yl)methyl]-1,3-thiazol-4-yl}phenol;
2,6-Di-*tert*-butyl-4-{2-[(4-methyl-1,4-diazepan-1-yl)methyl]-1,3-thiazol-4-yl}phenol;
N-{1-[4-(4-Anilinophenyl)-1,3-thiazol-2-yl]ethyl}-N-methylamin;
2,6-Di-*tert*-butyl-4-{2-[(isopropylamino)methyl]-1,3-thiazol-4-yl}phenol;
2,6-Di-*tert*-butyl-4-{2-[(cyclohexylamino)methyl]-1,3-thiazol-4-yl}phenol;
2,6-Di-*tert*-butyl-4-{2-[(4-isopropylpiperazin-1-yl)methyl]-1,3-thiazol-4-yl}phenol;
*N*-Methyl-1-[4-(10*H*-phenothiazin-2-yl)-1,3-thiazol-2-yl]ethanamin;
2,6-Di-*tert*-butyl-4-{2-[(4-ethylpiperazin-1-yl)methyl]-1,3-thiazol-4-yl}phenol;
*N*-{[4-(4-Anilinophenyl)-1,3-thiazol-2-yl]methyl}-*N*-ethylamin;
*N*-{[4-(10*H*-Phenothiazin-2-yl)-1,3-thiazol-2-yl]methyl}ethanamin;
2,6-Di-*tert*-butyl-4-(2-{[4-(dimethylamino)piperidin-1-yl]methyl}-1,3-thiazol-4-yl)phenol;
1-{[4-(3,5-Di-*tert*-butyl-4-hydroxyphenyl)-1,3-thiazol-2-yl]methyl}piperidin-4-ol;
4-Methylpentyl 2-[4-(1,1'-Biphenyl-4-yl)-1*H*-imidazol-2-yl]ethylcarbamat;
3,3-Dimethylbutyl 2-[4-(4-Pyrrolidin-1-ylphenyl)-1*H*-imidazol-2-yl]ethylcarbamat;
Isopentyl 2-[4-(1,1'-Biphenyl-4-yl)-1*H*-imidazol-2-yl]ethylcarbamat;
Hexyl 2-[4-(4'-Bromo-1,1'-biphenyl-4-yl)-1*H*-imidazol-2-yl]ethylcarbamat;
Benzyl 2-[4-(4-*tert*-Butylphenyl)-1*H*-imidazol-2-yl]ethylcarbamat;
3,3-Dimethylbutyl 2-[4-(1,1'-Biphenyl-4-yl)-1*H*-imidazol-2-yl]ethylcarbamat;
Hexyl 2-[4-(4-Pyrrolidin-1-ylphenyl)-1*H*-imidazol-2-yl]ethylcarbamat;
Hexyl 2-[4-(3,5-Di*tert*-butyl-4-hydroxyphenyl)-1*H*-imidazol-2-yl]ethylcarbamat;
3,3-Dimethylbutyl 2-[4-(3,5-Di*tert*-butyl-4-hydroxyphenyl)-1*H*-imidazol-2-yl]ethylcarbamat;
3,3-Dimethylbutyl 2-[4-(4-Methoxyphenyl)-1*H*-imidazol-2-yl]ethylcarbamat;
Benzyl 2-[4-(3,5-Di*tert*-butyl-4-hydroxyphenyl)-1*H*-imidazol-2-yl]ethylcarbamat;
Benzyl 2-[4-(4-Pyrrolidin-1-ylphenyl)-1*H*-imidazol-2-yl]ethylcarbamat;
2-Phenylethyl 2-[4-(1,1'-Biphenyl-4-yl)-1*H*-imidazol-2-yl]ethylcarbamat;
Butyl 2-[4-(4'-Fluoro-1,1'-biphenyl-4-yl)-1*H*-imidazol-2-yl]ethylcarbamat;
Butyl 2-[4-(1,1'-Biphenyl-4-yl)-5-methyl-1*H*-imidazol-2-yl]ethylcarbamat;
Butyl 2-[4-(4'-Methyl-1,1'-biphenyl-4-yl)-1*H*-imidazol-2-yl]ethylcarbamat;
Butyl 2-[4-(4'-Chloro-1,1'-biphenyl-4-yl)-1*H*-imidazol-2-yl]ethylcarbamat;
Butyl 2-[4-(2'-Fluoro-1,1'-biphenyl-4-yl)-1*H*-imidazol-2-yl]ethylcarbamat;
Butyl 2-[4-(2',4'-Difluoro-1,1'-biphenyl-4-yl)-1*H*-imidazol-2-yl]ethylcarbamat;
2,6-Di-*tert*-butyl-4-{2-[(propylamino)methyl]-1,3-thiazol-4-yl}phenol;
*N*-{[4-(10*H*-Phenothiazin-2-yl)-1,3-thiazol-2-yl]methyl}-*N*-propylamin;
*N*-{[4-(10*H*-Phenothiazin-2-yl)-1,3-thiazol-2-yl]methyl}butan-1-amin;
*N*-{[4-(10*H*-Phenothiazin-2-yl)-1,3-thiazol-2-yl]methyl}pentan-1-amin;
1-{[4-(3,5-Di-*tert*-butyl-4-hydroxyphenyl)-1,3-thiazol-2-yl]methyl}piperidin-3-ol;
1-{[4-(3,5-Di-*tert*-butyl-4-hydroxyphenyl)-1,3-thiazol-2-yl]methyl}pyrrolidin-3-ol;
[4-(10*H*-phenothiazin-2-yl)-1,3-thiazol-2-yl]methanol;
*N*,*N*-Dimethyl-*N*-{[4-(10*H*-phenothiazin-2-yl)-1,3-thiazol-2-yl]methyl}amin;
2-{2-[(4-Methylpiperazin-1-yl)methyl]-1,3-thiazol-4-yl}-10*H*-phenothiazin;
2-[2-(Piperidin-1-ylmethyl)-1,3-thiazol-4-yl]-10*H*-phenothiazin;
2-[2-(Piperazin-1-ylmethyl)-1,3-thiazol-4-yl]-10*H*-phenothiazin;
1-{[4-(3,5-Di-*tert*-butyl-4-hydroxyphenyl)-1,3-thiazol-2-yl]methyl}azetidin-3-ol;
2-[2-(Morpholin-4-ylmethyl)-1,3-thiazol-4-yl]-10*H*-phenothiazin;
2-[2-(Thiomorpholin-4-ylmethyl)-1,3-thiazol-4-yl]-10*H*-phenothiazin;
2-{2-[(4-Methyl-1,4-diazepan-1-yl)methyl]-1,3-thiazol-4-yl}-10*H*-phenothiazin;
(3R)-1-{[4-(3,5-Di-tert-butyl-4-hydroxyphenyl)-1,3-thiazol-2-yl]methyl}pyrrolidin-3-ol;
(3S)-1-{[4-(3,5-Di-tert-butyl-4-hydroxyphenyl)-1,3-thiazol-2-yl]methyl}pyrrolidin-3-ol;
2,6-Di-*tert*-butyl-4-[2-(pyrrolidin-1-ylmethyl)-1,3-thiazol-4-yl]phenol;
2,6-Di-*tert*-butyl-4-{2-[(butylamino)methyl]-1,3-thiazol-4-yl]phenol;
2-{2-[(4-Ethylpiperazin-1-yl)methyl]-1,3-thiazol-4-yl}-10*H*-phenothiazin;
*N*-Methyl-*N*-{[4-(10*H*-phenothiazin-2-yl)-1*H*-imidazol-2-yl]methyl}amin;
Methyl [4-(10*H*-Phenothiazin-2-yl)-1,3-thiazol-2-yl]methylcarbamat;
Butyl [4-(10*H*-Phenothiazin-2-yl)-1,3-thiazol-2-yl]methylcarbamat;
*N*-Neopentyl-N-{[4-(10*H*-phenothiazin-2-yl)-1,3-thiazol-2-yl]methyl}amin;
1-{[4-(10*H*-Phenothiazin-2-yl)-1,3-thiazol-2-yl]methyl}piperidin-4-ol;
*N*-{[4-(10*H*-Phenothiazin-2-yl)-1,3-thiazol-2-yl]methyl}acetamid;
*N*-{[4-(10*H*-Phenothiazin-2-yl)-1,3-thiazol-2-yl]methyl}butanamid;
2,6-Di-*tert*-butyl-4-{2-[(4-propylpiperazin-1-yl)methyl]-1,3-thiazol-4-yl}phenol;
2,6-Di-*tert*-butyl-4-{2-[2-methyl-1-(methylamino)propyl]-1,3-thiazol-4-yl}phenol;
*N*,2-Dimethyl-1-[4-(10*H*-phenothiazin-2-yl)-1,3-thiazol-2-yl]propan-1-amin;
*N*-{[4-(10*H*-Phenothiazin-2-yl)-1,3-thiazol-2-yl]methyl}hexanamid;
(3*R*)-1-{[4-(10*H*-Phenothiazin-2-yl)-1,3-thiazol-2-yl]methyl}pyrrolidin-3-ol;
(3*S*)-1-{[4-(10*H*-Phenothiazin-2-yl)-1,3-thiazol-2-yl]methyl}pyrrolidin-3-ol;
1-{[4-(10*H*-Phenothiazin-2-yl)-1,3-thiazol-2-yl]methyl}azetidin-3-ol;
2-{2-[(4-Propylpiperazin-1-yl)methyl]-1,3-thiazol-4-yl}-10*H*-phenothiazin;
2-{2-[(4-Acetylpiperazin-1-yl)methyl]-1,3-thiazol-4-yl}-10*H*-phenothiazin;
2-{2-[(4-Butylpiperazin-1-yl)methyl]-1,3-thiazol-4-yl}-10*H*-phenothiazin;
Methyl 4-{[4-(10*H*-Phenothiazin-2-yl)-1,3-thiazol-2-yl]methyl}piperazin-1-carboxylat;
4-[2-(Aminomethyl)-1-imidazol-4-yl]-2,6-di-*tert*-butylphenol;
4-[2-[(Benzylamino)methyl]-1,3-thiazol-4-yl}-2,6-di-*tert*-butylphenol;
4-[2-[(4-Acetylpiperazin-1-yl)methyl]-1,3-thiazol-4-yl}-2,6-di-*tert*-butylphenol;
*N*-Methyl-N-{[4-(10*H*-phenoxazin-2-yl)-1,3-thiazol-2-yl]methyl}amin;
4-[2-(Azetidin-1-ylmethyl)-1,3-thiazol-4-yl]-2,6-di-*tert*-butylphenol;
2,6-Di-*tert*-butyl-4-{2-[(4-butylpiperazin-1-yl)methyl]-1,3-thiazol-4-yl}phenol;
Butyl 2-[4-(3'-Chloro-1,1'-biphenyl-4-yl)-1*H*-imidazol-2-yl]ethylcarbamat;
Butyl 2-[4-(3'-Fluoro-1,1'-biphenyl-4-yl)-1*H*-imidazol-2-yl]ethylcarbamat;
Butyl 2-[4-(4-Isobutylphenyl)-1*H*-imidazol-2-yl]ethylcarbamat;
Benzyl 2-[4-(4-Isobutylphenyl)-1*H*-imidazol-2-yl]ethylcarbamat;
Butyl 2-[4-(3'-Chloro-4'-fluoro-1,1'-biphenyl-4-yl)-1*H*-imidazol-2-yl]ethylcarbamat;
Butyl 2-[4-(3',4'-Dichloro-1,1'-biphenyl-4-y)-1*H*-imidazol-2-yl]ethylcarbamat;
Butyl 2-[4-(4-Propylphenyl)-1*H*-imidazol-2-yl]ethylcarbamat;
Butyl 2-[4-(4-Ethylphenyl)-1*H*-imidazol-2-yl]ethylcarbamat;
Butyl 2-[4-(4'-Cyano-1,1'-biphenyl-4-yl)-1*H*-imidazol-2-yl]ethylcarbamat;
Butyl 2-[4-(1,1'-Biphenyl-4-yl)-5-ethyl-1*H*-imidazol-2-yl]ethylcarbamat;
Butyl 2-[4-(2'-Chloro-1,1'-biphenyl-4-yl)-1*H*-imidazol-2-yl]ethylcarbamat;
Butyl 2-[4-(2',3'-Difluoro-1,1'-biphenyl-4-yl)-1*H*-imidazol-2-yl]ethylcarbamat;
Butyl 2-[4-(2'-Bromo-1,1'-biphenyl-4-yl)-1*H*-imidazol-2-yl]ethylcarbamat;
Butyl 2-[4-(3',5'-Difluoro-1,1'-biphenyl-4-yl)-1*H*-imidazol-2-yl]ethylcarbamat;
Butyl 2-[4-(2'-Methoxy-1,1'-biphenyl-4-yl)-1*H*-imidazol-2-yl]ethylcarbamat;
Butyl 2-[4-(3'-Nitro-1,1'-biphenyl-4-yl)-1*H*-imidazol-2-yl]ethylcarbamat;
Butyl 2-[4-(2',5'-Difluoro-1,1'-biphenyl-4-yl)-1*H*-imidazol-2-yl]ethylcarbamat;
Butyl 2-[4-(3'-Methoxy-1,1'-biphenyl-4-yl)-1*H*-imidazol-2-yl]ethylcarbamat;
Methyl 4-{[4-(3,5-Di-*tert*-butyl-4-hydroxyphenyl)-1,3-thiazol-2-yl]methyl}piperazin-1-carboxylat;
[4-(3,5-Di-*tert*-butyl-4-hydroxyphenyl)-1,3-thiazol-2-yl]methylmethylcarbamat;
*N*-{[4-(3,5-Di-*tert*-butyl-4-hydroxyphenyl)-1,3-thiazol-2-yl]methyl}benzamid;
*N*-{[4-(3,5-Di-*tert*-butyl-4-hydroxyphenyl)-1,3-thiazol-2-yl]methyl}-2-phenylacetamid;
*N*-{[4-(3,5-Di-*tert*-butyl-4-hydroxyphenyl)-1,3-thiazol-2-yl]methyl}propanamid;
1-{[4-(3,5-Di-*tert*-butyl-4-hydroxyphenyl)-1,3-thiazol-2-yl]methyl}piperidin-4-yl-acetat;
1-{[4-(3,5-Di-*tert*-butyl-4-hydroxyphenyl)-1,3-thiazol-2-yl]methyl}pyrrolidin-3,4-diol;
oder ein pharmazeutisch annehmbares Salz einer solchen Verbindung.

3. Pharmazeutische Zusammensetzung, die als Wirkstoff mindestens eine der Verbindungen nach Anspruch 2 enthält.

4. Verwendung einer Verbindung, **dadurch gekennzeichnet, dass** sie aus den folgenden Verbindungen ausgewählt ist:
2,6-Di-*tert*-butyl-4-{2-[2-(methylamino)ethyl]-1,3-thiazol-4-yl}phenol;
2,6-Di-*tert*-butyl-4-[4-(hydroxymethyl)-1,3-oxazol-2-yl]phenol;
2,6-Di-*tert*-butyl-4-{2-[1-(methylamino)ethyl]-1,3-thiazol-4-yl}phenol;
2,6-Di-*tert*-butyl-4-[2-(methoxymethyl)-1,3-thiazol-4-yl]phenol;
2,6-Di-*tert*-butyl-4-(4-[(methylamino)methyl]-l,3-oxazol-2-yl)phenol;
N-{[4-(3,5-Di*tert*-butyl-4-hydroxyphenyl)-1,3-thiazol-2-yl]methyl}acetamid;
Ethyl [4-(3,5-Di-*tert*-butyl-4-hydroxyphenyl)-1,3-thiazol-2-yl]methylcarbamat;
2,6-Di-*tert*-butyl-4-[2-(morpholin-4-ylmethyl)-1,3-thiazol-4-yl]phenol;
2,6-Di-*tert*-butyl-4-[2-(thiomorpholin-4-ylmethyl)-1,3-thiazol-4-yl]phenol;
4-[2-(Anilinomethyl)-1,3-thiazol-4-yl]-2,6-di-*tert*-butylphenol;
2,6-Di-*tert*-butyl-4-(2-{[[2-(dimethylamino)ethyl](methyl)amino]methyl}-1,3-thiazol-4-yl)phenol;
2,6-Di-*tert*-butyl-4-{5-methyl-2-[(methylamino)methyl]-1,3-thiazol-4-yl}phenol;
1-[4-(10*H*-Phenothiazin-2-yl)-1,3-thiazol-2-yl]methanamin;
*N*-{[4-(3,5-Di-*tert*-butyl-4-hydroxyphenyl)-1,3-thiazol-2-yl]methyl}-*N*-methylacetamid;
1-[4-(3,5-Di-*tert*-butyl-4-methoxyphenyl)-1,3-thiazol-2-yl]-N-methanamin;
2,6-Di-*tert*-butyl-4-{2-[(ethylamino)methyl]-1,3-thiazol-4-yl}phenol;
2,6-Di-*tert*-butyl-4-{2-[(4-phenylpiperazin-1-yl)methyl]-1,3-thiazol-4-yl}phenol;
2,6-Di-*tert*-butyl-4-{2-[(4-methyl-1,4-diazepan-1-yl)methyl]-1,3-thiazol-4-yl}phenol;
N-{1-[4-(4-Anilinophenyl)-1,3-thiazol-2-yl]ethyl}-N-methylamin;
2,6-Di-*tert*-butyl-4-{2-[(isopropylamino)methyl]-1,3-thiazol-4-yl}phenol;
2,6-Di-*tert*-butyl-4-{2-[(cyclohexylamino)methyl]-1,3-thiazol-4-yl}phenol;
2,6-Di-*tert*-butyl-4-{2-[(4-isopropylpiperazin-1-yl)methyl]-1,3-thiazol-4-yl}phenol;
*N*-Methyl-1-[4-(10*H*-phenothiazin-2-yl)-1,3-thiazol-2-yl]ethanamin;
2,6-Di-*tert*-butyl-4-{2-[(4-ethylpiperazin-1-yl)methyl]-1,3-thiazol-4-yl}phenol;
*N*-{[4-(4-Anilinophenyl)-1,3-thiazol-2-yl]methyl}-*N*-ethylamin;
*N*-{[4-(10*H*-Phenothiazin-2-yl)-1,3-thiazol-2-yl]methyl}ethanamin;
2,6-Di-*tert*-butyl-4-(2-{[4-(dimethylamino)piperidin-1-yl]methyl}-1,3-thiazol-4-yl)phenol;
1-{[4-(3,5-Di-*tert*-butyl-4-hydroxyphenyl)-1,3-thiazol-2-yl]methyl}piperidin-4-ol;
4-Methylpentyl 2-[4-(1,1'-Biphenyl-4-yl)-1*H*-imidazol-2-yl]ethylcarbamat;
3,3-Dimethylbutyl 2-[4-(4-Pyrrolidin-1-ylphenyl)-1*H*-imidazol-2-yl] ethylcarbamat;
Isopentyl 2-[4-(1,1'-Biphenyl-4-yl)-1*H*-imidazol-2-yl]ethylcarbamat;
Hexyl 2-[4-(4'-Bromo-1,1'-biphenyl-4-yl)-1*H*-imidazol-2-yl]ethylcarbamat;
Benzyl 2-[4-(4-*tert*-Butylphenyl)-1*H*-imidazol-2-yl]ethylcarbamat;
,3-Dimethylbutyl 2-[4-(1,1'-Biphenyl-4-yl)-1*H*-imidazol-2-yl]ethylcarbamat;
Hexyl 2-[4-(4-Pyrrolidin-1-ylphenyl)-1*H*-imidazol-2-yl]ethylcarbamat;
Hexyl 2-[4-(3,5-Di-*tert*-butyl-4-hydroxyphenyl)-1*H*-imidazol-2-yl]ethylcarbamat;
3,3-Dimethylbutyl 2-[4-(3,5-Di-*tert*-butyl-4-hydroxyphenyl)-1*H*-imidazol-2-yl]ethylcarbamat;
3,3-Dimethylbutyl 2-[4-(4-Methoxyphenyl)-1*H*-imidazol-2-yl]ethylcarbamat;
Benzyl 2-[4-(3,5-Di-*tert*-butyl-4-hydroxyphenyl)-1*H*-imidazol-2-yl]ethylcarbamat;
Benzyl 2-[4-(4-Pyrrolidin-1-ylphenyl)-1*H*-imidazol-2-yl]ethylcarbamat;
2-Phenylethyl 2-[4-(1,1'-Biphenyl-4-yl)-1*H*-imidazol-2-yl]ethylcarbamat;
Butyl 2-[4-(4'-Fluoro-1,1'-biphenyl-4-yl)-1*H*-imidazol-2-yl]ethylcarbamat;
Butyl 2-[4-(1,1'-Biphenyl-4-yl)-5-methyl-1*H*-imidazol-2-yl]ethylcarbamat;
Butyl 2-[4-(4'-Methyl-1,1'-biphenyl-4-yl)-1*H*-imidazol-2-yl]ethylcarbamat;
Butyl 2-[4-(4'-Chloro-1,1'-biphenyl-4-yl)-1*H*-imidazol-2-yl]ethylcarbamat;
Butyl 2-[4-(2'-Fluoro-1,1'-biphenyl-4-yl)-1*H*-imidazol-2-yl]ethylcarbamat;
Butyl 2-[4-(2',4'-Difluoro-1,1'-biphenyl-4-yl)-1*H*-imidazol-2-yl]ethylcarbamat;
2,6-Di-*tert*-butyl-4-{2-[(propylamino)methyl]-1,3-thiazol-4-yl}phenol;
*N*-{[4-(10*H*-Phenothiazin-2-yl)-1,3-thiazol-2-yl]methyl}-*N*-propylamin;
*N*-{[4-(10*H*-Phenothiazin-2-yl)-1,3-thiazol-2-yl]methyl}butan-1-amin;
*N*-{[4-(10*H*-Phenothiazin-2-yl)-1,3-thiazol-2-yl]methyl}pentan-1-amin;
1-{[4-(3,5-Di-*tert*-butyl-4-hydroxyphenyl)-1,3-thiazol-2-yl]methyl}piperidin-3-ol;
1-{[4-(3,5-Di-*tert*-butyl-4-hydroxyphenyl)-1,3-thiazol-2-yl]methyl}pyrrolidin-3-ol;
[4-(10*H*-phenothiazin-2-yl)-1,3-thiazol-2-yl]methanol;
*N*,*N*-Dimethyl-*N*-{[4-(10*H*-phenothiazin-2-yl)-1,3-thiazol-2-yl]methyl}amin;
2 -{2-[(4-Methylpiperazin-1-yl)methyl]-1,3-thiazol-4-yl}-10*H*-phenothiazin;
2-[2-(Piperidin-1-ylmethyl)-1,3-thiazol-4-yl]-10*H*-phenothiazin;
2-[2-(Piperazin-1-ylmethyl)-1,3-thiazol-4-yl]-10*H*-phenothiazin;
1-{[4-(3,5-Di-*tert*-butyl-4-hydroxyphenyl)-1,3-thiazol-2-yl]methyl}azetidin-3-ol;
2-[2-(Morpholin-4-ylmethyl)-1,3-thiazol-4-yl]-10*H*-phenothiazin;
2-[2-(Thiomorpholin-4-ylmethyl)-1,3-thiazol-4-yl]-10*H*-phenothiazin;
2-{2-[(4-Methyl-1,4-diazepan-1-yl)methyl]-1,3-thiazol-4-yl}-10*H*-phenothiazin;
(3R)-1-{[4-(3,5-Di-tert-butyl-4-hydroxyphenyl)-1,3-thiazol-2-yl]methyl}pyrrolidin-3-ol;
(3S)-1-{[4-(3,5-Di-tert-butyl-4-hydroxyphenyl)-1,3-thiazol-2-yl]methyl}pyrrolidin-3-ol;
2,6-Di-*tert*-butyl-4-[2-(pyrrolidin-1-ylmethyl)-1,3-thiazol-4-yl]phenol;
2,6-Di-*tert*-butyl-4-{2-[(butylamino)methyl]-1,3-thiazol-4-yl]phenol;
2-{2-[(4-Ethylpiperazin-1-yl)methyl]-1,3-thiazol-4-yl}-10*H*-phenothiazin;
*N*-Methyl-*N*-{[4-(10*H*-phenothiazin-2-yl)-1*H*-imidazol-2-yl]methyl}amin;
Methyl [4-(10*H*-Phenothiazin-2-yl)-1,3-thiazol-2-yl]methylcarbamat;
Butyl [4-(10*H*-Phenothiazin-2-yl)-1,3-thiazol-2-yl]methylcarbamat;
*N*-Neopentyl-*N*-{[4-(10*H*-phenothiazin-2-yl)-1,3-thiazol-2-yl]methyl}amin;
1-{[4-(10*H*-Phenothiazin-2-yl)-1,3-thiazol-2-yl]methyl}piperidin-4-ol;
*N*-{[4-(10*H*-Phenothiazin-2-yl)-1,3-thiazol-2-yl]methyl}acetamid;
*N*-{[4-(10*H*-Phenothiazin-2-yl)-1,3-thiazol-2-yl]methyl}butanamid;
2,6-Di-*tert*-butyl-4-{2-[(4-propylpiperazin-1-yl)methyl]-1,3-thiazol-4-yl}phenol;
2,6-Di-*tert*-butyl-4-{2-[2-methyl-1-(methylamino)propyl]-1,3-thiazol-4-yl}phenol;
*N*,2-Dimethyl-1-[4-(10*H*-phenothiazin-2-yl)-1,3-thiazol-2-yl]propan-1-amin;
*N*-{[4-(10*H*-Phenothiazin-2-yl)-1,3-thiazol-2-yl]methyl}hexanamid;
(3*R*)-1-{[4-(10*H*-Phenothiazin-2-yl)-1,3-thiazol-2-yl]methyl}pyrrolidin-3-ol;
(3*S*)-1-{[4-(10*H*-Phenothiazin-2-yl)-1,3-thiazol-2-yl]methyl}pyrrolidin-3-ol;
1-{[4-(10*H*-Phenothiazin-2-yl)-1,3-thiazol-2-yl]methyl}azetidin-3-ol;
2-{2-[(4-Propylpiperazin-1-yl)methyl]-1,3-thiazol-4-yl}-10*H*-phenothiazin;
2-{2-[(4-Acetylpiperazin-1-yl)methyl]-1,3-thiazol-4-yl}-10*H*-phenothiazin;
2-{2-[(4-Butylpiperazin-1-yl)methyl]-1,3-thiazol-4-yl}-10*H*-phenothiazin;
Methyl 4-{[4-(10*H*-Phenothiazin-2-yl)-1,3-thiazol-2-yl]methyl}piperazin-1-carboxylat;
4-[2-(Aminomethyl)-1-imidazol-4-yl]-2,6-di-*tert*-butylphenol;
4-[2-[(Benzylamino)methyl]-1,3-thiazol-4-yl}-2,6-di-*tert*-butylphenol;
4-[2-[(4-Acetylpiperazin-1-yl)methyl]-1,3-thiazol-4-yl}-2,6-di-*tert*-butylphenol;
*N*-Methyl-N-{[4-(10*H*-phenoxazin-2-yl)-1,3-thiazol-2-yl]methyl}amin;
4-[2-(Azetidin-1-ylmethyl)-1,3-thiazol-4-yl]-2,6-di-*tert*-butylphenol;
2,6-Di-*tert*-butyl-4-{2-[(4-butylpiperazin-1-yl)methyl]-1,3-thiazol-4-yl}phenol;
Butyl 2-[4-(3'-Chloro-1,1'-biphenyl-4-yl)-1*H*-imidazol-2-yl]ethylcarbamat;
Butyl 2-[4-(3'-Fluoro-1,1'-biphenyl-4-yl)-1*H*-imidazol-2-yl]ethylcarbamat;
Butyl 2-[4-(4-Isobutytphenyl)-1*H*-imidazol-2-yl]ethylcarbamat;
Benzyl 2-[4-(4-lsobutylphenyl)-1*H*-imidazol-2-yl]ethylcarbamat;
Butyl 2-[4-(3'-Chloro-4'-fluoro-1,1'-biphenyl-4-yl)-1*H*-imidazol-2-yl]ethylcarbamat;
Butyl 2-[4-(3',4'-Dichloro-1,1'-biphenyl-4-yl)-1*H*-imidazol-2-yl]ethylcarbamat;
Butyl 2-[4-(4-Propylphenyl)-1*H*-imidazol-2-yl]ethylcarbamat;
Butyl 2-[4-(4-Ethylphenyl)-1*H*-imidazol-2-yl]ethylcarbamat;
Butyl 2-[4-(4'-Cyano-1,1'-biphenyl-4-yl)-1*H*-imidazol-2-yl]ethylcarbamat;
Butyl 2-[4-(1,1'-Biphenyl-4-yl)-5-ethyl-1*H*-imidazol-2-yl]ethylcarbamat;
Butyl 2-[4-(2'-Chloro-1,1'-biphenyl-4-yl)-1*H*-imidazol-2-yl]ethylcarbamat;
Butyl 2-[4-(2',3'-Difluoro-1,1'-biphenyl-4-yl)-1*H*-imidazol-2-yl]ethylcarbamat;
Butyl 2-[4-(2'-Bromo-1,1'-biphenyl-4-yl)-1*H*-imidazol-2-yl]ethylcarbamat;
Butyl 2-[4-(3',5'-Difluoro-1,1'-biphenyl-4-yl)-1*H*-imidazol-2-yl]ethylcarbamat;
Butyl 2-[4-(2'-Methoxy-1,1'-biphenyl-4-yl)-1*H*-imidazol-2-yl]ethylcarbamat;
Butyl 2-[4-(3'-Nitro-1,1'-biphenyl-4-yl)-1*H*-imidazol-2-yl]ethylcarbamat;
Butyl 2-[4-(2',5'-Difluoro-1,1'-biphenyl-4-yl)-1*H*-imidazol-2-yl]ethylcarbamat;
Butyl 2-[4-(3'-Methoxy-1,1'-biphenyl-4-yl)-1*H*-imidazol-2-yl]lethylcarbamat;
Methyl 4-{[4-(3,5-Di-*tert*-butyl-4-hydroxyphenyl)-1,3-thiazol-2-yl]methyl}piperazin-1-carboxylat;
Methyl [4-(3,5-Di-*tert*-butyl-4-hydroxyphenyl)-1,3-thiazol-2-yl]methylcarbamat;
*N*-{[4-(3,5-Di-*tert*-butyl-4-hydroxyphenyl)-1,3-thiazol-2-yl]methyl}benzamid;
*N*-{[4-(3,5-Di-*tert*-butyl-4-hydroxyphenyl)-1,3-thiazol-2-yl]methyl}-2-phenylacetamid;
*N*-{[4-(3,5-Di-*tert*-butyl-4-hydroxyphenyl)-1,3-thiazol-2-yl]methyl}propanamid;
1-{[4-(3,5-Di-*tert*-butyl-4-hydroxyphenyl)-1,3-thiazol-2-yl]methyl}piperidin-4-yl-acetat;
1-{[4-(3,5-Di-*tert*-butyl-4-hydroxyphenyl)-1,3-thiazol-2-yl]methyl}pyrrolidin-3,4-diol;
oder ein pharmazeutisch annehmbares Salz einer dieser Verbindungen
zur Herstellung eines Medikaments, das zur Behandlung einer der folgenden Störungen oder einer der folgenden Krankheiten bestimmt ist: Parkinson-Krankheit, Altersdemenzen, Alzheimer-Krankheit, Chorea Huntington, Multiple Sklerose, Schizophrenie, Depressionen, Psychosen, Migräne oder Schmerzen und insbesondere neuropathische Schmerzen.

5. Verwendung einer der folgenden Verbindungen:
2,6-Di-*tert*-butyl-4-{2-[2-(methylamino)ethyl]-1,3-thiazol-4-yl}phenol;
2,6-Di-*tert*-butyl-4-[4-(hydroxymethyl)-1,3-oxazol-2-yl]phenol;
2,6-Di-*tert*-butyl-4-{2-[1-(methylamino)ethyl]-1,3-thiazol-4-yl}phenol;
2,6-Di-*tert*-butyl-4-[2-(methoxymethyl)-1,3-thiazol-4-yl]phenol;
2,6-Di-*tert*-butyl-4-{4-[(methylamino)methyl]-1,3-oxazol-2-yl}phenol;
N-{[4-(3,5-Di*tert*-butyl-4-hydroxyphenyl)-1,3-thiazol-2-yl]methyl}acetamid;
Ethyl [4-(3,5-Di-*tert*-butyl-4-hydroxyphenyl)-1,3-thiazol-2-yl]methylcarbamat;
2,6-Di-*tert*-butyl-4-[2-(morpholin-4-ylmethyl)-1,3-thiazol-4-yl]phenol;
2,6-Di-*tert*-butyl-4-[2-(thiomorpholin-4-ylmethyl)-1,3-thiazol-4-yl]phenol;
4-[2-(Anilinomethyl)-1,3-thiazol-4-yl]-2,6-di-*tert*-butylphenol;
2,6-Di-*tert*-butyl-4-(2-{[[2-(dimethylamino)ethyl](methyl)amino]methyl}-1,3-thiazol-4-yl)phenol;
2,6-Di-*tert*-butyl-4-{5-methyl-2-[(methylamino)methyl]-1,3-thiazol-4-yl}phenol;
1-[4-(10*H*-Phenothiazin-2-yl)-1,3-thiazol-2-yl]methanamin;
*N*-{[4-(3,5-Di-*tert*-butyl-4-hydroxyphenyl)-1,3-thiazol-2-yl]methyl}-*N*-methylacetamid;
1-[4-(3,5-Di-*tert*-butyl-4-methoxyphenyl)-1,3-thiazol-2-yl]-N-methanamin;
2,6-Di-*tert*-butyl-4-{2-[(ethylamino)methyl]-1,3-thiazol-4-yl}phenol;
2,6-Di-*tert*-butyl-4-{2-[(4-phenylpiperazin-1-yl)methyl]-1,3-thiazol-4-yl}phenol;
2,6-Di-*tert*-butyl-4-{2-[(4-methyl-1,4-diazepan-1-yl)methyl]-1,3-thiazol-4-yl}phenol;
N-{1-[4-(4-Anilinophenyl)-1,3-thiazol-2-yl]ethyl}-N-methylamin;
2,6-Di-*tert*-butyl-4-{2-[(isopropylamino)methyl]-1,3-thiazol-4-yl}phenol;
2,6-Di-*tert*-butyl-4-{2-[(cyclohexylamino)methyl]-1,3-thiazol-4-yl}phenol;
2,6-Di-*tert*-butyl-4-{2-[(4-isopropylpiperazin-1-yl)methyl]-1,3-thiazol-4-yl}phenol;
*N*-Methyl-1-[4-(10*H*-phenothiazin-2-yl)-1,3-thiazol-2-yl]ethanamin;
2,6-Di-*tert*-butyl-4-{2-[(4-ethylpiperazin-1-yl)methyl]-1,3-thiazol-4-yl}phenol;
*N*-{[4-(4-Anilinophenyl)-1,3-thiazol-2-yl]methyl}-*N*-ethylamin;
*N*-{[4-(10*H*-Phenothiazin-2-yl)-1,3-thiazol-2-yl]methyl}ethanamin;
2,6-Di-*tert*-butyl-4-(2-{[4-(dimethylamino)piperidin-1-yl]methyl}-1,3-thiazol-4-yl)phenol;
1-{[4-(3,5-Di-*tert*-butyl-4-hydroxyphenyl)-1,3-thiazol-2-yl]methyl}piperidin-4-ol;
2,6-Di-*tert*-butyl-4-{2-[(propylamino)methyl]-1,3-thiazol-4-yl}phenol;
*N*-{[4-(10*H*-Phenothiazin-2-yl)-1,3-thiazol-2-yl]methyl}-*N*-propylamin;
*N*-{[4-(10*H*-Phenothiazin-2-yl)-1,3-thiazol-2-yl]methyl}butan-1-amin;
*N*-{[4-(10*H*-Phenothiazin-2-yl)-1,3-thiazol-2-yl]methyl}pentan-1-amin;
1-{[4-(3,5-Di-*tert*-butyl-4-hydroxyphenyl)-1,3-thiazol-2-yl]methyl}piperidin-3-ol;
1-{[4-(3,5-Di-*tert*-butyl-4-hydroxyphenyl)-1,3-thiazol-2-yl]methyl}pyrrolidin-3-ol;
[4-(10*H*-phenothiazin-2-yl)-1,3-thiazol-2-yl]methanol;
*N*,*N*-Dimethyl-*N*-{[4-(10*H*-phenothiazin-2-yl)-1,3-thiazol-2-yl]methyl}amin;
2-{2-[(4-Methylpiperazin-1-yl)methyl]-1,3-thiazol-4-yl}-10*H*-phenothiazin;
2-[2-(Piperidin-1-ylmethyl)-1,3-thiazol-4-yl]-10*H*-phenothiazin;
2-[2-(Piperazin-1-ylmethyl)-1,3-thiazol-4-yl]-10*H*-phenothiazin;
1-{[4-(3,5-Di-*tert*-butyl-4-hydroxyphenyl)-1,3-thiazol-2-yl]methyl}azetidin-3-ol;
2-[2-(Morpholin-4-ylmethyl)-1,3-thiazol-4-yl]-10*H*-phenothiazin;
2-[2-(Thiomorpholin-4-ylmethyl)-1,3-thiazol-4-yl]-10*H*-phenothiazin;
2-{2-[(4-Methyl-1,4-diazepan-1-yl)methyl]-1,3-thiazol-4-yl}-10*H*-phenothiazin;
(3R)-1-{[4-(3,5-Di-tert-butyl-4-hydroxyphenyl)-1,3-thiazol-2-yl]methyl}pyrrolidin-3-ol;
(3S)-1-{[4-(3,5-Di-tert-butyl-4-hydroxyphenyl)-1,3-thiazol-2-yl]methyl}pyrrolidin-3-ol;
2,6-Di-*tert*-butyl-4-[2-(pyrrolidin-1-ylmethyl)-1,3-thiazol-4-yl]phenol;
2,6-Di-*tert*-butyl-4-{2-[(butylamino)methyl]-1,3-thiazol-4-yl]phenol;
2-{2-[(4-Ethylpiperazin-1-yl)methyl]-1,3-thiazol-4-yl}-10*H*-phenothiazin;
*N*-Methyl-*N*-{[4-(10*H*-phenothiazin-2-yl)-1*H*-imidazol-2-yl]methyl}amin;
Methyl [4-(10*H*-Phenothiazin-2-yl)-1,3-thiazol-2-yl]methylcarbamat;
Butyl [4-(10*H*-Phenothiazin-2-yl)-1,3-thiazol-2-yl]methylcarbamat;
*N*-Neopentyl-N-{[4-(10*H*-phenothiazin-2-yl)-1,3-thiazol-2-yl]methyl}amin;
1-{[4-(10*H*-Phenothiazin-2-yl)-1,3-thiazol-2-yl]methyl}piperidin-4-ol;
*N*-{[4-(10*H*-Phenothiazin-2-yl)-1,3-thiazol-2-yl]methyl}acetamid;
*N*-{[4-(10*H*-Phenothiazin-2-yl)-1,3-thiazol-2-yl]methyl}butanamid;
2,6-Di-*tert*-butyl-4-{2-[(4-propylpiperazin-1-yl)methyl]-1,3-thiazol-4-yl}phenol;
2,6-Di-*tert*-butyl-4-{2-[2-methyl-1-(methylamino)propyl]-1,3-thiazol-4-yl}phenol;
*N*,2-Dimethyl-1-[4-(10*H*-phenothiazin-2-yl)-1,3-thiazol-2-yl]propan-1-amin;
*N*-{[4-(10*H*-Phenothiazin-2-yl)-1,3-thiazol-2-yl]methyl}hexanamid;
(3*R*)-1-{[4-(10*H*-Phenothiazin-2-yl)-1,3-thiazol-2-yl]methyl}pyrrolidin-3-ol;
(3*S*)-1-{[4-(10*H*-Phenothiazin-2-yl)-1,3-thiazol-2-yl]methyl}pyrrolidin-3-ol;
1-{[4-(10*H*-Phenothiazin-2-yl)-1,3-thiazol-2-yl]methyl}azetidin-3-ol;
2-{2-[(4-Propylpiperazin-1-yl)methyl]-1,3-thiazol-4-yl}-10*H*-phenothiazin;
2-{2-[(4-Acetylpiperazin-1-yl)methyl]-1,3-thiazol-4-yl}-10*H*-phenothiazin;
2-{2-[(4-Butylpiperazin-1-yl)methyl]-1,3-thiazol-4-yl}-10*H*-phenothiazin;
Methyl 4-{[4-(10*H*-Phenothiazin-2-yl)-1,3-thiazol-2-yl]methyl}piperazin-1-carboxylat;
4-[2-(Aminomethyl)-1-imidazol-4-yl]-2,6-di-*tert*-butylphenol;
4-[2-[(Benzylamino)methyl]-1,3-thiazol-4-yl}-2,6-di-*tert*-butylphenol;
4-[2-[(4-Acetylpiperazin-1-yl)methyl]-1,3-thiazol-4-yl}-2,6-di-*tert*-butylphenol;
*N*-Methyl-N-{[4-(10*H*-phenoxazin-2-yl)-1,3-thiazol-2-yl]methyl}amin;
4-[2-(Azetidin-1-ylmethyl)-1,3-thiazol-4-yl]-2,6-di-tert-butylphenol;
2,6-Di-*tert*-butyl-4-{2-[(4-butylpiperazin-1-yl)methyl]-1,3-thiazol-4-yl}phenol;
Methyl 4-{[4-(3,5-Di-*tert*-butyl-4-hydroxyphenyl)-1,3-thiazol-2-yl]methyl}piperazin-1-carboxylat;
Methyl [4-(3,5-Di-*tert*-butyl-4-hydroxyphenyl)-1,3-thiazol-2-yl]methylcarbamat;
*N*-{[4-(3,5-Di-*tert*-butyl-4-hydroxyphenyl)-1,3-thiazol-2-yl]methyl}benzamid;
*N*-{[4-(3,5-Di-*tert*-butyl-4-hydroxyphenyl)-1,3-thiazol-2-yl]methyl}-2-phenylacetamid;
*N*-{[4-(3,5-Di-*tert*-butyl-4-hydroxyphenyl)-1,3-thiazol-2-yl]methyl}propanamid;
1-{[4-(3,5-Di-*tert*-butyl-4-hydroxyphenyl)-1,3-thiazol-2-yl]methyl}piperidin-4-yl-acetat;
1-{[4-(3,5-Di-*tert*-butyl-4-hydroxyphenyl)-1,3-thiazol-2-yl]methyl}pyrrolidin-3,4-diol;
oder eines pharmazeutisch annehmbaren Salzes einer dieser Verbindungen,
für die Herstellung eines Medikaments, das für die Behandlung einer der folgenden Störungen oder einer der folgenden Krankheiten bestimmt ist: Parkinson-Krankheit, Altersdemenzen, Alzheimer-Krankheit, Chorea Huntington, Multiple Sklerose, Schizophrenie, Depressionen und Psychosen.

6. Verwendung nach Anspruch 5, **dadurch gekennzeichnet, dass** die verwendete Verbindung aus einer der folgenden Verbindungen ausgewählt ist:
2,6-Di-*tert*-butyl-4-{2-[1-(methylamino)ethyl]-1,3-thiazol-4-yl}phenol;
1-[4-(3,5-Di*tert*-butyl-4-methoxyphenyl)-1,3-thiazol-2-yl]-N-methylmethanamin;
2,6-Di-*tert*-butyl-4-{2-[(ethylamino)methyl]-1,3-thiazol-4-yl}phenol;
1-{[4-(3,5-Di-*tert*-butyl-4-hydroxyphenyl)-1,3-thiazol-2-yl]methyl}piperidin-4-ol;
1-{[4-(3,5-Di-*tert*-butyl-4-hydroxyphenyl)-1,3-thiazol-2-yl]methyl}pyrrolidin-3-ol;
1-{[4-(3,5-Di-*tert*-butyl-4-hydroxyphenyl)-1,3-thiazol-2-yl]methyl}azetidin-3-ol;
(3R)-1-{[4-(3,5-Di-tert-butyl-4-hydroxyphenyl)-1,3-thiazol-2-yl]methyl}pyrrolidin-3-ol;
(3S)-1-{[4-(3,5-Di-tert-butyl-4-hydroxyphenyl)-1,3-thiazol-2-yl]methyl}pyrrolidin-3-ol;
*N*,2-Dimethyl-1-[4-(10*H*-phenothiazin-2-yl)-1,3-thiazol-2-yl]propan-1-amin;
und die pharmazeutisch annehmbaren Salze dieser Verbindungen.

7. Verwendung einer der folgenden Verbindungen:
2,6-Di-*tert*-butyl-4-{2-[2-(methylamino)ethyl]-1,3-thiazol-4-yl}phenol;
2,6-Di-*tert*-butyl-4-{2-[1-(methylamino)ethyl]-1,3-thiazol-4-yl}phenol;
2,6-Di-*tert*-butyl-4-{4-[(methylamino)methyl]-1,3-oxazol-2-yl}phenol;
2,6-Di-*tert*-butyl-4-{5-methyl-2-[(methylamino)methyl]-1,3-thiazol-4-yl}phenol;
1-{[4-(3,5-Di*tert*-butyl-4-methoxyphenyl)-1,3-thiazol-2-yl]-N-methylmethanamin;
2,6-Di-*tert*-butyl-4-{2-[(ethylamino)methyl]-1,3-thiazol-4-yl}phenol;
4-Methylpentyl 2-[4-(1,1'-Biphenyl-4-yl)-1*H*-imidazol-2-yl]ethylcarbamat;
3,3-Dimethylbutyl 2-[4-(4-Pyrrolidin-1-ylphenyl)-1*H*-Imidazol-2-yl]ethylcarbamat;
Isopentyl 2-[4-(1,1'-Biphenyl-4-yl)-1*H*-imidazol-2-yl]ethylcarbamat;
Hexyl 2-[4-(4'-Bromo-1,1'-biphenyl-4-yl)-1*H*-imidazol-2-yl]ethylcarbamat;
Benzyl 2-[4-(4-*tert*-Butylphenyl)-1*H*-imidazol-2-yl]ethylcarbamat;
3,3-Dimethylbutyl 2-[4-(1,1'-Biphenyl-4-yl)-1*H*-imidazol-2-yl]ethylcarbamat;
Hexyl 2-[4-(4-Pyrrolidin-1-ylphenyl)-1*H*-imidazol-2-yl]ethylcarbamat;
Hexyl 2-[4-(3,5-Di-*tert*-butyl-4-hydroxyphenyl)-1*H*-imidazol-2-yl]ethylcarbamat;
Hexyl 2-[4-(3,5-Di-*tert*-butyl-4-hydroxyphenyl)-1*H*-imidazol-2-yl]ethylcarbamat;
3,3-Dimethylbutyl 2-[4-(4-Methoxyphenyl)-1*H*-imidazol-2-yl]ethylcarbamat;
Benzyl 2-[4-(3,5-Di*tert*-butyl-4-hydroxyphenyl)-1*H*-imidazol-2-yl]ethylcarbamat;
Benzyl 2-[4-(4-Pyrrolidin-1-ylphenyl)-1*H*-imidazol-2-yl]ethylcarbamat;
2-Phenylethyl 2-[4-(1,1'-Biphenyl-4-yl)-1*H*-imidazol-2-yl]ethylcarbamat;
Butyl 2-[4-(4'-Fluoro-1,1'-biphenyl-4-yl)-1*H*-imidazol-2-yl]ethylcarbamat;
Butyl 2-[4-(1,1'-Biphenyl-4-yl)-5-methyl-1*H-*imidazol-2-yl]ethylcarbamat;
Butyl 2-[4-(4'-Methyl-1,1'-biphenyl-4-yl)-1*H*-imidazol-2-yl]ethylcarbamat;
Butyl 2-[4-(4'-Chloro-1,1'-biphenyl-4-yl)-1*H*-imidazol-2-yl]ethylcarbamat;
Butyl 2-[4-(2'-Fluoro-1,1'-biphenyl-4-yl)-1*H*-imidazol-2-yl]ethylcarbamat;
Butyl 2-[4-(2',4'-Difluoro-1,1'-biphenyl-4-yl)-1*H*-imidazol-2-yl]ethylcarbamat;
Butyl 2-[4-(3'-Chloro-1,1'-biphenyl-4-yl)-1*H*-imidazol-2-yl]ethylcarbamat;
Butyl 2-[4-(3'-Fluoro-1,1'-biphenyl-4-yl)-1*H*-imidazol-2-yl]ethylcarbamat;
Butyl 2-[4-(4-Isobutylphenyl)-1*H*-imidazol-2-yl]ethylcarbamat;
Benzyl 2-[4-(4-Isobutylphenyl)-1*H*-imidazol-2-yl]ethylcarbamat;
Butyl 2-[4-(3'-Chloro-4'-fluoro-1,1'-biphenyl-4-yl)-1*H*-imidazol-2-yl]ethylcarbamat;
Butyl 2-[4-(3',4'-Dichloro-1,1'-biphenyl-4-yl)-1*H*-imidazol-2-yl]ethylcarbamat;
Butyl 2-[4-(4-Propylphenyl)-1*H*-imidazol-2-yl]ethylcarbamat;
Butyl -[4-(4-Ethylphenyl)-1*H*-imidazol-2-yl]ethylcarbamat;
Butyl 2-[4-(4'-Cyano-1,1'-biphenyl-4-yl)-1*H*-imidazol-2-yl]ethylcarbamat;
Butyl 2-[4-(1,1'-Biphenyl-4-yl)-5-ethyl-1*H*-imidazol-2-yl]ethylcarbamat;
Butyl 2-[4-(2'-Chloro-1,1'-biphenyl-4-yl)-1*H*-imidazol-2-yl]ethylcarbamat;
Butyl 2-[4-(2',3'-Difluoro-1,1'-biphenyl-4-yl)-1*H*-imidazol-2-yl]ethylcarbamat;
Butyl 2-[4-(2'-Bromo-1,1'-biphenyl-4-yl)-1*H*-imidazol-2-yl]ethylcarbamat;
Butyl 2-[4-(3',5'-Difluoro-1,1'-biphenyl-4-yl)-1*H*-imidazol-2-yl]ethylcarbamat;
Butyl 2-[4-(2'-Methoxy-1,1'-biphenyl-4-yl)-1*H*-imidazol-2-yl]ethylcarbamat;
Butyl 2-[4-(3'-Nitro-1,1'-biphenyl-4-yl)-1*H*-imidazol-2-yl]ethylcarbamat;
Butyl 2-[4-(2',5'-Difluoro-1,1'-biphenyl-4-yl)-1*H*-imidazol-2-yl]ethylcarbamat;
Butyl 2-[4-(3'-Methoxy-1,1'-biphenyl-4-yl)-1*H*-imidazol-2-yl]ethylcarbamat;
oder eines pharmazeutisch annehmbaren Salzes einer dieser Verbindungen,
zur Herstellung eines Medikaments, das zur Behandlung einer der folgenden Störungen oder einer der folgenden Krankheiten bestimmt ist: Parkinson-Krankheit, Alzheimer-Krankheit, Multiple Sklerose, Migräne oder Schmerzen und insbesondere neuropathische Schmerzen.
